(19) Europäisches Patentamt / European Patent Office / Office européen des brevets

(11) **EP 2 824 099 A1**

(12) **EUROPEAN PATENT APPLICATION**
published in accordance with Art. 153(4) EPC

(43) Date of publication:
**14.01.2015 Bulletin 2015/03**

(21) Application number: **13758233.4**

(22) Date of filing: **07.03.2013**

(51) Int Cl.:
***C07D 251/42*** *(2006.01)* ***A61K 31/53*** *(2006.01)*
***A61K 31/5377*** *(2006.01)* ***A61P 19/00*** *(2006.01)*
***A61P 29/00*** *(2006.01)* ***A61P 35/00*** *(2006.01)*
***A61P 35/02*** *(2006.01)* ***A61P 37/06*** *(2006.01)*
***A61P 43/00*** *(2006.01)*

(86) International application number:
**PCT/JP2013/056266**

(87) International publication number:
**WO 2013/133367 (12.09.2013 Gazette 2013/37)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **09.03.2012 JP 2012053543**
**26.04.2012 JP 2012100646**
**29.06.2012 JP 2012147111**
**25.12.2012 JP 2012280811**

(71) Applicant: **Carna Biosciences, Inc.**
**Kobe-shi, Hyogo 650-0047 (JP)**

(72) Inventors:
- **KAWAHATA, Wataru**
  **Kobe-shi**
  **Hyogo 651-1242 (JP)**
- **ASAMI, Tokiko**
  **Kobe-shi**
  **Hyogo 654-0154 (JP)**
- **SAWA, Masaaki**
  **Ibaraki-shi**
  **Osaka 567-0046 (JP)**
- **ASAMITSU, Yuko**
  **Kobe-shi**
  **Hyogo 651-0093 (JP)**
- **IRIE, Takayuki**
  **Kobe-shi**
  **Hyogo 655-0047 (JP)**
- **MIYAKE, Takahiro**
  **Gose-shi**
  **Nara 639-2334 (JP)**
- **KIYOI, Takao**
  **Kobe-shi**
  **Hyogo 657-0033 (JP)**

(74) Representative: **Sajda, Wolf E. et al**
**Meissner, Bolte & Partner GbR**
**Postfach 86 06 24**
**81633 München (DE)**

# (54) NOVEL TRIAZINE DERIVATIVE

(57) To provide a novel triazine derivative represented by the following formula (I):
A triazine derivative represented by the following formula (I) :

[Chemical Formula 1]

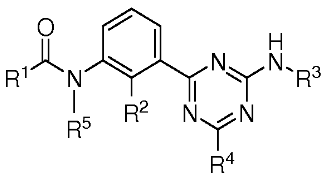

(I)

wherein
$R^1$ represents a substituted or unsubstituted aryl group, a substituted or unsubstituted heterocyclic ring, a substituted or unsubstituted heterocyclic fused ring, or a substituted or unsubstituted alkynyl group,
$R^2$ represents a hydrogen atom, a halogen atom, a substituted or unsubstituted lower alkyl group, or a substituted or unsubstituted alkoxy group,
$R^3$ represents a substituted or unsubstituted aryl group, a substituted or unsubstituted heterocyclic ring, or a substituted or unsubstituted heterocyclic fused ring,
$R^4$ represents a hydrogen atom, a substituted or unsubstituted lower alkyl group, a substituted or unsubstituted alkoxy group, a substituted or unsubstituted amino group, or a halogen atom, and

EP 2 824 099 A1

$R^5$ represents a hydrogen atom, a substituted or unsubstituted lower alkyl group, or $R^1$ and $R^5$ may be combined to form a saturated or unsaturated 5- to 6-membered ring, thereby forming a multiply fused ring,
or a pharmaceutically acceptable salt thereof.

**Description**

Technical Field

The present invention relates to a pharmaceutical, and particularly to a novel triazine derivative having a BTK inhibitory effect, or a pharmaceutically acceptable salt thereof.

Background Art]

Bruton's tyrosine kinase (BTK) is a member of the Tec family of non-receptor tyrosine kinases, and is an important signaling enzyme which is expressed in all hematopoietic cell types except for T lymphocytes and natural killer cells. BTK is an important control factor associated with survival, differentiation, proliferation and activation of B-cells, and takes an important role in signaling of B-cells (Non Patent Literatures 1 and 2). A B-cell receptor (BCR) of the cell surface signals into cells through BTK existing in the downstream of BCR and, therefore, it is considered that abnormal activation of the signaling pathway of B-cells accelerates proliferation and survival of cancer cells of B-cell lymphoma, chronic lymphocytic leukemia and the like (Non Patent Literature 3). It is known that BTK also plays an important role in the signal pathway of a large number of other cells, and it is said that BTK is involved in allergic diseases, self-immune diseases, inflammatory diseases and the like (Non Patent Literature 1). For example, it is known that BTK plays an important role for signaling of a high affinity IgE receptor (FcεRI) in mast cells, and degranulation decreases and the production of proinflammatory cytokines decreases in BTK-deficient mast cells (Non Patent Literature 4). It is suggested that BTK is involved in systemic lupus erythematosus (SLE) in a test of a BTK-deficient mouse (Non Patent Literature 5). Furthermore, the BTK mutant mouse exhibits resistance to the onset of collagen-induced arthritis (Non Patent Literature 6). Therefore, the compound having a BTK inhibitory activity is useful for the treatment of diseases which are involved in BTK signaling, for example, cancer, B-cell lymphoma, and chronic lymphocytic leukemia, and is also useful for the treatment of allergic diseases, self-immune diseases and inflammatory diseases.

Although a compound having a BTK inhibitory effect has hitherto been reported, it has not been reported that a novel triazine derivative or a pharmaceutically acceptable salt thereof of the present invention has a BTK inhibitory effect.

Citation List

Non Patent Literature

Non Patent Literature 1
Satterthwaite, A. B. andWitte, O N., Immunol. Rev., 2000, 175, 120-127
Non Patent Literature 2
Kurosaki T., Curr. Opin. Immunol., 2000, 12, 276-281
Non Patent Literature 3
Davis R. E. et al., Nature, 2010, 463, 88-92
Non Patent Literature 4
Ellmeier W. et al., FEBS J., 2011, 278, 1990-2000
Non Patent Literature 5
Halcomb K. E., Mol. Immunol., 2008, 46(2), 233-241 Non Patent Literature 6
Jansson L. and Holmdahl R., Clin. Exp. Immunol., 1993, 94, 459-465

Summary of the Invention

Technical Problem

An object of the present invention is to provide a pharmaceutical, particularly a novel triazine derivative having a BTK inhibitory effect, or a pharmaceutically acceptable salt thereof.

Solution to the Problem

The present invention is achieved by the following aspects (1) to (4):

(1) A triazine derivative represented by the following formula (I) :

[Chemical Formula 1]

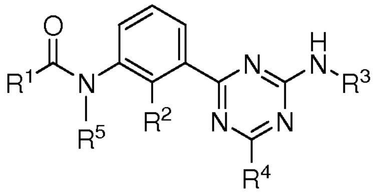

(I)

wherein

$R^1$ represents a substituted or unsubstituted aryl group, a substituted or unsubstituted heterocyclic ring, a substituted or unsubstituted heterocyclic fused ring, or a substituted or unsubstituted alkynyl group,
$R^2$ represents a hydrogen atom, a halogen atom, a substituted or unsubstituted lower alkyl group, or a substituted or unsubstituted alkoxy group,
$R^3$ represents a substituted or unsubstituted aryl group, a substituted or unsubstituted heterocyclic ring, or a substituted or unsubstituted heterocyclic fused ring,
$R^4$ represents a hydrogen atom, a substituted or unsubstituted lower alkyl group, a substituted or unsubstituted alkoxy group,
a substituted or unsubstituted amino group, or a halogen atom, and
$R^5$ represents a hydrogen atom, a substituted or unsubstituted lower alkyl group, or $R^1$ and $R^5$ may be combined to form a saturated or unsaturated 5- to 6-membered ring, thereby forming a multiply fused ring,
or a pharmaceutically acceptable salt thereof;

(2) The triazine derivative according to (1), wherein $R^1$ is a substituted or unsubstituted aryl group, or a pharmaceutically acceptable salt thereof;
(3) The triazine derivative according to (1), wherein $R^2$ is a substituted or unsubstituted lower alkyl group, or a pharmaceutically acceptable salt thereof; and (4) The triazine derivative according to (1), wherein $R^5$ and $R^1$ are combined to form a saturated or unsaturated 5- to 6-membered ring, thereby forming a multiply fused ring, or a pharmaceutically acceptable salt thereof.

Advantageous Effects of the Invention

The present inventors have intensively studied so as to achieve the above object and found that a novel triazine derivative represented by formula (I) shown above and a pharmaceutically acceptable salt thereof have an excellent BTK inhibitory effect, and thus completing the present invention. The compound provided by the present invention is useful as a preventive or therapeutic pharmaceutical (pharmaceutical composition) for diseases which are known to be involved in abnormal cell response through BTK, for example, self-immune diseases, inflammatory diseases, bone diseases, and cancers such as lymphoma. The compound is also useful, as a BTK inhibitor, for reagents to be used in tests and researches.

Description of Embodiments

The present invention will be described in detail below.
A novel triazine derivative of the present invention is a compound represented by formula (I) shown below:

Chemical Formula 2

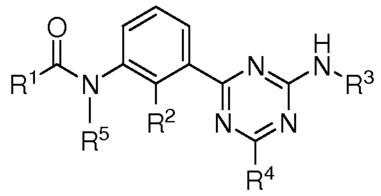

(I)

wherein

$R^1$ represents a substituted or unsubstituted aryl group, a substituted or unsubstituted heterocyclic ring, a substituted or unsubstituted heterocyclic fused ring, or a substituted or unsubstituted alkynyl group,
$R^2$ represents a hydrogen atom, a halogen atom, a substituted or unsubstituted lower alkyl group, or a substituted or unsubstituted alkoxy group,
$R^3$ represents a substituted or unsubstituted aryl group, a substituted or unsubstituted heterocyclic ring, or a substituted or unsubstituted heterocyclic fused ring,
$R^4$ represents a hydrogen atom, a substituted or unsubstituted lower alkyl group, a substituted or unsubstituted alkoxy group,
a substituted or unsubstituted amino group, or a halogen atom, and
$R^5$ represents a hydrogen atom, a substituted or unsubstituted lower alkyl group, or $R^1$ and $R^5$ may be combined to form a saturated or unsaturated 5- to 6-membered ring, thereby forming a multiply fused ring.

In formula (I) shown above, examples of the halogen atom include fluorine, chlorine, and bromine.

An aryl group moiety of the substituted or unsubstituted aryl group may be any of aryl groups having 6 to 14 carbon atoms, and specific examples thereof include phenyl, naphthyl, and indenyl.

A heterocyclic ring moiety of the substituted or unsubstituted heterocyclic ring includes an alicyclic heterocyclic group and an aromatic heterocyclic group. The alicyclic heterocyclic group includes, for example, 3- to 8-membered heterocyclic group having at least one heteroatom selected from a nitrogen atom, a sulfur atom, and an oxygen atom. Specific examples thereof include pyrrolidinyl, piperidinyl, piperazinyl, morpholinyl, and thiomorpholinyl. The aromatic heterocyclic group includes, for example, 5- or 6-membred monocyclic aromatic heterocyclic group having at least one heteroatom selected from a nitrogen atom, a sulfur atom, and an oxygen atom. Specific examples thereof include imidazolyl, pyrazolyl, thienyl, thiazolyl, and pyridyl.

A heterocyclic fused ring moiety of the substituted or unsubstituted heterocyclic fused ring includes, for example, a fused heterocyclic group which is 3- to 8-membered ring-fused bicyclic group and has at least one heteroatom selected from a nitrogen atom, a sulfur atom, and an oxygen atom. Specific examples thereof include tetrahydroisoquinolyl, benzothiophenyl, benzimidazolyl, benzooxazolyl, benzothiazolyl, indolyl, isoquinolyl, and phthalimide.

A lower alkyl group moiety of the substituted or unsubstituted lower alkyl group may be any of linear, branched and cyclic alkyl groups having 1 to 3 carbon atoms, and specific examples thereof include a methyl group, an isopropyl group, and a cyclopropyl group.

An alkoxy group moiety of the substituted or unsubstituted alkoxy group may be any of linear, branched, or cyclic alkyl group having 1 to 3 carbon atoms, and specific examples thereof include a methoxy group, an ethoxy group, an isopropyloxy group, and a cyclopropyloxy group.

The substituted or unsubstituted amino group may be more specifically any of amino groups having linear, branched, or cyclic alkyl group having 1 to 3 carbon atoms, and specific examples thereof include an amino group, a methylamino group, and a dimethylamino group.

An alkynyl group moiety of the substituted or unsubstituted alkynyl group may be any of linear or branched group having 2 to 6 carbon atoms, and specific examples thereof include ethynyl group, propargyl group, 2-butynyl group. A substituted moiety of the substituted or unsubstituted alkynyl group may be any of a substituted or unsubstituted aryl ring, a substituted or unsubstituted heterocyclic ring, or a substituted or unsubstituted heterocyclic fused ring, and specific examples thereof include aryl group.

It is possible to have, as a substituent of the substituted or unsubstituted aryl group, the substituted or unsubstituted heterocyclic ring, the substituted or unsubstituted heterocyclic fused ring, the substituted or unsubstituted lower alkyl group, the substituted or unsubstituted alkoxy group, or the substituted or unsubstituted amino group, one, or two or more of any kind of substituent (s) at any chemically possible position. When the above group have two or more

substituents, the respective substituents may be the same or different, and examples of the substituent include a halogen atom, a substituted or unsubstituted alkyl group, a substituted or unsubstituted alkoxy group, a substituted or unsubstituted amino group, a nitro group, a cyano group, a hydroxy group, a substituted or unsubstituted alkylamino group, a substituted or unsubstituted carbamoyl group, a carboxyl group, a formyl group, an acetyl group, a benzoyl group, and a substituted or unsubstituted acylamino group.

A multiply fused ring which can be formed by combining $R^5$ and $R^1$ to form a saturated or unsaturated 5- to 6-membered ring includes, for example, 3- to 8-membered ring-fused heterocyclic group having heteroatoms such as a nitrogen atom, a sulfur atom, and an oxygen atom. Specific examples thereof include oxoisoquinolyl, oxodihydroisoquinolyl, oxophthalazinyl, and oxothienopyrrolyl.

Isomers may sometimes exist in the compound (I) of the present invention, depending on the kind of the substituent. In the present description, the isomers may be sometimes described by a chemical structure of only one form thereof. The present invention includes all isomers (geometrical isomer, optical isomer, tautomer, etc.) which can be structurally formed, and also includes isomers alone, or a mixture thereof.

Examples of the pharmaceutically acceptable salt of the compound (I) of the present invention include inorganic acid salts with hydrochloric acid, sulfuric acid, carbonic acid, and phosphoric acid, etc; and organic acid salts with fumaric acid, maleic acid, methanesulfonic acid, and p-toluenesulfonic acid, etc. The present invention also includes ammonium salts, in addition to alkali metal salts with sodium and potassium; alkaline earth metal salts with magnesium and calcium; organic amine salts with lower alkylamine and lower alcoholamine; and basic amino acid salts with lysine, arginine, and ornithine.

The compound (I) of the present invention and a pharmaceutically acceptable salt thereof can be produced, for example, by methods shown below. When defined groups vary under the conditions of an implemental method in the production method shown below, or are unsuited to carry out the method, it is possible to easily produce them by a method which is usually used in organic synthetic chemistry, for example, a method of applying means such as protection or deprotection of a functional group [T. W. Greene, Protective Groups in Organic Synthesis 3rd Edition, John Wiley&Sons, Inc. , 1999]. If necessary, the order of a reaction step such as introduction of substituents can also be changed.

Meanings of abbreviations and symbols used in the following description are as follows.

DCM: dichloromethane
DCC: *N,N'*-dicyclohexylcarbodiimide
EDC: 1-ethyl-3-(3-dimethylaminopropyl)carbodiimide hydrochloride
HOBt: 1-hydroxybenzotriazole
THF: tetrahydrofuran
DIEA: N,N-diisopropylethylamine
DMF: dimethylformamide
DMSO: dimethyl sulfoxide
TEA: triethylamine
$CDCl_3$: deuterated chloroform

Method for Production of the Compound (I) of the Present Invention

The compound of the present invention represented by formula (I) can be produced, for example, according to Scheme 1:

Scheme

Chemical Formula 3

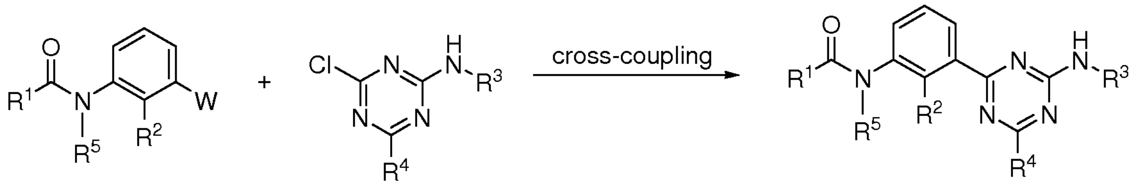


(II) (III) (I)

wherein $R^1$, $R^2$, $R^3$, $R^4$, and $R^5$ are as defined above, and W represents a boronyl group or a boronic ester group.

The compound (I) of the present invention can be produced by a cross-coupling reaction such as Suzuki coupling reaction, using a compound (II) and a compound (III) (see, for example, known literatures (N. Miyaura et al., J. Am. Chem. Soc., 107, 972 (1985)., N. Miyaura, A. Suzuki, Chem. Rev. 95, 2457 (1995) with respect to the conditions of the Suzuki coupling reaction)). That is, the reaction can be carried out in the presence of a metal catalyst such as palladium or nickel, if necessary, using a base and additives. Examples of a solvent used in the reaction include THF, dioxane, toluene, dimethoxyethane, methanol, ethanol, and acetonitrile. It is also suitable to use two or more kinds of these solvents, or to use them in combination with water. The solvent is preferably a mixed solvent of THF and water, or a mixed solvent of toluene, methanol and water, or dioxane. The compound (II) is preferably used in an equivalent or excess amount, and more preferably in an amount of from 1 equivalent to 10 equivalents, based on the compound (III). If necessary, a base may be added so as to accelerate the reaction, and sodium carbonate, cesium carbonate, and potassium carbonate are usually used as the base. The amount of the base to be used is from 1 equivalent to 10 equivalents, and preferably from 1 equivalent to 5 equivalents, based on the compound (III). It is possible to use, as a metal catalyst, a commercially available palladium catalyst (for example, $PdCl_2$(dppf), $Pd_2(dba)_3$, $Pd(PPh_3)_4$, etc.) which is used in the cross-coupling, and the catalyst is preferably used in a catalytic amount, that is, an amount of from 0.1 equivalent to 0.5 equivalent based on the compound (III).

If necessary, additives can be added so as to accelerate the reaction. The additive includes, for example, *rac*-BINAP and can be used in the amount of from 0.01 equivalent to 1 equivalent based on the compound (III). It is possible to synthesize by reacting at a temperature ranging from 0°C to 200°C for several minutes to several days, and preferably from 10°C to 100°C for 1 hour to 36 hours. It is also possible to synthesize by reacting under the temperature condition of from 60°C to 150°C for several minutes to several hours, using a microwave synthesis equipment.

The compound (II) used as a starting material of Scheme 1 can be produced, for example, by the method shown in Scheme 2:

Scheme 2

Chemical Formula 4

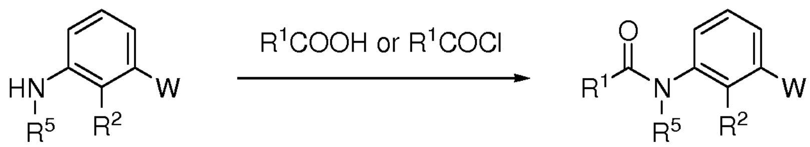


(IV) (II)

wherein $R^1$, $R^2$, $R^5$, and W are as defined above.

The compound (II) can be produced by subjecting an amine (IV) and carboxylic acid ($R^1COOH$) or an acid chloride ($R^1COCl$) to an amidation reaction which is often used in conventional organic chemistry.

That is, the compound (II) can be obtained by subjecting an amine (IV) and 1 to 5 molar equivalents, and preferably 1 to 1.5 molar equivalents of carboxylic acid ($R^1COOH$) to amidation condensation in a solvent in the presence of a base such as triethylamine, using a condensing agent. The solvent may be any solvent as long as it is inert to the reaction, and is not particularly limited. For example, chloroform, dichloromethane, diethyl ether, or THF can be used alone, or a mixed solvent thereof can be used. It is possible to use, as the condensing agent, commercially available *N,N'*-dicyclohexylcarbodiimide (DCC), 1-ethyl-3-(3-dimethylaminopropyl)carbodiimide hydrochloride (EDC), 1,1-carbonyldiimidazole (CDI), 2-chloro-1-methylpyridinium iodine, and 1-propylphosphonic acid cyclic anhydride (PPA). The reaction can be carried out at a temperature of from -10°C to a boiling point of the solvent to be used for 1 hour to 1 week. Preferably, it is possible to synthesize by reacting at a temperature of from 0°C to ambient temperature for 1 hour to 1 day. If necessary, it is also possible to synthesize by adding a reaction reagent such as 1-hydroxybenzotriazole (HOBt).

The compound (II) can be obtained by reacting an amine (IV) with 1 to 5 molar equivalents, and preferably 1 to 1.5 molar equivalents of an acid chloride ($R^1COCl$) in a solvent in the presence of a base such as pyridine or triethylamine. The solvent is not particularly limited and, for example, chloroform, dichloromethane, diethyl ether, pyridine, or THF can be used alone, or a mixed solvent thereof can be used. The reaction can be carried out at a temperature of from -10°C to a boiling point of the solvent to be used for 1 hour to 1 week. Preferably, it is possible to synthesize by reacting at a temperature of from 0°C to ambient temperature for 1 hour to 1 day.

It is also possible to synthesize the compound (II) from an amine (IV) and carboxylic acid ($R^1COOH$) by the mixed acid anhydride method.

It is desired that any of these amidation reactions are carried out in an inert gas (argon, nitrogen, etc.) atmosphere under anhydrous conditions.

The amine (IV) can be obtained as a commercially available product, or can be obtained by a well-known procedure or the procedure according to it.

The compound (II) can also be produced, for example, by introducing W into a compound (V) as shown in Scheme 3:

Scheme 3

[Chemical Formula 5]

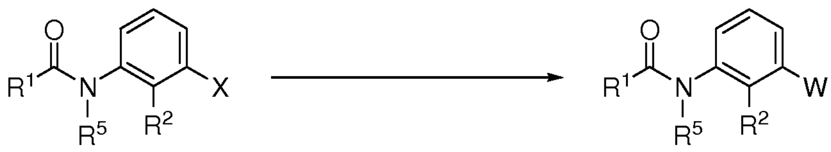

(V) (II)

wherein $R^1$, $R^2$, $R^5$, and W are as defined above, and X represents a halogen.

The compound (II) can be produced by activating the compound (V) with *n*-butyllithium, and then reacting the activated compound with a boric acid ester. That is, the compound (II) can be obtained by lithiation of the compound (V) with 1 to 5 molar equivalents, and preferably 1 to 1.5 molar equivalents of *n*-butyllithium, and reacting the lithiated compound with 1 to 5 molar equivalents, and preferably 1 to 1.5 molar equivalents of a boric acid ester.

The solvent may be any solvent as long as it is inert to the reaction and is not particularly limited, and THF can be preferably used.

The reaction temperature is usually from -100°C to -30°C, and preferably from -80°C to -60°C. The reaction time is not particularly limited, and is usually from 0.1 hour to 12 hours, and the reaction time of from 0.2 hour to 6 hours is exemplified as a preferable example.

The compound (II) can also be obtained by reacting the compound (V) with 1 to 5 molar equivalents, and preferably 1 to 1.5 molar equivalents of metallic magnesium and a catalytic amount of iodine in an ether-based solvent at a temperature of from -10°C to a boiling point of the solvent to be used to obtain a Grignard reagent, and then reacting the Grignard reagent with 1 to 5 molar equivalents, and preferably 1 to 1.5 molar equivalents of a boric acid ester. The reaction temperature is usually from -30°C to -100°C, and preferably from -60°C to -80°C. The reaction time is not particularly limited and is usually from 0.1 hour to 12 hours, and the reaction time of from 0.2 hour to 6 hours is exemplified as a preferable example.

Furthermore, the compound (II) can be obtained by subjecting the compound (V) and 1 to 5 molar equivalents, and preferably 1 to 3 molar equivalents of a diboron ester to a coupling reaction in the presence of a metal catalyst such as palladium and nickel and a base in an organic solvent.

It is possible to use, as the metal catalyst, a commercially available palladium catalyst (for example, $PdCl_2$(dppf), $Pd_2(dba)_3$, $Pd(PPh_3)_4$, etc.) which is used in the cross-coupling, and the catalyst is preferably used in a catalytic amount, that is, an amount of from 0.1 equivalent to 0.5 equivalent based on the compound (V) to be used in the cross-coupling. Potassium acetate is usually used as the base. The amount of the base to be used is from 1 equivalent to 10 equivalents based on the compound (V), and preferably from 1 equivalent to 5 equivalents, based on the compound (V).

The solvent may be any solvent as long as it is inert to the reaction and is not particularly limited, and dioxane can be preferably used.

The reaction temperature is usually from 0°C to 200°C, and preferably from 10°C to 100°C. The reaction time is not particularly limited and the reaction time of from 0.2 hour to 48 hours is usually exemplified, and the reaction time of from 1 hour to 36 hours is exemplified as a preferable example.

It is desired that any of these reactions are carried out in an inert gas (argon, nitrogen etc.) atmosphere, under anhydrous conditions.

The compound (V) can be obtained as a commercially available product, or can be obtained by a well-known procedure or the procedure according to it.

The compound (III) to be used as a starting material of Scheme 1 can be produced, for example, by the method shown in Scheme 4:

Scheme 4

Chemical Formula 6

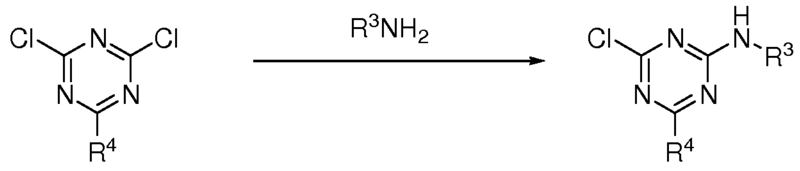


(III)

wherein $R^3$ and $R^4$ are as defined above.

The compound (III) can be obtained by reacting an amine ($R^3NH_2$) with 1 to 5 molar equivalents, and preferably 1 to 1.5 molar equivalents of 2,4-dichloro-1,3,5-triazine in a polar solvent and, if necessary, in the presence of a base catalyst.

The solvent may be any solvent as long as it is inert to the reaction and is not particularly limited, and dimethylformamide can be preferably used.

The reaction temperature is usually from 0°C to 200°C, and preferably from 10°C to 100°C. The reaction time is not particularly limited and the reaction time of from 0.2 hour to 48 hours is usually exemplified, and the reaction time of from 1 hour to 36 hours is exemplified as a preferable examples.

2,4-Dichloro-1,3,5-triazine and a derivative thereof, which are starting materials of Scheme 4, can be obtained as commercially available products, or can be obtained by a well-known procedure or the procedure according to it. An amine ($R^3NH_2$) also can be obtained as a commercially available product, or can be used by a well-known procedure or the procedure according to it.

The compound (I) of the present invention in which $R^4$ is a hydrogen atom can also be produced, for example, according to Scheme 5:

Scheme 5

Chemical Formula 7

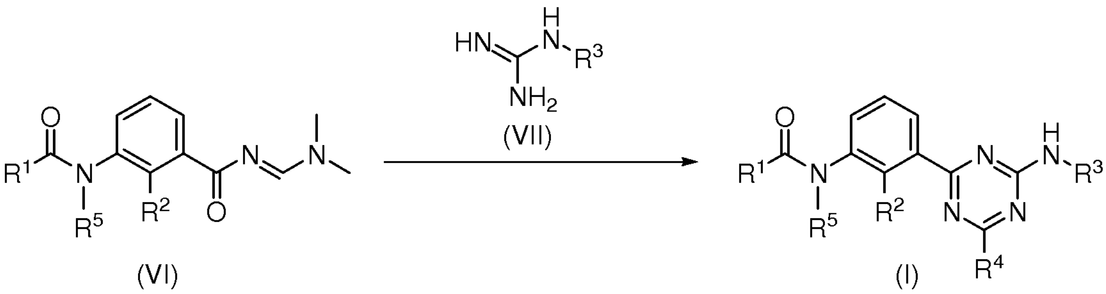

(VI) (VII) (I)

wherein $R^1$, $R^2$, $R^3$, and $R^5$ are as defined above, and $R^4$ is a hydrogen atom.

The compound (I) can be obtained by reacting the compound (VI) with 1 to 5 molar equivalents, and preferably 1 to 1.5 molar equivalents of the compound (VII) in an organic solvent in the presence of base.

The solvent may be any solvent as long as it is inert to the reaction and is not particularly limited, and dioxane can be preferably used.

Examples of the base to be used include sodium methoxide, sodium ethoxide, and potassium t-butoxide, and potassium t-butoxide can be preferably used.

The reaction temperature is usually from 0°C to 200°C, and preferably from 10°C to 100°C. The reaction time is not particularly limited and is usually from 0.2 hour to 48 hours, and the reaction time of from 1 hour to 36 hours is exemplified as a preferable example.

The compound (VII) can be obtained as a commercially available product, or can be obtained by a well-known procedure or the procedure according to it.

The compound (VI) to be used as a starting material of Scheme 5 can be produced, for example, by a method shown in Scheme 6:

Scheme 6

Chemical Formula 8

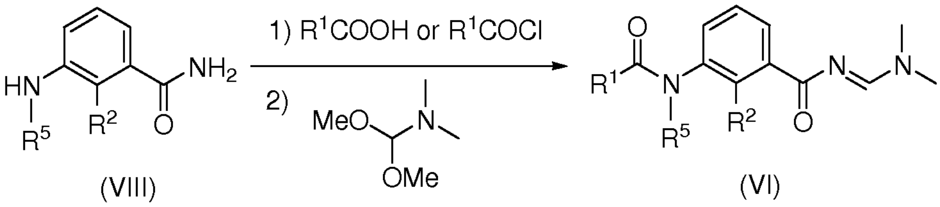


(VIII) (VI)

wherein $R^1$, $R^2$, and $R^5$ are as defined above.

The compound (VI) can be produced by reacting the compound, which is obtained by reacting an amine (VIII) with carboxylic acid ($R^1COOH$) or an acid chloride ($R^1COCl$), with N,N-dimethylformamide dimethyl acetal.

In the amidation reaction, the same conditions as those of the amidation reaction of Scheme 2 can be used. The amide obtained above can be obtained by reacting with 1 to 10 molar equivalents of N,N-dimethylformamide dimethyl acetal in an organic solvent or without using the solvent.

The solvent may be any solvent as long as it is inert to the reaction and is not particularly limited, and THF can be preferably used.

The reaction temperature is usually from 0°C to 200°C, and preferably from 10°C to 100°C. The reaction time is not particularly limited and is usually from 0.2 hour to 48 hours, and the reaction time of from 1 hour to 36 hours is exemplified as a preferable example. It is also suitable that reaction is carried out under microwave irradiation conditions.

The amine (VIII) can be obtained as a commercially available product, or can be obtained by a well-known procedure or the procedure according to it.

The compound (I) of the present invention can also be produced, for example, according to Scheme 7:

Scheme 7

Chemical Formula 9

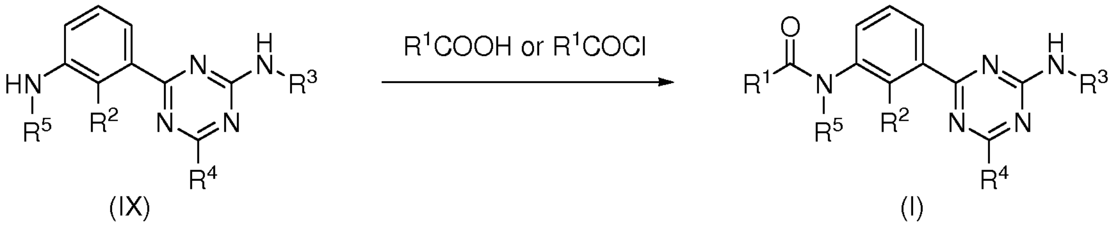


(IX) (I)

wherein $R^1$, $R^2$, $R^3$, $R^4$, and $R^5$ are as defined above.

The compound (I) of the present invention can be produced by subjecting an amine (IX) and carboxylic acid ($R^1COOH$) or an acid chloride ($R^1COCl$) to an amidation reaction. The conditions of the amidation reaction are the same as those in the method of producing the compound (II) according to Scheme 2 shown above.

The compound (IX) to be used as a starting material of Scheme 7 can be produced, for example, by a method shown in Scheme 8:

Scheme 8

Chemical Formula 10

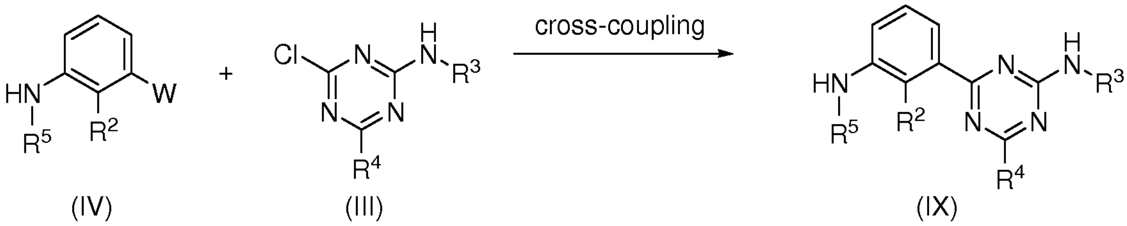


(IV) + (III) (IX)

wherein $R^2$, $R^3$, $R^4$, $R^5$, and W are as defined above.

The compound (IX) can also be produced by a cross-coupling reaction of the compound (IV) with the compound (III).

In carrying out the cross-coupling reaction, the compound (IX) can also be produced by optionally protecting or deprotecting an amino group of the compound (IV), appropriately combining methods to be usually used in organic synthetic chemistry. For example, it is possible to use protection or deprotection of a functional group of an amino group of the compound (IV) [T. W. Greene, Protective Groups in Organic Synthesis 3rd Edition, John Wiley & Sons, Inc. , 1999] and a nitro group derivative which is an amino group precursor of the compound (IV).

The compound (IV) can be obtained as a commercially available product, or can be obtained by a well-known procedure or the procedure according to it.

The compound (V) to be used as a starting material of Scheme 3 can be produced, for example, by a method shown in Scheme 9:

Scheme 9

Chemical Formula 11

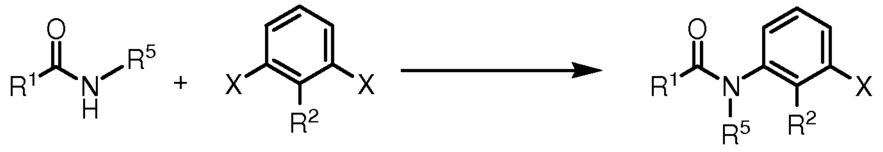

(X) (XI) (V)

wherein $R^1$, $R^2$, $R^5$, and X are as defined above.

The compound (V) can be obtained by reacting an amide (X) with 1 to 5 molar equivalents, and preferably 1.5 to 3 molar equivalents of compound (XI) in a polar solvent in the presence of metal catalyst and base.

The solvent may be any solvent as long as it is inert to the reaction and is not particularly limited, and DMSO can be preferably used.

In carrying out the coupling reaction, the compound (V) can also be produced by optionally protecting or deprotecting of the compound (XI), appropriately combining methods to be usually used in organic synthetic chemistry. For example, it is possible to use protection or deprotection of a functional group, such as hydroxy or amino group of the compound (XI) [T. W. Greene, Protective Groups in Organic Synthesis 3rd Edition, John Wiley&Sons, Inc., 1999] and aldehyde derivative which is hydroxy group precursor of the compound (XI).

The reaction can be carried out at a temperature of from 80°C to 200°C for 0.5 hour to 200 hours, and preferably from 100°C to 150°C for 1 hour to 100 hours. It is also possible to perform the reaction using microwave synthesis equipment.

It is possible to use, as the metal catalyst, a commercially available palladium catalyst (for example, $PdCl_2$(dppf) , $Pd_2(dba)_3$, $Pd(PPh_3)_4$, etc.) or copper (I) iodide which is used in the coupling reaction, and the catalyst is preferably used in a catalytic amount, that is, an amount of from 0.01 equivalent to 2 equivalent based on the amide (X) to be used in the coupling.

Examples of the base to be used include potassium carbonate, sodium carbonate, cesium carbonate and sodium hydrogen carbonate, and cesium carbonate and sodium hydrogen carbonate can be preferably used. The amount of the base to be used is from 1 molar equivalent to 10 molar equivalents based on the amide (X), and preferably from 2 molar equivalent to 5 molar equivalents, based on the amide (X) . And if necessary, xantphos can be used as additive to the reaction in the amount of 0.1 equivalent to 0.5 equivalent based on the amide (X).

The amide (X) and the compound (XI) can be obtained as a commercially available product, or can be obtained by a well-known procedure or the procedure according to it.

In the scheme shown above, a boronyl group represented by W may be in the form of a salt of alkali metal and alkaline earth metal, and specific examples of the boronic ester group include boronic ester groups such as a boronic acid dimethyl ester group, a boronic acid diethyl ester group, a boronic acid dibutyl ester group, a boronic acid dicyclohexyl group, a boronic acid ethylene glycol ester group, a boronic acid propylene glycol ester group (a boronic acid 1,2-propanediol ester group, a boronic acid 1,3-propanediol ester group), a boronic acid neopentyl glycol ester group, a boronic acid catechol ester group, a boronic acid glycerin ester group, a boronic acid trimethylolethane ester group, a boronic acid diethanolamine ester group, and a boronic acid triethanolamine ester group; and boronic acid anhydride groups.

It is possible to obtain the compound (I) having the desired functional group at the desired position of the present invention by appropriately using the above methods in combination, and then carrying out a method usually used in

organic synthetic chemistry (for example, an alkylation reaction of an amino group, an oxidizing reaction of alkylthio group into a sulfoxide group or a sulfone group, a reaction of converting an alkoxy group into a hydroxyl group, or a reaction of inversely converting the group).

Applications of Compound (I) of the Present Invention

The compound (I) or a pharmaceutically acceptable salt thereof of the present invention can be prepared into a form of a conventional pharmaceutical formulation (pharmaceutical composition), which is suited for oral administration, parenteral administration, or local administration.

Formulations for oral administration include solid formulations such as tablets, granules, powders, and capsules; and liquid formulations such as syrups. These formulations can be prepared by a conventional method. The solid formulations can be prepared by using conventional pharmaceutical carriers, for example, starches such as lactose and corn starch; crystalline celluloses such as micro crystalline cellulose; and hydroxypropyl cellulose, calcium carboxymethyl cellulose, talc, and magnesium stearate. Capsules can be prepared by encasing thus prepared granules or powders in capsules. Syrups can be prepared by dissolving or suspending the compound (I) or a pharmaceutically acceptable salt thereof of the present invention in an aqueous solution containing sucrose and carboxymethyl cellulose.

Formulations for parenteral administration include injections such as instillation. Injection formulations can also be prepared by a conventional method, and can be appropriately incorporated into isotonic agents (for example, mannitol, sodium chloride, glucose, sorbitol, glycerol, xylitol, fructose, maltose, mannose), stabilizers (for example, sodium sulfite, albumin), and antiseptics (for example, benzyl alcohol, methyl *p*-oxybenzoate).

The dosage of the compound (I) or a pharmaceutically acceptable salt thereof of the present invention can vary depending on severity of disease, age and body weight of the patient, and dosage form, and is usually within a range from 1 mg to 1,000 mg per day for adults. The compound or a pharmaceutically acceptable salt thereof can be administered once, or dividedly administered twice or three times according to an oral or parenteral route.

The compound (I) or a pharmaceutically acceptable salt thereof of the present invention can also be used, as a BTK inhibitor, for reagents to be used in tests and researches.

[Examples]

The present invention will be more specifically described below by way of Examples and Test Examples, but the present invention is not limited to these Examples.

Identification of the compound was carried out by hydrogen nuclear magnetic resonance spectrum ($^1$H-NMR) and mass spectrum (MS). $^1$H-NMR is measured at 400 MHz, unless otherwise specified, and exchangeable hydrogen cannot be sometimes clearly observed depending on the compound and measurement conditions. br. means a broad signal (broad).

HPLC preparative chromatography was carried out by a commercially available ODS column in a gradient mode using water/methanol (containing formic acid) as eluents, unless otherwise specified.

Referential Example 1, Examples 1-5, 33, 39, 42, 43 and 44 were prepared as follows.

**Referential Example 1 (synthesis of starting material)**

4-(*tert*-Butyl)-*N*-[2-methyl-3-(4,4,5,5-tetramethyl-1,3,2-dio xoboran-2-yl)phenyl]benzamide

Chemical Formula 12

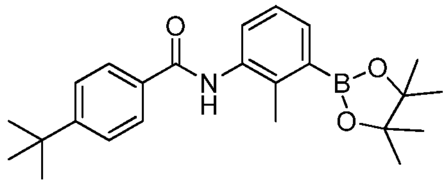

First Step

Under nitrogen atmosphere, 3-bromo-2-methylaniline (5.01g, 26.9 mmol) was dissolved in DCM (100 mL). To

this solution, cooled with ice bath, TEA (7.5 mL, 53.9 mmol) and 4-*tert*-butylbenzoyl chloride (5.3 g, 26.9 mmol) were added dropwise and stirred at 0 °C for 4 h. The reaction mixture was diluted with DCM (100 mL), washed with water (2x30 mL), 1N hydrochloric acid solution (30 mL), saturated sodium hydrogen carbonate solution (30 mL) and brine (30 mL), then the organic layer was dried over sodium sulfate, filtered and concentrated. The crude material was suspended in hexane, then the precipitate was collected by filtration, washed with hexane then dried to afford *N*-(3-bromo-2-methylphenyl)-4-(tert-butyl)benzamide (9.0 g).

$^1$H NMR (400 MHz, DMSO-$d_6$) δ 10.06 (s, 1H), 7.92 (d, J = 8.4 Hz, 2H), 7.54 (m, 3H), 7.33 (d, J = 7.7 Hz, 1H), 7.18 (t, J = 8.0 Hz, 1H), 2.27 (s, 3H), 1.32 (s, 9H)

Second Step

Under nitrogen atmosphere, *N*-(3-bromo-2-methylphenyl)-4-(tert-butyl)benzamide (500 mg, 1.44 mmol) which was afforded in the First Step was dissolved in dioxane (10 mL). To this solution, bis(pinacolato)diboron (733 mg, 2.89 mmol), dichloro[1,1'-bis(diphenylphosphino)ferrocene]palladium(II) dichloromethane adduct (118 mg, 0.14 mmol) and potassium acetate (424 mg, 4.33 mmol) were added and refluxed for 16 h. Solvents were removed under reduced pressure, and the crude material was purified by chromatography on silica gel, eluted with hexane/ethyl acetate to afford 4-(*tert*-butyl)-*N*-[2-methyl-3-(4,4,5,5-tetramethyl-1,3,2-dio xaborolan-2-yl)phenyl]benzamide (410 mg).

$^1$H NMR (400 MHz, DMSO-$d_6$) δ 9.80 (s, 1H), 7.92 (d, J = 8.3 Hz, 2H), 7.54 (m, 3H), 7.39 (d, J = 7.6 Hz, 1H), 7.21 (t, J = 7.6 Hz, 1H), 2.37 (s, 3H), 1.32 (s, 9H), 1.32 (s, 12H) ; LCMS (m/z): 394.0 $[M+H]^+$.

**Example 1**

4-(*tert*-Butyl)-N-(2-methyl-3-{4-[(3,4,5-trimethoxyphenyl)am ino]-1,3,5-triazin-2-yl}phenyl)benzamide

Chemical Formula 13

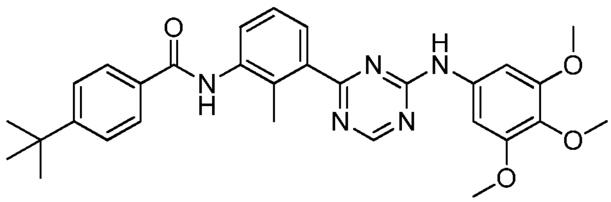

First Step

To a solution of 2, 4-dichloro-1, 3, 5-triazine (300 mg, 2.0 mmol) in DMF (5 mL), cooled with ice bath, DIEA (0.5 mL, 3.0 mmol) and 3,4,5-trimethoxyaniline (366 mg, 2.0 mmol) were added then stirred at 0 °C for 16 h. The reaction mixture was diluted with water (15 mL), extracted with ethyl acetate (3x50 mL). The combined organic layer was washed with water (20 mL), dried over sodium sulfate, filtered and concentrated. The crude material was purified by chromatography on silica gel, eluted with hexane/ethyl acetate to afford 4-chloro-*N*-(3,4,5-trimethoxyphenyl)-1,3,5-triazin-2-amine (400 mg).

$^1$H NMR (400 MHz, DMSO-$d_6$) δ 10.62 (s, 1H), 8.62 (s, 1H), 7.05 (s, 2H), 3.76 (s, 6H), 3.65 (s, 3H) ; LCMS (m/z): 297.0 $[M+H]^+$.

Second Step

To a solution of 4-chloro-*N*-(3,4,5-trimethoxyphenyl)-1,3,5-triazin-2-amine (50.0 mg, 0.17 mmol) and 4-(*tert*-butyl)-*N*-[2-methyl-3-(4,4,5,5,-tetramethyl-1,3,2-di oxaborolan-2-yl)phenyl]benzamide (66 mg, 0.17 mmol) which was afforded in Referential Example 1 in dimethoxyethane (3 mL), tetrakis(triphenylphosphine)palladium (0) (19 mg, 0.017 mmol) and potassium carbonate (47 mg, 0.34 mmol) in water solution (1 mL) were added then heated with the microwave synthesizer at 110 °C for 20 min. The reaction mixture was extracted with ethyl acetate (2x40 mL), and the combined organic layer was washed with water (5 mL), dried over sodium sulfate, filtered and concentrated. The crude material was purified by chromatography on silica gel, eluted with hexane/ethyl acetate to afford the titled compound (27 mg).

$^1$H NMR (400 MHz, DMSO-$d_6$) δ 10.20 (s, 1H), 9.98 (s, 1H), 8.85 (s, 1H), 7.94 (d, J = 8.3 Hz, 2H), 7.56 (d, J =

8.4 Hz, 2H), 7.47 (d, J = 7.5 Hz, 1H), 7.37 (t, J = 7.8 Hz, 1H), 7.20 (m, 3H), 3.76 (s, 6H), 3.64 (s, 3H), 2.39 (s, 3H), 1.33 (s, 9H) ; LCMS (m/z): 528 $[M+H]^+$.

**Example 2**

4-(*tert*-Butyl)-*N*-[2-methyl-3-(4-{[4-(morpholine-4-carbonyl) phenyl]amino}-1,3,5-triazin-2-yl)phenyl]benzamide

Chemical Formula 14

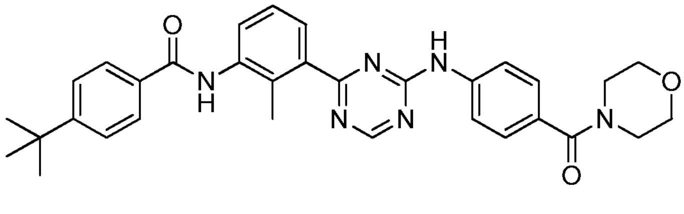

The titled compound (15 mg) was similarly prepared according to the procedure described in the Example 1, using (4-aminophenyl)morpholin-4-ylmethanone (412 mg, 2.0 mmol) instead of 3,4, 5-trimethoxyaniline as $R^3NH_2$ starting materials.

$^1H$ NMR (400 MHz, DMSO-$d_6$) $\delta$ 10.57 (s, 1H), 9.98 (s, 1H), 8.91 (s, 1H), 7.95 (d, J = 8.1 Hz, 2H), 7.85 (d, J = 8.0 Hz, 2H), 7.71 - 7.33 (m, 7H), 3.72 - 3.39 (m, 8H), 2.38 (s, 3H), 1.33 (s, 9H) ; LCMS (m/z): 551.0 $[M+H]^+$.

**Example 3**

4-[(4-{3-[4-(*tert*-Butyl)benzamido]-2-methylphenyl}-1,3,5-tr iazin-2-yl)amino]benzoic acid

Chemical Formula 15

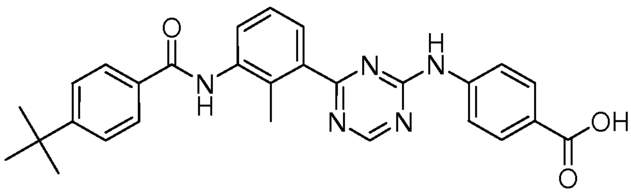

First Step

To a solution of 2, 4-dichloro-1, 3, 5-triazine (2.0 g, 13.3 mmol) in DMF (33 mL), cooled with ice bath, DIEA (3.5 mL, 20.0 mmol) and ethyl 4-aminobenzoate (2.2 g, 13.3 mmol) were added then stirred at 0°C for 20 min. The precipitate was collected by filtration, washed with water then dried under reduced pressure to afford ethyl 4-[(4-chloro-1,3,5-triazin-2-yl)amino]benzoate (2.45 g).

$^1H$ NMR (400 MHz, DMSO-$d_6$) $\delta$ 11.09 (s, 1H), 8.74 (s, 1H), 8.00 - 7.93 (m, 2H), 7.84 (d, J = 8.6 Hz, 2H), 4.30 (q, J = 7.1 Hz, 2H), 1.32 (t, J = 7.1 Hz, 3H) ; ;LCMS (m/z): 279 $[M+H]^+$.

Second Step

4-(*tert*-Butyl)-*N*-[2-methyl-3-(4,4,5,5-tetramethyl-1,3,2-dio xaborolan-2-yl)phenyl]benzamide (353 mg, 0.9 mmol), which was afforded in Referential Example 1, was dissolved in a mixed solution of dimethoxyethane (15 mL) and water (1 mL). To this solution, ethyl 4-[(4-chloro-1,3,5-triazin-2-yl)amino]benzoate (250 mg, 0.9 mmol) which was afforded in the First Step, tetrakis(triphenylphosphine)palladium (0) (104 mg, 0.09 mmol) and potassium carbonate (248

mg, 1.79 mmol) were added. The reaction vessel was degassed under vacuum, then filled with nitrogen, and then heated with the microwave synthesizer at 110 °C for 20 min. The reaction mixture was diluted with water, and extracted with ethyl acetate for 2 times. The combined organic layer was washed with saturated sodium hydrogen carbonate solution and brine, dried over sodium sulfate, filtered and concentrated. The crude material was purified by chromatography on silica gel, eluted with hexane/ethyl acetate to afford ethyl 4-[(4-{3-[4-(*tert*-butyl)benzamido]-2-methylphenyl}-1,3,5-tr iazin-2-yl)amino]benzoate (183 mg).

$^1$H NMR (400 MHz, DMSO-$d_6$) $\delta$ 10.74 (s, 1H), 9.98 (s, 1H), 8.96 (s, 1H), 7.95 (d, J = 4.7 Hz, 6H), 7.70 (dd, J = 7.8, 1.3 Hz, 1H), 7.60 - 7.48 (m, 3H), 7.39 (t, J = 7.8 Hz, 1H), 4.30 (q, J = 7.1 Hz, 2H), 2.41 (s, 3H), 1.33 (s, 9H) 1.32 (t, J = 7.1 Hz, 3H) ; LCMS (m/z): 509.9 [M+H]$^+$.

<u>Third Step</u>

To a solution of ethyl 4-[(4-{3-[4-(*tert*-butyl)benzamido]-2-methylphenyl}-1,3,5-tr iazin-2-yl)amino]benzoate (730 mg) in THF (16.4 mL), ethanol (8.2 mL) and 2N sodium hydroxide solution (4.1 mL) were added at ambient temperature then stirred for overnight. The reaction mixture was neutralized by 2N hydrochloric acid solution, water was added thereto, and then extracted with ethyl acetate for 2 times. The combined organic layer was washed with water and brine, dried over sodium sulfate, filtered and concentrated to afford the titled compound (690 mg).

$^1$H NMR (400 MHz, DMSO-$d_6$) $\delta$ 12.71 (s, 1H), 10.70 (s, 1H), 9.97 (s, 1H), 8.95 (s, 1H), 7.99 - 7.88 (m, 5H), 7.69 (dd, J = 7.7, 1.4 Hz, 1H), 7.65 - 7.47 (m, 4H), 7.39 (t, J = 7.8 Hz, 1H), 2.40 (s, 3H), 1.33 (s, 9H) ; LCMS (m/z): 482.0 [M+H]$^+$.

<u>Example 4</u>

4-(*tert*-Butyl)-N-[2-methyl-3-(4-{[4-(4-methylpiperazine-1-c arbonyl)phenyllaminol-1,3,5-triazin-2-yl)phenyl]benzamide

<u>Chemical Formula 16</u>

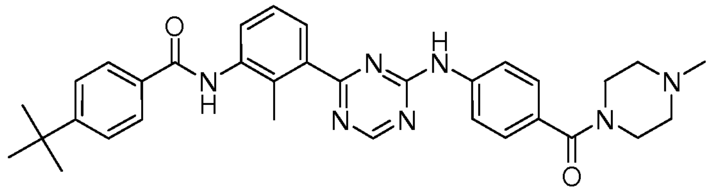

4-[(4-{3-[4-(*tert*-Butyl)benzamido]-2-methylphenyl}-1,3,5-tr iazin-2-yl)amino]benzoic acid (50 mg, 0.1 mmol) and 1-methylpiperazine (10 mg, 0.1 mmol) were dissolved in DMF (0.5 mL), then EDC (30 mg, 0.16 mmol) was added to the solution and stirred at ambient temperature for 3 days. Water was added to the reaction mixture, and then extracted with ethyl acetate for 2 times. The combined organic layer was washed with saturated sodium hydrogen carbonate solution and brine, dried over sodium sulfate, filtered and concentrated. The crude material was purified by chromatography on silica gel, eluted with hexane/ethyl acetate followed by chloroform/methanol to afford the titled compound (24 mg).

$^1$H NMR (400 MHz, DMSO-$d_6$) $\delta$ 10.55 (s, 1H), 9.97 (s, 1H), 8.91 (s, 1H), 7.99 - 7.91 (m, 2H), 7.88 - 7.81 (m, 2H), 7.68 (dd, J = 7.8, 1.5 Hz, 1H), 7.60 - 7.46 (m, 3H), 7.44 - 7.34 (m, 3H), 3.64 - 3.54 (m, 2H), 2.39 (s, 3H), 2.33 - 2.28 (m, 5H), 2.21 - 2.16 (m, 4H), 1.33 (s, 9H) ; LCMS (m/z): 564.2 [M+H]$^+$.

**<u>Example 5</u>**

*N*-[3-(4-Amino-6-{[4-(morpholine-4-carbonyl)phenyl]amino}-1, 3,5-triazin-2-yl)-2-methylphenyl]-4-(tert-butyl)benzamide

Chemical Formula 17

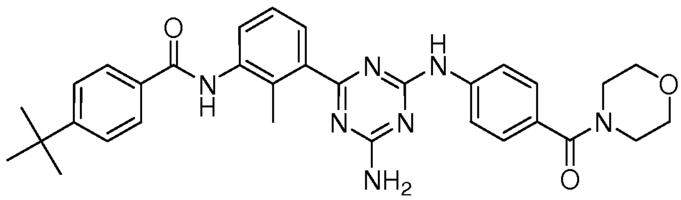

First Step

To a solution of 2-amino-4,6-dichloro-1,3,5-triazine (300 mg, 1.82 mmol) in DMF (3 mL), cooled with ice bath, DIEA (0.23 mL, 1.33 mmol) and (4-aminophenyl)morpholin-4-ylmethanone (250 mg, 1.21 mmol) were added then stirred at ambient temperature for 16 h. The reaction mixture was diluted with ethyl acetate (100 mL), washed with water and brine, dried over sodium sulfate, filtered and concentrated to afford {4-[(4-amino-6-chloro-1,3,5-triazin-2-yl)amino]phenyl}(morp holino)methanone (325 mg).

$^1$H NMR (400 MHz, DMSO-$d_6$) δ 10.15 (s, 1H), 7.81 (d, J = 8.3 Hz, 2H), 7.68 (s, 2H), 7.40 - 7.32 (m, 2H), 3.65 - 3.53 (m, 4H), 3.58 - 3.44 (m, 4H).; LCMS (m/z): 335.0 $[M+H]^+$.

Second Step

To a solution of {4-[(4-amino-6-chloro-1,3,5-triazin-2-yl)amino]phenyl}(morp holino)methanone (50.0 mg, 0.15 mmol) and 4-(*tert*-butyl)-N-[2-methyl-3-(4,4,5,5-tetramethyl-1,3,2-dio xaborolan-2-yl)phenyl]benzamide (59 mg, 0.15 mmol) which was afforded in Referential Example 1 in dimethoxyethane (3 mL), tetrakis(triphenylphosphine)palladium (0) (17 mg, 0.015 mmol) and potassium carbonate (41 mg, 0.3 mmol) in water solution (1 mL) were added then heated with the microwave synthesizer at 110 °C for 20 min. The reaction mixture was extracted with ethyl acetate (2x40 mL), and the combined organic layer was washed with water (5 mL), dried over sodium sulfate, filtered and concentrated. The crude material was purified by chromatography on silica gel, eluted with hexane/ethyl acetate to afford the titled compound (27 mg).

$^1$H NMR (400 MHz, DMSO-$d_6$) δ 9.92 (s, 1H), 9.82 (s, 1H), 7.98 - 7.86 (m, 4H), 7.59 - 7.47 (m, 3H), 7.44 - 7.23 (m, 6H), 3.65 - 3.53 (m, 4H), 3.58 -3.44 (m, 4H), 2.34 (s, 3H), 1.33 (s, 9H) ; LCMS (m/z): 565.9 $[M+H]^+$.

Example 33

2-[3-(4-Amino-6-{[4-(morpholine-4-carbonyl)phenyl]amino}-1, 3,5-triazin-2-yl)-2-methylphenyl]-5-(*tert*-butyl)isoindolin-1-one

Chemical Formula 18

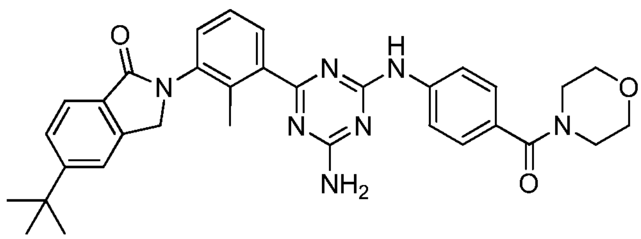

First Step

To a solution of 4-*tert*-butylphthalic anhydride (4.39 g, 21.5 mmol) in acetic acid (40 mL), 3-bromo-2-methylaniline (2.65 mL, 21.5 mmol) was added and stirred at 100 °C for 1 h. The reaction mixture was concentrated under reduced pressure, and diluted with ethyl acetate (300 mL), washed with water (100 mL), saturated sodium hydrogen carbonate solution (100 mL) and brine (100 mL), dried over sodium sulfate, filtered and concentrated. The crude material was

suspended in hexane, then the precipitate was collected by filtration, washed with hexane then dried to afford 2-(3-bromo-2-methylphenyl)-5-(tert-butyl)isoindoline-1,3-di one (7.0 g).

$^1H$ NMR (400 MHz, $CDCl_3$) δ 8.00 (dd, J = 1.7, 0.7 Hz, 1H), 7.93 - 7.77 (m, 2H), 7.67 (dd, J = 7.6, 1.8 Hz, 1H), 7.24 - 7.12 (m, 2H), 2.27 (s, 3H), 1.42 (s, 9H).

Second Step

2-(3-Bromo-2-methylphenyl)-5-(*tert*-butyl)isoindoline-1,3-di one (1.0 g, 2.7 mmol) was suspended in methanol (20 mL), and sodium borohydride (203 mg, 5.37 mmol) was added, then stirred at ambient temperature for 10 min. The reaction mixture was concentrated under reduced pressure, then diluted with DCM (200 mL), washed with water (100 mL) and brine (100 mL), dried over sodium sulfate, filtered and concentrated. The crude material was diluted with DCM (10 mL), and trifluoroacetic acid (10 mL) was added then stirred at ambient temperature for 10 min. To this reaction mixture, triethylsilane (6.4 mL) was added then stirred at ambient temperature for further 30 min. The reaction mixture was concentrated under reduced pressure, and diluted with DCM (100 mL), washed with water (50 mL) and brine (20 mL), dried over sodium sulfate, filtered and concentrated. The crude material was purified by chromatography on silica gel, eluted with hexane/ethyl acetate to afford 2-(3-bromo-2-methylphenyl)-5-(*tert*-butyl)isoindolin-1-one (242 mg).

$^1H$ NMR (400 MHz, $CDCl_3$) δ 7.88 (dd, J = 8.1, 0.6 Hz, 1H), 7.59 (dt, J = 8.2, 1.9 Hz, 2H), 7.55 - 7.50 (m, 1H), 7.21 (dd, J = 7.9, 1.4 Hz, 1H), 7.14 (td, J = 7.9, 0.7 Hz, 1H), 4.69 (s, 2H), 2.31 (s, 3H), 1.40 (s, 9H).

Third Step

Under nitrogen atmosphere, 2-(3-bromo-2-methylphenyl)-5-(*tert*-butyl)isoindolin-1-one (200 mg, 0.56 mmol) was dissolved in dioxane (3.7 mL). To this solution, bis(pinacolato)diboron (284 mg, 1.12 mmol), dichloro[1,1'-bis(diphenylphosphino)ferrocene]palladium(II) dichloromethane adduct (46 mg, 0.056 mmol) and potassium acetate (164 mg, 1.68 mmol) were added and refluxed for 16 h. Water (50 mL) was added to the reaction mixture, and extracted with ethyl acetate (3x25 mL). The combined organic layer was washed with saturated sodium hydrogen carbonate solution and brine, dried over sodium sulfate, filtered and concentrated. The crude material was purified by chromatography on silica gel, eluted with hexane/ethyl acetate to afford 5-(*tert*-butyl)-2-[2-methyl-3-(4,4,5,5-tetramethyl-1,3,2-dio xaborolan-2-yl)phenyl]isoindolin-1-one (206 mg).

$^1H$ NMR (400 MHz, $CDCl_3$) δ 7.91 - 7.85 (m, 1H), 7.81 (dd, J = 7.0, 2.0 Hz, 1H), 7.57 (dd, J = 8.1, 1.6 Hz, 1H), 7.54 - 7.49 (m, 1H), 7.41 - 7.18 (m, 2H), 4.66 (s, 2H), 2.43 (s, 3H), 1.40 (s, 9H), 1.35 (s, 12H).

Fourth Step

To a solution of {4-[(4-amino-6-chloro-1,3,5-triazin-2-yl)amino]phenyl}(morp holino)methanone (69.0 mg, 0.21 mmol) which was afforded in Example 5, First Step and 5-(*tert*-butyl)-2-[2-methyl-3-(4,4,5,5-tetramethyl-1,3,2-dio xaborolan-2-yl)phenyl]isoindolin-1-one (126 mg, 0.31 mmol) in dimethoxyethane (3 mL), tetrakis(triphenylphosphine)palladium (0) (24 mg, 0.021 mmol) and potassium carbonate (57 mg, 0.4 mmol) in water solution (1 mL) were added then heated with the microwave synthesizer at 110 °C for 20 min. The reaction mixture was diluted with water (50 mL), and extracted with ethyl acetate (3x25 mL). The combined organic layer was washed with saturated sodium hydrogen carbonate solution and brine, dried over sodium sulfate, filtered and concentrated. The crude material was purified by chromatography on silica gel, eluted with hexane/ethyl acetate to afford the titled compound (30 mg).

$^1H$ NMR (400 MHz, DMSO-$d_6$) δ 9.84 (s, 1H), 7.89 (d, J = 8.3 Hz, 2H), 7.77 - 7.66 (m, 2H), 7.66 - 7.58 (m, 2H), 7.49 (dd, J = 7.9, 1.5 Hz, 1H), 7. 43 - 7.31 (m, 3H), 7.32 - 7.21 (m, 2H), 4.85 (s, 2H), 3.75 - 3.37 (m, 8H), 2.26 (s, 3H), 1.36 (s, 9H); LCMS (m/z): 578.1 $[M+H]^+$.

Example 39

*N*-[3-(4-Amino-6-{(4-(morpholine-4-carbonyl)phenyl]amino}-1, 3,5-triazin-2-yl)-2-(hydroxymethyl)phenyl]-4-(tert-butyl)be nzamide

Chemical Formula 19

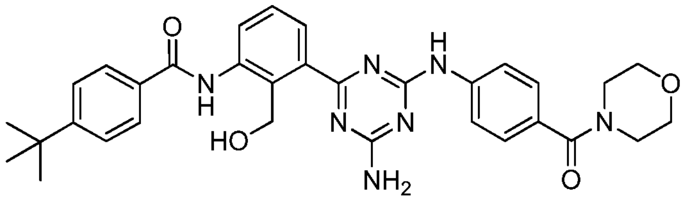

First Step

To a solution of (2-bromo-6-nitrobenzyloxy)(tert-butyl)dimethylsilane (14.5g, 41.9 mmol) in ethanol (291 mL), iron powder (23.4 g, 419 mmol), ammonium chloride (44.8 g, 837 mmol) and water (58 mL) were added and stirred at 80 °C for 3 h. The reaction mixture was filtered and the filtrate was concentrated under reduced pressure. To this crude material, water (500 mL) was added then extracted with chloroform (3x300 mL). The combined organic layer was washed with water and brine, dried over sodium sulfate, filtered and concentrated to afford 3-bromo-2-{[(*tert*-butyldimethylsilyl)oxy]methyl}aniline (12.1 g).

$^1H$ NMR (400 MHz, $CDCl_3$) δ 6.95 - 6.85 (m, 2H), 6.59 (m, 1H), 4.95 (s, 2H), 4.48 (s, 2H), 0.89 (s, 9H), 0.10 (s, 6H).

Second Step

Under nitrogen atmosphere, 3-bromo-2-{[(*tert*-butyldimethylsilyl)oxy]methyl}aniline (2.0 g, 6.32 mmol) was dissolved in THF (63 mL). To this solution, cooled with ice bath, TEA (1.76 mL, 12.65 mmol) and 4-*tert*-butylbenzoyl chloride (1.36 mL, 6.96 mmol) were added and stirred at 0 °C for 4 h. The reaction mixture was diluted with DCM (300 mL), and washed with water (100 mL), 1N hydrochloric acid solution (100 mL), saturated sodium hydrogen carbonate solution (100 mL) and brine (100 mL), dried over sodium sulfate, filtered and concentrated. The resulting crude material was dissolved in dioxane (39 mL) under nitrogen atmosphere, and bis(pinacolato)diboron (2.98 g, 11.75 mmol), dichloro[1,1'-bis(diphenylphosphino)ferrocene]palladium(II) dichloromethaneadduct (480 mg,0. 588 mmol) and potassium acetate (1.73 g, 17.63 mmol) were added and stirred at 80 °C for 16 h. Water (100 mL) was added to the reaction mixture, and extracted with ethyl acetate (3x100 mL). The combined organic layer was washed with saturated sodium hydrogen carbonate solution and brine, dried over sodium sulfate, filtered and concentrated. The crude material was purified by chromatography on silica gel, eluted with hexane/ethyl acetate to afford 4-(*tert*-butyl)-*N*-(2-{[(*tert*-butyldimethylsilyl)oxy]methyl}-3-(4,4,5,5-tetramethyl-1,3,2-dioxaborolan-2-yl)phenyl)benza mide (3.0 g).

$^1H$ NMR (400 MHz, $CDCl_3$) δ 10.06 (s, 1H), 8.46 (dd, J = 8.1, 1.4 Hz, 1H), 7.96 - 7.84 (m, 2H), 7.61 (dd, J = 7.5, 1.4 Hz, 1H), 7.53 - 7.42 (m, 2H), 7.36 (t, J = 7.8 Hz, 1H), 5.27 (s, 2H), 1.38 - 1.34 (m, 21H), 0.88 (s, 9H), 0.13 (s, 6H).

Third Step

To a stirred solution of {4-[(4-amino-6-chloro-1,3,5-triazin-2-yl)amino]phenyl}(morp holino)methanone (256 mg, 0.76 mmol) which similarly prepared according to the procedure described in the Example 5, First Step and 4-(*tert*-butyl)-*N*-(2-{[(tert-butyldimethylsilyl)oxy]methyl}-3-(4,4,5,5-tetramethyl-1,3,2-dioxaborolan-2-yl)phenyl)benza mide (400 mg, 0.76 mmol) in dimethoxyethane (11.5 mL), tetrakis(triphenylphosphine)palladium (0) (88 mg, 0.076 mmol) and potassium carbonate (211 mg, 1.53 mmol) in water solution (3.8 mL) were added then heated with the microwave synthesizer at 110 °C for 20 min. Water (100 mL) was added to the reaction mixture, and extracted with ethyl acetate (3x50 mL). The combined organic layer was washed with saturated sodium hydrogen bicarbonate solution and brine, dried over sodium sulfate, filtered and concentrated. The crude material was purified by chromatography on silica gel, eluted with hexane/ethyl acetate to afford *N*-[3-(4-amino-6-{[4-(morpholine-4-carbonyl)phenyl]amino}-l, 3,5-triazin-2-yl)-2-{[(tert-butyldimethylsilyl)oxy]methyl}p henyl]-4-(tert-butyl)benzamide (144 mg).

$^1H$ NMR (400 MHz, DMSO-$d_{66}$) δ 10.04 (s, 1H), 9.82 (s, 1H), 7.92 - 7.84 (m, 3H), 7.84 - 7.75 (m, 1H), 7.60 - 7.52 (m, 2H), 7.50 - 7.30 (m, 5H), 7.31 - 7.10 (m, 2H), 5.11 (s, 2H), 3.68 - 3.55 (m, 4H), 3.56 - 3.43 (m, 4H), 1.33 (s, 9H), 0.67 (s, 9H), 0.17 (s, 6H).

Fourth Step

To a solution of *N*-[3-(4-amino-6-{[4-(morpholine-4-carbonyl)phenyl]amino}-1, 3,5-triazin-2-yl)-2-{[(tert-butyldimethylsilyl)oxy]methyl}p henyl]-4-(*tert*-butyl)benzamide (144 mg, 0.2 mmol) in THF (4.1 mL), tetrabutylammonium fluoride THF solution (1 mol/L, 0.41 mL) was added and stirred at ambient temperature for 16 h. Solvents were removed

under reduced pressure, and the crude material was purified by chromatography on silica gel, eluted with hexane/ethyl acetate to afford the titled compound (62 mg).

$^1$H NMR (400 MHz, DMSO-$d_6$) δ 10.41 (s, 1H), 9.86 (s, 1H), 8.01 (d, J = 7.9 Hz, 1H), 7.94 - 7.86 (m, 4H), 7.63 - 7.54 (m, 2H), 7.51 (dd, J = 7.8, 1.4 Hz, 1H), 7.42 (t, J = 7.8 Hz, 1H), 7.36 (d, J = 8.6 Hz, 2H), 7.35 - 7.19 (m, 2H), 5.67 (t, J = 5.6 Hz, 1H), 4.83 (d, J = 5.5 Hz, 2H), 3.73 - 3.55 (m, 4H), 3.55 - 3.35 (m, 4H), 1.33 (s, 9H) ; LCMS (m/z): 582.1 $[M+H]^+$.

**Example 42**

5-[3-(4-Amino-6-{[4-(morpholine-4-carbonyl)phenyl]amino}-1, 3,5-triazin-2-yl)-2-methylphenyl]-2-(*tert*-butyl)-4*H*-thieno[ 2,3-c]pyrrol-6(5*H*)-one

Chemical Formula 20

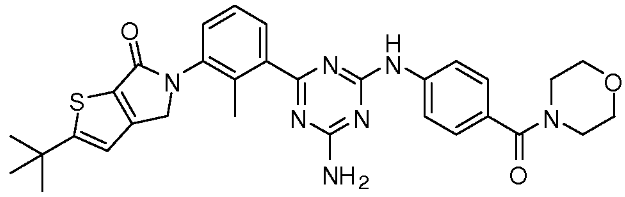

First Step

Under nitrogen atmosphere, aluminum chloride (6.4 g, 48.0 mmol) was dissolved in DCM (7.3 mL). To this solution, methyl 3-methylthiophene-2-carboxylate (5.0 g, 32.0 mmol) in DCM solution (3.6 mL) was added dropwise during a period of 5 min at -80 °C then stirred for 5 min. To this reaction mixture, 2-chloro-2-methylpropane (3.55 g, 38.4 mmol) in DCM solution (3.6 mL) was added dropwise during a period of 5 min. The reaction mixture was allowed to warm gradually to ambient temperature and stirred for 14 h. The reaction mixture was poured into ice then extracted with DCM (3x300 mL). The combined organic layer was washed with water, saturated sodium hydrogen carbonate solution and brine, dried over sodium sulfate, filtered and concentrated. The crude material was purified by chromatography on silica gel, eluted with hexane/ethyl acetate to afford methyl 5-(*tert*-butyl)-3-methylthiophene-2-carboxylate (4.74 g).

$^1$H NMR (400 MHz, $CDCl_3$) δ6.67 (s, 1H), 3.83 (s, 3H), 2.49 (s, 3H), 1.37 (s, 9H) ; LCMS (m/z): 213.0 $[M+H]^+$.

Second Step

Methyl 5-(*tert*-butyl)-3-methylthiophene-2-carboxylate (3.15g, 14.84 mmol) was suspended in carbon tetrachloride (40 mL), then N-bromosuccinimide (3.17 g, 17.8 mmol) and 2,2'-azobis(2-methylpropionitrile) (122 mg, 0.74 mmol) were added and stirred at 85 °C for 14 h. The reaction mixture was filtered, and the filtrate was concentrated under reduced pressure. The crude material was purified by chromatography on silica gel, eluted with hexane/ethyl acetate to afford methyl 3-(bromomethyl)-5-(tert-butyl)thiophene-2-carboxylate (4.15 g).

$^1$H NMR (400 MHz, $CDCl_3$) δ6.92 (s, 1H), 4.86 (s, 2H), 3.87 (s, 3H), 1.39 (s, 9H).

Third Step

Under nitrogen atmosphere, methyl 3-(bromomethyl)-5-(tert-butyl)thiophene-2-carboxylate (1.5g, 5.15mmol) was dissolved in acetonitrile (25 mL). To this solution, 3-bromo-2-methylaniline (2.88g, 15.45mmol) and cesium carbonate (1.85g, 5.67 mmol) were added then stirred at ambient temperature for 16 h. Solvents were removed under reduced pressure, then the crude material was purified by chromatography on silica gel, eluted with hexane/ethyl acetate to afford methyl 3-{[(3-bromo-2-methylphenyl)amino]methyl}-5-(*tert*-butyl)thi ophene-2-carboxylate (1.43 g).

$^1$H NMR (400 MHz, $CDCl_3$) δ6.95-6.90 (m, 2H), 6.85 (s, 1H), 6.58 (dd, J = 8.0, 4.0Hz, 1H), 4.62-4.58 (m, 1H), 4.60 (s, 2H), 3.86 (s, 3H), 2.29 (s, 3H), 1.35 (s, 9H).

Fourth Step

To a solution of methyl 3-{[(3-bromo-2-methylphenyl)amino]methyl}-5-(tert-butyl)thi ophene-2-carboxylate (1.39

g, 3.5 mmol) in a mixed solution of THF and methanol (1:1, 10 mL), lithium hydroxide (838 mg, 35 mmol) in water solution (5 mL) was added and stirred at 45 °C for 14 h. The reaction mixture was concentrated under reduced pressure, and 2N hydrochloric acid solution (20 mL) was added. The precipitate was collected by filtration, washed with hexane then dried to afford 3-{[(3-bromo-2-methylphenyl)amino]methyl}-5-(tert-butyl)thi ophene-2-carboxylic acid (1.15 g).

$^1$H NMR (400 MHz, DMSO-$d_6$) $\delta$7.00 (dd, J = 8.0, 1.2Hz, 1H), 6.93 (t, J = 8.0 Hz, 1H), 6.87 (s, 1H), 6.61 (d, J = 8.0 Hz, 1H), 4.59 (s, 2H), 2.31 (s, 3H), 1.37 (s, 9H) ; LCMS (m/z): 382.0 $[M+H]^+$.

Fifth Step

Under nitrogen atmosphere, to a solution of 3-{[(3-bromo-2-methylphenyl)amino]methyl}-5-(tert-butyl)thi ophene-2-carboxylic acid (1.04 g, 2.72 mmol) in DCM (25 mL), thionyl chloride (0.79 mL, 10.9 mmol) was added and stirred at ambient temperature for 16 h. The reaction mixture was concentrated under reduced pressure, then the crude material was purified by chromatography on silica gel, eluted with hexane/ethyl acetate to afford 5-(3-bromo-2-methylphenyl)-2-(*tert*-butyl)-4*H*-thieno[2,3-*c*]p yrrol-6(5H)-one (605 mg).

$^1$H NMR (400 MHz, DMSO-$d_6$) $\delta$7.57 (dd, J = 8.0, 1.2Hz, 1H), 7.20 (dd, J = 8. 0, 1.2Hz, 1H), 7.12 (t, J = 8.0Hz, 1H), 6. 88 (s, 1H), 4.56 (s, 2H), 2.33 (s, 3H), 1.45 (s, 9H) ;LCMS (m/z) : 364.1 $[M+H]^+$.

Sixth Step

Under nitrogen atmosphere, 5-(3-bromo-2-methylphenyl)-2-(*tert*-butyl)-4*H*-thieno[2,3-*c*]p yrrol-6(5H)-one (460 mg, 1.26 mmol) was dissolved in dioxane (8.0 mL). To this solution, bis(pinacolato)diboron (641 mg, 2.53 mmol), dichloro[1,1'-bis(diphenylphosphino)ferrocene]palladium(II) dichloromethaneadduct (103 mg, 0.126 mmol) and potassium acetate (372 mg, 3.79 mmol) were added and stirred at 80 °C for 16 h. Water (50 mL) was added to the reaction mixture, and extracted with ethyl acetate (2x25 mL). The combined organic layer was washed with saturated sodium hydrogen carbonate solution and brine, dried over sodium sulfate, filtered and concentrated. The crude material was purified by chromatography on silica gel, eluted with hexane/ethyl acetate to afford 2-(*tert*-butyl)-5-[2-methyl-3-(4,4,5,5-tetramethyl-1,3,2-dio xaborolan-2-yl)phenyl]-4*H*-thieno[2,3-*c*]pyrrol-6(5*H*)-one 210 mg).

$^1$H NMR (400 MHz, DMSO-$d_6$) $\delta$7.79 (dd, J = 7.6, 1.6Hz, 1H), 7.30 (dd, J = 7. 6, 1.6Hz, 1H), 7. 23 (t, J = 7.6Hz, 1H), 6.87 (s, 1H), 4.53 (s, 2H), 2.44 (s, 3H), 1.45 (s, 9H), 1.34 (s, 12H) ; LCMS (m/z): 412.1 $[M+H]^+$.

Seventh Step

To a stirred solution of {4-[(4-amino-6-chloro-1,3,5-triazin-2-yl)amino]phenyl}(morp holino)methanone (81.1 mg, 0.24 mmol) which was afforded in the Example 5, First step and 2-(*tert*-butyl)-5-[2-methyl-3-(4,4,5,5-tetramethyl-1,3,2-dio xaborolan-2-yl)phenyl]-4*H*-thieno[2,3-*c*]pyrrol-6(5*H*)-one (100 mg, 0.24 mmol) in dimethoxyethane (3 mL), tetrakis(triphenylphosphine)palladium (0) (28 mg, 0.024 mmol) and potassium carbonate (67 mg, 0.49 mmol) in water solution (1.2 mL) were added then heated with the microwave synthesizer at 110 °C for 40 min. Water (50 mL) was added to the reaction mixture, and extracted with ethyl acetate (2x25 mL). The combined organic layer was washed with saturated sodium hydrogen carbonate solution and brine, dried over sodium sulfate, filtered and concentrated. The crude material was purified by chromatography on silica gel, eluted with hexane/ethyl acetate to afford the titled compound (50 mg).

$^1$H NMR (400 MHz, DMSO-$d_6$) $\delta$9.84 (s, 1H), 7.89 (d, J = 8. 4Hz, 2H), 7.61(d, J=7.6Hz, 1H), 7.48(dd, J=7.6, 1.6Hz, 1H), 7.36 (t, J = 7.6Hz, 1H), 7.35 (d, J = 8.4Hz, 2H), 7.34 - 7.16 (m, 2H), 7.14 (s, 1H), 4.76 (s, 2H), 3.65 - 3.57(m, 4H), 3.56 - 3.40(m, 4H), 2.28 (s, 3H), 1.42 (s, 9H) ; LCMS (m/z): 584.1 $[M+H]^+$.

**Example 43**

2-(3-{4-Amino-6-[(4-morpholinophenyl)amino]-1,3,5-triazin-2 -yl}-2-methylphenyl)-6-cyclopropylphthalazin-1(2*H*)-one

Chemical Formula 21

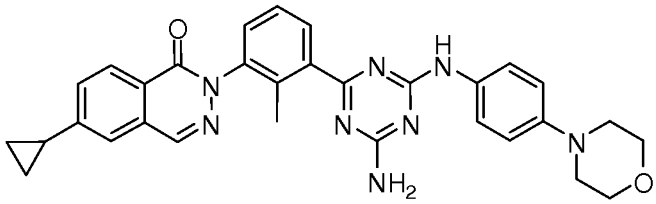

First Step

6-Bromophthalazin-1(2*H*)-one (1.00 g, 4.44 mmol), cyclopropylboronic acid (0.57 g, 6.67 mmol), tricyclohexylphosphine (0.13 g, 0.44 mmol) and potassium phosphate (0.57 g, 6.67 mmol) were suspended in a mixed solution of toluene (20mL) and water (1mL). To this mixture, palladium(II) acetate (0.20 g, 0.89 mmol) was added under nitrogen atmosphere at ambient temperature and stirred at 100 °C for 3 h. Cooled to ambient temperature, the reaction mixture was filtered and insoluble material was washed with water and ethyl acetate. The filtrate was extracted with ethyl acetate, washed with water and brine, dried over sodium sulfate, filtered and concentrated. The crude material was purified by chromatography on silica gel, eluted with hexane/ethyl acetate to afford 6-cyclopropylphthalazin-1(2*H*)-one (0.13 g).
$^1$H NMR (400 MHz, DMSO-$d_6$) $\delta$ 12.52 (s, 1H), 8.25 (s, 1H), 8.08 (d, J = 8.2 Hz, 1H), 7.60 (d, J = 1.7 Hz, 1H), 7.56 (dd, J = 8.3, 1.8 Hz, 1H), 2.18 - 2.00 (m, 1H), 1.14 - 1.08 (m, 2H), 0.89 - 0.83 (m, 2H) ; LCMS (m/z): 187.1 $[M+H]^+$.

Second Step

6-Cyclopropylphthalazin-1(2H)-one (56 mg, 0.30 mmol), 1,3-dibromo-2-methylbenzene (150 mg, 0.60 mmol), cesium carbonate (195 mg, 0.60 mmol) and copper (I) iodide (11 mg, 0.06 mmol) were dissolved in DMSO (2 mL). The reaction vessel was filled with nitrogen during a period of 5 min, and then stirred at 150 °C for 18 h. Cooled to ambient temperature, the reaction mixture was poured into cold water, and extracted with ethyl acetate for 2 times. The combined organic layer was washed with brine, dried over sodium sulfate, filtered and concentrated. The crude material was purified by chromatography on silica gel, eluted with hexane/ethyl acetate to afford 2-(3-bromo-2-methylphenyl)-6-cyclopropylphthalazin -1(2*H*)-one (17 mg).
$^1$H NMR (400 MHz, DMSO-$d_6$) $\delta$ 11.23 - 10.60 (m, 1H), 7.69 - 7.63 (m, 2H), 7.50 - 7.45 (m, 1H), 7.45 - 7.33 (m, 1H), 7.21 - 7.08 (m, 1H), 6.95 - 6.83 (m, 1H), 2.23 - 2.12 (m, 4H), 1.13 - 1.06 (m, 2H), 0.90 - 0.84 (m, 2H).; LCMS (m/z): 355.1, 357.1 $[M+H]^+$.

Third Step

To a solution of 2-(3-bromo-2-methylphenyl)-6-cyclopropylphthalazin-1(2*H*)-on e (16 mg, 0.045 mmol) in dioxane (0.45 mL), bis(pinacolato)diboron (23 mg, 0.09 mmol), dichloro[1,1'-bis(diphenylphosphino)ferrocene]palladium(II) dichloromethane adduct (3.7 mg, 0.0045 mmol) and potassium acetate (13 mg, 0.13 mmol) were added and stirred at 80 °C for 20h. Cooled to ambient temperature, water was added to the reaction mixture, and extracted with ethyl acetate. The organic layer was dried over sodium sulfate, filtered and concentrated. The crude material was purified by chromatography on silica gel, eluted with hexane/ethyl acetate to afford 6-cyclopropyl-2-[2-methyl-3-(4,4,5,5-tetramethyl-1,3,2-diox aborolan-2-yl)phenyllphthalazin-1(2*H*)-one (9 mg).
LCMS (m/z): 403.3 $[M+H]^+$.

Fourth Step

To a solution of 2-amino-4,6-dichloro-1,3,5-triazine (124 mg, 0.75 mmol) in THF (1.2 mL), cooled with ice bath, DIEA (0.18 mL, 1.33 mmol) and 4-morpholinoaniline (89 mg, 0.50 mmol) in THF solution (0.8 mL) were added slowly then stirred with ice bath for 1 h and then at ambient temperature for further 16 h. The precipitate was collected by filtration, washed with ethyl acetate then dried to afford 2-amino-4-chloro-6-[(4-morpholinophenyl)amino]-1,3,5-triazine (124 mg).
$^1$H NMR (400 MHz, DMSO-$d_6$) $\delta$ 9.85 - 9.45 (m, 1H), 7.65 - 7.20 (m, 4H), 6.88 (d, J = 9.0 Hz, 2H), 3.77 - 3.69 (m, 4H), 3.08 - 3.01 (m, 4H).; LCMS (m/z): 307.1 $[M+H]^+$.

Fifth Step

2-Amino-4-chloro-6-[(4-morpholinophenyl)amino]-1,3,5-triazi ne (7 mg, 0.022 mmol) and 6-cyclopropyl-2-[2-methyl-3-(4,4,5,5-tetramethyl-1,3,2-diox aborolan-2-yl)phenyl]phthalazin-1(2*H*)-one (9 mg, 0.022 mmol) which was afforded in the above mentioned Third Step were dissolved in dimethoxyethane (0.6 mL). Under nitrogen atmosphere, tetrakis(triphenylphosphine)palladium(0) (2.6mg, 0.0022mmol) and potassium carbonate (6 mg, 0.45 mmol) in water solution (0.2 mL) were added to this solution then heated with the microwave synthesizer at 110 °C for 20 min. Cooled to ambient temperature, filtered to remove insoluble material. The crude material was purified by HPLC preparative chromatography (water/methanol (containing formic acid) as eluents) to afford the titled compound (2.7 mg).

$^1$H NMR (400 MHz, DMSO-$d_6$) δ 11.13 - 10.65 (m, 1H), 9.28 (br. s, 1H), 7.72 - 7.53 (m, 4H), 7.50 - 7.43 (m, 1H), 7.42 - 7.35 (m, 1H), 7.33 - 7.20 (m, 1H), 7.08 - 6.78 (m, 5H), 3.77 - 3.68 (m, 4H), 3.08 - 2.95 (m, 4H), 2.30 - 2.15 (m, 4H), 1.15 - 1.00 (m, 2H), 0.95 - 0.80 (m, 2H).; LCMS (m/z): 547.3 $[M+H]^+$.

**Example 44**

2-(3-{4-Amino-6-[(4-morpholinophenyl)amino]-1,3,5-triazin-2 -yl}-2-(hydroxymethyl)phenyl)-6-cycropropyl-8-fluoro-3,4-di hydroisoquinolin-1(2*H*)-one

Chemical Formula 22

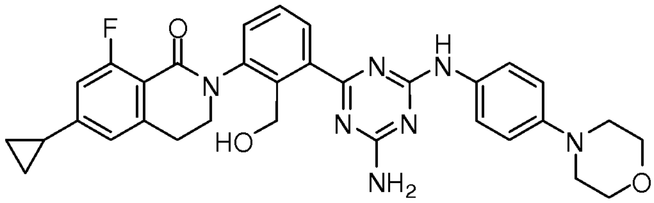

First Step

Under nitrogen atmosphere, 6-cyclopropyl-8-fluoro-3,4-dihydroisoquinolin-1(2*H*)-one (1.56g, 7.6 mmol), 1,6-dibromobenzaldehyde (4.0g, 15.2 mmol), copper (I) iodide (1.45g, 7.6 mmol) and sodium hydrogen carbonate (1.28 g, 15.2 mmol) were suspended in DMSO (15 mL), then stirred at 110 °C for 2 days. Cooled to ambient temperature, water and ethyl acetate were added. Filtered through Celite pad to remove insoluble material, then the filtrate was extracted with ethyl acetate for 3 times. The combined organic layer was washed with brine, dried over sodium sulfate, filtered and concentrated. The crude material was purified by chromatography on silica gel, eluted with chloroform only followed by hexane/ethyl acetate to afford 2-bromo-6-(6-cyclopropyl-8-fluoro-1-oxo-3,4-dihydroisoquino lin-1(2*H*)-yl)benzaldehyde (2.07 g).

$^1$H NMR (400 MHz, $CDCl_3$) δ 10.21 (s, 1H), 7.58 (dd, J = 8.1, 1.1 Hz, 1H), 7.42 (t, J = 8.0 Hz, 1H), 7.31 - 7.22 (m, 1H), 6.73 (d, J = 1.6 Hz, 1H), 6.68 (dd, J = 12.3, 1.7 Hz, 1H), 4.05 - 3.75 (m, 2H), 3.55 - 2.80 (m, 2H), 1.93 - 1.85 (m, 1H), 1.12 - 1.02 (m, 2H), 0.81 - 0.73 (m, 2H); LCMS (m/z): 387.9 / 389.9 $[M+H]^+$.

Second Step

To a solution of 2-bromo-6-(6-cyclopropyl-8-fluoro-1-oxo-3,4-dihydroisoquino lin-1(2*H*)-yl)benzaldehyde (2.53 g, 6.52 mmol) in a mixed solvent of DCM and isopropanol (2:1, 36 mL) cooled in an ice bath, sodium borohydride (0.12 g, 3.26 mmol) was added and the suspension mixture was allowed to warm to ambient temperature and then stirred vigorously for 1 h. The reaction mixture was poured into ice water, extracted with chloroform for 2 times. The combined organic layer was washed with brine, dried over sodium sulfate, filtered and concentrated. The crude alcoholic material (2.61 g) was dissolved in DCM (50mL). Cooled with ice bath, then acetyl chloride (1.39mL, 19.6 mmol) and pyridine (1.39mL, 19.6mmol) were added, and the reaction mixture was allowed to warm to ambient temperature then stirred at ambient temperature for 1 h. The reaction mixture was poured into ice water, extracted with chloroform for 2 times. The combined organic layer was washed with brine, dried over sodium sulfate, filtered and concentrated. A mixed solution of ethyl acetate and diisopropyl ether (1:5) was added to the crude material, then the precipitate was collected by filtration, washed with diisopropyl ether then dried to afford 2-bromo-6-(6-cyclopropyl-8-fluoro-1-oxo-3,4-dihydroisoquino lin-2(1*H*)-yl)benzyl acetate (2.20 g).

$^1$H NMR (400 MHz, $CDCl_3$) $\delta$ 7.60 (dd, J = 7.9, 1.4 Hz, 1H), 7.28 (t, J = 7.9 Hz, 1H), 7.23 (dd, J = 7. 9, 1.4 Hz, 1H), 6.75 - 6.67 (m, 2H), 5.28 - 5.12 (m, 2H), 3.97 - 3.87 (m, 1H), 3.78 - 3.68 (m, 1H), 3.31 - 3.19 (m, 1H), 3.02 - 2.93 (m, 1H), 2.08 - 2.02 (m, 3H), 1.94 - 1.86 (m, 1H), 1.12 - 1.04 (m, 2H), 0.81 - 0.73 (m, 2H); LCMS (m/z): 431.8 / 433.8 $[M+H]^+$.

Third Step

Under nitrogen atmosphere, 2-bromo-6-(6-cyclopropyl-8-fluoro-1-oxo-3,4-dihydroisoquino lin-2(1*H*)-yl)benzyl acetate (2.10 g, 4.86 mmol) was dissolved in dioxane (24.0 mL). To this solution, bis(pinacolato)diboron (2.47 g, 9.72 mmol), dichloro[1,1'-bis(diphenylphosphino)ferrocene]palladium(II) dichloromethaneadduct (397mg, 0.486mmol) and potassium acetate (1.43 g, 14.6 mmol) were added and stirred at 95 °C for 24 h. To complete the reaction, supplementary amount of bis(pinacolato)diboron (2.47 g, 9.72 mmol), dichloro[1,1'-bis(diphenylphosphino)ferrocene]palladium(II) dichloromethaneadduct (397mg, 0.486mmol) and potassium acetate (1.43g, 14.6 mmol) were added to the reaction mixture and stirred at 95 °C for further 24 h. Cooled to ambient temperature, water was added to the reaction mixture, and extracted with ethyl acetate. The combined organic layer was washed with saturated sodium hydrogen carbonate solution and brine, dried over sodium sulfate, filtered and concentrated. The crude material was purified by chromatography on silica gel, eluted with hexane/ethyl acetate to afford 2-(6-cyclopropyl-8-fluoro-1-oxo-3,4-dihydroisoquinolin-2(1*H*)-yl)-6-(4,4,5,5-tetramethyl-1,3,2-dioxaborolan-2-yl) benzyl acetate (2.02 g).

$^1$H NMR (400 MHz, $CDCl_3$) $\delta$ 7.81 (dd, J = 7.5, 1.5 Hz, 1H), 7.41 (t, J = 7.5 Hz, 1H), 7.32 (dd, J = 7.5, 1. 5 Hz, 1H), 6.75 - 6.65 (m, 2H), 5.51 (d, J = 11.6 Hz, 1H), 5.21 (d, J = 11.6 Hz, 1H), 3.93-3.85 (m, 1H), 3.78-3.69 (m, 1H), 3.28-3.19 (m, 1H), 3.03 - 2.91 (m, 1H), 1.99 (s, 3H), 1.91 -1.87 (m, 1H), 1.34 (s, 6H), 1.34 (s, 6H), 1.12 - 1.02 (m, 2H), 0.81 - 0.72 (m, 2H); LCMS (m/z): 480.2 $[M+H]^+$.

Fourth Step

To a stirred solution of 2-amino-4-chloro-6-[(4-morpholinophenyl)amino]-1,3,5-triazi ne (1.31 g, 4.26 mmol) which similarly prepared according to the procedure described in the Example 43, Fourth Step and 2-(6-cyclopropyl-8-fluoro-1-oxo-3,4-dihydroisoquinolin-2(1*H*)-yl)-6-(4,4,5,5-tetramethyl-1,3,2-dioxaborolan-2-yl) benzyl acetate (2.0 g, 4.26 mmol) in dimethoxyethane (15 mL), tetrakis(triphenylphosphine)palladium (0) (246 mg, 0.21 mmol) and potassium carbonate (1.18 g, 8.51 mmol) in water solution (7.5 mL) were added then stirred at 100 °C for 6 h. Water was added to the reaction mixture, then the precipitate was collected by filtration, washed with water. The crude material was purified by chromatography on silica gel, eluted with hexane/ethyl acetate to afford a mixture of 2-{4-amino-6-[(4-morpholinophenyl)amino]-1,3,5-triazin-2-yl }-6-(6-cyclopropyl-8-fluoro-1-oxo-3,4-dihydroisoquinolin-2( 1*H*)-yl) benzyl acetate and its de-acetylate (2.0g). To a solution of this mixed material (2.0 g) in methanol (15 mL), potassium carbonate (828 mg, 6 mmol) was added and stirred at 70 °C for 30 min. Water was added to the reaction mixture, and the precipitate was collected by filtration, washed with water and hexane then dried to afford the titled compound (1.59 g).

$^1$H NMR (400 MHz, DMSO-$d_6$) $\delta$ 9.49 (s, 1H), 7.75 (dd, J = 7.4, 1.8 Hz, 1H), 7.69 - 7.52 (m, 2H), 7.50 - 7.41 (m, 2H), 7.35-7.10 (m, 2H), 7.00 - 6.81 (m, 4H), 5.05 (s, 1H), 4.56 (dd, J = 11.8, 4. 5 Hz, 1H), 4.34 (dd, J = 11.8, 9.6 Hz, 1H), 3.92-3.83 (m, 1H), 3.82-3.73 (m, 1H), 3.66-3.60(m, 4H), 3.26-3.15 (m, 1H), 3.13 - 2.96 (m, 5H), 2.08-1.95 (m, 1H), 1.10 - 0.98 (m, 2H), 0.87 - 0.72 (m, 2H); LCMS (m/z): 582.3 $[M+H]^+$.

**Examples 6-32, 34-38, 40, 41, 45-282**

Each of the Example compounds shown in the following [Table 1-1] to [Table 1-27] were prepared according to the procedure described in the above Examples or modified procedure well known in the art of organic chemistry if needed, using appropriate starting materials (those materials are obtained from commercial sources, or are prepared by literature procedures or modifications of literature procedures known to persons skilled in the art).

The physicochemical data of each compound were shown in the following [Table 2-1] to [Table 2-15].

**Table 1-1**

| Ex. No. | Structure | Name |
|---|---|---|
| 6 | 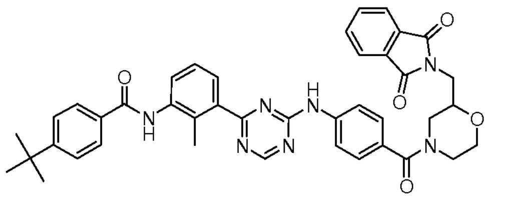 | 4-(*tert*-Butyl)-*N*-(3-{ 4-[(4-{2-[(1,3-dioxoi soindolin-2-yl)methyl ]morpholine-4-carbony l}phenyl)amino]-1,3,5 -triazin-2-yl}-2-meth ylphenyl)benzamide |
| 7 | 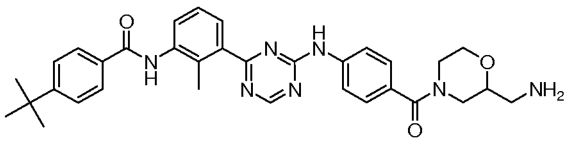 | *N*-{3-[4-({4-[2-(Amino methyl)morpholine-4-c arbonyl]phenyl}amino) -1,3,5-triazin-2-yl]-2-methlphenyl}-4-(*ter t*-butyl)benzamide |
| 8 | 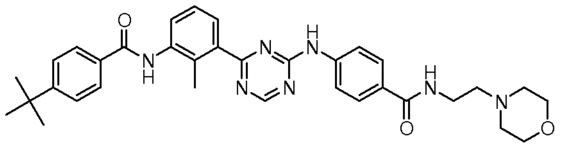 | 4-(*tert*-Butyl)-N-{2-m ethyl-3-[4-({4-[(2-mo rpholinoethyl)carbamo yl]phenyl}amino)-1,3, 5-triazin-2-yl]phenyl }benzamide |
| 9 | 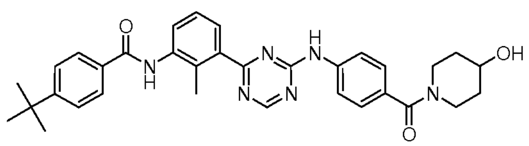 | 4-(*tert*-Butyl)-*N*-[3-( 4-{[4-(4-hydroxypiper idine-1-carbonyl)phen yl]amino}-1,3,5-triaz in-2-yl)-2-methylphen yl]benzamide |
| 10 | 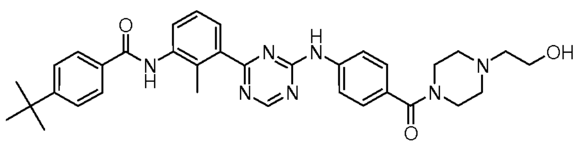 | 4-(*tert*-Butyl)-*N*-{3-[ 4-({4-[4-(2-hydroxyet hyl)piperazine-1-carb onyl]phenyl}amino)-1, 3,5-triazin-2-yl]-2-m ethylphenyl}benzamide |
| 11 | 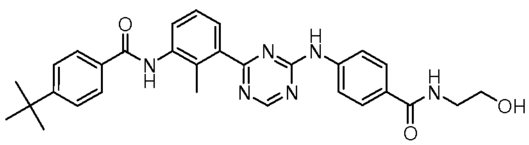 | 4-(*tert*-Butyl)-*N*-{3-[ 4-({4-[(2-hydroxyethy l)carbamoyl]phenyl}am ino)-1,3,5-triazin-2-yl]-2-methylphenyl}be nzamide |
| 12 | 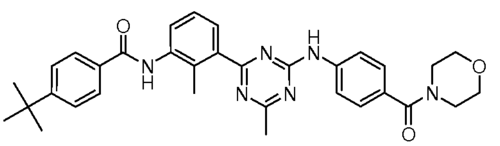 | 4-(*tert*-Butyl)-*N*-[2-m ethyl-3-(4-methyl-6-{ [4-(morpholine-4-carb onyl)phenyl]amino}-1, 3,5-triazin-2-yl)phen yl]benzamide |
| 13 | 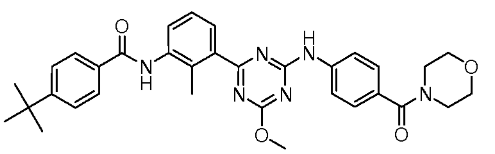 | 4-(*tert*-Butyl)-*N*-[3-( 4-methoxy-6-{[4-(morp holine-4-carbonyl)phe nyl]aminol-1,3,5-tria zin-2-yl)-2-methylphe nyl]benzamide |
| 14 | 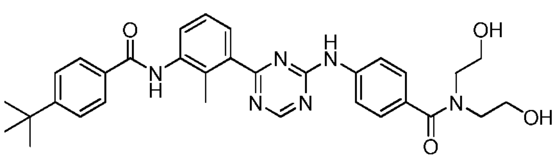 | 4-[(4-{3-[4-(tert-But yl)benzamido]-2-methy lphenyl}-1,3,5-triazi n-2-yl)amino]-N,N-bis (2-hydroxyethyl)benza mide |

(continued)

| Ex. No. | Structure | Name |
|---|---|---|
| 15 | 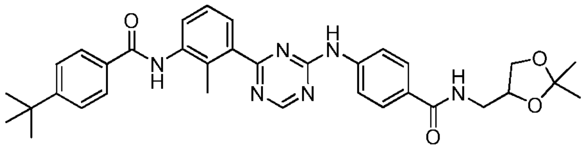 | 4-(*tert*-Butyl)-*N*-(3-{ 4-[(4-{[(2,2-dimethyl -1,3-dioxolan-4-yl)me thyl]carbamoyl}phenyl )amino]-1,3,5-triazin -2-yl}-2-methylphenyl )benzamide |
| 16 | 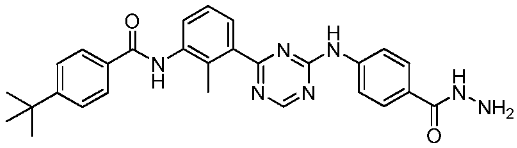 | 4-(*tert*-Butyl)-*N*-[3-( 4-{[4-(hydrazinecarbo nyl)phenyl]amino}-1,3 ,5-triazin-2-yl)-2-me thylphenyl]benzamide |
| 17 | 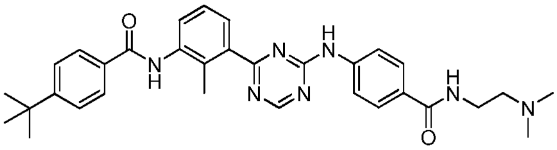 | 4-(*tert*-Butyl)-*N*-(3-{ 4-[(4-{[2-(dimethylam ino)ethyl]carbamoyl}p henyl)amino]-1,3,5-tr iazin-2-yl}-2-methylp henyl)benzamide |
| 18 | 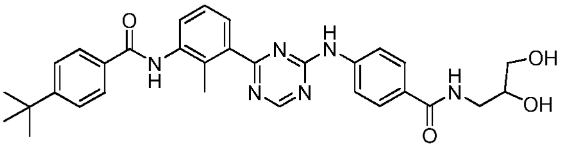 | 4-(*tert*-Butyl)-*N*-{3-[ 4-({4-[(2,3-dihydroxy propyl)carbamoyl]phen yl}amino)-1,3,5-triaz in-2-yl]-2-methylphen yl}benzamide |

**Table 1-2**

| | | |
|---|---|---|
| 19 | 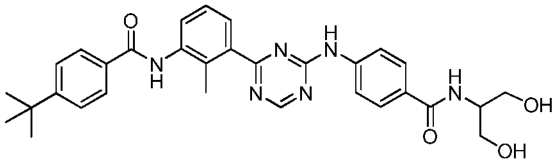 | 4-(*tert*-Butyl)-*N*-{3-[ 4-({4-[(1,3-dihydroxy propan-2-yl)carbamoyl ]phenyl}amino)-1,3,5-triazin-2-yl]-2-methy lphenyl}benzamide |
| 20 | 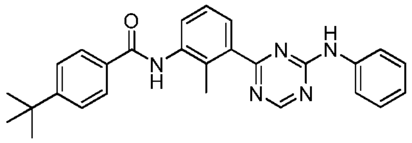 | 4-(*tert*-Butyl)-*N*-{2-m ethyl-3-[4-(phenylami no)-1,3,5-triazin-2-y l]phenyl}benzamide |
| 21 | 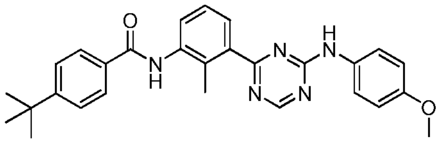 | 4-(*tert*-Butyl)-*N*-(3-{ 4-[(4-methoxyphenyl)a mino]-1,3,5-triazin-2 -yl}-2-methylphenyl)b enzamide |
| 22 | 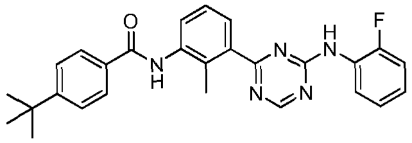 | 4-(*tert*-Butyl)-*N*-(3-{ 4-[(2-fluorophenyl)am ino]-1,3,5-triazin-2-yl}-2-methylphenyl)be nzamide |
| 23 | 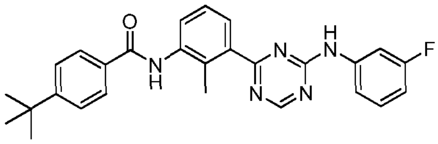 | 4-(*tert*-Butyl)-*N*-(3-{ 4-[(3-fluorophenyl)am ino]-1,3,5-triazin-2-yl}-2-methylphenyl)be nzamide |

(continued)

| | | |
|---|---|---|
| 24 | 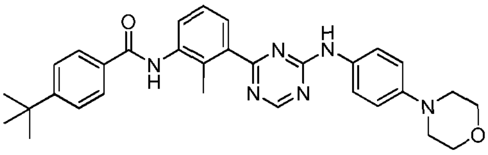 | 4-(*tert*-Butyl)-*N*-(2-m ethyl-3-{4-[(4-morpho linophenyl)amino]-1,3 ,5-triazin-2-yl}pheny l)benzamide |
| 25 | 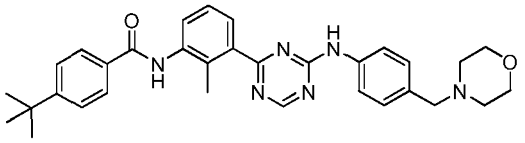 | 4-(*tert*-Butyl)-*N*-[2-m ethyl-3-(4-{[4-(morph olinomethyl)phenyl]am ino}-1,3,5-triazin-2-yl)phenyl]benzamide |
| 26 | 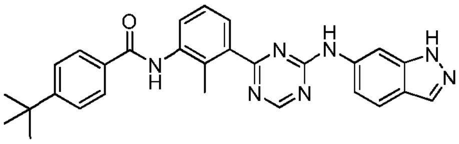 | *N*-(3-{4-[(1*H*-Indazol-6-yl)amino]-1,3,5-tri azin-2-yl}-2-methylph enyl)-4-(*tert*-butyl)b enzamide |
| 27 | 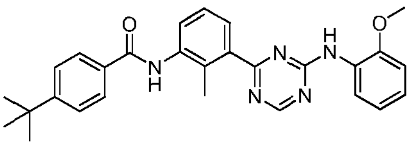 | 4-(*tert*-Butyl)-*N*-(3-{ 4-[(2-methoxyphenyl)a mino]-1,3,5-triazin-2 -yl}-2-methylphenyl)b enzamide |
| 28 | 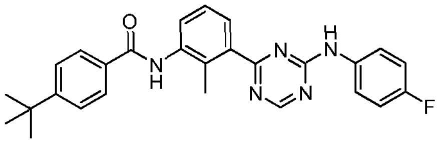 | 4-(*tert*-Butyl)-*N*-(3-{ 4-[(4-fluorophenyl)am ino]-1,3,5-triazin-2-yl}-2-methylphenyl)be nzamide |
| 29 | 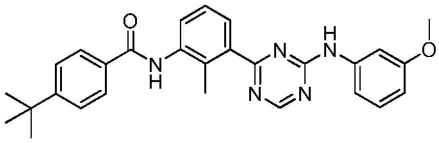 | 4-(*tert*-Butyl)-*N*-(3-{ 4-[(3-methoxyphenyl)a mino]-1,3,5-triazin-2 -yl}-2-methylphenyl)b enzamide |
| 30 | 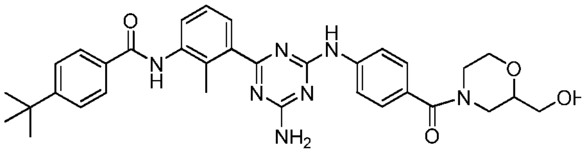 | *N*-{3-[4-Amino-6-({4-[ 2-(hydroxymethyl)morp holine-4-carbonyl]phe nyl}amino)-1,3,5-tria zin-2-yl]-2-methylphe nyl}-4-(tert-butyl)be nzamide |
| 31 | 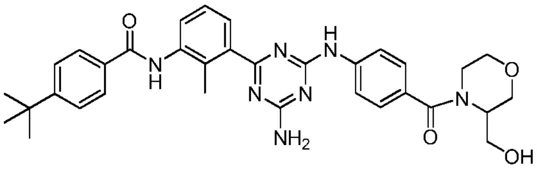 | *N*-{3-[4-Amino-6-({4-[ 3-(hydroxymethyl)morp holine-4-carbonyl]phe nyl}amino)-1,3,5-tria zin-2-yl]-2-methylphe nyl}-4-(tert-butyl)be nzamide |

**Table 1-3**

| | | |
|---|---|---|
| 32 | 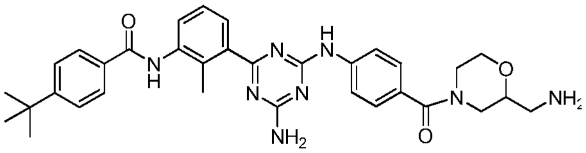 | *N*-{3-[4-Amino-6-({4-[ 2-(aminomethyl)morpho line-4-carbonyl]pheny l}amino)-1,3,5-triazi n-2-yl]-2-methylpheny l}-4-(*tert*-butyl)ben amide |

(continued)

| | | |
|---|---|---|
| 34 | 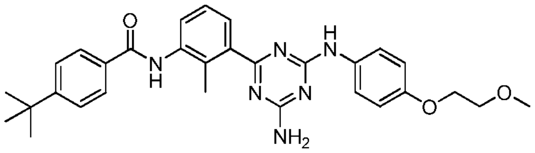 | *N*-[3-(4-Amino-6-{[4-( 2-methoxyethoxy)pheny l]amino}-1,3,5-triazi n-2-yl)-2-methylpheny l]-4-(*tert*-butyl)benz amide |
| 35 | 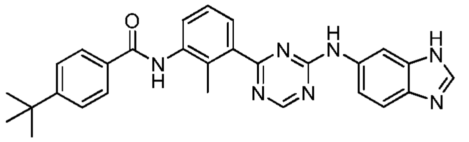 | *N*-(3-{4-[(1*H*-Benzo[*d*] imidazol-6-yl)amino]-1,3,5-triazin-2-yl}-2 -methylphenyl)-4-(*ter t*-butyl)benzamide |
| 36 | 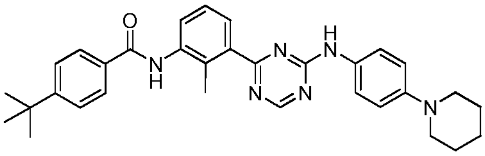 | 4-(*tert*-Butyl)-*N*-[2-m ethyl-3-(4-{[4-(piper idin-1-yl)phenyl]amin o}-1,3,5-triazin-2-yl )phenyl]benzamide |
| 37 | 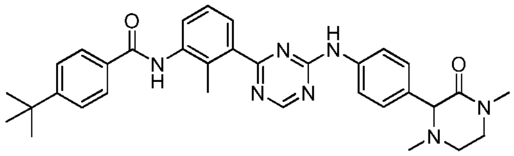 | 4-(*tert*-Butyl)-*N*-[3-( 4-{[4-(1,4-dimethyl-3 -oxopiperazin-2-yl)ph enyl]aminol-1,3,5-tri azin-2-yl)-2-methylph enyl]benzamide |
| 38 | 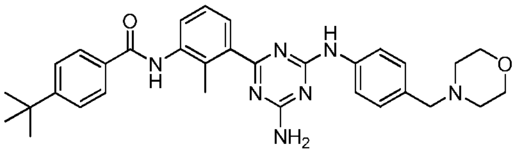 | *N*-[3-(4-Amino-6-{[4-( morpholinomethyl)phen yl]amino}-1,3,5-triaz in-2-yl)-2-methylphen yl]-4-(tert-butyl)ben zamide |
| 40 | 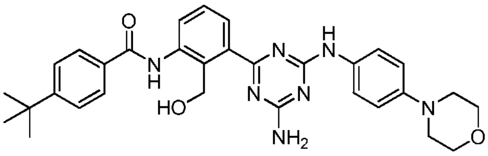 | *N*-(3-{4-Amino-6-[(4-m orpholinophenyl)amino ]-1,3,5-triazin-2-yl} -2-(hydroxymethyl)phe nyl)-4-(tert-butyl)be nzamide |
| 41 | 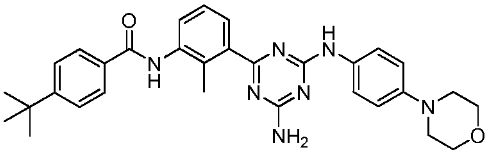 | *N*-(3-{4-Amino-6-[(4-m orpholinophenyl)amino ]-1,3,5-triazin-2-yl} -2-methylphenyl)-4-(*t ert*-butyl)benzamide |
| 45 | 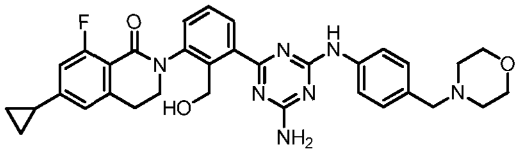 | 2-[3-(4-Amino-6-{[4-( morpholinomethyl)phen yl]amino}-1,3,5-triaz in-2-yl)-2-(hydroxyme thyl)phenyl]-6-cyclop ropyl-8-fluoro-3,4-di hydroisoquinolin-1(2*H* )-one |
| 46 | 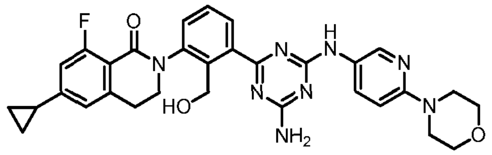 | 2-(3-{4-Amino-6-[(6-m orpholinopyridin-3-yl )amino]-1,3,5-triazin -2-yl}-2-(hydroxymeth yl)phenyl)-6-cyclopro pyl-8-fluoro-3,4-dihy droisoquinolin-1(2H)-one |
| 47 | 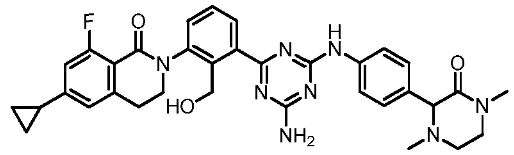 | 2-[3-(4-Amino-6-{[4-( 1,4-dimethyl-3-oxopip erazin-2-yl)phenyl]am ino}-1,3,5-triazin-2-yl)-2-(hydroxymethyl) phenyl]-6-cyclopropyl -8-fluoro-3,4-dihydro isoquinolin-1(2H)-one |

(continued)

| | | |
|---|---|---|
| 48 | 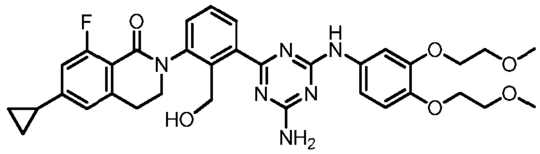 | 2-[3-(4-Amino-6-{[3,4 -bis(2-methoxyethoxy) phenyl]amino}-1,3,5-t riazin-2-yl)-2-(hydro xymethyl)phenyl]-6-cy clopropyl-8-fluoro-3, 4-dihydroisoquinolin-1(2*H*)-one |

**Table 1-4**

| | | |
|---|---|---|
| 49 | 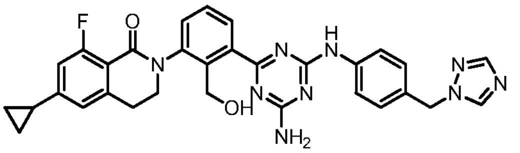 | 2-{3-[4-({4-[(1*H*-1,2, 4-Triazol-1-yl)methyl ]phenyl}amino)-6-amin o-1,3,5-triazin-2-yl] -2-(hydroxymethyl)phe nyl}-6-cyclopropyl-8-fluoro-3,4-dihydroiso quinolin-1(2H)-one |
| 50 | 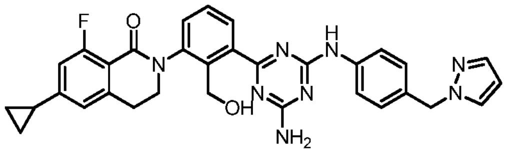 | 2-{3-[4-({4-[(1*H*-Pyra zol-1-yl)methyl]pheny l}amino)-6-amino-1,3 5-triazin-2-yl]-2-(hy droxymethyl)phenyl}-6 -cyclopropyl-8-fluoro -3,4-dihydroisoquinol in-1(2*H*)-one |
| 51 | 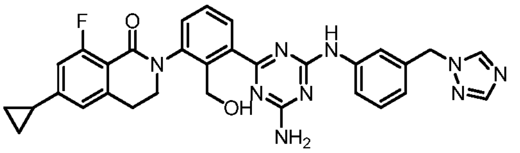 | 2-{3-[4-({3-[(1*H*-1,2, 4-Triazol-1-yl)methyl ]phenyl}amino)-6-amin o-1,3,5-triazin-2-yl] -2-(hydroxymethyl)phe nyl}-6-cyclopropyl-8-fluoro-3,4-dihydroiso quinolin-1(2H)-one |
| 52 | 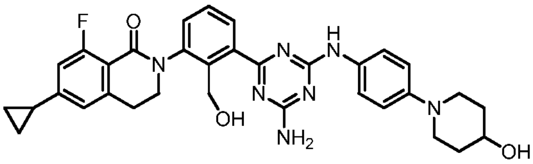 | 2-[3-(4-Amino-6-{[4-( 4-hydroxypiperidin-1-yl)phenyl]amino}-1,3, 5-triazin-2-yl)-2-(hy droxymethyl)phenyl]-6 -cyclopropyl-8-fluoro -3,4-dihydroisoquinol in-1(2*H*)-one |
| 53 | 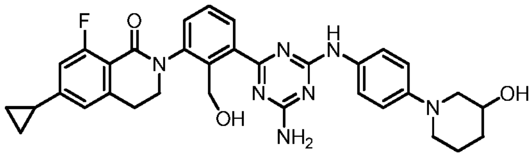 | 2-[3-(4-Amino-6-{[4-( 3-hydroxypiperidin-1-yl)phenyl]amino}-1,3, 5-triazin-2-yl)-2-(hy droxymethyl)phenyl]-6 -cyclopropyl-8-fluoro -3,4-dihydroisoquinol in-1(2*H*)-one |
| 54 | 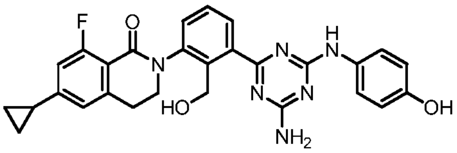 | 2-(3-{4-Amino-6-[(4-h ydroxyphenyl)amino]-1 ,3,5-triazin-2-yl}-2-(hydroxymethyl)phenyl )-6-cyclopropyl-8-flu oro-3,4-dihydroisoqui nolin-1(2H)-one |
| 55 | 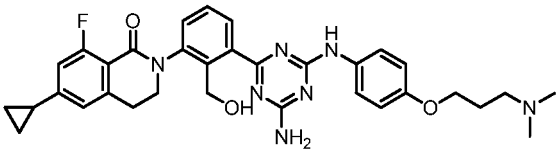 | 2-{3-[4-Amino-6-({4-[ 3-(dimethylamino)prop oxy]phenyl}amino)-1,3 ,5-triazin-2-yl]-2-(h ydroxymethyl)phenyl}-6-cyclopropyl-8-fluor o-3,4-dihydroisoquino lin-1(2H)-one |
| 56 | 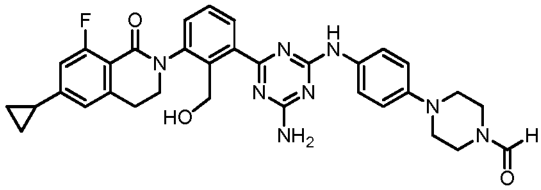 | 4-[4-({4-Amino-6-[3-( 6-cyclopropyl-8-fluor o-1-oxo-3,4-dihydrois oquinolin-2(1*H*)-yl)-2 -(hydroxymethyl)pheny l]-1,3,5-triazin-2-yl }amino)phenyl]piperaz ine-1-carbaldehyde |

(continued)

| | | |
|---|---|---|
| 57 | 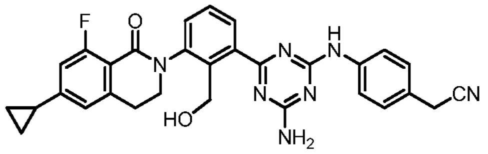 | 2-[4-({4-Amino-6-[3-( 6-cyclopropyl-8-fluor o-1-oxo-3,4-dihydrois oquinolin-2(1H)-yl)-2 -(hydroxymethyl)pheny l]-1,3,5-triazin-2-yl }amino)phenyl]acetoni trile |
| 58 | 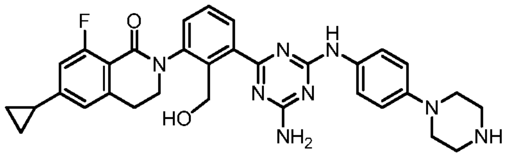 | 2-[3-(4-Amino-6-{[4-( piperazin-1-yl)phenyl ]amino}-1,3,5-triazin -2-yl)-2-(hydroxymeth yl)phenyl]-6-cyclopro pyl-8-fluoro-3,4-dihy droisoquinolin-1(2H)-one |

**Table 1-5**

| | | |
|---|---|---|
| 59 | 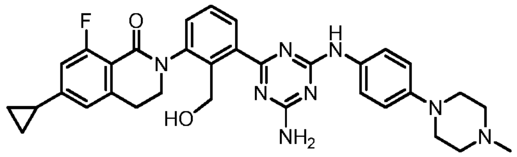 | 2-[3-(4-Amino-6-{[4-( 4-methylpiperazin-1-y l)phenyl]amino}-1,3,5 -triazin-2-yl)-2-(hyd roxymethyl)phenyl]-6-cyclopropyl-8-fluoro-3,4-dihydroisoquinoli n-1(2*H*)-one |
| 60 | 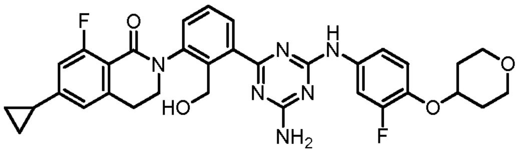 | 2-{3-[4-Amino-6-({3-f luoro-4-[(tetrahydro-2*H*-pyran-4-yl)oxy]phe nyl}amino)-1,3,5-tria zin-2-yl]-2-(hydroxym ethyl)phenyl}-6-cyclo propyl-8-fluoro-3,4-d ihydroisoquinolin-1(2 *H*)-one |
| 61 | 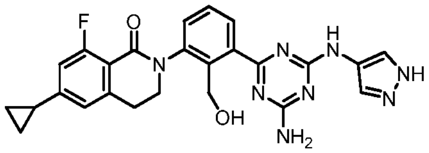 | 2-(3-{4-[(1*H*-Pyrazol-4-yl)amino]-6-amino-1 ,3,5-triazin-2-yl}-2-(hydroxymethyl)phenyl )-6-cyclopropyl-8-flu oro-3,4-dihydroisoqui nolin-1(2H)-one |
| 62 | 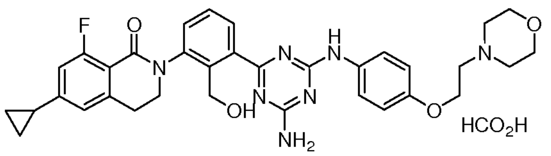 | 2-[3-(4-Amino-6-{[4-( 2-morpholinoethoxy)ph enyl]aminol-1,3,5-tri azin-2-yl)-2-(hydroxy methyl)phenyl]-6-cycl opropyl-8-fluoro-3,4-dihydroisoquinolin-1( 2*H*)-one formate |
| 63 | 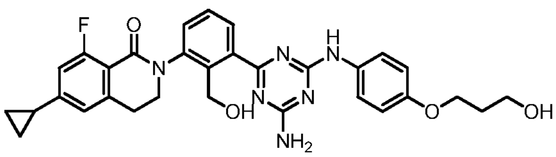 | 2-[3-(4-Amino-6-{[4-( 3-hydroxypropoxy)phen yl]amino}-1,3,5-triaz in-2-yl)-2-(hydroxyme thyl)phenyl]-6-cyclop ropyl-8-fluoro-3,4-di hydroisoquinolin-1(2*H* )-one |
| 64 | 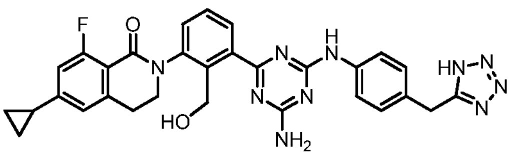 | 2-{3-[4-({4-[(1*H*-Tetr azol-5-yl)methyl]phen yl}amino)-6-amino-1,3 ,5-triazin-2-yl]-2-(h ydroxymethyl)phenyl}-6-cyclopropyl-8-fluor o-3,4-dihydroisoquino lin-1(2H)-one |
| 65 | 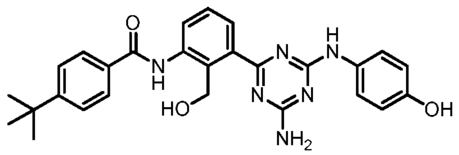 | *N*-(3-{4-Amino-6-[(4-h ydroxyphenyl)amino]-1 ,3,5-triazin-2-yl}-2-(hydroxymethyl)phenyl )-4-(*tert*-butyl)benza mide |

(continued)

| | | |
|---|---|---|
| 66 | 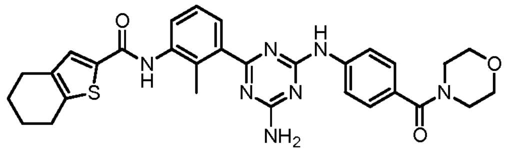 | *N*-[3-(4-Amino-6-{[4-( morpholine-4-carbonyl )phenyl]amino}-1,3,5-triazin-2-yl)-2-methy lphenyl]-4,5,6,7-tetr ahydrobenzo[*b*]thiophe ne-2-carboxamide |
| 67 | 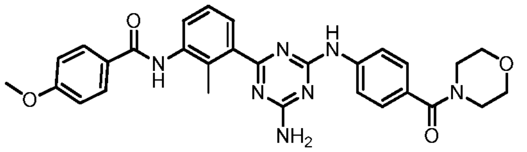 | *N*-[3-(4-Amino-6-{[4-( morpholine-4-carbonyl )phenyl]amino}-1,3,5-triazin-2-yl)-2-methy lphenyl]-4-methoxyben zamide |
| 68 | 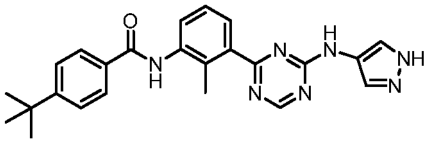 | *N*-(3-{4-[(1*H*-Pyrazol-4-yl)amino]-1,3,5-tri azin-2-yl}-2-methylph enyl)-4-(*tert*-butyl)b enzamide |

**Table 1-6**

| | | |
|---|---|---|
| 69 | 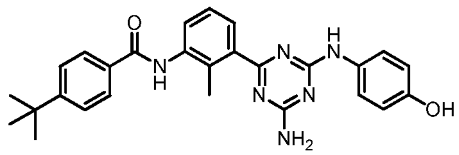 | *N*-(3-{4-Amino-6-[(4-h ydroxyphenyl)amino]-1 ,3,5-triazin-2-yl}-2-methylphenyl)-4-(tert -butyl)benzamide |
| 70 | 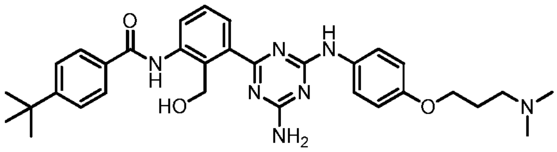 | *N*-{3-[4-Amino-6-({4-[ 3-(dimethylamino)prop oxy]phenyl}amino)-1,3 ,5-triazin-2-yl]-2-(h ydroxymethyl)phenyl}-4-(tert-butyl)benzami de |
| 71 | 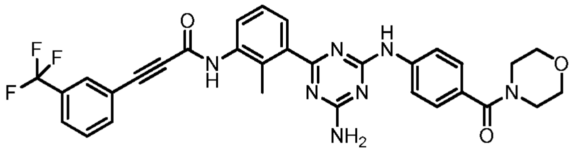 | *N*-[3-(4-Amino-6-{[4-( morpholine-4-carbonyl )phenyl]amino}-1,3,5-triazin-2-yl)-2-methy lphenyl]-3-[3-(triflu oromethyl)phenyl]prop -2-ynamide |
| 72 | 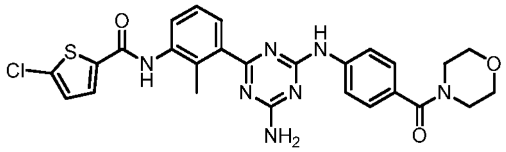 | *N*-[3-(4-Amino-6-{[4-( morpholine-4-carbonyl )phenyl]amino}-1,3,5-triazin-2-yl)-2-methy lphenyl]-5-chlorothio phene-2-carboxamide |
| 73 | 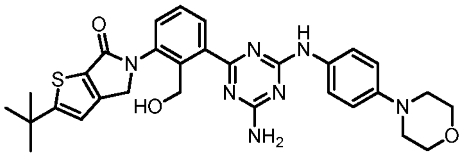 | 5-(3-{4-Amino-6-[(4-m orpholinophenyl)amino ]-1,3,5-triazin-2-yl} -2-(hydroxymethyl)phe nyl)-2-(tert-butyl)-4 *H*-thieno[2,3-c]pyrrol -6(5*H*)-one |
| 74 | 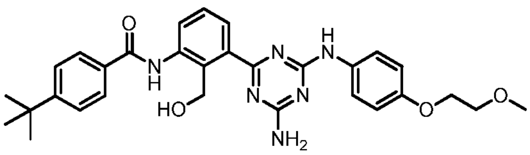 | *N*-[3-(4-Amino-6-{[4-( 2-methoxyethoxy)pheny l]amino}-1,3,5-triazi n-2-yl)-2-(hydroxymet hyl)phenyl]-4-(*tert*-b utyl)benzamide |

(continued)

| | | |
|---|---|---|
| 75 | 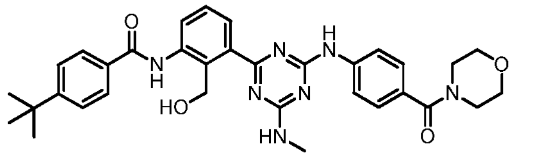 | 4-(*tert*-Butyl)-*N*-[2-( hydroxymethyl)-3-(4-( methylamino)-6-{[4-(m orpholine-4-carbonyl) phenyl]amino}-1,3,5-t riazin-2-yl)phenyl]be nzamide |
| 76 | 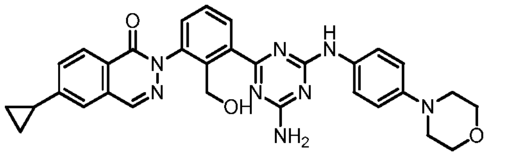 | 2-(3-{4-Amino-6-[(4-m orpholinophenyl)amino ]-1,3,5-triazin-2-yl} -2-(hydroxymethyl)phe nyl)-6-cyclopropylpht halazin-1(2H)-one |
| 77 | 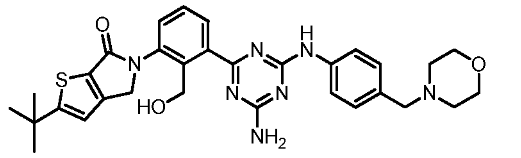 | 5-[3-(4-Amino-6-{[4-( morpholinomethyl)phen yl]amino}-1,3,5-triaz in-2-yl)-2-(hydroxyme thyl)phenyl]-2-(*tert*-butyl)-4H-thieno[2,3-c]pyrrol-6(5H)-one |
| 78 | 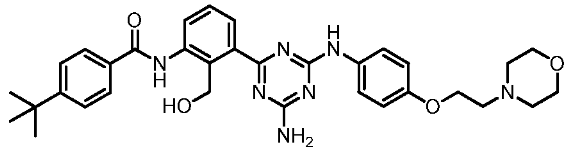 | *N*-[3-(4-Amino-6-{[4-( 2-morpholinoethoxy)ph enyl]aminol-1,3,5-tri azin-2-yl)-2-(hydroxy methyl)phenyl]-4-(*ter t*-butyl)benzamide |

**Table 1-7**

| | | |
|---|---|---|
| 79 | 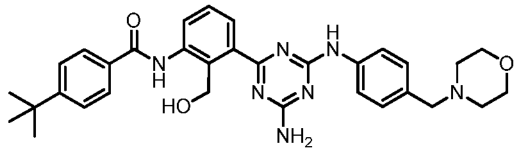 | *N*-[3-(4-Amino-6-{[4-( morpholinomethyl)phen yl]amino}-1,3,5-triaz in-2-yl)-2-(hydroxyme thyl)phenyl]-4-(*tert*-butyl)benzamide |
| 80 | 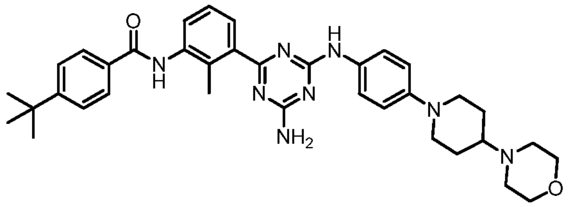 | *N*-[3-(4-Amino-6-{[4-( 4-morpholinopiperidin -1-yl)phenyl]amino}-1 ,3,5-triazin-2-yl)-2-methylphenyl]-4-(*tert* -butyl)benzamide |
| 81 | 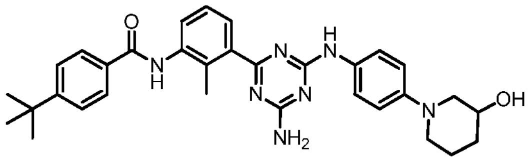 | *N*-[3-(4-Amino-6-{[4-( 3-hydroxypiperidin-1-yl)phenyl]amino}-1,3, 5-triazin-2-yl)-2-met hylphenyl]-4-(*tert*-bu tyl)benzamide |
| 82 | 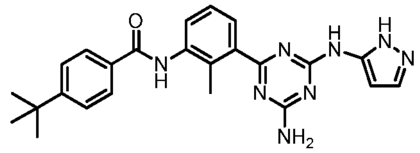 | *N*-(3-{4-[(1*H*-Pyrazol-5-yl)amino]-6-amino-1 ,3,5-triazin-2-yl}-2-methylphenyl)-4-(tert -butyl)benzamide |
| 83 | 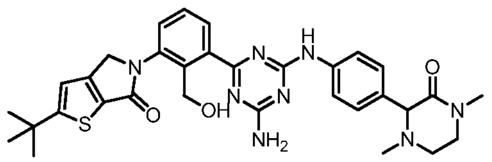 | 5-[3-(4-Amino-6-{[4-( 1,4-dimethyl-3-oxopip erazin-2-yl)phenyl]am ino}-1,3,5-triazin-2-yl)-2-(hydroxymethyl) phenyl]-2-(*tert*-butyl)-4*H*-thieno[2,3-*c*]pyr rol-6(5H)-one |

(continued)

| | | |
|---|---|---|
| 84 | 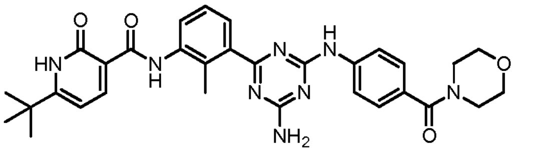 | *N*-[3-(4-Amino-6-{[4-( morpholine-4-carbonyl )phenyl]amino}-1,3,5-triazin-2-yl)-2-methy lphenyl]-6-(*tert*-buty l)-2-oxo-1,2-dihydrop yridine-3-carboxamide |
| 85 | 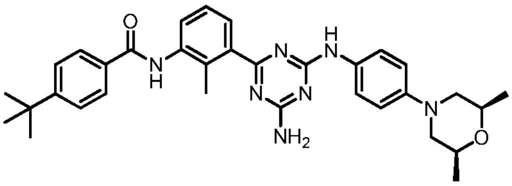 | *N*-[3-(4-Amino-6-{[4-( (2S,6R)-2,6-dimethylm orpholino)phenyl]amin o}-1,3,5-triazin-2-yl )-2-methylphenyl]-4-( *tert*-butyl)benzamide |
| 86 | 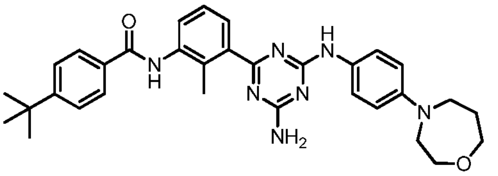 | *N*-[3-(4-{[4-(1,4-Oxaz epan-4-yl)phenyl]amin o}-6-amino-1,3,5-tria zin-2-yl)-2-methylphe nyl]-4-(*tert*-butyl)be nzamide |
| 87 | 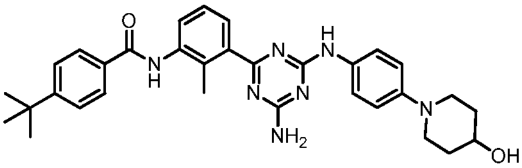 | *N*-[3-(4-Amino-6-{[4-( 4-hydroxypiperidin-1-yl)phenyl]amino}-1,3, 5-triazin-2-yl)-2-met hylphenyl]-4-(*tert*-bu tyl)benzamide |
| 88 | 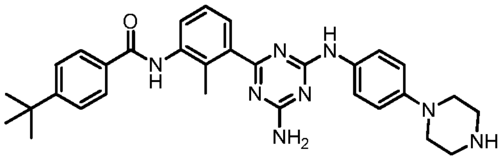 | *N*-[3-(4-Amino-6-{[4-( piperazin-1-yl)phenyl ]amino}-1,3,5-triazin -2-yl)-2-methylphenyl ]-4-(*tert*-butyl)benza mide |

**Table 1-8**

| | | |
|---|---|---|
| 89 | 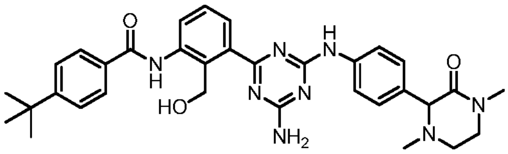 | *N*-[3-(4-Amino-6-{[4-( 1,4-dimethyl-3-oxopip erazin-2-yl)phenyl]am ino}-1,3,5-triazin-2-yl)-2-(hydroxymethyl) phenyl]-4-(*tert*-butyl )benzamide |
| 90 | 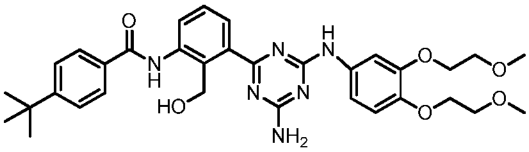 | *N*-[3-(4-Amino-6-{[3,4 -bis(2-methoxyethoxy) phenyl]amino}-1,3,5-t riazin-2-yl)-2-(hydro xymethyl)phenyl]-4-(*t ert*-butyl)benzamide |
| 91 | 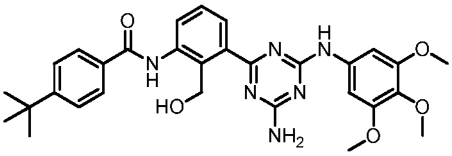 | *N*-(3-{4-Amino-6-[(3,4 ,5-trimethoxyphenyl)a mino]-1,3,5-triazin-2 -yl}-2-(hydroxymethyl )phenyl)-4-(*tert*-buty l)benzamide |
| 92 | 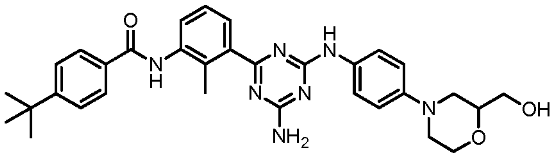 | *N*-{3-[4-Amino-6-({4-[ 2-(hydroxymethyl)morp holino]phenyl}amino)-1,3,5-triazin-2-yl]-2 -methylphenyl}-4-(*ter t*-butyl)benzamide |

(continued)

| | | |
|---|---|---|
| 93 | 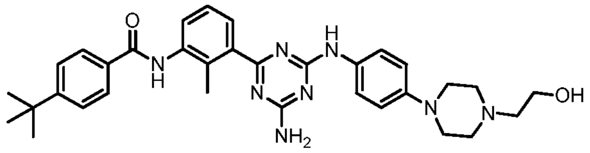 | *N*-{3-[4-Amino-6-({4-[ 4-(2-hydroxyethyl)pip erazin-1-yl]phenyl}am ino)-1,3,5-triazin-2-yl]-2-methylphenyl}-4 -(*tert*-butyl)benzamid e |
| 94 | 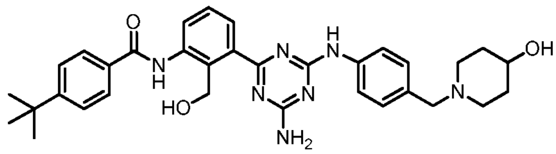 | *N*-{3-[4-Amino-6-({4-[ (4-hydroxypiperidin-1 -yl)methyl]phenyl}ami no)-1,3,5-triazin-2-y l]-2-(hydroxymethyl)p henyl}-4-(*tert*-butyl) benzamide |
| 95 | 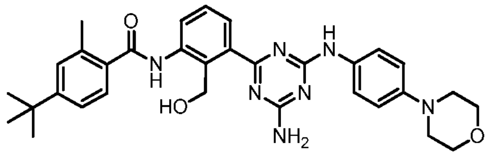 | *N*-(3-{4-Amino-6-[(4-m orpholinophenyl)amino ]-1,3,5-triazin-2-yl} -2-(hydroxymethyl)phe nyl)-4-(*tert*-butyl)-2 -methylbenzamide |
| 96 | 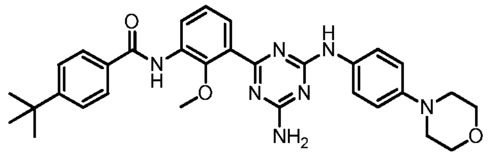 | *N*-(3-{4-Amino-6-[(4-m orpholinophenyl)amino ]-1,3,5-triazin-2-yl} -2-methoxyphenyl)-4-( *tert*-butyl)benzamide |
| 97 | 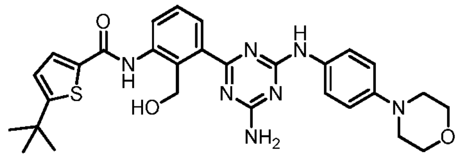 | *N*-(3-{4-Amino-6-[(4-m orpholinophenyl)amino ]-1,3,5-triazin-2-yl} -2-(hydroxymethyl)phe nyl)-5-(*tert*-butyl)th iophene-2-carboxamide |
| 98 | 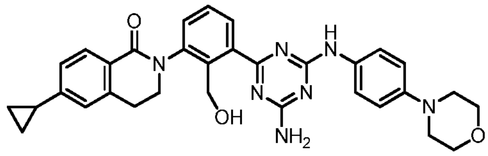 | 2-(3-{4-Amino-6-[(4-m orpholinophenyl)amino ]-1,3,5-triazin-2-yl} -2-(hydroxymethyl)phe nyl)-6-cyclopropyl-3, 4-dihydroisoquinolin-1(2*H*)-one |

**Table 1-9**

| | | |
|---|---|---|
| 99 | 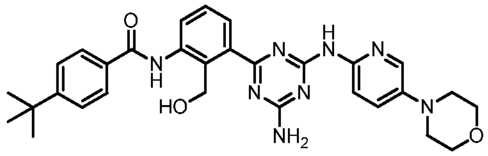 | *N*-(3-{4-Amino-6-[(5-m orpholinopyridin-2-yl )amino]-1,3,5-triazin -2-yl}-2-(hydroxymeth yl)phenyl)-4-(*tert*-bu tyl)benzamide |
| 100 | 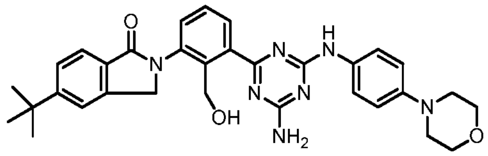 | 2-(3-{4-Amino-6-[(4-m orpholinophenyl)amino ]-1,3,5-triazin-2-yl} -2-(hydroxymethyl)phe nyl)-5-(*tert*-butyl)is oindolin-1-one |
| 101 | 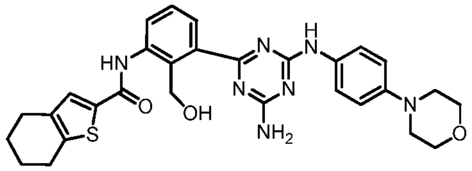 | *N*-(3-{4-Amino-6-[(4-m orpholinophenyl)amino ]-1,3,5-triazin-2-yl} -2-(hydroxymethyl)phe nyl)-4,5,6,7-tetrahyd robenzo[*b*]thiophene-2 -carboxamide |

(continued)

| | | |
|---|---|---|
| 102 | 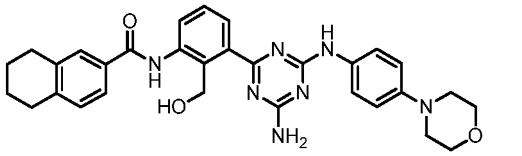 | *N*-(3-{4-Amino-6-[(4-m orpholinophenyl)amino ]-1,3,5-triazin-2-yl} -2-(hydroxymethyl)phe nyl)-5,6,7,8-tetrahyd ronaphthalene-2-carbo xamide |
| 103 | 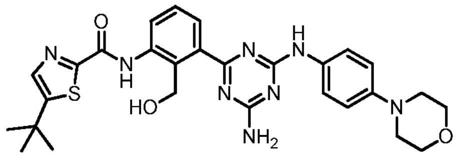 | *N*-(3-{4-Amino-6-[(4-m orpholinophenyl)amino ]-1,3,5-triazin-2-yl} -2-(hydroxymethyl)phe nyl)-5-(*tert*-butyl)th iazole-2-carboxamide |
| 104 | 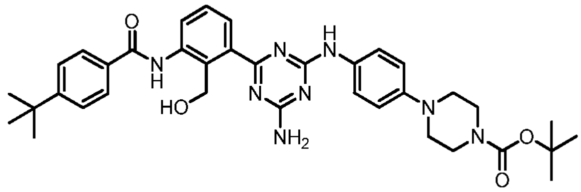 | *tert*-Butyl 4-{4-[(4-amino-6-{3-[ 4-(*tert*-butyl)benzami do]-2-(hydroxymethyl) phenyl}-1,3,5-triazin -2-yl)amino]phenyl}pi perazine-1-carboxylat e |
| 105 | 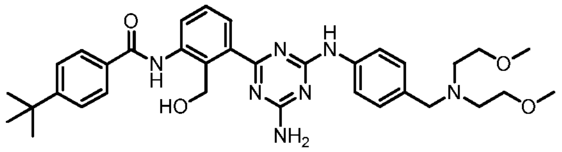 | *N*-(3-{4-Amino-6-[(4-{ [bis(2-methoxyethyl)a mino]methyl}phenyl)am ino)-1,3,5-triazin-2-yl]-2-(hydroxymethyl) phenyl}-4-(*tert*-butyl)benzamide |
| 106 | 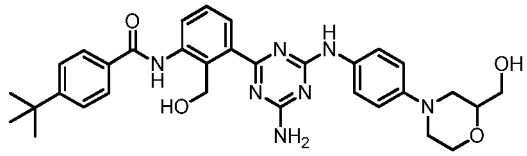 | *N*-{3-[4-Amino-6-({4-[ 2-(hydroxymethyl)morp holino]phenyl}amino)-1,3,5-triazin-2-yl]-2 -(hydroxymethyl)pheny l}-4-(*tert*-butyl)ben amide |
| 107 | 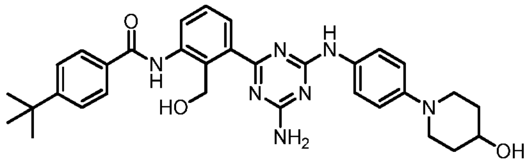 | *N*-[3-(4-Amino-6-{[4-( 4-hydroxypiperidin-1-yl)phenyl]amino}-1,3, 5-triazin-2-yl)-2-(hy droxymethyl)phenyl]-4 -(*tert*-butyl)benzamid e |
| 108 | 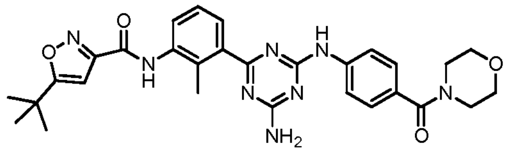 | *N*-[3-(4-Amino-6-{[4-( morpholine-4-carbonyl )phenyl]amino}-1,3,5-triazin-2-yl)-2-methy lphenyl]-5-(*tert*-buty l)isoxazole-3-carboxa mide |

**Table 1-10**

| | | |
|---|---|---|
| 109 | 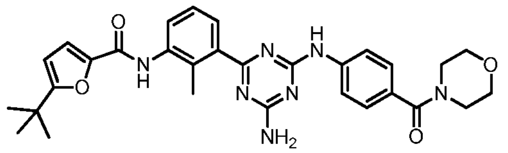 | *N*-[3-(4-Amino-6-{[4-( morpholine-4-carbonyl )phenyl]amino}-1,3,5-triazin-2-yl)-2-methy lphenyl]-5-(*tert*-buty l)furan-2-carboxamide |
| 110 | 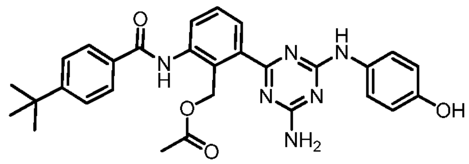 | 2-{4-Amino-6-[(4-hydr oxyphenyl)amino]-1,3, 5-triazin-2-yl}-6-[4-(*tert*-butyl)benzamido ]benzyl acetate |

(continued)

| | | |
|---|---|---|
| 111 | 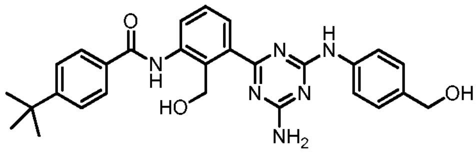 | *N*-[3-(4-Amino-6-{[4-( hydroxymethyl)phenyl] amino}-1,3,5-triazin-2-yl)-2-(hydroxymethy l)phenyl]-4-(*tert*-but yl)benzamide |
| 112 | 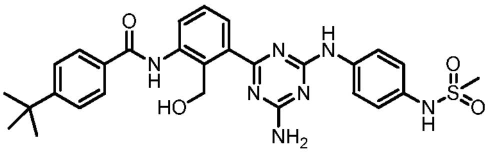 | *N*-[3-(4-Amino-6-{[4-( methylsulfonamido)phe nyl]aminol-1,3,5-tria zin-2-yl)-2-(hydroxym ethyl)phenyl]-4-(*tert* -butyl)benzamide |
| 113 | 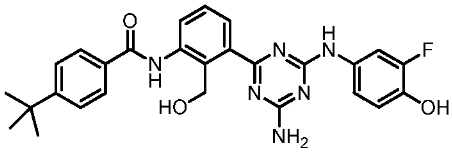 | *N*-(3-{4-Amino-6-[(3-f luoro-4-hydroxyphenyl )amino]-1,3,5-triazin -2-yl}-2-(hydroxymeth yl)phenyl)-4-(*tert*-bu tyl)benzamide |
| 114 | 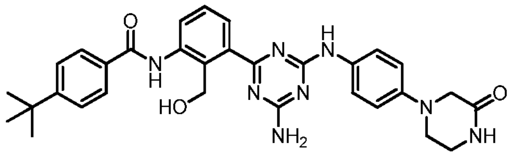 | *N*-[3-(4-Amino-6-{[4-( 3-oxopiperazin-1-yl)p henyl]amino}-1,3,5-tr iazin-2-yl)-2-(hydrox ymethyl)phenyl]-4-(*te rt*-butyl)benzamide |
| 115 | 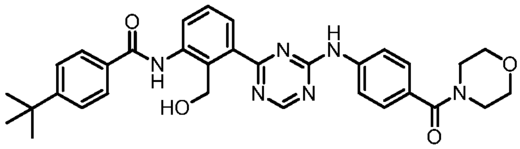 | 4-(*tert*-Butyl)-*N*-[2-( hydroxymethyl)-3-(4-{ [4-(morpholine-4-carb onyl)phenyl]amino}-1, 3,5-triazin-2-yl)phen yl]benzamide |
| 116 | 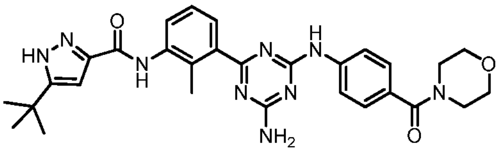 | *N*-[3-(4-Amino-6-{[4-( morpholine-4-carbonyl )phenyl]amino}-1,3,5-triazin-2-yl)-2-methy lphenyl]-5-(*tert*-buty 1)-1*H*-pyrazole-3-carb oxamide |
| 117 | 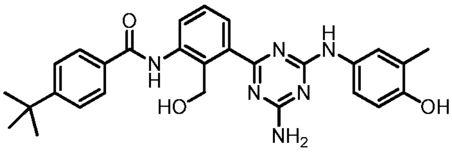 | *N*-(3-{4-Amino-6-[(4-h ydroxy-3-methylphenyl )amino]-1,3,5-triazin -2-yl}-2-(hydroxymeth yl)phenyl)-4-(*tert*-bu tyl)benzamide |
| 118 | 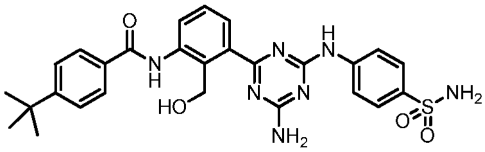 | *N*-(3-{4-Amino-6-[(4-s ulfamoylphenyl)amino] -1,3,5-triazin-2-yl}-2-(hydroxymethyl)phen yl)-4-(*tert*-butyl)ben zamide |

**Table 1-11**

| | | |
|---|---|---|
| 119 | 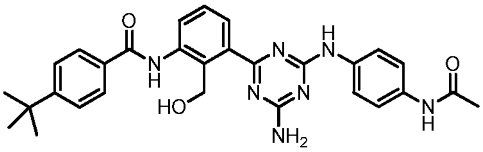 | *N*-(3-{4-[(4-Acetamido phenyl)amino]-6-amino -1,3,5-triazin-2-yl}-2-(hydroxymethyl)phen yl)-4-(*tert*-butyl)ben zamide |

(continued)

| | | |
|---|---|---|
| 120 | 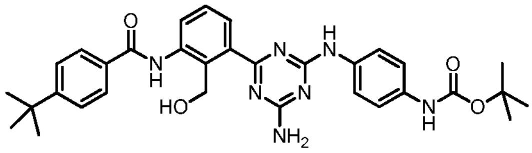 | *tert*-Butyl {4-[(4-amino-6-{3-[4-(*tert*-butyl)benzamido ]-2-(hydroxymethyl)phenyl}-1,3,5-triazin-2 -yl)amino]phenyl}carb amate |
| 121 | 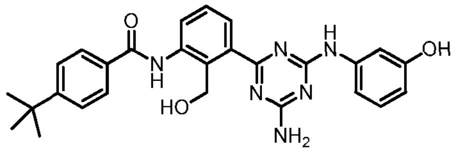 | *N*-(3-{4-Amino-6-[(3-h ydroxyphenyl)amino]-1 ,3,5-triazin-2-yl}-2-(hydroxymethyl)phenyl )-4-(*tert*-butyl)benza mide |
| 122 | 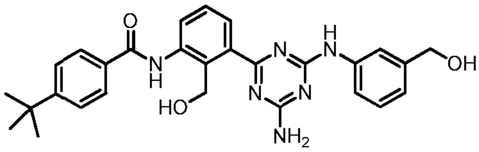 | *N*-[3-(4-Amino-6-{[3-( hydroxymethyl)phenyl] amino}-1,3,5-triazin-2-yl)-2-(hydroxymethy l)phenyl]-4-(*tert*-but yl)benzamide |
| 123 | 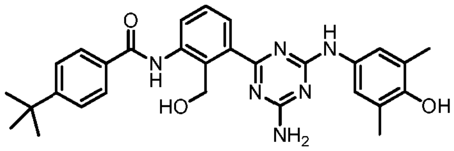 | *N*-(3-{4-Amino-6-[(4-h ydroxy-3,5-dimethylph enyl)amino]-1,3,5-tri azin-2-yl}-2-(hydroxy methyl)phenyl)-4-(*ter t*-butyl)benzamide |
| 124 | 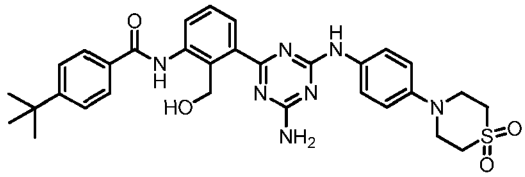 | *N*-[3-(4-Amino-6-{[4-( 1,1-dioxidothiomorpho lino)phenyl]amino}-1, 3,5-triazin-2-yl)-2-( hydroxymethyl)phenyl] -4-(*tert*-butyl)benzam ide |
| 125 | 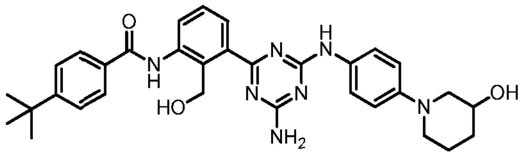 | *N*-[3-(4-Amino-6-{[4-( 3-hydroxypiperidin-1-yl)phenyl]amino}-1,3, 5-triazin-2-yl)-2-(hy droxymethyl)phenyl]-4 -(*tert*-butyl)benzamid e |
| 126 | 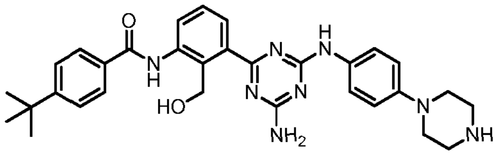 | *N*-[3-(4-Amino-6-{[4-( piperazin-1-yl)phenyl ]amino}-1,3,5-triazin -2-yl)-2-(hydroxymeth yl)phenyl]-4-(*tert*-bu tyl)benzamide |
| 127 | 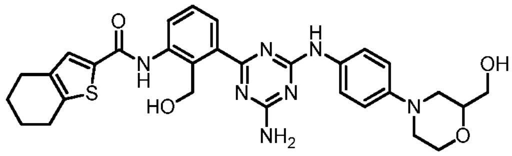 | *N*-{3-[4-Amino-6-({4-[ 2-(hydroxymethyl)morp holino]phenyl}amino)-1,3,5-triazin-2-yl]-2 -(hydroxymethyl)pheny 1}-4,5,6,7-tetrahydro benzo[*b*]thiophene-2-c arboxamide |
| 128 | 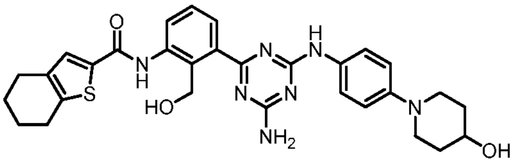 | *N*-[3-(4-Amino-6-{[4-( 4-hydroxypiperidin-1-yl)phenyl]amino}-1,3, 5-triazin-2-yl)-2-(hy droxymethyl)phenyl]-4 ,5,6,7-tetrahydrobenz o[*b*]thiophene-2-carbo xamide |

**Table 1-12**

| | | |
|---|---|---|
| 129 | 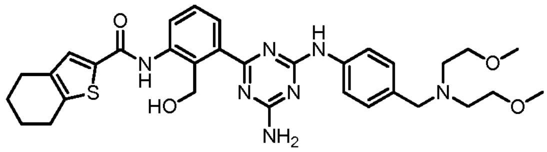 | *N*-(3-{4-Amino-6-[(4-{ [bis(2-methoxyethyl)a mino]methyl}phenyl)am ino]-1,3,5-triazin-2-yl}-2-(hydroxymethyl) phenyl)-4,5,6,7-tetra hydrobenzo[*b*]thiophen e-2-carboxamide |
| 130 | 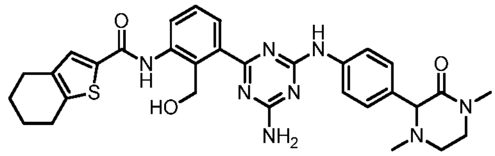 | *N*-[3-(4-Amino-6-{[4-( 1,4-dimethyl-3-oxopip erazin-2-yl)phenyl]am ino}-1,3,5-triazin-2-yl)-2-(hydroxymethyl) phenyl]-4,5,6,7-tetra hydrobenzo[*b*]thiophen e-2-carboxamide |
| 131 | 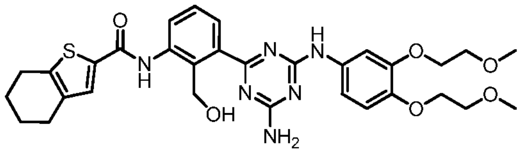 | *N*-[3-(4-Amino-6-{[3,4 -bis(2-methoxyethoxy) phenyl]amino}-1,3,5-t riazin-2-yl)-2-(hydro xymethyl)phenyl]-4,5, 6,7-tetrahydrobenzo[*b* ]thiophene-2-carboxam ide |
| 132 | 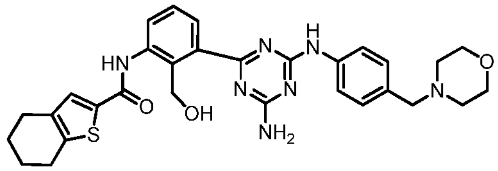 | *N*-[3-(4-Amino-6-{[4-( morpholinomethyl)phen yl]amino}-1,3,5-triaz in-2-yl)-2-(hydroxyme thyl)phenyl]-4,5,6,7-tetrahydrobenzo[*b*]thi ophene-2-carboxamide |
| 133 | 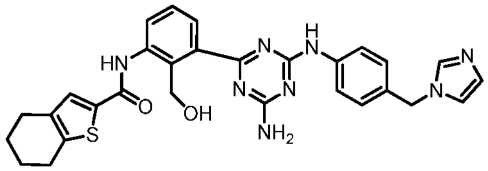 | *N*-{3-[4-({4-[(1*H*-Imid azol-1-yl)methyl]phen yl}amino)-6-amino-1,3 ,5-triazin-2-yl]-2-(h ydroxymethyl)phenyl}-4,5,6,7-tetrahydroben zo[*b*]thiophene-2-carb oxamide |
| 134 | 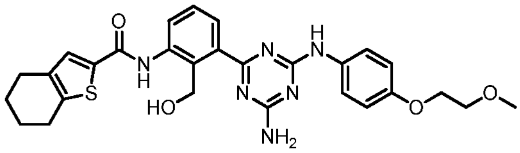 | *N*-[3-(4-Amino-6-{[4-( 2-methoxyethoxy)pheny l]amino}-1,3,5-triazi n-2-yl)-2-(hydroxymet hyl)phenyl]-4,5,6,7-t etrahydrobenzo[*b*]thio phene-2-carboxamide |
| 135 | 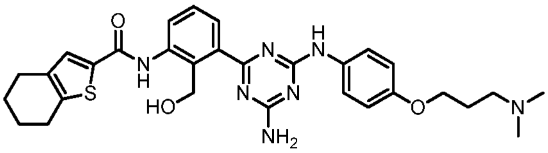 | *N*-{3-[4-Amino-6-({4-[ 3-(dimethylamino)prop oxy]phenyl}amino)-1,3 ,5-triazin-2-yl]-2-(h ydroxymethyl)phenyl}-4,5,6,7-tetrahydroben zo[*b*]thiophene-2-carb oxamide |
| 136 | 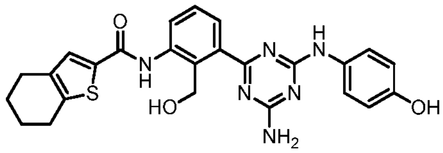 | *N*-(3-{4-Amino-6-[(4-h ydroxyphenyl)amino]-1 ,3,5-triazin-2-yl}-2-(hydroxymethyl)phenyl )-4,5,6,7-tetrahydrob enzo[*b*]thiophene-2-ca rboxamide |
| 137 | 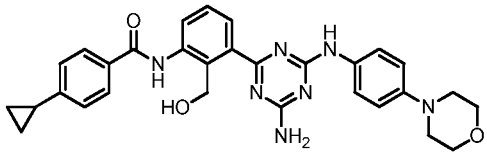 | *N*-(3-{4-Amino-6-[(4-m orpholinophenyl)amino ]-1,3,5-triazin-2-yl} -2-(hydroxymethyl)phe nyl)-4-cyclopropylben zamide |

(continued)

| | | |
|---|---|---|
| 138 | 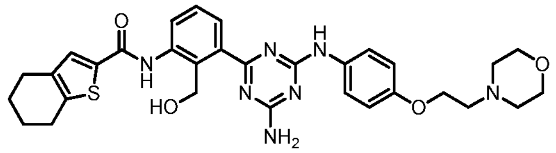 | *N*-[3-(4-Amino-6-{[4-( 2-morpholinoethoxy)ph enyl]aminol-1,3,5-tri azin-2-yl)-2-(hydroxy methyl)phenyl]-4,5,6, 7-tetrahydrobenzo[*b*]t hiophene-2-carboxamid e |

**Table 1-13**

| | | |
|---|---|---|
| 139 | 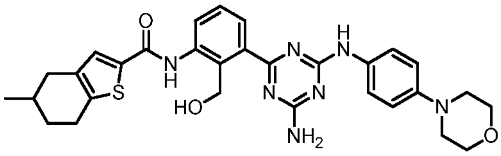 | *N*-(3-{4-Amino-6-[(4-m orpholinophenyl)amino ]-1,3,5-triazin-2-yl} -2-(hydroxymethyl)phe nyl)-5-methyl-4,5,6,7 -tetrahydrobenzo[*b*]th iophene-2-carboxamide |
| 140 | 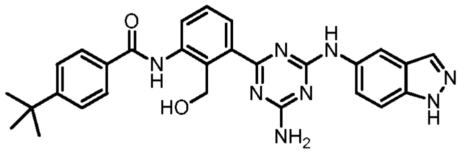 | *N*-(3-{4-[(1*H*-Indazol-5-yl)amino]-6-amino-1 ,3,5-triazin-2-yl}-2-(hydroxymethyl)phenyl )-4-(*tert*-butyl)benzamide |
| 141 | 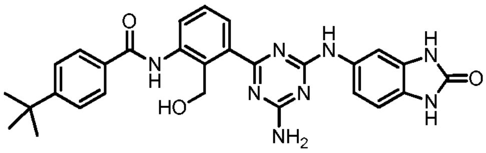 | *N*-(3-{4-Amino-6-[(2-o xo-2,3-dihydro-1*H*-ben zo[d]imidazol-5-yl)am ino]-1,3,5-triazin-2-yl}-2-(hydroxymethyl) phenyl)-4-(tert-butyl )benzamide |
| 142 | 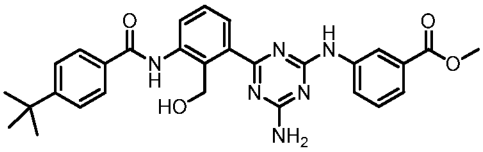 | Methyl 3-[(4-amino-6-{3-[4-( *tert*-butyl)benzamido] -2-(hydroxymethyl)phe nyl}-1,3,5-triazin-2-yl)amino]benzoate |
| 143 | 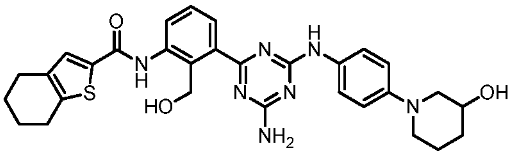 | *N*-[3-(4-Amino-6-{[4-( 3-hydroxypiperidin-1-yl)phenyl]amino}-1,3, 5-triazin-2-yl)-2-(hy droxymethyl)phenyl]-4 ,5,6,7-tetrahydrobenz o[*b*]thiophene-2-carbo xamide |
| 144 | 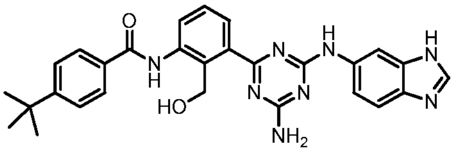 | *N*-(3-{4-[(1*H*-Benzo[*d*] imidazol-6-yl)amino]-6-amino-1,3,5-triazin -2-yl}-2-(hydroxymeth yl)phenyl)-4-(*tert*-bu tyl)benzamide |
| 145 | 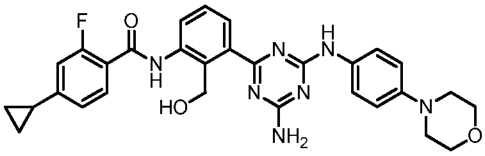 | *N*-(3-{4-Amino-6-[(4-m orpholinophenyl)amino ]-1,3,5-triazin-2-yl} -2-(hydroxymethyl)phe nyl)-4-cyclopropyl-2-fluorobenzamide |
| 146 | 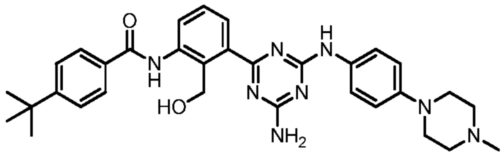 | *N*-[3-(4-Amino-6-{[4-( 4-methylpiperazin-1-y l)phenyl]amino}-1,3,5 -triazin-2-yl)-2-(hyd roxymethyl)phenyl]-4-(tert-butyl)benzamide |

(continued)

| | | |
|---|---|---|
| 147 | 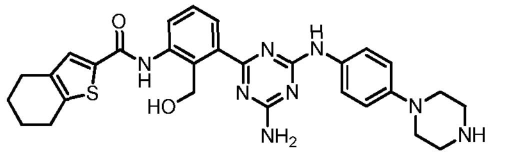 | *N*-[3-(4-Amino-6-{[4-( piperazin-1-yl)phenyl ]amino}-1,3,5-triazin -2-yl)-2-(hydroxymeth yl)phenyl]-4,5,6,7-te trahydrobenzo[*b*]thiop hene-2-carboxamide |
| 148 | 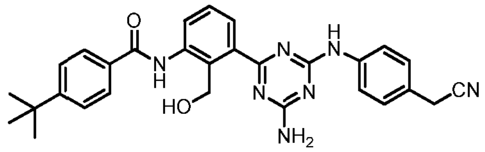 | *N*-[3-(4-Amino-6-{[4-( cyanomethyl)phenyl]am ino}-1,3,5-triazin-2-yl)-2-(hydroxymethyl) phenyl]-4-(*tert*-butyl )benzamide |

**Table 1-14**

| | | |
|---|---|---|
| 149 | 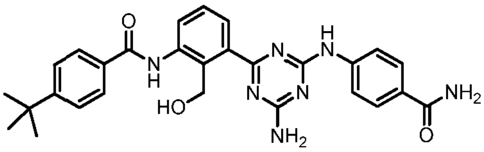 | N-(3-{4-Amino-6-[(4-c arbamoylphenyl)amino] -1,3,5-triazin-2-yl}-2-(hydroxymethyl)phen yl)-4-(*tert*-butyl)ben zamide |
| 150 | 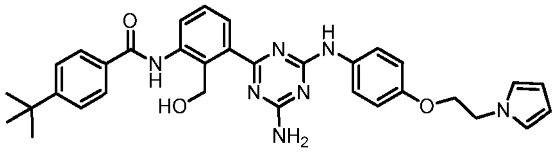 | *N*-{3-[4-({4-[2-(1*H*-Py rrol-1-yl)ethoxy]phen yl}amino)-6-amino-1,3 ,5-triazin-2-yl]-2-(h ydroxymethyl)phenyl}-4-(tert-butyl)benzami de |
| 151 | 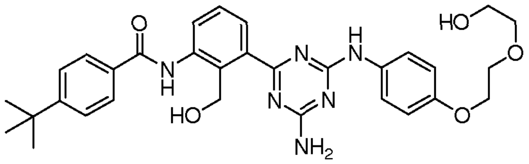 | *N*-{3-[4-Amino-6-({4-[ 2-(2-hydroxyethoxy)et hoxy]phenyl}amino)-1, 3,5-triazin-2-yl]-2-( hydroxymethyl)phenyl} -4-(*tert*-butyl)benzamide |
| 152 | 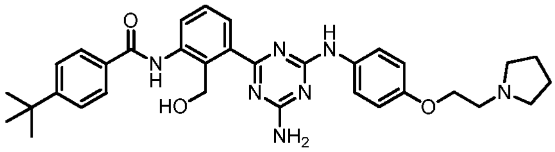 | *N*-{3-[4-Amino-6-({4-[ 2-(pyrrolidin-1-yl)et hoxy]phenyl}amino)-1, 3,5-triazin-2-yl]-2-( hydroxymethyl)phenyl} -4-(*tert*-butyl)benzam ide |
| 153 | 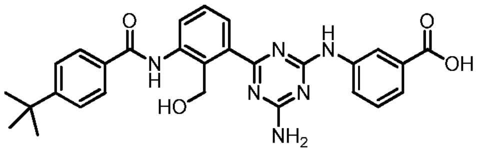 | 3-[(4-Amino-6-{3-[4-( *tert*-butyl)benzamido] -2-(hydroxymethyl)phe nyl}-1,3,5-triazin-2-yl)amino]benzoic acid |
| 154 | 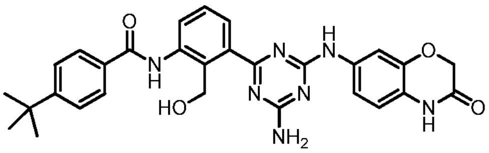 | *N*-(3-{4-Amino-6-[(3-0 xo-3,4-dihydro-2*H*-ben zo[*b*][1,4]oxazin-7-yl )amino]-1,3,5-triazin -2-yl}-2-(hydroxymeth yl)phenyl)-4-(*tert*-bu tyl)benzamide |
| 155 | 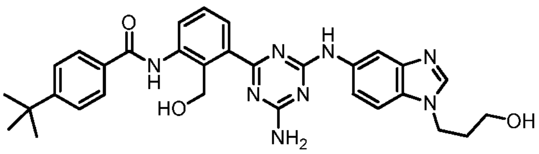 | *N*-[3-(4-Amino-6-{[1-( 3-hydroxypropyl)-1*H*-b enzo[*d*]imidazol-5-yl] amino}-1,3,5-triazin-2-yl)-2-(hydroxymethy l)phenyl]-4-(*tert*-but yl)benzamide |

(continued)

| | | |
|---|---|---|
| 156 | 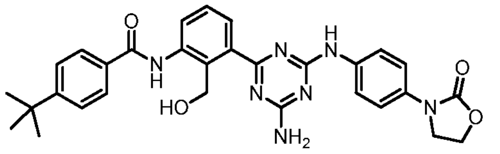 | *N*-[3-(4-Amino-6-{[4-( 2-oxooxazolidin-3-yl) phenyl]amino}-1,3,5-t riazin-2-yl)-2-(hydro xymethyl)phenyl]-4-(*t ert*-butyl)benzamide |
| 157 | 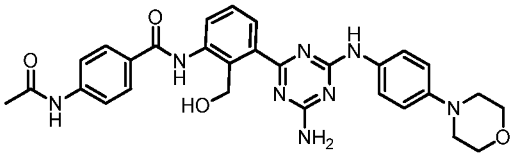 | 4-Acetamido-*N*-(3-{4-a mino-6-[(4-morpholino phenyl)amino]-1,3,5-t riazin-2-yl}-2-(hydro xymethyl)phenyl)benzamide |
| 158 | 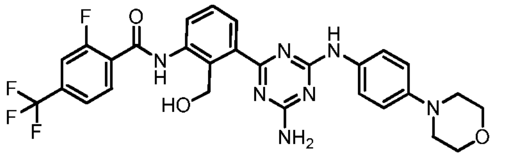 | *N*-(3-{4-Amino-6-[(4-m orpholinophenyl)amino ]-1,3,5-triazin-2-yl} -2-(hydroxymethyl)phe nyl)-2-fluoro-4-(trif luoromethyl)benzamide |

**Table 1-15**

| | | |
|---|---|---|
| 159 | 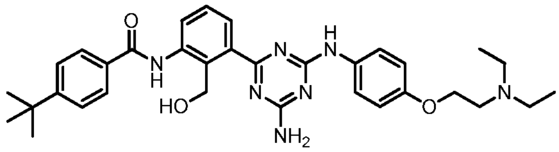 | *N*-{3-[4-Amino-6-({4-[ 2-(diethylamino)ethox y]phenyl}amino)-1,3,5 -triazin-2-yl]-2-(hyd roxymethyl)phenyl}-4-(tert-butyl)benzamide |
| 160 | 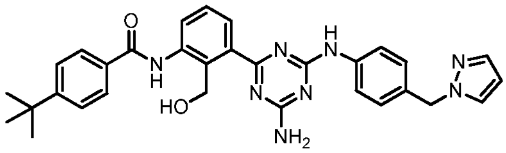 | *N*-{3-[4-({4-[(1*H*-Pyra zol-1-yl)methyl]pheny l}amino)-6-amino-1,3 5-triazin-2-yl]-2-(hy droxymethyl)phenyl}-4 -(*tert*-butyl)benzamide |
| 161 | 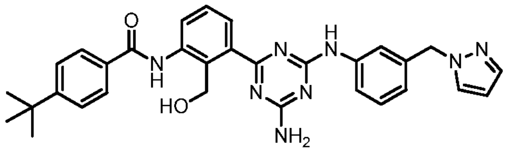 | *N*-{3-[4-({3-[(1*H*-Pyra zol-1-yl)methyl]pheny l}amino)-6-amino-1,3 5-triazin-2-yl]-2-(hy droxymethyl)phenyl}-4 -(*tert*-butyl)benzamide |
| 162 | 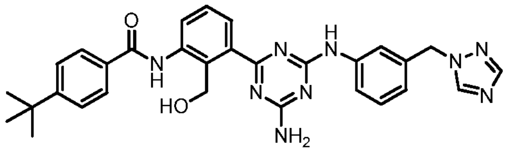 | *N*-{3-[4-({3-[(1*H*-1,2, 4-Triazol-1-yl)methyl ]phenyl}amino)-6-amin o-1,3,5-triazin-2-yl] -2-(hydroxymethyl)phe nyl}-4-(tert-butyl)be nzamide |
| 163 | 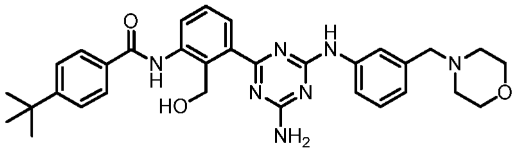 | *N*-[3-(4-Amino-6-{[3-( morpholinomethyl)phen yl]amino}-1,3,5-triaz in-2-yl)-2-(hydroxyme thyl)phenyl]-4-(*tert*-butyl)benzamide |
| 164 | 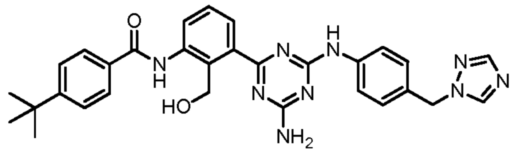 | *N*-{3-[4-({4-[(1*H*-1,2, 4-Triazol-1-yl)methyl ]phenyl}amino)-6-amin o-1,3,5-triazin-2-yl] -2-(hydroxymethyl)phe nyl}-4-(tert-butyl)benzamide |

(continued)

| | | |
|---|---|---|
| 165 | 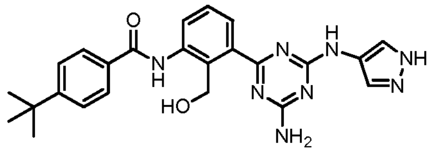 | *N*-(3-{4-[(1*H*-Pyrazol-4-yl)amino]-6-amino-1 ,3,5-triazin-2-yl}-2-(hydroxymethyl)phenyl )-4-(*tert*-butyl)benzamide |
| 166 | 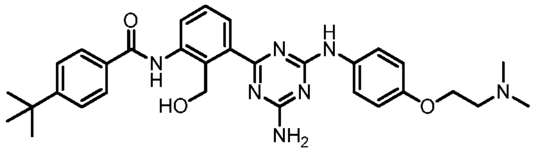 | *N*-{3-[4-Amino-6-({4-[ 2-(dimethylamino)etho xy]phenyl}amino)-1,3, 5-triazin-2-yl]-2-(hy droxymethyl)phenyl}-4 -(*tert*-butyl)benzamide |
| 167 | 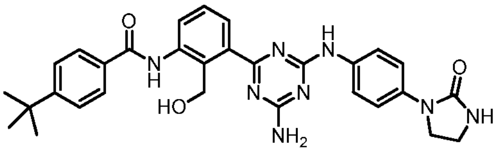 | *N*-[3-(4-Amino-6-{[4-( 2-oxoimidazolidin-1-y l)phenyl]amino}-1,3,5 -triazin-2-yl)-2-(hyd roxymethyl)phenyl]-4-(tert-butyl)benzamide |
| 168 | 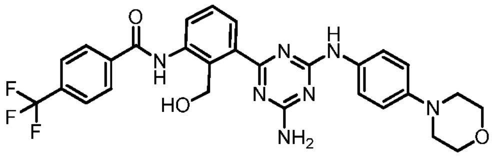 | *N*-(3-{4-Amino-6-[(4-m orpholinophenyl)amino ]-1,3,5-triazin-2-yl} -2-(hydroxymethyl)phe nyl)-4-(trifluoromethyl)benzamide |

**Table 1-16**

| | | |
|---|---|---|
| 169 | 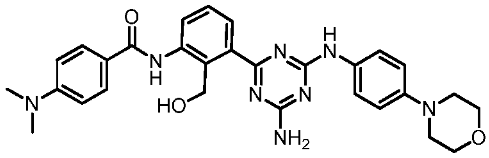 | *N*-(3-{4-Amino-6-[(4-m orpholinophenyl)amino ]-1,3,5-triazin-2-yl} -2-(hydroxymethyl)phe nyl)-4-(dimethylamino)benzamide |
| 170 | 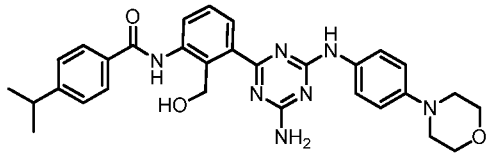 | *N*-(3-{4-Amino-6-[(4-m orpholinophenyl)amino ]-1,3,5-triazin-2-yl} -2-(hydroxymethyl)phe nyl)-4-isopropylbenzamide |
| 171 | 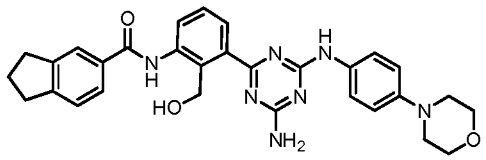 | *N*-(3-{4-Amino-6-[(4-m orpholinophenyl)amino ]-1,3,5-triazin-2-yl} -2-(hydroxymethyl)phe nyl)-2,3-dihydro-1*H*-i ndene-5-carboxamide |
| 172 | 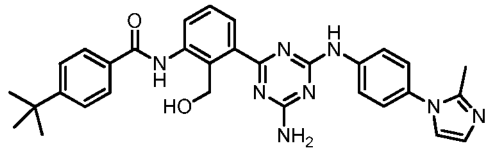 | *N*-[3-(4-Amino-6-{[4-( 2-methyl-1*H*-imidazol-1-yl)phenyl]amino}-1, 3,5-triazin-2-yl)-2-( hydroxymethyl)phenyl] -4-(*tert*-butyl)benzamide |
| 173 | 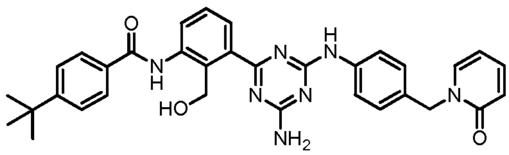 | *N*-{3-[4-Amino-6-({4-[ (2-oxopyridin-1(2*H*)-y l)methyl]phenyl}amino )-1,3,5-triazin-2-yl] -2-(hydroxymethyl)phe nyl}-4-(tert-butyl)benzamide |

(continued)

| | | |
|---|---|---|
| 174 | 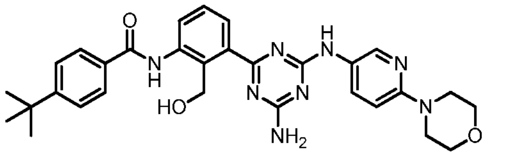 | *N*-(3-{4-Amino-6-[(6-m orpholinopyridin-3-yl )amino]-1,3,5-triazin -2-yl}-2-(hydroxymethyl)phenyl)-4-(*tert*-bu tyl)benzamide |
| 175 | 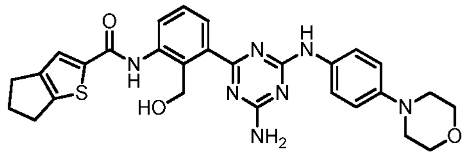 | *N*-(3-{4-Amino-6-[(4-m orpholinophenyl)amino ]-1,3,5-triazin-2-yl} -2-(hydroxymethyl)phe nyl)-5,6-dihydro-4*H*-cyclopenta[*b*]thiophene -2-carboxamide |
| 176 | 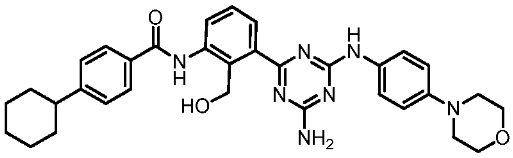 | *N*-(3-{4-Amino-6-[(4-m orpholinophenyl)amino ]-1,3,5-triazin-2-yl} -2-(hydroxymethyl)phe nyl)-4-cyclohexylbenzamide |
| 177 | 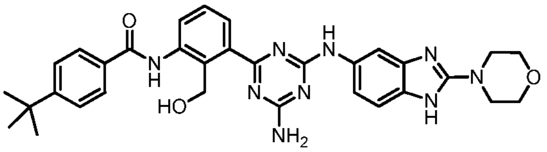 | *N*-(3-{4-Amino-6-[(2-m orpholino-1*H*-benzo[*d*] imidazol-5-yl)amino]-1,3,5-triazin-2-yl}-2 -(hydroxymethyl)phenyl)-4-(*tert*-butyl)benzamide |
| 178 | 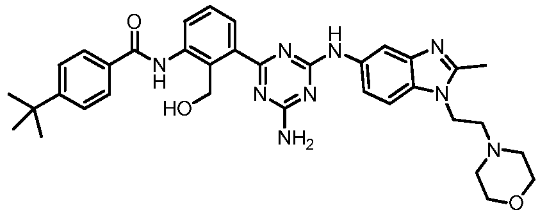 | *N*-[3-(4-Amino-6-{[2-m ethyl-1-(2-morpholino ethyl)-1*H*-benzo[*d*]imi dazol-5-yl]amino}-1,3 ,5-triazin-2-yl)-2-(h ydroxymethyl)phenyl]-4-(tert-butyl)benzamide |

**Table 1-17**

| | | |
|---|---|---|
| 179 | 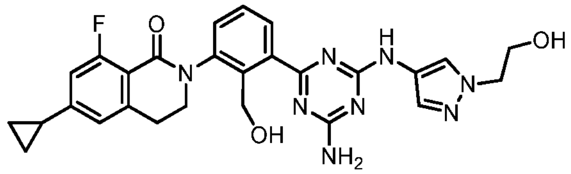 | 2-[3-(4-Amino-6-{[1-( 2-hydroxyethyl)-1*H*-py razol-4-yl]amino}-1,3 ,5-triazin-2-yl)-2-(h ydroxymethyl)phenyl]-6-cyclopropyl-8-fluor o-3,4-dihydroisoquino lin-1(2*H*)-one |
| 180 | 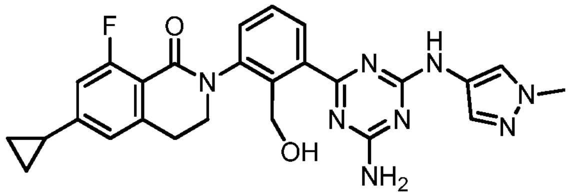 | 2-(3-{4-Amino-6-[(1-m ethyl-1*H*-pyrazol-4-yl )amino]-1,3,5-triazin -2-yl}-2-(hydroxymeth yl)phenyl)-6-cyclopro pyl-8-fluoro-3,4-dihy droisoquinolin-1(2H)-one |
| 181 | 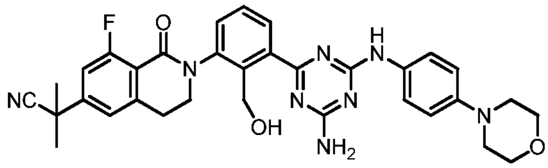 | 2-[2-(3-{4-Amino-6-[( 4-morpholinophenyl)am ino]-1,3,5-triazin-2-yl}-2-(hydroxymethyl) phenyl)-8-fluoro-1-oxo-1,2,3,4-tetrahydroi soquinolin-6-yl]-2-me thylpropanenitrile |

(continued)

| | | |
|---|---|---|
| 182 | 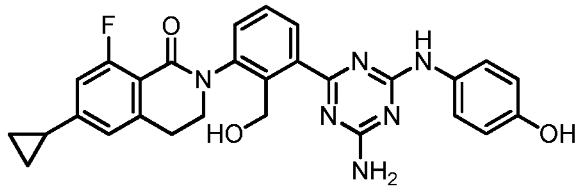 | 2-(3-{4-Amino-6-[(4-h ydroxyphenyl)amino]-1 ,3,5-triazin-2-yl}-2-(hydroxymethyl)phenyl )-6-cyclopropyl-8-flu oro-3,4-dihydroisoquinolin-1(2*H*)-one |
| 183 | 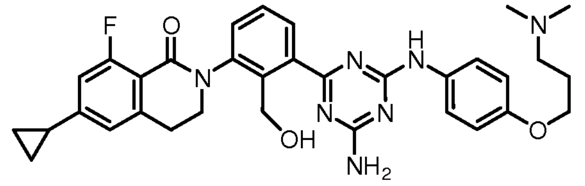 | 2-{3-[4-Amino-6-({4-[ 3-(dimethylamino)prop oxy]phenyl}amino)-1,3 ,5-triazin-2-yl]-2-(h ydroxymethyl)phenyl}-6-cyclopropyl-8-fluor O-3,4-dihydroisoquino lin-1(2*H*)-one |
| 184 | 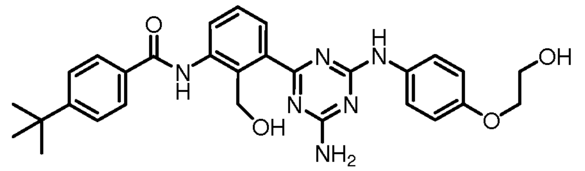 | *N*-[3-(4-Amino-6-{[4-( 2-hydroxyethoxy)pheny 1]aminol-1,3,5-triazi n-2-yl)-2-(hydroxymethyl)phenyl]-4-(*tert*-b utyl)benzamide |
| 185 | 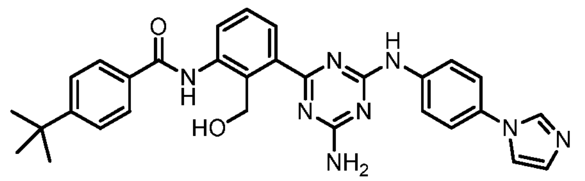 | *N*-[3-(4-{[4-(1*H*-Imida zol-1-yl)phenyl]amino }-6-amino-1,3,5-triaz in-2-yl)-2-(hydroxyme thyl)phenyl]-4-(tert-butyl)benzamide |
| 186 | 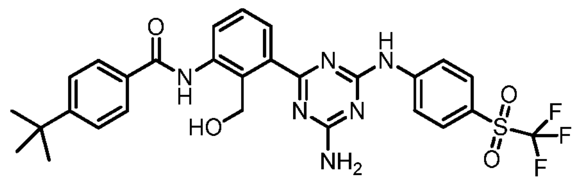 | *N*-{3-[4-Amino-6-({4-[ (trifluoromethyl)sulf onyl]phenyl}amino)-1, 3,5-triazin-2-yl]-2-( hydroxymethyl)phenyl} -4-(*tert*-butyl)benzamide |
| 187 | 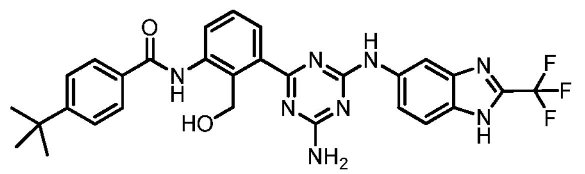 | *N*-[3-(4-Amino-6-{[2-( trifluoromethyl)-1*H*-b enzo[*d*]imidazol-5-yl] amino}-1,3,5-triazin-2-yl)-2-(hydroxymethy 1)phenyl]-4-(*tert*-butyl)benzamide |
| 188 | 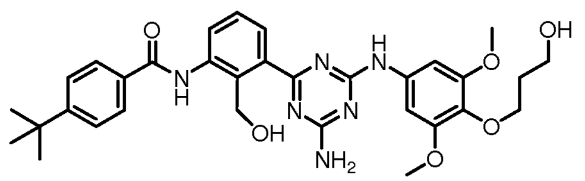 | *N*-[3-(4-Amino-6-{[4-( 3-hydroxypropoxy)-3,5 -dimethoxyphenyl]amin o}-1,3,5-triazin-2-yl )-2-(hydroxymethyl)ph enyl]-4-(tert-butyl)b enzamide |

**Table 1-18**

| | | |
|---|---|---|
| 189 | 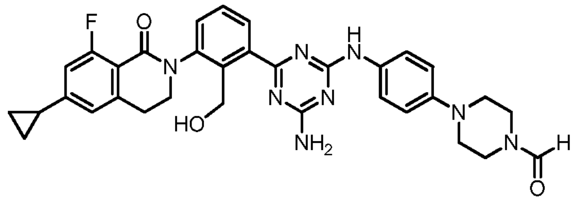 | 4-[4-({4-Amino-6-[3-( 6-cyclopropyl-8-fluor o-1-oxo-3,4-dihydrois oquinolin-2(1*H*)-yl)-2 -(hydroxymethyl)pheny 1]-1,3,5-triazin-2-yl }amino)phenyl]piperaz ine-1-carbaldehyde |

(continued)

| | | |
|---|---|---|
| 190 | 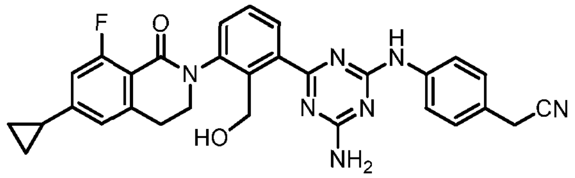 | 2-[4-({4-Amino-6-[3-( 6-cyclopropyl-8-fluor o-1-oxo-3,4-dihydrois oquinolin-2(1*H*)-yl)-2 -(hydroxymethyl)pheny 1]-1,3,5-triazin-2-yl }amino)phenyl]acetonitrile |
| 191 | 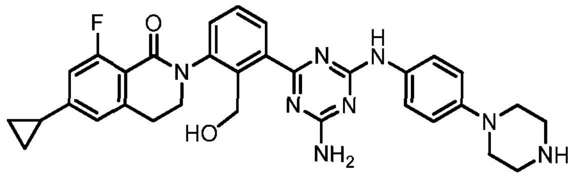 | 2-[3-(4-Amino-6-{[4-( piperazin-1-yl)phenyl ]amino}-1,3,5-triazin -2-yl)-2-(hydroxymeth yl)phenyl]-6-cyclopro pyl-8-fluoro-3,4-dihy droisoquinolin-1(2H)-one |
| 192 | 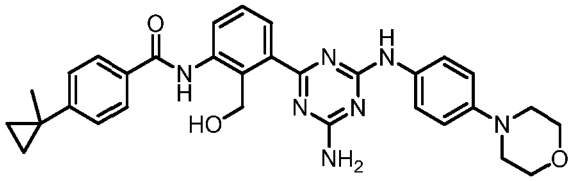 | *N*-(3-{4-Amino-6-[(4-m orpholinophenyl)amino ]-1,3,5-triazin-2-yl} -2-(hydroxymethyl)phe nyl)-4-(1-methylcyclopropyl)benzamide |
| 193 | 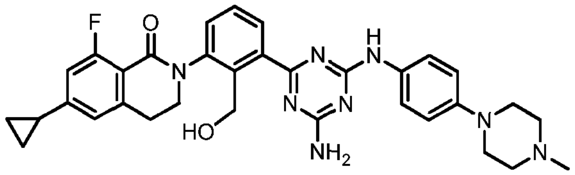 | 2-[3-(4-Amino-6-{[4-( 4-methylpiperazin-1-y 1)phenyl]amino}-1,3,5 -triazin-2-yl)-2-(hyd roxymethyl)phenyl]-6-cyclopropyl-8-fluoro-3,4-dihydroisoquinolin-1(2*H*)-one |
| 194 | 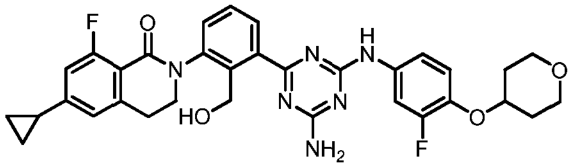 | 2-{3-[4-Amino-6-({3-f luoro-4-[(tetrahydro-2*H*-pyran-4-yl)oxy]phe nyl}amino)-1,3,5-tria zin-2-yl]-2-(hydroxym ethyl)phenyl}-6-cyclo propyl-8-fluoro-3,4-d ihydroisoquinolin-1(2 *H*)-one |
| 195 | 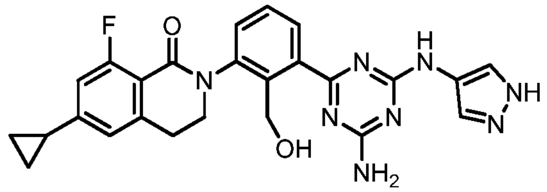 | 2-(3-{4-[(1*H*-Pyrazol-4-yl)amino]-6-amino-1 ,3,5-triazin-2-yl}-2-(hydroxymethyl)phenyl )-6-cyclopropyl-8-flu oro-3,4-dihydroisoquinolin-1(2*H*)-one |
| 196 | 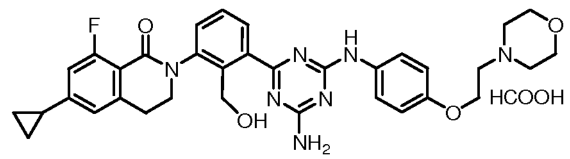 | 2-[3-(4-Amino-6-{[4-( 2-morpholinoethoxy)ph enyl]amino}-1,3,5-tri azin-2-yl)-2-(hydroxy methyl)phenyl]-6-cycl opropyl-8-fluoro-3,4-dihydroisoquinolin-1( 2*H*)-one formate |
| 197 | 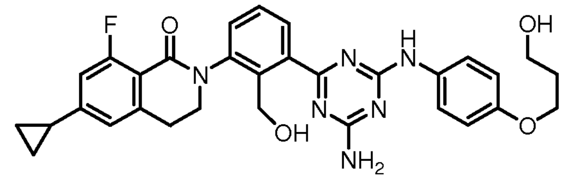 | 2-[3-(4-Amino-6-{[4-( 3-hydroxypropoxy)phen yl]amino}-1,3,5-triaz in-2-yl)-2-(hydroxyme thyl)phenyl]-6-cyclop ropyl-8-fluoro-3,4-di hydroisoquinolin-1(2*H* )-one |

(continued)

| | | |
|---|---|---|
| 198 | 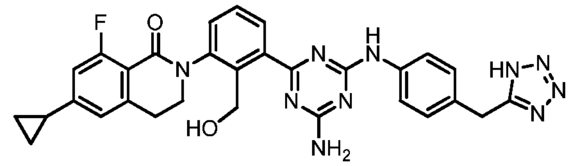 | 2-{3-[4-({4-[(1*H*-Tetr azol-5-yl)methyl]phen yl}amino)-6-amino-1,3 ,5-triazin-2-yl]-2-(h ydroxymethyl)phenyl}-6-cyclopropyl-8-fluor O-3,4-dihydroisoquinolin-1(2*H*)-one |

**Table 1-19**

| | | |
|---|---|---|
| 199 | 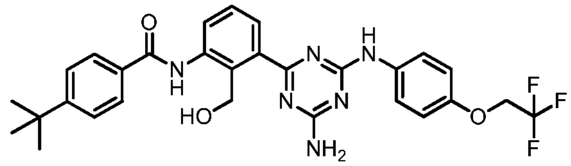 | *N*-[3-(4-Amino-6-{[4-( 2,2,2-trifluoroethoxy )phenyl]amino}-1,3,5-triazin-2-yl)-2-(hydr oxymethyl)phenyl]-4-( *tert*-butyl)benzamide |
| 200 | 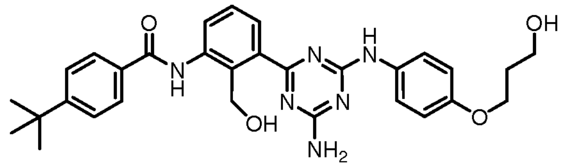 | *N*-[3-(4-Amino-6-{[4-( 3-hydroxypropoxy)phen yl]amino}-1,3,5-triaz in-2-yl)-2-(hydroxyme thyl)phenyl]-4-(tert-butyl)benzamide |
| 201 | 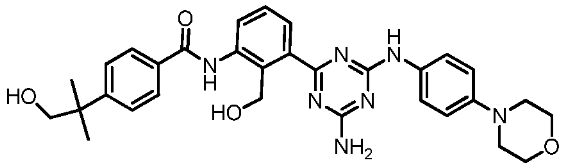 | *N*-(3-{4-Amino-6-[(4-m orpholinophenyl)amino ]-1,3,5-triazin-2-yl} -2-(hydroxymethyl)phe nyl)-4-(1-hydroxy-2-m ethylpropan-2-yl)benzamide |
| 202 | 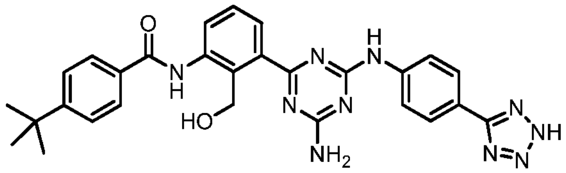 | *N*-[3-(4-{[4-(2*H*-Tetra zol-5-yl)phenyl]amino }-6-amino-1,3,5-triaz in-2-yl)-2-(hydroxyme thyl)phenyl]-4-(tert-butyl)benzamide |
| 203 | 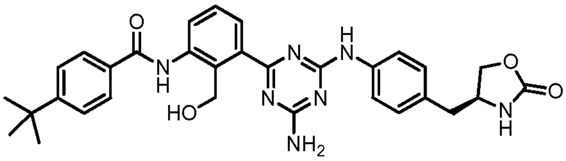 | *N*-{3-[4-Amino-6-({4-[ (2-oxooxazolidin-4-yl )methyl]phenyl}amino) -1,3,5-triazin-2-yl]-2-(hydroxymethyl)phen yl}-4-(*tert*-butyl)benzamide |
| 204 | 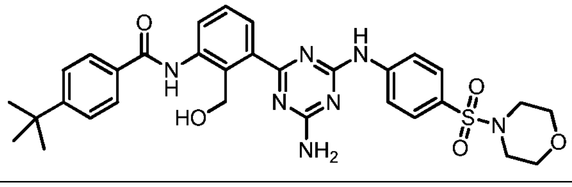 | *N*-[3-(4-Amino-6-{[4-( morpholinosulfonyl)ph enyl]amino}-1,3,5-tri azin-2-yl)-2-(hydroxy methyl)phenyl]-4-(ter*t*-butyl)benzamide |
| 205 | 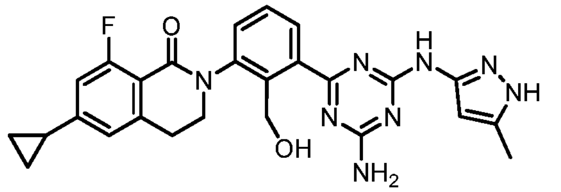 | 2-(3-{4-Amino-6-[(5-m ethyl-1*H*-pyrazol-3-yl )amino]-1,3,5-triazin -2-yl}-2-(hydroxymeth yl)phenyl)-6-cyclopro pyl-8-fluoro-3,4-dihy droisoquinolin-1(2H)-one |

(continued)

| | | |
|---|---|---|
| 206 | 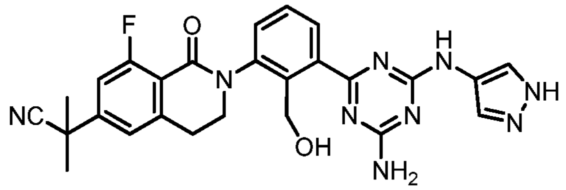 | 2-[2-(3-{4-[(1*H*-Pyraz ol-4-yl)amino]-6-amin o-1,3,5-triazin-2-yl} -2-(hydroxymethyl)phe nyl)-8-fluoro-1-oxo-1 ,2,3,4-tetrahydroisoq uinolin-6-yl]-2-methylpropanenitrile |
| 207 | 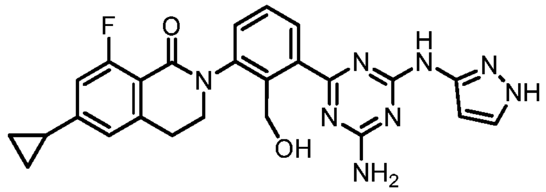 | 2-(3-{4-[(1*H*-Pyrazol-3-yl)amino]-6-amino-1 ,3,5-triazin-2-yl}-2-(hydroxymethyl)phenyl )-6-cyclopropyl-8-flu oro-3,4-dihydroisoquinolin-1(2*H*)-one |
| 208 | 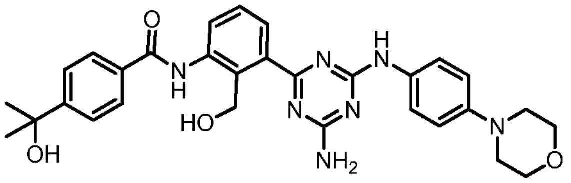 | *N*-(3-{4-Amino-6-[(4-m orpholinophenyl)amino ]-1,3,5-triazin-2-yl} -2-(hydroxymethyl)phe nyl)-4-(2-hydroxyprop an-2-yl)benzamide |

**Table 1-20**

| | | |
|---|---|---|
| 209 | 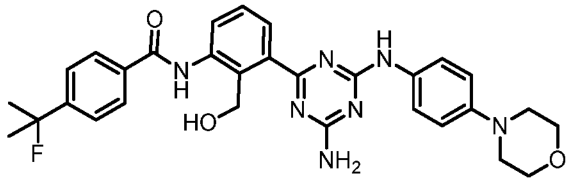 | *N*-(3-{4-Amino-6-[(4-m orpholinophenyl)amino ]-1,3,5-triazin-2-yl} -2-(hydroxymethyl)phe nyl)-4-(2-fluoropropan-2-yl)benzamide |
| 210 | 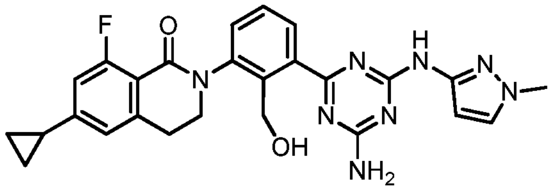 | 2-(3-{4-Amino-6-[(1-m ethyl-1*H*-pyrazol-3-yl )amino]-1,3,5-triazin -2-yl}-2-(hydroxymeth yl)phenyl)-6-cyclopro pyl-8-fluoro-3,4-dihy droisoquinolin-1(2H)-one |
| 211 | 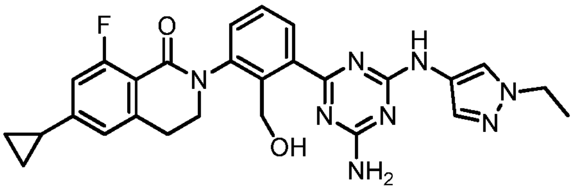 | 2-(3-{4-Amino-6-[(1-e thyl-1*H*-pyrazol-4-yl) amino]-1,3,5-triazin-2-yl}-2-(hydroxymethy l)phenyl)-6-cycloprop yl-8-fluoro-3,4-dihyd roisoquinolin-1(2*H*)-o ne |
| 212 | 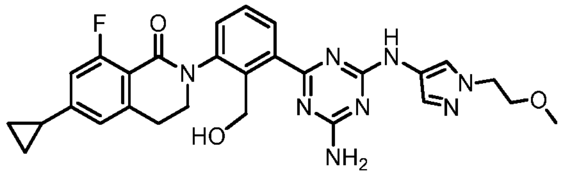 | 2-[3-(4-Amino-6-{[1-( 2-methoxyethyl)-1*H*-py razol-4-yl]amino}-1,3 ,5-triazin-2-yl)-2-(h ydroxymethyl)phenyl]-6-cyclopropyl-8-fluor O-3,4-dihydroisoquinolin-1(2*H*)-one |

(continued)

| | | |
|---|---|---|
| 213 | 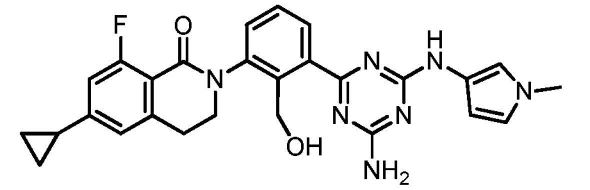 | 2-(3-{4-Amino-6-[(1-m ethyl-1*H*-pyrrol-3-yl) amino]-1,3,5-triazin-2-yl}-2-(hydroxymethy l)phenyl)-6-cycloprop yl-8-fluoro-3,4-dihydroisoquinolin-1(2*H*)-o ne |
| 214 | 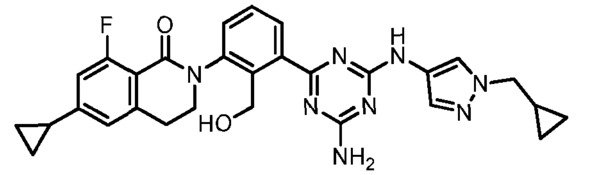 | 2-[3-(4-Amino-6-{[1-( cyclopropylmethyl)-1*H* -pyrazol-4-yl]amino}-1,3,5-triazin-2-yl)-2 -(hydroxymethyl)pheny l]-6-cyclopropyl-8-fl uoro-3,4-dihydroisoquinolin-1(2*H*)-one |
| 215 | 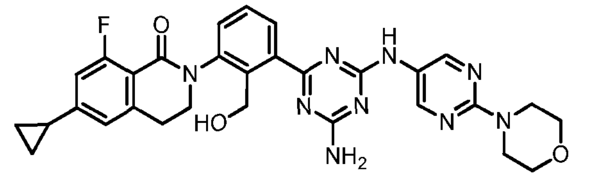 | 2-(3-{4-Amino-6-[(2-m orpholinopyrimidin-5-yl)amino]-1,3,5-triaz in-2-yl}-2-(hydroxyme thyl)phenyl)-6-cyclop ropyl-8-fluoro-3,4-di hydroisoquinolin-1(2*H* )-one |
| 216 | 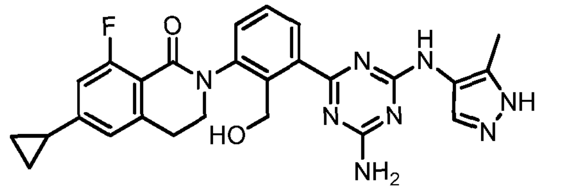 | 2-(3-{4-Amino-6-[(5-m ethyl-1*H*-pyrazol-4-yl )amino]-1,3,5-triazin -2-yl}-2-(hydroxymeth yl)phenyl)-6-cyclopro pyl-8-fluoro-3,4-dihy droisoquinolin-1(2H)-one |
| 217 | 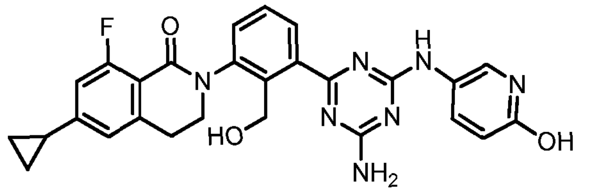 | 2-(3-{4-Amino-6-[(6-h ydroxypyridin-3-yl)am ino]-1,3,5-triazin-2-yl}-2-(hydroxymethyl) phenyl)-6-cyclopropyl -8-fluoro-3,4-dihydroisoquinolin-1(2*H*)-one |
| 218 | 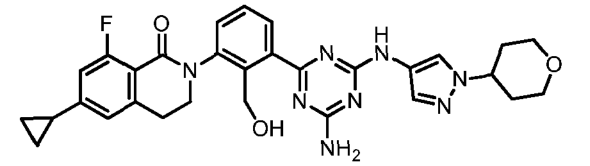 | 2-[3-(4-Amino-6-{[1-( tetrahydro-2*H*-pyran-4 -yl)-1*H*-pyrazol-4-yl] amino}-1,3,5-triazin-2-yl)-2-(hydroxymethy l)phenyl]-6-cycloprop yl-8-fluoro-3,4-dihydroisoquinolin-1(2*H*)-one |

**Table 1-21**

| | | |
|---|---|---|
| 219 | 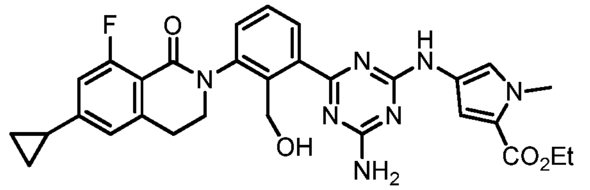 | Ethyl 4-({4-amino-6-[3-(6-c yclopropyl-8-fluoro-1 -oxo-3,4-dihydroisoqu inolin-2(1*H*)-yl)-2-(hydroxymethyl)phenyl]-1,3,5-triazin-2-yl}am ino)-1-methyl-1*H*-pyrr ole-2-carboxylate |
| 220 | 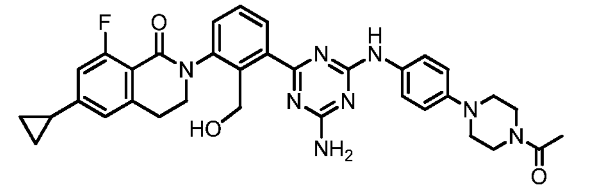 | 2-[3-(4-{[4-(4-Acetyl piperazin-1-yl)phenyl ]amino}-6-amino-1,3,5 -triazin-2-yl)-2-(hyd roxymethyl)phenyl]-6-cyclopropyl-8-fluoro-3,4-dihydroisoquinolin-1(2*H*)-one |

(continued)

| | | |
|---|---|---|
| 221 | 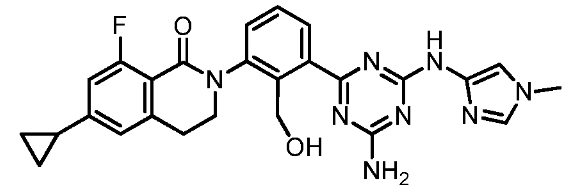 | 2-(3-{4-Amino-6-[(1-m ethyl-1*H*-imidazol-4-y l)amino]-1,3,5-triazi n-2-yl}-2-(hydroxymet hyl)phenyl)-6-cyclopr opyl-8-fluoro-3,4-dih ydroisoquinolin-1(2*H*) -one |
| 222 | 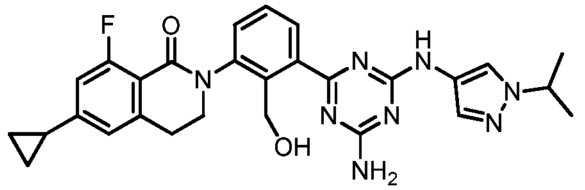 | 2-(3-{4-Amino-6-[(1-i sopropyl-1*H*-pyrazol-4-yl)amino]-1,3,5-tria zin-2-yl}-2-(hydroxym ethyl)phenyl)-6-cyclopropyl-8-fluoro-3,4-d ihydroisoquinolin-1(2 *H*)-one |
| 223 | 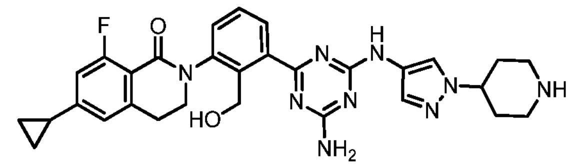 | 2-[3-(4-Amino-6-{[1-( piperidin-4-yl)-1*H*-py razol-4-yl]amino}-1,3 ,5-triazin-2-yl)-2-(h ydroxymethyl)phenyl]-6-cyclopropyl-8-fluor O-3,4-dihydroisoquinolin-1(2*H*)-one |
| 224 | 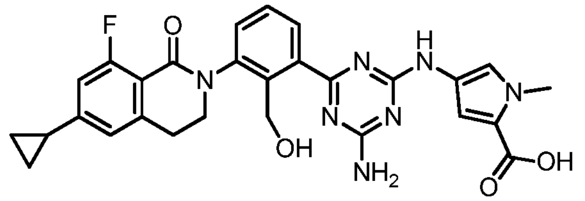 | 4-({4-Amino-6-[3-(6-c yclopropyl-8-fluoro-1 -oxo-3,4-dihydroisoqu inolin-2(1*H*)-yl)-2-(h ydroxymethyl)phenyl]-1,3,5-triazin-2-yl}am ino)-1-methyl-1*H*-pyrr ole-2-carboxylic acid |
| 225 | 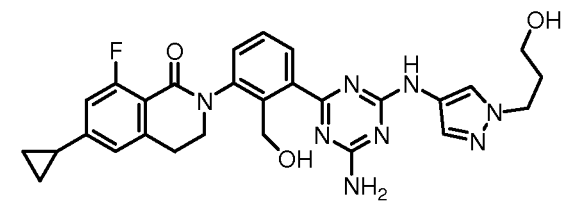 | 2-[3-(4-Amino-6-{[1-( 3-hydroxypropyl)-1*H*-p yrazol-4-yl]amino}-1, 3,5-triazin-2-yl)-2-( hydroxymethyl)phenyl] -6-cyclopropyl-8-fluo ro-3,4-dihydroisoquinolin-1(2*H*)-one |
| 226 | 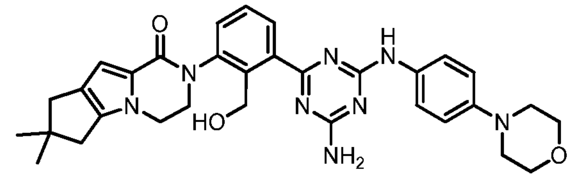 | 2-(3-{4-Amino-6-[(4-m orpholinophenyl)amino ]-1,3,5-triazin-2-yl} -2-(hydroxymethyl)phe nyl)-7,7-dimethyl-2,3 ,4,6,7,8-hexahydro-1*H* -cyclopenta[4,5]pyrrolo[1,2-*a*]pyrazin-1-one |
| 227 | 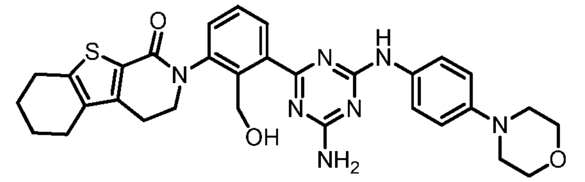 | 2-(3-{4-Amino-6-[(4-m orpholinophenyl)amino ]-1,3,5-triazin-2-yl} -2-(hydroxymethyl)phe nyl)-3,4,5,6,7,8-hexa hydrobenzo[4,5]thieno [2,3-*c*]pyridin-1(2*H*)-one |
| 228 | 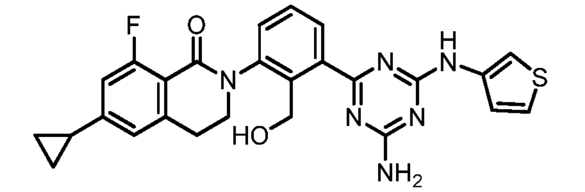 | 2-{3-[4-Amino-6-(thio phen-3-ylamino)-1,3,5 -triazin-2-yl]-2-(hyd roxymethyl)phenyl}-6-cyclopropyl-8-fluoro-3,4-dihydroisoquinolin-1(2*H*)-one |

**Table 1-22**

| | | |
|---|---|---|
| 229 | 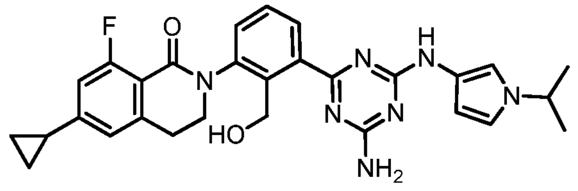 | 2-(3-{4-Amino-6-[(1-i sopropyl-1*H*-pyrrol-3-yl)amino]-1,3,5-triaz in-2-yl}-2-(hydroxyme thyl)phenyl)-6-cyclop ropyl-8-fluoro-3,4-di hydroisoquinolin-1(2*H* )-one |
| 230 | 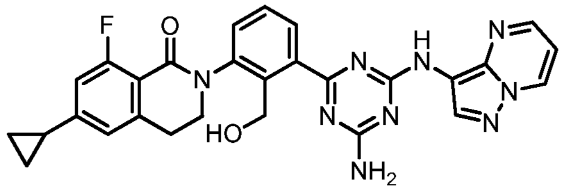 | 2-{3-[4-Amino-6-(pyra zolo[1,5-a]pyrimidin-3-ylamino)-1,3,5-tria zin-2-yl]-2-(hydroxym ethyl)phenyl}-6-cyclo propyl-8-fluoro-3,4-d ihydroisoquinolin-1(2 *H*)-one |
| 231 | 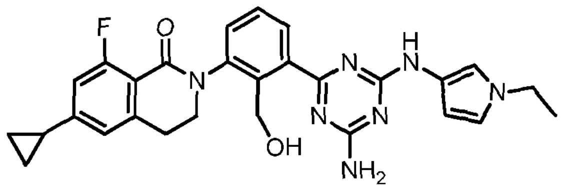 | 2-(3-{4-Amino-6-[(1-e thyl-1*H*-pyrrol-3-yl)a mino]-1,3,5-triazin-2 -yl}-2-(hydroxymethyl )phenyl)-6-cyclopropy 1-8-fluoro-3,4-dihydroisoquinolin-1(2*H*)-on e |
| 232 | 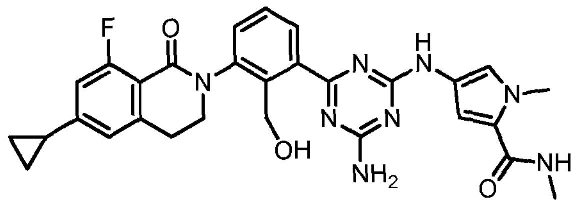 | 4-({4-Amino-6-[3-(6-c yclopropyl-8-fluoro-1 -oxo-3,4-dihydroisoqu inolin-2(1*H*)-yl)-2-(h ydroxymethyl)phenyl]-1,3,5-triazin-2-yl}am ino)-*N*,1-dimethyl-1*H*-pyrrole-2-carboxamide |
| 233 | 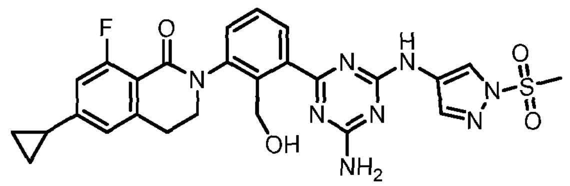 | 2-[3-(4-Amino-6-{[1-( methylsulfonyl)-1*H*-py razol-4-yl]amino}-1,3 ,5-triazin-2-yl)-2-(h ydroxymethyl)phenyl]-6-cyclopropyl-8-fluor O-3,4-dihydroisoquino lin-1(2H)-one |
| 234 | 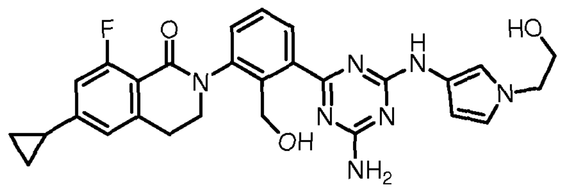 | 2-[3-(4-Amino-6-{[1-( 2-hydroxyethyl)-1*H*-py rrol-3-yl]amino}-1,3, 5-triazin-2-yl)-2-(hy droxymethyl)phenyl]-6 -cyclopropyl-8-fluoro -3,4-dihydroisoquinolin-1(2*H*)-one |
| 235 | 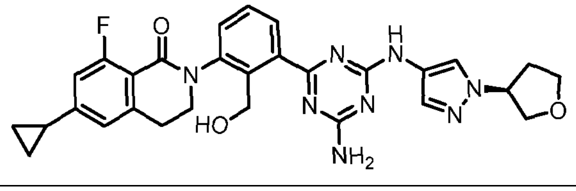 | (*S*)-2-[3-(4-Amino-6-{ [1-(tetrahydrofuran-3 -yl)-1*H*-pyrazol-4-yl] amino}-1,3,5-triazin-2-yl)-2-(hydroxymethy l)phenyl]-6-cycloprop yl-8-fluoro-3,4-dihydroisoquinolin-1(2*H*)-one |
| 236 | 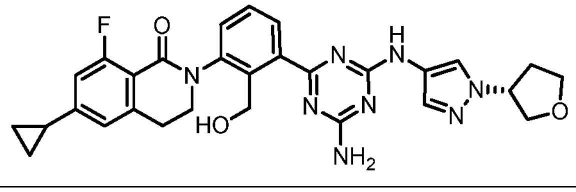 | (*R*)-2-[3-(4-Amino-6-{ [1-(tetrahydrofuran-3 -yl)-1*H*-pyrazol-4-yl] amino}-1,3,5-triazin-2-yl)-2-(hydroxymethy l)phenyl]-6-cycloprop yl-8-fluoro-3,4-dihydroisoquinolin-1(2*H*)-o ne |

(continued)

| | | |
|---|---|---|
| 237 | 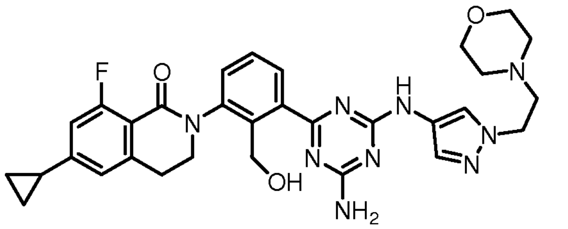 | 2-[3-(4-Amino-6-{[1-( 2-morpholinoethyl)-1*H* -pyrazol-4-yl]amino}-1,3,5-triazin-2-yl)-2 -(hydroxymethyl)pheny l]-6-cyclopropyl-8-f1 uoro-3,4-dihydroisoquinolin-1(2*H*)-one |
| 238 | 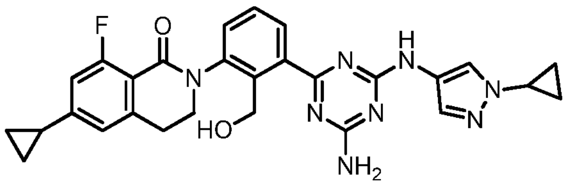 | 2-(3-{4-Amino-6-[(1-c yclopropyl-1*H*-pyrazol -4-yl)amino]-1,3,5-tr iazin-2-yl}-2-(hydrox ymethyl)phenyl)-6-cyc lopropyl-8-fluoro-3,4 -dihydroisoquinolin-1(2*H*)-one |

**Table 1-23**

| | | |
|---|---|---|
| 239 | 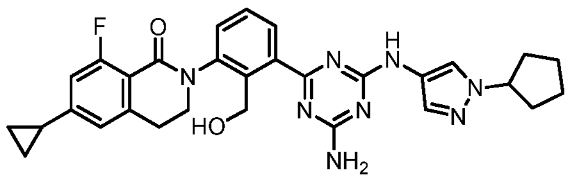 | 2-(3-{4-Amino-6-[(1-c yclopentyl-1*H*-pyrazol -4-yl)amino]-1,3,5-tr iazin-2-yl}-2-(hydrox ymethyl)phenyl)-6-cyclopropyl-8-fluoro-3,4 -dihydroisoquinolin-1 (2*H*)-one |
| 240 | 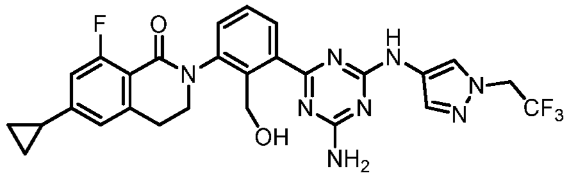 | 2-[3-(4-Amino-6-{[1-( 2,2,2-trifluoroethyl) -1*H*-pyrazol-4-yl]amin o}-1,3,5-triazin-2-yl )-2-(hydroxymethyl)ph enyl]-6-cyclopropyl-8 -fluoro-3,4-dihydroisoquinolin-1(2*H*)-one |
| 241 | 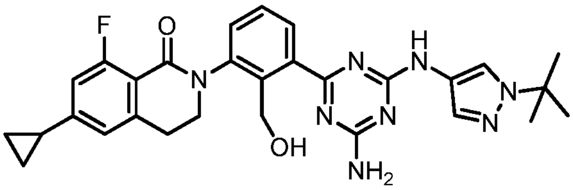 | 2-[3-(4-Amino-6-{[1-( *tert*-butyl)-1*H*-pyrazo l-4-yl]amino}-1,3,5-t riazin-2-yl)-2-(hydro xymethyl)phenyl]-6-cy clopropyl-8-fluoro-3, 4-dihydroisoquinolin-1(2*H*)-one |
| 242 | 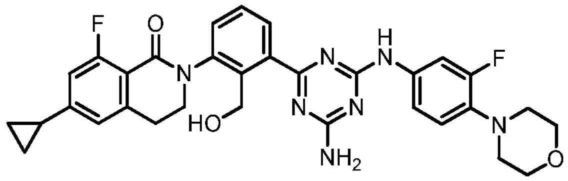 | 2-(3-{4-Amino-6-[(3-f luoro-4-morpholinophe nyl)amino]-1,3,5-tria zin-2-yl}-2-(hydroxym ethyl)phenyl)-6-cyclo propyl-8-fluoro-3,4-d ihydroisoquinolin-1(2 *H*)-one |
| 243 | 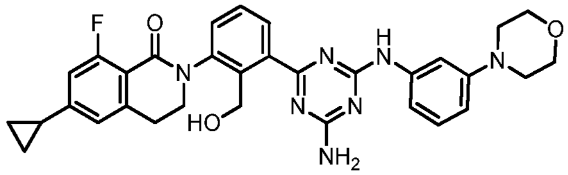 | 2-(3-{4-Amino-6-[(3-m orpholinophenyl)amino ]-1,3,5-triazin-2-yl} -2-(hydroxymethyl)phe nyl)-6-cyclopropyl-8-fluoro-3,4-dihydroisoquinolin-1(2*H*)-one |
| 244 | 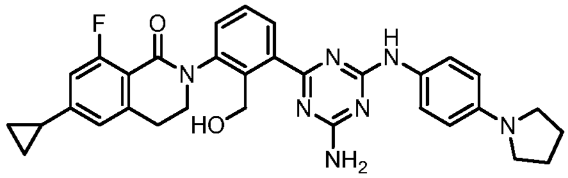 | 2-[3-(4-Amino-6-{[4-( pyrrolidin-1-yl)pheny l]amino}-1,3,5-triazi n-2-yl)-2-(hydroxymet hyl)phenyl]-6-cyclopr opyl-8-fluoro-3,4-dihydroisoquinolin-1(2*H*) -one |

(continued)

| | | |
|---|---|---|
| 245 | 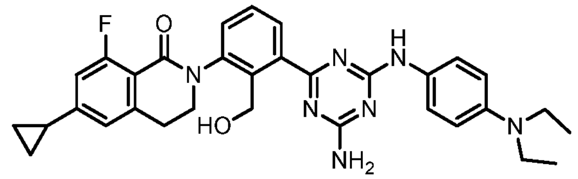 | 2-[3-(4-Amino-6-{[4-( diethylamino)phenyl]a mino}-1,3,5-triazin-2 -yl)-2-(hydroxymethyl )phenyl]-6-cyclopropy 1-8-fluoro-3,4-dihydroisoquinolin-1(2*H*)-on e |
| 246 | 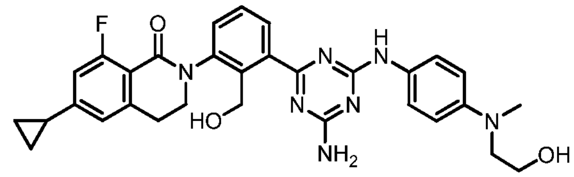 | 2-{3-[4-Amino-6-({4-[ (2-hydroxyethyl)(meth yl)amino]phenyl}amino )-1,3,5-triazin-2-yl] -2-(hydroxymethyl)phe nyl}-6-cyclopropyl-8-fluoro-3,4-dihydroisoquinolin-1(2*H*)-one |
| 247 | 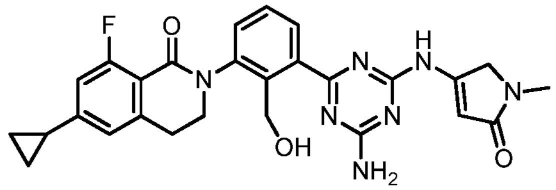 | 2-(3-{4-Amino-6-[(1-m ethyl-5-oxo-2,5-dihyd ro-1*H*-pyrrol-3-yl)ami no]-1,3,5-triazin-2-y l}-2-(hydroxymethyl)p henyl)-6-cyclopropyl-8-fluoro-3,4-dihydroisoquinolin-1(2*H*)-one |
| 248 | 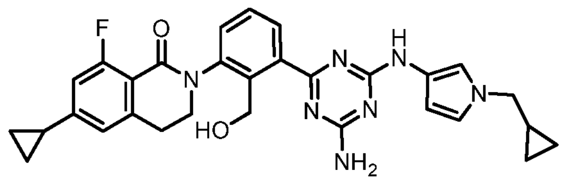 | 2-[3-(4-Amino-6-{[1-( cyclopropylmethyl)-1*H* -pyrrol-3-yl]amino}-1 ,3,5-triazin-2-yl)-2-(hydroxymethyl)phenyl ]-6-cyclopropyl-8-flu oro-3,4-dihydroisoquinolin-1(2*H*)-one |

**Table 1-24**

| | | |
|---|---|---|
| 249 | 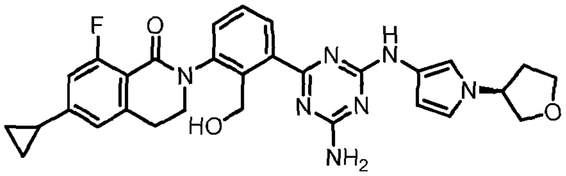 | (*S*)-2-[3-(4-Amino-6-{ [1-(tetrahydrofuran-3 -yl)-1*H*-pyrrol-3-yl]a mino}-1,3,5-triazin-2 -yl)-2-(hydroxymethyl )phenyl]-6-cyclopropy 1-8-fluoro-3,4-dihydr oisoquinolin-1(2H)-one |
| 250 | 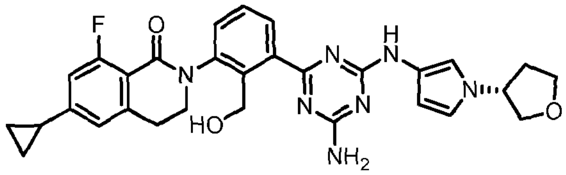 | (*R*)-2-[3-(4-Amino-6-{ [1-(tetrahydrofuran-3 -yl)-1*H*-pyrrol-3-yl]a mino}-1,3,5-triazin-2 -yl)-2-(hydroxymethyl )phenyl]-6-cyclopropy 1-8-fluoro-3,4-dihydroisoquinolin-1(2*H*)-one |
| 251 | 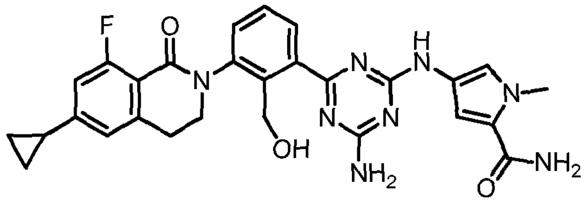 | 4-({4-Amino-6-[3-(6-c yclopropyl-8-fluoro-1 -oxo-3,4-dihydroisoqu inolin-2(1*H*)-yl)-2-(h ydroxymethyl)phenyl]-1,3,5-triazin-2-yl}am ino)-1-methyl-1*H*-pyrr ole-2-carboxamide |
| 252 | 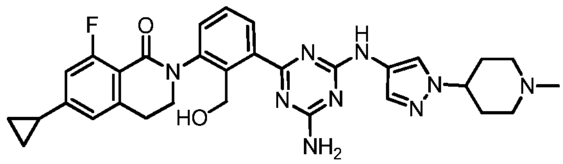 | 2-[3-(4-Amino-6-{[1-( 1-methylpiperidin-4-y l)-1*H*-pyrazol-4-yl]am ino}-1,3,5-triazin-2-yl)-2-(hydroxymethyl) phenyl]-6-cyclopropyl -8-fluoro-3,4-dihydroisoquinolin-1(2*H*)-one |

(continued)

| | | |
|---|---|---|
| 253 | 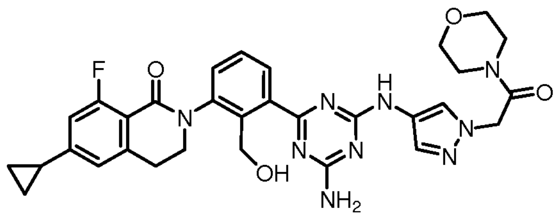 | 2-[3-(4-Amino-6-{[1-( 2-morpholino-2-oxoeth yl)-1*H*-pyrazol-4-yl]a mino}-1,3,5-triazin-2 -yl)-2-(hydroxymethyl )phenyl]-6-cyclopropy 1-8-fluoro-3,4-dihydroisoquinolin-1(2*H*)-one |
| 254 | 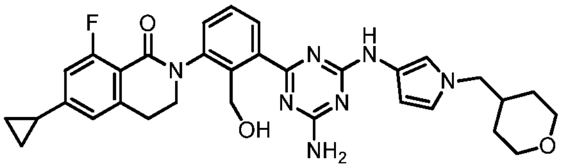 | 2-{3-[4-Amino-6-({1-[ (tetrahydro-2*H*-pyran-4-yl)methyl]-1*H*-pyrro l-3-yl}amino)-1,3,5-t riazin-2-yl]-2-(hydro xymethyl)phenyl}-6-cy clopropyl-8-fluoro-3, 4-dihydroisoquinolin-1(2*H*)-one |
| 255 | 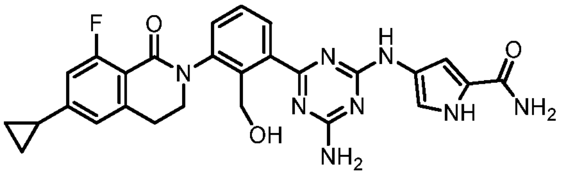 | 4-({4-Amino-6-[3-(6-c yclopropyl-8-fluoro-1 -oxo-3,4-dihydroisoqu inolin-2(1*H*)-yl)-2-(h ydroxymethyl)phenyl]-1,3,5-triazin-2-yl}am ino)-1*H*-pyrrole-2-car boxamide |
| 256 | 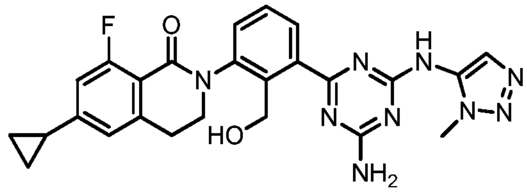 | 2-(3-{4-Amino-6-[(1-m ethyl-1*H*-1,2,3-triazo l-5-yl)amino]-1,3,5-t riazin-2-yl}-2-(hydro xymethyl)phenyl)-6-cyclopropyl-8-fluoro-3, 4-dihydroisoquinolin-1(2*H*)-one |
| 257 | 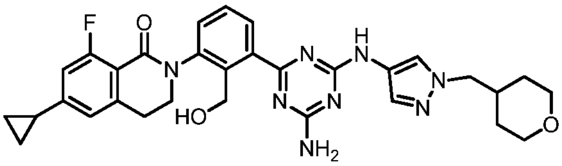 | 2-{3-[4-Amino-6-({1-[ (tetrahydro-2*H*-pyran-4-yl)methyl]-1*H*-pyraz ol-4-yl}amino)-1,3,5-triazin-2-yl]-2-(hydr oxymethyl)phenyl}-6-c yclopropyl-8-fluoro-3 ,4-dihydroisoquinolin -1(2*H*)-one |
| 258 | 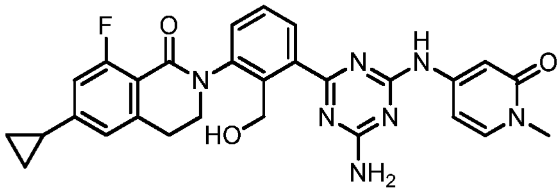 | 2-(3-{4-Amino-6-[(1-m ethyl-2-oxo-1,2-dihyd ropyridin-4-yl)amino] -1,3,5-triazin-2-yl}-2-(hydroxymethyl)phen yl)-6-cyclopropyl-8-f luoro-3,4-dihydroisoq uinolin-1(2*H*)-one |

**Table 1-25**

| | | |
|---|---|---|
| 259 | 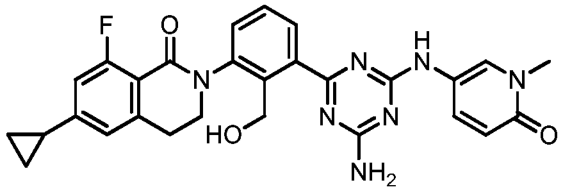 | 2-(3-{4-Amino-6-[(1-m ethyl-6-oxo-1,6-dihyd ropyridin-3-yl)amino] -1,3,5-triazin-2-yl}-2-(hydroxymethyl)phen yl)-6-cyclopropyl-8-f luoro-3,4-dihydroisoquinolin-1(2*H*)-one |

(continued)

| | | |
|---|---|---|
| 260 | 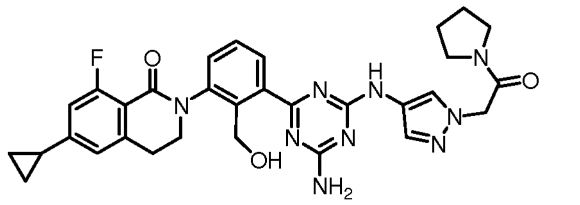 | 2-{3-[4-Amino-6-({1-[ 2-oxo-2-(pyrrolidin-1 -yl)ethyl]-1*H*-pyrazol -4-yl}amino)-1,3,5-tr iazin-2-yl]-2-(hydrox ymethyl)phenyl}-6-cyc lopropyl-8-fluoro-3,4 -dihydroisoquinolin-1(2*H*)-one |
| 261 | 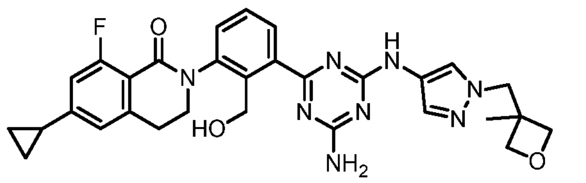 | 2-{3-[4-Amino-6-({1-[ (3-methyloxetan-3-yl) methyl]-1*H*-pyrazol-4-yl}amino)-1,3,5-triaz in-2-yl]-2-(hydroxyme thyl)phenyl}-6-cyclop ropyl-8-fluoro-3,4-di hydroisoquinolin-1(2*H* )-one |
| 262 | 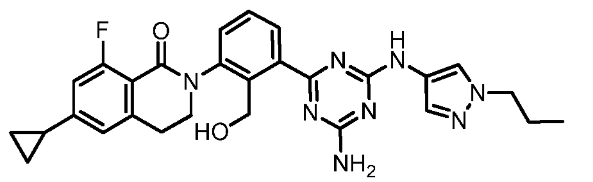 | 2-(3-{4-Amino-6-[(1-p ropyl-1*H*-pyrazol-4-yl )amino]-1,3,5-triazin -2-yl}-2-(hydroxymeth yl)phenyl)-6-cyclopro pyl-8-fluoro-3,4-dihy droisoquinolin-1(2H)-one |
| 263 | 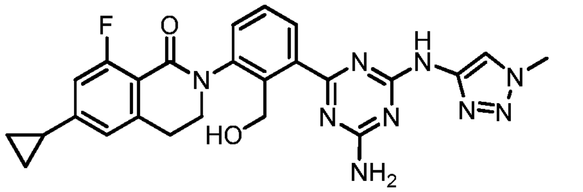 | 2-(3-{4-Amino-6-[(1-methyl-1*H*-1,2,3-triazo 1-4-yl)amino]-1,3,5-t riazin-2-yl}-2-(hydro xymethyl)phenyl)-6-cy clopropyl-8-fluoro-3, 4-dihydroisoquinolin-1(2*H*)-one |
| 264 | 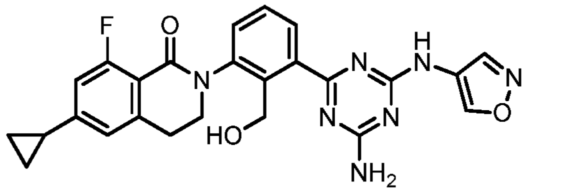 | 2-{3-[4-Amino-6-(isox azol-4-ylamino)-1,3,5 -triazin-2-yl]-2-(hyd roxymethyl)phenyl}-6-cyclopropyl-8-fluoro-3,4-dihydroisoquinolin-1(2*H*)-one |
| 265 | 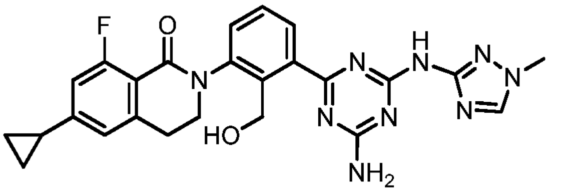 | 2-(3-{4-Amino-6-[(1-m ethyl-1*H*-1,2,4-triazo l-3-yl)amino]-1,3,5-t riazin-2-yl}-2-(hydro xymethyl)phenyl)-6-cy clopropyl-8-fluoro-3, 4-dihydroisoquinolin-1(2*H*)-one |
| 266 | 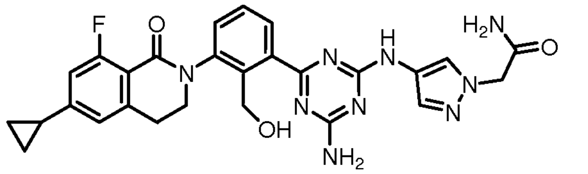 | 2-[4-({4-Amino-6-[3-( 6-cyclopropyl-8-fluor o-1-oxo-3,4-dihydrois oquinolin-2(1*H*)-yl)-2 -(hydroxymethyl)pheny 1]-1,3,5-triazin-2-yl }amino)-1*H*-pyrazol-1-yl]acetamide |
| 267 | 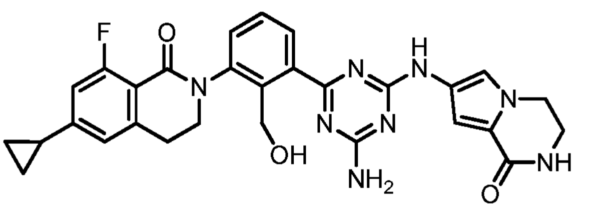 | 2-(3-{4-Amino-6-[(1-o xo-1,2,3,4-tetrahydro pyrrolo[1,2-*a*]pyrazin -7-yl)amino]-1,3,5-tr iazin-2-yl}-2-(hydrox ymethyl)phenyl)-6-cyc lopropyl-8-fluoro-3,4 -dihydroisoquinolin-1 (2*H*)-one |

(continued)

| | | |
|---|---|---|
| 268 | 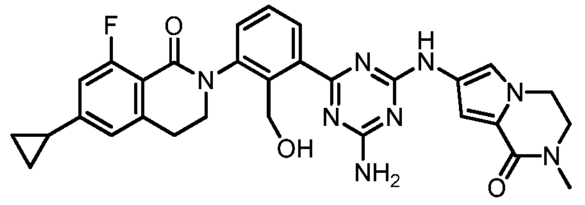 | 2-(3-{4-Amino-6-[(2-m ethyl-1-oxo-1,2,3,4-t etrahydropyrrolo[1,2-a]pyrazin-7-yl)amino] -1,3,5-triazin-2-yl}-2-(hydroxymethyl)phen yl)-6-cyclopropyl-8-f luoro-3,4-dihydroisoquinolin-1(2*H*)-one |

**Table 1-26**

| | | |
|---|---|---|
| 269 | 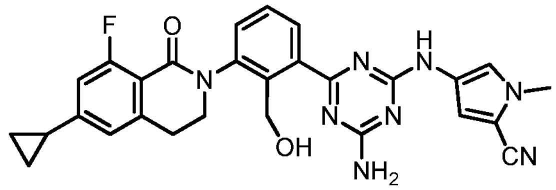 | 4-({4-Amino-6-[3-(6-c yclopropyl-8-fluoro-1 -oxo-3,4-dihydroisoqu inolin-2(1*H*)-yl)-2-(h ydroxymethyl)phenyl]-1,3,5-triazin-2-yl}am ino)-1-methyl-1*H*-pyrr ole-2-carbonitrile |
| 270 | 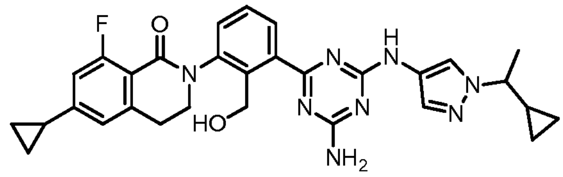 | 2-[3-(4-Amino-6-{[1-( 1-cyclopropylethyl)-1 *H*-pyrazol-4-yl]amino} -1,3,5-triazin-2-yl)-2-(hydroxymethyl)phen yl]-6-cyclopropyl-8-f luoro-3,4-dihydroisoquinolin-1(2*H*)-one |
| 271 | 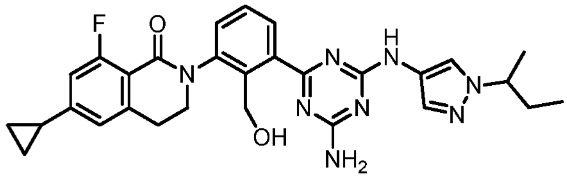 | 2-[3-(4-Amino-6-{[1-( sec-butyl)-1H-pyrazol -4-yl]amino}-1,3,5-tr iazin-2-yl)-2-(hydrox ymethyl)phenyl]-6-cyc lopropyl-8-fluoro-3,4 -dihydroisoquinolin-1 (2*H*)-one |
| 272 | 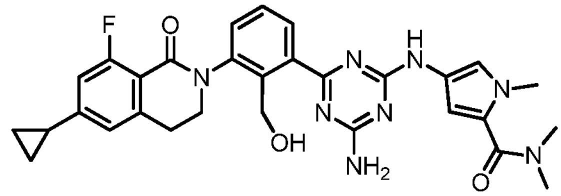 | 4-({4-Amino-6-[3-(6-c yclopropyl-8-fluoro-1 -oxo-3,4-dihydroisoqu inolin-2(1*H*)-yl)-2-(h ydroxymethyl)phenyl]-1,3,5-triazin-2-yl}am ino)-*N,N*,1-trimethyl-1*H*-pyrrole-2-carboxamide |
| 273 | 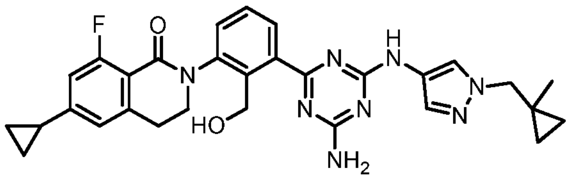 | 2-{3-[4-Amino-6-({1-[ (1-methylcyclopropyl) methyl]-1*H*-pyrazol-4-yl}amino)-1,3,5-triaz in-2-yl]-2-(hydroxyme thyl)phenyl}-6-cyclopropyl-8-fluoro-3,4-di hydroisoquinolin-1(2*H* )-one |
| 274 | 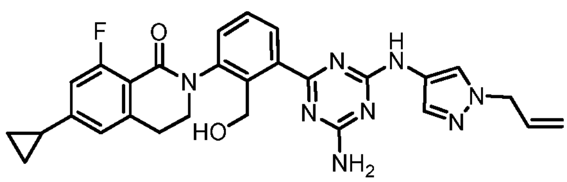 | 2-(3-{4-[(1-Allyl-1*H*-pyrazol-4-yl)amino]-6 -amino-1,3,5-triazin-2-yl}-2-(hydroxymethy l)phenyl)-6-cycloprop yl-8-fluoro-3,4-dihydroisoquinolin-1(2*H*)-o ne |
| 275 | 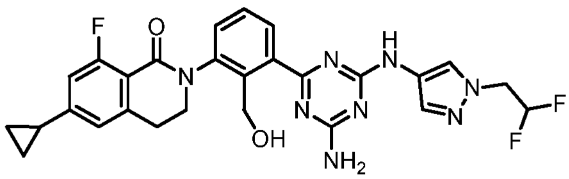 | 2-[3-(4-Amino-6-{[1-( 2,2-difluoroethyl)-1*H* -pyrazol-4-yl]amino}-1,3,5-triazin-2-yl)-2 -(hydroxymethyl)pheny l]-6-cyclopropyl-8-f1 uoro-3,4-dihydroisoquinolin-1(2*H*)-one |

(continued)

| | | |
|---|---|---|
| 276 | 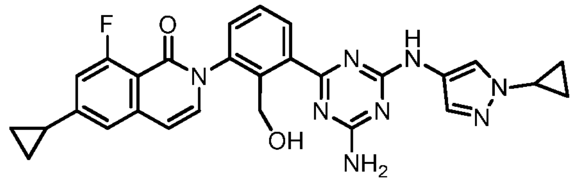 | 2-(3-{4-Amino-6-[(1-c yclopropyl-1*H*-pyrazol -4-yl)amino]-1,3,5-tr iazin-2-yl}-2-(hydrox ymethyl)phenyl)-6-cyc lopropyl-8-fluoroisoquinolin-1(2*H*)-one |
| 277 | 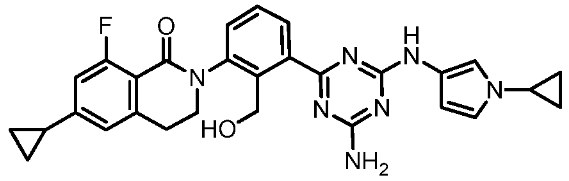 | 2-(3-{4-Amino-6-[(1-cyclopropyl-1*H*-pyrrol-3-yl)amino]-1,3,5-tri azin-2-yl}-2-(hydroxy methyl)phenyl)-6-cycl opropyl-8-fluoro-3,4-dihydroisoquinolin-1( 2*H*)-one |
| 278 | 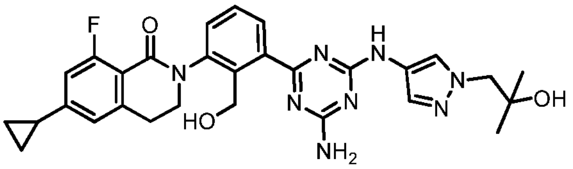 | 2-[3-(4-Amino-6-{[1-( 2-hydroxy-2-methylpro pyl)-1*H*-pyrazol-4-yl] amino}-1,3,5-triazin-2-yl)-2-(hydroxymethy l)phenyl]-6-cycloprop yl-8-fluoro-3,4-dihydroisoquinolin-1(2*H*)-one |

**Table 1-27**

| | | |
|---|---|---|
| 279 | 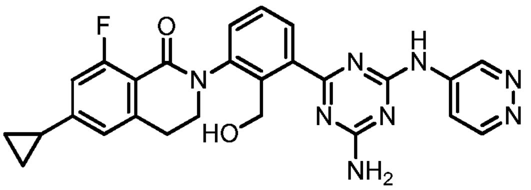 | 2-{3-[4-Amino-6-(pyri dazin-4-ylamino)-1,3, 5-triazin-2-yl]-2-(hy droxymethyl)phenyl}-6 -cyclopropyl-8-fluoro -3,4-dihydroisoquinol in-1(2*H*)-one |
| 280 | 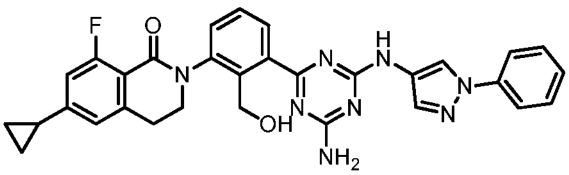 | 2-(3-{4-Amino-6-[(1-p henyl-1*H*-pyrazol-4-yl )amino]-1,3,5-triazin -2-yl}-2-(hydroxymeth yl)phenyl)-6-cyclopro pyl-8-fluoro-3,4-dihy droisoquinolin-1(2*H*)-one |
| 281 | 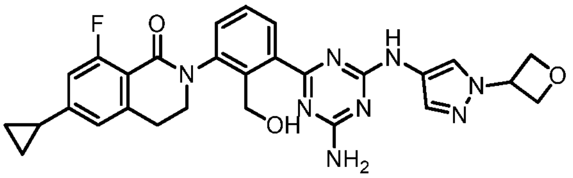 | 2-[3-(4-Amino-6-{[1-( oxetan-3-yl)-1*H*-pyraz ol-4-yl]amino}-1,3,5-triazin-2-yl)-2-(hydr oxymethyl)phenyl]-6-c yclopropyl-8-fluoro-3 ,4-dihydroisoquinolin -1(2*H*)-one |
| 282 | 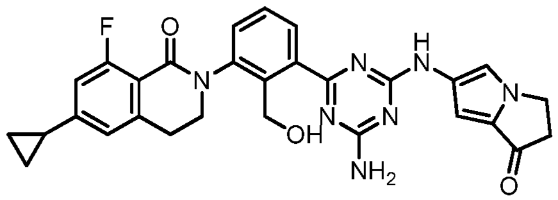 | 2-(3-{4-Amino-6-[(1-o xo-2,3-dihydro-1*H*-pyr rolizin-6-yl)amino]-1 ,3,5-triazin-2-yl}-2-(hydroxymethyl)phenyl )-6-cyclopropyl-8-flu oro-3,4-dihydroisoquinolin-1(2*H*)-one |

**Table 2-1**

| Ex. No. | $^1$H-NMR δ (ppm) | LCMS m/z $[M+H]^+$ |
|---|---|---|
| 6 | (DMSO-d6) : δ 10.55 (s, 1H), 9.96 (s, 1H), 8.91 (s, 1H), 7.98 - 7.80 (m, 8H), 7.68 (dd, J = 7.9, 1.4 Hz, 1H), 7.59 - 7.46 (m, 3H), 7.45 - 7.33 (m, 3H), 3.77-3.70 (m, 8H), 3.43 (dd, J = 11.7, 9.0 Hz, 1H), 2.39 (s, 3H), 1.33 (s, 9H). | 710.1 |
| 7 | (DMSO-d6) : δ 10.56 (s, 1H), 9.97 (s, 1H), 8.91 (s, 1H), 7.99 - 7.91 (m, 2H), 7.86 (d, J = 8.5 Hz, 2H), 7.67 (dd, J = 7.8, 1.5 Hz, 1H), 7.60 - 7.46 (m, 3H), 7.46 - 7.34 (m, 3H), 3.86 - 3.81 (m, 9H), 3.64 - 3.56 (m, 1H), 3.50 - 3.39 (m, 1H), 2.39 (s, 3H), 1.33 (s, 9H). | 580.1 |
| 8 | (DMSO-d6) : δ 10.59 (s, 1H), 9.98 (s, 1H), 8.92 (s, 1H), 8.34 - 8.26 (m, 1H), 7. 99 - 7.91 (m, 2H), 7.90 - 7.79 (m, 4H), 7.69 (d, J = 7.4 Hz, 1H), 7.60 - 7.46 (m, 3H), 7.39 (t, J = 7. 7 Hz, 1H), 3.64 - 3.53 (m, 8H), 3.42 - 3.33 (m, 2H), 2.48 - 2.38 (m, 5H), 1.33 (s, 9H). | 594.2 |
| 9 | (DMSO-d6) : δ 10.54 (s, 1H), 9.97 (s, 1H), 8.91 (s, 1H), 7.99 - 7.91 (m, 2H), 7.87 - 7.80 (m, 2H), 7.68 (dd, J = 7.7, 1.5 Hz, 1H), 7.59 - 7.46 (m, 3H), 7.40 - 36 (m, 3H), 4.77 (d, J = 3.9 Hz, 1H), 3.75 - 3.10 (m, 9H), 2.39 (s, 3H), 1.33 (s, 9H). | 565.2 |
| 10 | (DMSO-d6) : δ 10.55 (s, 1H), 9.97 (s, 1H), 8.91 (s, 1H), 7.99 - 7.91 (m, 2H), 7.88 - 7.81 (m, 2H), 7.68 (dd, J = 7.7, 1.4 Hz, 1H), 7.60 - 7.46 (m, 3H), 7.44 - 7.34 (m, 3H), 4.44 - 4.41 (m, 1H), 3.64 - 3.56 (m, 4H), 3 . 52 - 3.48 (m, 2H), 2.46 - 2.37 (m, 9H), 1.33 (s, 9H). | 594.2 |
| 11 | (DMSO-d6) : δ 10.58 (s, 1H), 9.98 (s, 1H), 8.93 (s, 1H), 8.33 (t, J = 5.7 Hz, 1H), 7.95 (d, J = 8.5 Hz, 2H), 7.80 - 7.90 (m, 4H), 7.69 (d, J = 7.4 Hz, 1H), 7.60 - 7.46 (m, 3H), 7.39 (t, J = 7.8 Hz, 1H), 4.71 (t, J = 5.6 Hz, 1H), 3.45 - 3.55 (m, 2H), 3.37 - 3.27 (m, 2H), 2.40 (s, 3H), 1.33 (s, 9H). | 525.2 |
| 12 | (DMSO-d6) : δ 10.45 (s, 1H), 9.95 (s, 1H), 7.99 - 7.92 (m, 2H), 7.87 (d, J = 8.2 Hz, 2H), 7.67 - 7.58 (m, 2H), 7. 59 - 7.51 (m, 2H), 7.48 (d, J = 7.3 Hz, 1H), 7.41 (d, J = 8.6 Hz, 1H), 7.36 (t, J=7.8 Hz, 1H), 3.65 - 3.55 (m, 4H), 3.52 - 42 (m, 4H), 2.52 (s, 3H), 2.37 (s, 3H), 1.33 (s, 9H). | 656.0 |
| 13 | (DMSO-d6) : δ 10.49 (s, 1H), 9.97 (s, 1H), 8.00 - 7.91 (m, 2H), 7.86 (d, J = 8.3 Hz, 2H), 7.70 - 7.64 (m, 1H), 7.59 - 7.52 (m, 2H), 7.48 (d, J = 7.5 Hz, 1H), 7.42 (d, J = 8.4 Hz, 2H), 7.37 (t, J = 7.7 Hz, 1H), 4.01 (s, 3H), 3.64 - 3.56 (m, 4H), 3.57 - 3.43 (m, 4H), 2.39 (s, 3H), 1.33 (s, 9H). | 580.9 |
| 14 | (DMSO-d6): δ 10.51 (d, J = 2.2 Hz, 1H), 9.99 - 9.94 (m, 1H), 8.90 (s, 1H), 7.99 - 7.91 (m, 2H), 7.85 - 7.77 (m, 2H), 7.68 (dd, J = 7.6, 1.5 Hz, 1H), 7.60 - 7.46 (m, 3H), 7.44 - 7.34 (m, 3H), 4.82 - 4.76 (m, 2H), 3.64-3.50 (m, 8H), 2.39 (s, 3H), 1.33 (s, 9H). | 569.0 |
| 15 | (DMSO-d6) : δ 10.59 (s, 1H), 9.98 (s, 1H), 8.93 (s, 1H), 8.48 (t, J = 5.9 Hz, 1H), 7.95 (d, J = 8.5 Hz, 2H), 7.91 - 7.81 (m, 4H), 7.73 - 7.66 (m, 1H), 7.60 - 7.46 (m, 3H), 7.39 (t, J = 7.8 Hz, 1H), 4.25 - 4.14 (m, 1H), 3.98 (dd, J = 8.3, 6.2 Hz, 1H), 3.70 (dd, J = 8.3, 5.8 Hz, 1H), 3.65 - 3.56 (m, 1H), 3.47-3.31 (m, 1H), 2.40 (s, 3H), 1.36 - 1.34 (m, 6H), 1.33 (s, 9H). | 595.2 |
| 16 | (DMSO-d6) : δ 10.61 - 10.56 (m, 1H), 9.98 (s, 1H), 9.66 (s, 1H), 8.92 (s, 1H), 7.95 (d, J = 8.5 Hz, 2H), 7.89 - 7.78 (m, 4H), 7.68 (dd, J = 7.7, 1.5 Hz, 1H), 7.60 - 7.46 (m, 3H), 7.39 (t, J = 7.8 Hz, 1H), 4.45 (s, 2H), 2.39 (s, 3H), 1.33 (s, 9H). | 496.0 |
| 17 | (DMSO-d6) : δ 10.58 (s, 1H), 9.98 (s, 1H), 8.92 (s, 1H), 8.33 - 8.25 (m, 1H), 7. 99 - 7.91 (m, 2H), 7.90 - 7.79 (m, 4H), 7.69 (d, J = 7.7 Hz, 1H), 7.60 - 7.46 (m, 3H), 7.39 (t, J = 7.8 Hz, 1H), 3.42 - 3.22 (m, 4H), 2.40 (s, 3H), 2.21 (s, 6H), 1.33 (s, 9H). | 552.0 |
| 18 | (DMSO-d6) : δ 10.59 (s, 1H), 9.97 (s, 1H), 8.93 (s, 1H), 8.30 (t, J = 5.8 Hz, 1H), 7.95 (d, J = 8.5 Hz, 2H), 7.89-7.84 (m, 4H), 7.69 (d, J = 8.1 Hz, 1H), 7.58 - 7.54 (m, 2H), 7.52 - 7. 48 (m, 1H), 7.39 (t, J = 7.8 Hz, 1H), 4.80 (d, J = 4.8 Hz, 1H), 4.56 (t, J = 5.8 Hz, 1H), 3.67-3.57 (m, 1H), 3.44 - 3.30 (m, 3H), 3.25 - 3.13 (m, 1H), 2.40 (s, 3H), 1.33 (s, 9H). | 555.0 |
| 19 | (DMSO-d6): δ10.60 - 10.55 (m, 1H), 9.98 (s, 1H), 8.93 (s, 1H), 7.95 (d, J = 8.5 Hz, 2H), 7.89-7.80 (m, 5H), 7.73 - 7.66 (m, 1H), 7.58 - 7.54 (m, 2H), 7.50 (dd, J = 7.9, 1.5 Hz, 1H), 7.39 (t, J = 7.8 Hz, 1H), 4.68 - 4.60 (m, 2H), 3.96 (dt, J = 7.9, 5.8 Hz, 1H), 3.51 (t, J = 5.8 Hz, 4H), 2.41 (s, 3H), 1.33 (s, 9H). | 555.0 |
| 20 | (MeOH-d4): δ 8.72 (s, 1H), 7.99 - 7.85 (m, 2H), 7.75 - 7.65 (m, 3H), 7.60 - 7.46 (m, 3H), 7.41 - 7.27 (m, 3H), 7.10 (t, J = 7.4 Hz, 1H), 2.45 (s, 3H), 1.36 (s, 9H). | 438.4 |

(continued)

| Ex. No. | $^1$H-NMR δ (ppm) | LCMS m/z [M+H]$^+$ |
|---|---|---|
| 21 | (MeOH-d4): δ 8.66 (s, 1H), 7.93 (d, J = 8.2 Hz, 2H), 7.67 (dd, J = 7. 8, 1. 5 Hz, 1H), 7.61 - 7. 41 (m, 5H), 7.37 (t, J = 7.8 Hz, 1H), 6.91 (d, J = 8.4 Hz, 2H), 3.78 (s, 3H), 2.43 (s, 3H), 1.37 (s, 9H). | 468.4 |
| 22 | (DMSO-d6) : δ 10.04 (s, 1H), 9.93 (s, 1H), 8.80 (s, 1H), 7.93 (d, J = 8.2 Hz, 2H), 7.67 - 7.51 (m, 4H), 7.45 (d, J = 7.5 Hz, 1H), 7.40 - 7.17 (m, 4H), 2.31 (s, 3H), 1.33 (s, 9H). | 456.4 |
| 23 | (MeOH-d4): δ 8.78 (s, 1H), 7.96 - 7.86 (m, 2H), 7.77 - 7.66 (m, 2H), 7.60 - 7.24 (m, 6H), 6.86 - 6.76 (m, 1H), 2.46 (s, 3H), 1.36 (s, 9H). | 456.4 |
| 24 | (MeOH-d4): δ 8.66 (s, 1H), 7.93 (d, J = 8.0 Hz, 2H), 7.67 (d, J = 7.8 Hz, 1H), 7.60 - 7.53 (m, 4H), 7.50 (d, J = 7.9 Hz, 1H), 7.36 (t, J = 7.8 Hz, 1H), 6.97 (d, J = 8.3 Hz, 2H), 3.85 - 3.80 (m, 4H), 3.13 - 3.08 (m, 4H), 2.44 (s, 3H), 1.37 (s, 9H). | 523.4 |
| 25 | (DMSO-d6) : δ 10.33 (s, 1H), 9.96 (s, 1H), 8.84 (s, 1H), 7.95 (d, J = 8.2 Hz, 2H), 7.73 - 7.60 (m, 3H), 7.59 - 7.52 (m, 2H), 7.48 (d, J = 7.5 Hz, 1H), 7.37 (t, J = 7.9 Hz, 1H), 7.31 - 7.18 (m, 2H), 3.59 - 3.52 (m, 4H), 3.39 (dd, J = 15.5, 8.9 Hz, 2H), 2.40 - 2.31 (m, 7H), 1.33 (s, 9H). | 537.4 |
| 26 | (MeOH-d4) : δ 8.79 (s, 1H), 8.36 (s, 1H), 8.00 - 7.85 (m, 4H), 7.77 - 7.65 (m, 2H), 7.67-7.46 (m, 5H), 7.40 (t, J = 7.7 Hz, 1H), 7.27 (d, J = 8.6 Hz, 1H), 2.47 (s, 3H), 1.37 (s, 9H). | 478.2 |
| 27 | (MeOH-d4): δ 8.73 (s, 1H), 8.14 - 8.07 (m, 1H), 7.93 (d, J = 8.5 Hz, 2H), 7.70 (d, J = 7.6 Hz, 1H), 7. 61 - 7.48 (m, 3H), 7. 42 - 7.31 (m, 1H), 7. 19 - 7.09 (m, 1H), 7.05 (d, J = 7.9 Hz, 1H), 6.97 (t, J = 7.6 Hz, 1H), 3.91 (s, 3H), 2.44 (s, 3H), 1.37 (s, 9H). | 468.6 |
| 28 | (MeOH-d4): δ 8.48 (d, J = 12.4 Hz, 1H), 7.91 (d, J = 8.2 Hz, 2H), 7.63 - 7.52 (m, 3H), 7.44 - 7.30 (m, 3H), 7. 14 - 7.07 (m, 1H), 7.04 (s, 1H), 6.93 - 6.83 (m, 1H), 2.11 (s, 3H), 1.36 (s, 9H). | 456.4 |

**Table 2-2**

| | | |
|---|---|---|
| 29 | (DMSO-d6) : δ 10.33 (s, 1H), 9.97 (s, 1H), 8.87 (s, 1H), 7.99 - 7.91 (m, 2H), 7.67 (d, J = 7.8 Hz, 1H), 7.60 - 7.50 (m, 2H), 7.52 - 7.45 (m, 2H), 7.42 - 7.30 (m, 2H), 7.25 (t, J = 8.1 Hz, 1H), 6.70 - 6.63 (m, 1H), 3.75 (s, 3H), 2.38 (s, 3H), 1.33 (s, 9H). | 468.6 |
| 30 | (DMSO-d6): δ 9.93 (s, 1H), 9.80 (s, 1H), 7.94 (d, J = 8.5 Hz, 2H), 7.87 (d, J = 8.3 Hz, 2H), 7.58 - 7.53 (m, 2H), 7.51 (dd, J = 7.6, 1.5 Hz, 1H), 7.40 (dd, J = 7. 9, 1.4 Hz, 1H), 7.35 (d, J=8.6 Hz, 2H), 7.30 (t, J = 7.7 Hz, 1H), 7.25 (s, 2H), 4.91 (t, J = 5.6 Hz, 1H), 3.96 - 3.36 (m, 9H), 2.33 (s, 3H), 1.33 (s, 9H). | 596.1 |
| 31 | (DMSO-d6) : δ 9.92 (s, 6H), 9.82 (s, 1H), 7.96 - 7.92 (m, 2H), 7.89 (d, J = 8.3 Hz, 2H), 7.58 - 7.53 (m, 2H), 7.51 (dd, J = 7.7, 1.5 Hz, 1H), 7.40 (dd, J = 7.9, 1.5 Hz, 1H), 7.37 - 7.33 (m, 2H), 7.30 (t, J = 7.7 Hz, 1H), 7.25 (s, 2H), 4.77 (s, 1H), 3.91 - 3.73 (m, 2H), 3.64 - 3.56 (m, 1H), 3.51-3.33 (m, 6H), 2.34 (s, 3H), 1.33 (s, 9H). | 596.1 |
| 32 | (DMSO-d6) : δ 9.93 (s, 1H), 9.82 (s, 1H), 7.97 - 7.92 (m, 2H), 7.89 (d, J = 8.2 Hz, 2H), 7.55 (dd, J = 8.4, 1.5 Hz, 2H), 7.51 (d, J = 7.6 Hz, 1H), 7.40 (d, J = 7.8 Hz, 1H), 7.37 - 7.33 (m, 2H), 7.31 (t, J = 7.9 Hz, 1H), 7.28-7.18 (m, 2H), 4.00-3.37 (m, 9H), 2.33 (s, 3H), 1.33 (s, 9H). | 595.1 |
| 34 | (DMSO-d6) : δ 9.92 (s, 1H), 9.43 (s, 1H), 7.94 (d, J = 8.5 Hz, 2H), 7.66 (s, 2H), 7.59 - 7.51 (m, 2H), 7.51 - 7.44 (m, 1H), 7.39 (d, J = 8.0 Hz, 1H), 7.29 (t, J = 7.7 Hz, 1H), 7.10 (s, 2H), 6.90 - 6.83 (m, 2H), 4.08-4.01 (m, 2H), 3.67 - 3.60 (m, 2H), 3.30 (s, 3H), 2.32 (s, 3H), 1.33 (s, 9H). | 527.1 |
| 35 | (MeOH-d4): δ 8.73 (s, 1H), 8.32 - 8.01 (m, 1H), 7.93 (d, J = 8.3 Hz, 2H), 7.71 (d, J = 7.7 Hz, 1H), 7. 65 - 7.48 (m, 6H), 7.49 - 7.16 (m, 1H), 2.47 (s, 3H), 1.37 (s, 9H). | 478 |
| 36 | (DMSO-d6) : δ 10.08 (s, 1H), 9.93 (s, 1H), 8.76 (s, 1H), 7.95 (d, J = 8.2 Hz, 2H), 7.72 - 7.40 (m, 6H), 7.35 (t, J = 7.9 Hz, 1H), 6.94 - 6.89 (m, 2H), 3.13 - 3.05 (m, 4H), 2.36 (s, 3H), 1.70 - 1.54 (m, 4H), 1.58 - 1.45 (m, 2H), 1.33 (s, 9H). | 521 |

(continued)

| | | |
|---|---|---|
| 37 | (MeOH-d4): δ 8.74 (s, 1H), 7.93 (d, J = 8.2 Hz, 2H), 7.75 - 7.67 (m, 3H), 7.60 - 7.47 (m, 3H), 7.42 - 7.27 (m, 3H), 3.78 - 3.66 (m, 2H), 3.09 - 2.99 (m, 1H), 2.96 (s, 3H), 2.74 - 2.62 (m, 2H), 2.45 (s, 3H), 2.16 (s, 3H), 1.37 (s, 9H). | 564 |
| 38 | (DMSO-d6): δ 9.91 (s, 1H), 9.55 (s, 1H), 7.94 (d, J = 8.5 Hz, 2H), 7. 73 (d, J=8.1 Hz, 2H), 7.68 - 7.46 (m, 3H), 7.39 (dd, J = 7.9, 1.4 Hz, 1H), 7.29 (t, J = 7.7 Hz, 1H), 7.22-7.17 (m, 2H), 7.16 - 7.05 (m, 2H), 3.64 - 3.52 (m, 8H), 3.39 (s, 2H), 2.33 (s, 3H), 1.33 (s, 9H). | 552.1 |
| 40 | (DMSO-d6) : δ 10.39 (s, 1H), 9.42 (s, 1H), 7.96 (s, 1H), 7.90 (d, J = 8.5 Hz, 2H), 7.62 (s, 2H), 7.58 (d, J = 8.5 Hz, 2H), 7.49 (dd, J = 7.8, 1.5 Hz, 1H), 7.40 (t, J = 7.8 Hz, 1H), 7.16 (s, 2H), 6.88 (d, J = 8.9 Hz, 2H), 5.69 (t, J=5.7 Hz, 1H), 4.79 (d, J=5.7 Hz, 2H), 3.76 - 3.69 (m, 4H), 3.07-3.00 (m, 4H), 1.33 (s, 9H). | 554.1 |
| 41 | (DMSO-d6): δ 9.90 (s, 1H), 9.34 (s, 1H), 7.98 - 7.90 (m, 2H), 7.64 - 7.51 (m, 4H), 7.51 - 7.44 (m, 1H), 7.38 (d, J = 7.7 Hz, 1H), 7.28 (t, J = 7.7 Hz, 1H), 7.04 (s, 2H), 6.92 - 6.83 (m, 2H), 3.77-3.69 (m, 4H), 3.07 - 3.00 (m, 4H), 2.32 (s, 3H), 1.33 (s, 9H). | 538.1 |
| 45 | (DMSO-d6) δ 9.67 (s, 1H), 7.76 (dd, J = 7.4, 1.7 Hz, 1H), 7.73 (d, J = 8.2 Hz, 2H), 7.51 - 7.42 (m, 2H), 7.41 - 7.25 (m, 2H), 7.24 - 7.18 (m, 2H), 6.95 (d, J = 1.7 Hz, 1H), 6.89 (dd, J = 12.7, 1.7 Hz, 1H), 4. 99 (s, 1H), 4.58 (d, J = 11.8 Hz, 1H), 4.35 (d, J = 11.8 Hz, 1H), 3.93 - 3.83 (m, 1H), 3.82 - 3.74 (m, 1H), 3.56 (t, J = 4.6 Hz, 4H), 3.41 (s, 2H), 3.27 - 3.15 (m, 1H), 3.11 - 3.01 (m, 1H), 2.45 - 2.25 (m, 4H), 2.05 - 1.93 (m, 1H), 1.12 - 1.00 (m, 2H), 0.86 - 0.72 (m, 2H). | 596.2 |
| 46 | (DMSO-d6) δ 9.53 (s, 1H), 8.57 (s, 1H), 7.87 (d, J = 9.2 Hz, 1H), 7.74 (d, J = 7.2 Hz, 1H), 7.52 - 7.37 (m, 2H), 7.35 - 7.00 (m, 2H), 6.95 (s, 1H), 6.89 (d, J = 12.6 Hz, 1H), 6.82 (d, J = 9.2Hz, 1H), 5.05 (s, 1H), 4.57 (dd, J = 11.9, 4.6 Hz, 1H), 4.34 (t, J = 10.8 Hz, 1H), 3.90 - 3.82 (m, 1H), 3.81 - 3.65 (m, 1H), 3.70 (t, J = 4.8 Hz, 4H), 3.37 (t, J = 4.8 Hz, 4H), 3.27 - 3.13 (m, 1H), 3.12 - 3.00 (m, 1H), 2.10 - 1.90 (m, 1H), 1.09 - 0.97 (m, 2H), 0.88 - 0.75 (m, 2H). | 583.1 |
| 47 | (DMSO-d6) δ 9.66 (s, 1H), 7.77 (dd, J = 7.4, 1.7 Hz, 1H), 7.66 (d, J = 8.2 Hz, 2H), 7.52 - 7.42 (m, 2H), 7.40 - 7.20 (m, 2H), 7.19 (d, J = 8.4 Hz, 2H), 6.95 (d, J = 1.7 Hz, 1H), 6. 89 (dd, J = 12.7, 1.7 Hz, 1H), 5.01 (s, 1H), 4.68 - 4.50 (m, 1H), 4.44 - 4.23 (m, 1H), 3.93 - 3.84 (m, 1H), 3.85 - 3.75 (m, 1H), 3.63 - 3.50 (m, 2H), 3.28 - 3.15 (m, 2H), 3.12 - 3.03 (m, 1H), 3.00 - 2.85 (m, 1H), 2.84 (s, 3H), 2.62 - 2.52 (m, 1H), 2.06 (s, 3H), 2.03 - 1.95 (m, 1H), 1.13 - 0.97 (m, 2H), 0.87 - 0.73 (m, 2H). | 623.2 |
| 48 | (DMSO-d6): δ 9.51 (s, 1H), 7.77 (d, J = 7.2 Hz, 1H), 7.57 - 7.52 (m, 1H), 7.51 - 7.41 (m, 2H), 7.40 - 7.10 (m, 3H), 6.95 (d, J = 1.6 Hz, 1H), 6.93 - 6.84 (m, 2H), 5.03 (s, 1H), 4.58 (dd, J = 11.8, 4.5 Hz, 1H), 4.34 (dd, J = 11.8, 9.5 Hz, 1H), 4.12 - 4.06 (m, 2H), 4.06 - 4.02 (m, 2H), 3.92 - 3.84 (m, 1H), 3.82 - 3.75 (m, 1H), 3.67 (t, J = 4.7 Hz, 2H), 3.65 - 3.59 (m, 2H), 3.32 (s, 6H), 3.26 - 3.14 (m, 1H), 3.12 - 3.03 (m, 1H), 2.05 - 1.93 (m, 1H), 1.10 - 0.98 (m, 2H), 0.87 - 0.70 (m, 2H). | 645.4 |
| 49 | (DMSO-d6): δ 9.74 (s, 1H), 8.63 (s, 1H), 7.97 (s, 1H), 7.80 - 7.72 (m, 3H), 7.58 - 7.30 (m, 4H), 7.27 - 7.20 (m, 2H), 6.98 - 6.85 (m, 2H), 5.35 (s, 2H), 4.98 (dd, J = 9.3, 4. 7 Hz, 1H), 4.59 (dd, J = 11.9, 4.6 Hz, 1H), 4.35 (dd, J = 11.9, 9.4 Hz, 1H), 3.94 - 3.73 (m, 2H), 3.27 - 3.15 (m, 1H), 3.13 - 3.01 (m, 1H), 2.05 - 1.94 (m, 1H), 1.10 - 0.99 (m, 2H), 0.90 - 0.77 (m, 2H). | 578.2 |
| 50 | (DMSO-d6): δ 9.70 (s, 1H), 7.82 - 7.70 (m, 4H), 7.52 - 7.40 (m, 3H), 7.41 - 7.32 (m, 1H), 7.30 - 7.21 (m, 1H), 7.21 - 7.12 (m, 2H), 6.98 - 6.85 (m, 2H), 6.26 (t, J = 2.1 Hz, 1H), 5.27 (s, 2H), 4. 99 (dd, J = 9.3, 4. 7 Hz, 1H), 4.59 (dd, J=11.8, 4.6 Hz, 1H), 4.35 (dd, J = 11.9, 9.4 Hz, 1H), 3.93 - 3.73 (m, 2H), 3.27 - 3.14 (m, 1H), 3.13 - 3.01 (m, 1H), 2.05 - 1.93 (m, 1H), 1.10 - 0.99 (m, 2H), 0.86 - 0.77 (m, 2H). | 577.2 |
| 51 | (DMSO-d6) : δ 9.73 (s, 1H), 8.68 (s, 1H), 7.99 (s, 1H), 7.87 - 7.66 (m, 3H), 7.56 - 7.33 (m, 3H), 7.28 (t, J = 7.9 Hz, 1H), 7.19 (s, 1H), 6.98 - 6.85 (m, 3H), 5.40 (s, 2H), 4.98 (dd, J = 9.3, 4. 6 Hz, 1H), 4.60 (dd, J = 11.9, 4. 6 Hz, 1H), 4.35 (dd, J = 11.9, 9.4 Hz, 1H), 3.94 - 3.74 (m, 2H), 3.21 (ddd, J = 15.1, 9.6, 5.1 Hz, 1H), 3.13 - 3.02 (m, 1H), 2.05 - 1.94 (m, 1H), 1.10 - 0.99 (m, 2H), 0.90 - 0.77 (m, 2H). | 578.2 |
| 52 | (DMSO-d6): δ 9.55 (s, 1H), 7.75 (dd, J = 7.3, 1.9 Hz, 1H), 7.60 (s, 2H), 7.48 (t, J = 7.6 Hz, 1H), 7. 44 (dd, J = 7. 8, 1.8 Hz, 1H), 7. 40 - 7.10 (m, 2H), 7.05 - 6.85 (m, 3H), 5.20 - 4.60 (m, 1H), 4.57 (d, J = 11.9 Hz, 1H), 4.35 (d, J = 11.9 Hz, 1H), 3.93 - 3.73 (m, 2H), 3.71 - 3.35 (m, 5H), 3.30 - 3.14 (m, 2H), 3.13 - 3.03 (m, 1H), 2.98 - 2.71 (m, 1H), 2.04 - 1.95 (m, 1H), 1.92 - 1.75 (m, 2H), 1.65 - 1.40 (m, 2H), 1.09 - 1.00 (m, 2H), 0.85 - 0.76 (m, 2H). | 596.1 |

**Table 2-3**

| | | |
|---|---|---|
| 53 | (DMSO-d6): δ 9.45 (s, 1H), 7.75 (dd, J = 7.4, 1.8 Hz, 1H), 7.63 - 7.51 (m, 2H), 7.47 (t, J = 7.6 Hz, 1H), 7.43 (dd, J = 8.0, 1.6 Hz, 1H), 7.35 - 7.05 (m, 2H), 6.98 - 6.82 (m, 4H), 5.06 (br.s, 1H), 4.79 (d, J = 4.6 Hz, 1H), 4.60 - 4.50 (m, 1H), 4.40 - 4.30 (m, 1H), 3.93 - 3.73 (m, 2H), 3.64 - 3.47 (m, 2H), 3.30 - 3.16 (m, 2H), 3.12 - 3.02 (m, 1H), 2.65 - 2.30 (m, 2H), 2.07 - 1.93 (m, 1H), 1.90 - 1.80 (m, 1H), 1.78 - 1.68 (m, 1H), 1.60 - 1.45 (m, 1H), 1.28 - 1.18 (m, 1H), 1.09 - 1.01 (m, 2H), 0.85 - 0.78 (m, 2H). | 596.1 |
| 54 | (DMSO-d6) δ 9.41 (s, 1H), 9.10 (s, 1H), 7.74 (dd, J = 7.4, 1.8 Hz, 1H), 7.58 - 7.37 (m, 4H), 7.23 (s, 2H), 6.95 (d, J = 1.6 Hz, 1H), 6.89 (dd, J = 12.6, 1.6 Hz, 1H), 6.77 - 6.60 (m, 2H), 5.06 (d, J = 11.9 Hz, 1H), 4.54 (d, J = 10.5 Hz, 1H), 4.33 (t, J = 10.7 Hz, 1H), 3.93 - 3.70 (m, 2H), 3.20 (s, 1H), 3.08 (dd, J = 16.1, 5.6 Hz, 1H), 2.07 - 1.92 (m, 1H), 1.13 - 0.98 (m, 2H), 0.86 - 0.69 (m, 2H). | 513.1 |
| 55 | (DMSO-d6) δ 9.53 (s, 1H), 7.75 (dd, J = 7.4, 1.8 Hz, 1H), 7. 71 - 7.53 (m, 2H), 7.53 - 7.40 (m, 2H), 7.38 - 7.02 (m, 2H), 6.95 (d, J = 1.7 Hz, 1H), 6.92 - 6.82 (m, 3H), 5.03 (s, 1H), 4.56 (dd, J = 12.0, 4.5 Hz, 1H), 4.34 (dd, J = 11.9, 9.5 Hz, 1H), 3.97 (d, J = 6.4 Hz, 1H), 3.92 - 3.72 (m, 2H), 3.29 - 3.14 (m, 1H), 3.13 - 3.00 (m, 1H), 2.40 - 2.27 (m, 3H), 2.14 (s, 6H), 2.08 - 1.91 (m, 1H), 1.90 - 1.74 (m, 2H), 1 . 15 - 0.95 (m, 2H), 0 . 90 - 0.74 (m, 2H). | 598.1 |
| 56 | (DMSO-d6) δ 9.50 (s, 1H), 8.08 (s, 1H), 7.75 (dd, J = 7.4, 1.8 Hz, 1H), 7.66 - 7.56 (m, 2H), 7.50 - 7.43 (m, 2H), 7.27 (br s, 2H), 6.96 - 6.86 (m, 4H), 5.04 (br s, 1H), 4.56 (dd, J = 11.8, 4.5 Hz, 1H), 4.34 (dd, J = 11.8, 9.6 Hz, 1H), 3.93 - 3.84 (m, 1H), 3.84 - 3.74 (m, 1H), 3.62 - 3.46 (m, 4H), 3.25 - 3.14 (m, 1H), 3.13 - 2.98 (m, 5H), 2.05 - 1.95 (m, 1H), 1.10 - 1.03 (m, 2H), 0.85 - 0.74 (m, 2H). | 609.3 |
| 57 | (DMSO-d6) δ 9.76 (s, 1H), 7.85 - 7.78 (d, J = 8.3 Hz, 2H), 7. 76 (dd, J = 7.5, 1.8 Hz, 1H), 7.48 (t, J = 7.5 Hz, 1H), 7.45 (dd, J = 7.5, 1.8 Hz, 1H), 7.35 - 7.20 (br s, 2H), 7.27 (d, J = 8.3 Hz, 2H), 6.95 (s, 1H), 6.89 (dd, J = 12.6, 1.7 Hz, 1H), 4.99 (dd, J = 9.3, 4.6 Hz, 1H), 4. 60 (dd, J = 11.9, 4. 6 Hz, 1H), 4.35 (dd, J = 11.9, 9.3 Hz, 1H), 3. 97 (s, 2H), 3. 94 - 3.84 (m, 1H), 3.84 - 3.75 (m, 1H), 3.25 - 3.16 (m, 1H), 3.14 - 3.03 (m, 1H), 2.05 - 1.95 (m, 1H), 1.09 - 1.03 (m, 2H), 0.85 - 0.77 (m, 2H). | 536.3 |
| 58 | (DMSO-d6) δ9.46 (s, 1H), 7.75 (dd, J = 7.6, 1.8 Hz, 1H), 7.68 - 7.53 (m, 2H), 7.47 (t, J = 7. 6, 1H), 7. 43 (dd, J = 7. 6, 1.8 Hz, 1H), 7.25 (br s, 2H), 6.97 - 6.85 (m, 4H), 5.05 (br s, 1H), 4.56 (dd, J = 11.9, 4.5 Hz, 1H), 4.34 (dd, J = 11.9, 9.6 Hz, 1H), 3. 92 - 3.75 (m, 2H), 3.26 - 3.15 (m, 1H), 3.07 (dt, J = 16.0, 5.2 Hz, 1H), 3.03 - 2.95 (m, 4H), 2.87 - 2.78 (m, 4H), 2.04 - 1.96 (m, 1H), 1.08 - 1.02 (m, 2H), 0.85 - 0.76 (m, 2H). | 581.4 |
| 59 | (DMSO-d6) δ9.47 (s, 1H), 7.75 (dd, J = 7.3, 1.8 Hz, 1H), 7. 64 - 7.51 (m, 2H), 7.50 - 7.40 (m, 2H), 7.25 (br s, 2H), 6.95 (d, J = 1.6 Hz, 1H), 6.92 - 6.84 (m, 3H), 5.05 (br s, 1H), 4.56 (dd, J = 11.9, 4.5 Hz, 1H), 4.34 (dd, J = 11.9, 9.6Hz, 1H), 3.92 - 3.83 (m, 1H), 3.83 - 3.75 (m, 1H), 3.26 - 3.15 (m, 1H), 3.15 - 3.03 (m, 5H), 2.48 - 2.40 (m, 4H), 2.22 (s, 3H), 2.03 - 1.95 (m, 1H), 1.10 - 0.98 (m, 2H), 0.86 - 0.78 (m, 2H). | 595.4 |
| 60 | (DMSO-d6) δ 9.72 (s, 1H), 7.91 (s, 1H), 7.75 (dd, J = 7.4, 1.8 Hz, 1H), 7. 54 - 7.43 (m, 2H), 7.43 - 7.23 (m, 2H), 7.16 (t, J = 9.3 Hz, 1H), 6.95 (d, J = 1.6 Hz, 1H), 6.89 (dd, J = 12.6, 1.7 Hz, 1H), 4.96 (d, J = 7.6 Hz, 1H), 4.59 (dd, J = 12.0, 4.6 Hz, 1H), 4.45 (dt, J = 8.7, 4. 5 Hz, 1H), 4.35 (dd, J = 11.9, 9.3 Hz, 1H), 4.02 - 3.61 (m, 5H), 3.45 (ddd, J = 11.9, 9.3, 2.9 Hz, 2H), 3.21 (ddd, J = 15.0, 9.6, 5.0 Hz, 1H), 3.07 (dt, J = 15.9, 5.2 Hz, 1H), 2.07 - 1.83 (m, 3H), 1.59 (dtd, J = 13.0, 9.0, 4.0 Hz, 2H), 1.11 - 0.98 (m, 2H), 0.87 - 0.71 (m, 2H). | 615.1 |
| 61 | (DMSO-d6) δ 12.57 (s, 1H), 9.69 -9.38 (m, 1H), 8.05 (s, 1H), 7.87 - 7.71 (m, 1H), 7.70 - 7.52 (m, 1H), 7.52 - 7.40 (m, 1H), 7.30 (s, 1H), 6. 95 (d, J = 1.6 Hz, 1H), 6.89 (dd, J = 12.7, 1.7 Hz, 1H), 5.25 - 4.91 (m, 1H), 4.54 (dd, J = 12.0, 4.5 Hz, 1H), 4.34 (dd, J = 11.9, 9.7 Hz, 1H), 3.99 - 3.73 (m, 2H), 3.21 (ddd, J = 15.1, 9.6, 5. 1 Hz, 1H), 3.08 (dt, J = 15.9, 5.2 Hz, 1H), 2.00 (ddd, J = 13.3, 8.6, 4.9 Hz, 1H), 1.12 - 0.98 (m, 3H), 0 . 90 - 0.72 (m, 3H). | 487.1 |
| 62 | (DMSO-d6) δ 9.66 - 9.35 (m, 1H), 8.17 (s, 1H), 7.75 (dd, J = 7.4, 1.8 Hz, 1H), 7.71 - 7.58 (m, 2H), 7. 57 - 7.40 (m, 2H), 7.39 - 7.01 (m, 2H), 6.97 - 6.93 (m, 1H), 6.92 - 6.85 (m, 3H), 5.13 - 4.94 (m, 1H), 4.62 - 4.51 (m, 1H), 4.41 - 4.28 (m, 1H), 4.09 - 4.03 (m, 2H), 3.93 - 3.83 (m, 1H), 3.83 - 3.73 (m, 1H), 3.60-3.55 (m, 4H), 3.13 - 3.06 (m, 2H), 2.71 - 2.64 (m, 2H), 2.48 - 2.44 (m, 4H), 2.05 - 1.94 (m, 1H), 1.09 - 1.01 (m, 2H), 0.85 - 0.77 (m, 2H). | 626.1 |

(continued)

| | | |
|---|---|---|
| 63 | (DMSO-d6) δ 9.55 (s, 1H), 7.75 (dd, J = 7.4, 1.8 Hz, 1H), 7.72 - 7.57 (m, 2H), 7.50 - 7.42 (m, 2H), 7.38 - 7.05 (m, 2H), 6.95 (s, 1H), 6.93 - 6.84 (m, 3H), 5.15 - 4.95 (m, 1H), 4.61 - 4.51 (m, 2H), 4.38 - 4.30 (m, 1H), 4.05 - 3.96 (m, 2H), 3.92 - 3.74 (m, 2H), 3.59-3.52 (m, 2H), 3.22 - 3.16 (m, 1H), 3.12 - 3.03 (m, 1H), 2.05 - 1.96 (m, 1H), 1.89 - 1.80 (m, 2H), 1.09 - 1.04 (m, 2H), 0.84 - 0.79 (m, 2H).. | 571.1 |
| 64 | (DMSO-d6) δ9.69 (s, 1H), 7.77 - 7.70 (m, 3H), 7.50 - 7.42 (m, 2H), 7.36 (br s, 2H), 7.19 (d, J = 8.5 Hz, 2H), 6.95 (d, J = 1.6 Hz, 1H), 6.89 (dd, J = 12.7, 1.6 Hz, 1H), 4.99 (dd, J = 9.4, 4.6 Hz, 1H), 4. 59 (dd, J = 12.0, 4.6 Hz, 1H), 4.41 - 4.30 (m, 1H), 4.23 (s, 2H), 3.93 - 3.83 (m, 1H), 3.83 - 3.75 (m, 1H), 3.26 - 3.15 (m, 1H), 3.12 - 3.03 (m, 1H), 2.04 - 1.96 (m, 1H), 1.09 - 1.02 (m, 2H), 0.84 - 0.77 (m, 2H). | 579.3 |
| 65 | (DMSO-d6) : δ 10.38 (s, 1H), 9.34 (s, 1H), 9.10 (s, 1H), 7.95 (d, J = 8.0 Hz, 1H), 7.92 - 7.87 (m, 2H), 7.62 - 7.54 (m, 2H), 7.52 - 7.45 (m, 3H), 7.40 (t, J = 7.9 Hz, 1H), 7.12 (s, 2H), 6.69 (d, J = 8.5 Hz, 2H), 5.66 (t, J = 5.7 Hz, 1H), 4.77 (d, J = 5.8 Hz, 2H), 1.33 (s, 9H). | 485.2 |
| 66 | (DMSO-d6): δ 9.83 (s, 1H), 9.80 (s, 1H), 7.88 (d, J = 8.2 Hz, 2H), 7.66 (s, 1H), 7.50 (d, J = 7.5 Hz, 1H), 7.38 - 7.32 (m, 3H), 7.31 - 7.15 (m, 3H), 3.66 - 3.55 (m, 4H), 3.55 - 3.43 (m, 4H), 2.80 - 2.71 (m, 2H), 2.65 - 2.57 (m, 2H), 2.31 (s, 3H), 1.85 - 1.70 (m, 4H). | 570.2 |
| 67 | (DMSO-d6) : δ 9.84 (s, 1H), 9.80 (s, 1H), 7.99 (d, J = 8.8 Hz, 2H), 7.89 (d, J = 8.2 Hz, 2H), 7.50 (d, J = 7.7 Hz, 1H), 7.39 (d, J = 7.6 Hz, 1H), 7.34 (d, J = 8.4 Hz, 2H), 7.32 - 7.14 (m, 3H), 7.07 (d, J = 8.7 Hz, 2H), 3.84 (s, 3H), 3.64 - 3.56 (m, 4H), 3.55 - 3.44 (m, 4H), 2.33 (s, 3H). | 540.2 |
| 68 | (DMSO-d6): δ 10.66 - 10.58 (m, 1H), 10.06-9.99 (m, 1H), 9.80 (s, 1H), 8.00 - 7.89 (m, 3H), 7.88 - 7.80 (m, 1H), 7.58 - 7.50 (m, 3H), 7.43 - 7.13 (m, 3H), 2.23 (s, 3H), 1.33 (s, 9H). | 428.2 |
| 69 | (DMSO-d6) : δ 9.90 (s, 1H), 9.27 (s, 1H), 9.08 (s, 1H), 7.94 (d, J = 8.5 Hz, 2H), 7.62 - 7.41 (m, 5H), 7.42 - 7.34 (m, 1H), 7.28 (t, J = 7.7 Hz, 1H), 7.02 (s, 2H), 6.72 - 6.64 (m, 2H), 2.32 (s, 3H), 1.33 (s, 9H). | 469.2 |

**Table 2-4**

| | | |
|---|---|---|
| 70 | (DMSO-d6) : δ 10.39 (s, 1H), 9.45 (s, 1H), 8.00 - 7.93 (m, 1H), 7. 93 - 7.85 (m, 2H), 7.63 (s, 2H), 7.61 - 7.53 (m, 2H), 7.52 - 7.46 (m, 1H), 7.45 - 7.37 (m, 1H), 7.16 (d, J = 14.8 Hz, 2H), 6.89 - 6.81 (m, 2H), 5.68 (t, J = 5.7 Hz, 1H), 4.79 (d, J = 5.7 Hz, 2H), 3.99 - 3.91 (m, 2H), 2.38 - 2.29 (m, 2H), 2.13 (s, 6H), 1.87 - 1.76 (m, 2H), 1.33 (s, 9H). | 570.3 |
| 71 | (DMSO-d6) : δ 10.50 (s, 1H), 9.81 (s, 1H), 8.06 - 8.02 (m, 1H), 8.01 - 7.96 (m, 1H), 7.94 - 7.85 (m, 3H), 7.79 - 7.73 (m, 1H), 7.50 (d, J = 7.5 Hz, 1H), 7.41 (d, J = 8.0 Hz, 1H), 7.37 - 7.18 (m, 5H), 3.64 - 3.56 (m, 4H), 3.55 - 3.44 (m, 4H), 2.34 (s, 3H). | 602.2 |
| 72 | (DMSO-d6) : δ 10.13 (s, 1H), 9.80 (s, 1H), 7.92 - 7.84 (m, 3H), 7.53 (d, J = 7.3 Hz, 1H), 7.40 - 7.16 (m, 7H), 3.66 - 3.55 (m, 4H), 3.55 - 3.42 (m, 4H), 2.32 (s, 3H). | 550.1 |
| 73 | (DMSO-d6) : δ 9.50 (s, 1H), 7.78 (dd, J = 5.1, 4.1 Hz, 1H), 7.67 - 7.54 (m, 2H), 7.51 - 7.48 (m, 2H), 7.35 - 7.10 (m, 3H), 6.89 (d, J = 9.0 Hz, 2H), 5.13 - 4.99 (m, 1H), 4.81 (s, 2H), 4.42 (d, J = 6.8 Hz, 2H), 3.77 - 3.70 (m, 4H), 3.08 - 3.02 (m, 4H), 1.42 (s, 9H). | 572.3 |
| 74 | (DMSO-d6) : δ 10.39 (s, 1H), 9.47 (s, 1H), 7.92 - 7.88 (m, 2H), 7.65 (s, 2H), 7.60 - 7.56 (m, 2H), 7.52 - 7.45 (m, 1H), 7.40 (t, J = 7.8 Hz, 1H), 7.28 - 7.04 (m, 2H), 6.95 - 6.79 (m, 3H), 5.68 (t, J = 5.7 Hz, 1H), 4.79 (d, J = 5.7 Hz, 2H), 4.05 (t, J = 4.7 Hz, 2H), 3.64 (t, J = 4.6 Hz, 2H), 3.30 (s, 3H), 1.33 (s, 9H). | 543.3 |
| 75 | (DMSO-d6): δ 10.30 (s, 1H), 9.81 - 9.54 (m, 1H), 8.14 - 7.99 (m, 2H), 7.96 - 7.81 (m, 4H), 7.63 - 7.47 (m, 3H), 7.45 - 7.32 (m, 3H), 5.50 (s, 1H), 5.03 - 4.73 (m, 2H), 3.65 - 3.56 (m, 4H), 3.53 - 3.46 (m, 4H), 2.91 (s, 3H), 1.33 (s, 9H). | 596.2 |
| 76 | (DMSO-d6) : δ 9.47 (s, 1H), 8.46 (s, 1H), 8.17 (d, J = 8.2 Hz, 1H), 7.86 (dd, J = 7.6, 1.6 Hz, 1H), 7.71 (d, J = 1.8 Hz, 1H), 7.68 - 7.44 (m, 5H), 7.22 (s, 2H), 6.89 (d, J = 9.1 Hz, 2H), 5.16 - 4.90 (m, 1H), 4.52 - 4.19 (m, 2H), 3.81-3.67 (m, 4H), 3.11 - 2.98 (m, 4H), 2.25 - 2.15 (m, 1H), 1.21 - 1.11 (m, 2H), 0.95 - 0.86 (m, 2H). | 563.3 |
| 77 | (DMSO-d6): δ 9.68 (s, 1H), 7.82 - 7.76 (m, 1H), 7.76 - 7.69 (m, 2H), 7.53 - 7.47 (m, 2H), 7.37 (s, 2H), 7.21 (d, J = 8.5 Hz, 2H), 7.16 (s, 1H), 5.00 (s, 1H), 4.82 (s, 2H), 4.44 (d, J = 6.9 Hz, 2H), 3.60 - 3.53 (m, 4H), 3.41 (s, 2H), 2.37 - 2.30 (m, 4H), 1.42 (s, 9H). | 586.3 |

(continued)

| | | |
|---|---|---|
| 78 | (DMSO-d6) : δ 10.39 (s, 1H), 9.46 (s, 1H), 7.97 (d, J = 7.9 Hz, 1H), 7.94 - 7.86 (m, 2H), 7.71 - 7.16 (m, 2H), 7.62 - 7.54 (m, 2H), 7.52 - 7.45 (m, 1H), 7.40 (t, J = 7.8 Hz, 1H), 7.16 (s, 2H), 6.91 - 6.84 (m, 2H), 5.68 (t, J = 5.7 Hz, 1H), 4.79 (d, J = 5.7 Hz, 2H), 4.04 (t, J = 5.8 Hz, 2H), 3.57 (t, J = 4.7 Hz, 4H), 2.69 - 2.64 (m, 2H), 2.50 - 2.42 (m, 4H), 1.33 (s, 9H). | 598.3 |
| 79 | (DMSO-d6) : δ 10.39 (s, 1H), 9.60 (s, 1H), 7.99 (d, J = 7.9 Hz, 1H), 7.93 - 7.87 (m, 2H), 7.73 (d, J = 8.1 Hz, 2H), 7.61 - 7.56 (m, 2H), 7.50 (dd, J = 7.8, 1.4 Hz, 1H), 7.41 (t, J = 7.8 Hz, 1H), 7.31 - 7.09 (m, 4H), 5.67 (t, J = 5.6 Hz, 1H), 4.81 (d, J = 5.6 Hz, 2H), 3.56 (t, J = 4.6 Hz, 4H), 3.40 (s, 2H), 2.39 - 2.25 (m, 4H), 1.33 (s, 9H). | 568.3 |
| 80 | (DMSO-d6) : δ 9.90 (s, 1H), 9.30 (s, 1H), 8.01 - 7.87 (m, 2H), 7.62 - 7.43 (m, 5H), 7.38 (d, J = 7.8 Hz, 1H), 7.28 (t, J = 7.8 Hz, 1H), 7.11 - 6.94 (m, 2H), 6.86 (d, J = 8.7 Hz, 2H), 3.68-3.51 (m, 6H), 2.64 - 2.54 (m, 2H), 2.49 - 2.44 (m, 4H), 2.32 (s, 3H), 2.28 - 2.16 (m, 1H), 1.89 - 1.79 (m, 2H), 1.56 - 1.40 (m, 2H), 1.33 (s, 9H). | 621.4 |
| 81 | (DMSO-d6) : δ 9.89 (s, 1H), 9.30 (s, 1H), 7.94 (d, J = 8.1 Hz, 2H), 7.62 - 7.51 (m, 4H), 7.48 (d, J = 7.6 Hz, 1H), 7.38 (d, J = 7.9 Hz, 1H), 7.28 (t, J = 7.7 Hz, 1H), 7.10 - 6.91 (m, 2H), 6.84 (d, J = 8.7 Hz, 2H), 4.77 (d, J = 4.7 Hz, 1H), 3.64 - 3.54 (m, 1H), 3.53 - 3.45 (m, 1H), 3.42 - 3.35 (m, 1H), 2.62 - 2.55 (m, 1H), 2.46 - 2.38 (m, 1H), 2.32 (s, 3H), 1.93 - 1.82 (m, 1H), 1.80 - 1.68 (m, 1H), 1.60 - 1.45 (m, 1H), 1.33 (s, 9H), 1.26 - 1.19 (m, 1H). | 552.3 |
| 82 | (Methanol-d4): δ 7.92 (d, J = 8.5 Hz, 2H), 7.67 - 7.60 (m, 3H), 7.59 - 7.49 (m, 6H), 7.47 (d, J = 8.0 Hz, 1H), 7.34 (t, J = 7.8 Hz, 1H), 2.41 (s, 3H), 1.37 (d, J = 3.3 Hz, 9H). | 443.2 |
| 83 | (DMSO-d6) δ 9.66 (s, 1H), 7.87 - 7.75 (m, 1H), 7. 71 - 7. 60 (m, 2H), 7.54 - 7.46 (m, 2H), 7.43 - 7.21 (m, 2H), 7.21 - 7.14 (m, 3H), 5.15 - 4.93 (m, 1H), 4.82 (s, 2H), 4.45 (d, J = 6.9 Hz, 2H), 3.69 - 3.49 (m, 2H), 3.27 - 3.20 (m, 1H), 2.97 (dt, J = 11.9, 3.6 Hz, 1H), 2.84 (s, 3H), 2.61 - 2.50 (m, 1H), 2.06 (s, 3H), 1.42 (s, 9H). | 613.0 |
| 84 | (DMSO-d6) δ 12.39 (s, 1H), 12.19 (s, 1H), 9.82 (s, 1H), 8.46 - 8.39 (m, 1H), 8.37 (d, J = 7.2 Hz, 1H), 7.89 (d, J = 8.3 Hz, 2H), 7.66 - 7.55 (m, 3H), 7.38 - 7.22 (m, 3H), 6.50 (dd, J = 7.7, 2.0 Hz, 1H), 3.67 - 3.56 (m, 4H), 3.57 - 3.42 (m, 4H), 2.45 (s, 3H), 1.33 (s, 9H). | 583.3 |
| 85 | (DMSO-d6): δ 9.90 (s, 1H), 9.32 (s, 1H), 7.94 (d, J = 8.4 Hz, 2H), 7.64 - 7.52 (m, 4H), 7.48 (d, J = 7.5 Hz, 1H), 7.38 (d, J = 7.8 Hz, 1H), 7.28 (t, J = 7.7 Hz, 1H), 7.13 - 6.94 (m, 2H), 6.87 (d, J = 8.7 Hz, 2H), 3.74 - 3.63 (m, 2H), 3.48 (d, J = 11.6 Hz, 2H), 2.33 (s, 3H), 2.24 - 2.14 (m, 2H), 1.33 (s, 9H), 1.14 (d, J = 6.2 Hz, 6H). | 566.3 |
| 86 | (DMSO-d6) δ 9.89 (s, 1H), 9.19 (s, 1H), 7.94 (d, J = 8.5 Hz, 2H), 7. 58 - 7.43 (m, 5H), 7.38 (d, J = 7.7 Hz, 1H), 7.28 (t, J = 7.7 Hz, 1H), 6.97 (s, 2H), 6.67 (d, J = 8.6 Hz, 2H), 3.74 - 3.64 (m, 2H), 3.59 - 3.48 (m, 6H), 2.32 (s, 3H), 1.92 - 1.84 (m, 2H), 1.33 (s, 9H). | 552.3 |
| 87 | (DMSO-d6) δ 9.90 (s, 1H), 9.31 (s, 1H), 7.94 (d, J = 8.2 Hz, 2H), 7.66 - 7.43 (m, 5H), 7.38 (d, J = 7.8 Hz, 1H), 7.28 (t, J = 7.7 Hz, 1H), 7.13 - 6.93 (m, 2H), 6.86 (d, J = 8.6 Hz, 2H), 4.65 (d, J = 4.2 Hz, 1H), 3.66 - 3.52 (m, 1H), 3.49 - 3.39 (m, 2H), 2.82 - 2.69 (m, 2H), 2.32 (s, 3H), 1.87 - 1.74 (m, 2H), 1.56 - 1.40 (m, 2H), 1.33 (s, 9H). | 552.3 |
| 88 | (DMSO-d6) δ 10.75 (s, 1H), 10.10 (s, 1H), 9.25 (s, 2H), 8.79 (s, 1H), 7.96 (d, J = 8.3 Hz, 2H), 7.71 - 7.38 (m, 7H), 7.01 (d, J = 8.5 Hz, 2H), 3.42 - 3.30 (m, 4H), 3.27 - 3.16 (m, 4H), 2.36 (s, 3H), 1.33 (s, 9H). | 537.3 |
| 89 | (DMSO-d6) : δ 10.39 (s, 1H), 9.59 (s, 1H), 7.99 (d, J = 7.9 Hz, 1H), 7.94 - 7.86 (m, 2H), 7.70 - 7.62 (m, 2H), 7.62 - 7.54 (m, 2H), 7.50 (dd, J = 7.7, 1.5 Hz, 1H), 7.41 (t, J = 7.9 Hz, 1H), 7.25 - 7.13 (m, 4H), 5.68 (t, J = 5.6 Hz, 1H), 4.82 (d, J = 5.5 Hz, 2H), 3.64-3.55 (m, 2H), 3.01 - 2.92 (m, 1H), 2.84 (s, 3H), 2.05 (s, 3H), 1.81 - 1.72 (m, 2H), 1.33 (s, 9H). | 595.3 |

**Table 2-5**

| | | |
|---|---|---|
| 90 | (DMSO-d6) : δ 10.39 (s, 1H), 9.45 (s, 1H), 7.96 (d, J = 8.1 Hz, 1H), 7.92 - 7.88 (m, 2H), 7.68 - 7. 48 (m, 5H), 7.41 (t, J = 7.9 Hz, 1H), 7.24 - 7.19 (m, 2H), 6.89 (d, J = 8.8 Hz, 1H), 5.68 (t, J = 5.7 Hz, 1H), 4.79 (d, J = 5.7 Hz, 2H), 4.11 - 4.01 (m, 4H), 3.68 - 3.59 (m, 4H), 3.32 (s, 6H), 1.33 (s, 9H). | 617.3 |

(continued)

| | | |
|---|---|---|
| 91 | (DMSO-d6) : δ 10.37 (s, 1H), 9.49 (s, 1H), 7.96 - 7.88 (m, 3H), 7. 61 - 7.50 (m, 3H), 7.41 (t, J = 7.8 Hz, 1H), 7.34 - 7.15 (m, 4H), 5.64 (t, J = 5.8 Hz, 1H), 4.80 (d, J = 5.9 Hz, 2H), 3.76 (s, 6H), 3.62 (s, 3H), 1.33 (s, 9H). | 559.3 |
| 92 | (DMSO-d6) : δ 9.90 (s, 1H), 9.34 (s, 1H), 7.94 (d, J = 8.5 Hz, 2H), 7.67 - 7.52 (m, 4H), 7.48 (d, J = 7.6 Hz, 1H), 7.39 (d, J = 7.9 Hz, 1H), 7.28 (t, J = 7.8 Hz, 1H), 7.15 - 6.94 (m, 2H), 6.87 (d, J = 8.7 Hz, 2H), 4.75 (t, J = 5.7 Hz, 1H), 3.97 - 3.87 (m, 1H), 3.68 - 3.36 (m, 6H), 2.67 - 2.56 (m, 1H), 2.41 - 2.27 (m, 4H), 1.33 (s, 9H). | 568.3 |
| 93 | (DMSO-d6): δ 9.90 (s, 1H), 9.32 (s, 1H), 7.94 (d, J = 8.1 Hz, 2H), 7.55 (d, J = 8.2 Hz, 4H), 7.47 (d, J = 7.6 Hz, 1H), 7.38 (d, J = 7.7 Hz, 1H), 7.28 (t, J = 7.7 Hz, 1H), 7.14-6.93 (m, 2H), 6.86 (d, J = 8.6 Hz, 2H), 4.51 - 4.37 (m, 1H), 3.59 - 3.48 (m, 2H), 3.16 - 2.96 (m, 4H), 2.64 - 2.39 (m, 6H), 2.32 (s, 3H), 1.33 (s, 9H). | 581.3 |
| 94 | (DMSO-d6) : δ 10.39 (s, 1H), 9.58 (s, 1H), 7.99 (d, J = 8.0 Hz, 1H), 7.94 - 7.86 (m, 2H), 7.71 (d, J = 8.1 Hz, 2H), 7.62 - 7.55 (m, 2H), 7.50 (dd, J = 7.8, 1.4 Hz, 1H), 7.41 (t, J = 7.9 Hz, 1H), 7.28 - 7.14 (m, 4H), 5.67 (t, J = 5.6 Hz, 1H), 4.81 (d, J = 5.4 Hz, 2H), 4.50 (d, J = 4.2 Hz, 1H), 3.49 - 3.37 (m, 1H), 3.36 (s, 2H), 2.70 - 2.60 (m, 2H), 2.05 - 1.94 (m, 2H), 1.72 - 1.64 (m, 2H), 1.43 - 1.35 (m, 2H), 1.33 (s, 9H). | 582.3 |
| 95 | (DMSO-d6) : δ 9.98 (s, 1H), 9.50 (s, 1H), 7.88 (s, 1H), 7.60 (s, 2H), 7.54 - 7.47 (m, 2H), 7.45 - 7.29 (m, 3H), 7.23 - 7.18 (m, 2H), 6.89 (d, J = 8.9 Hz, 2H), 4.74 (s, 2H), 3.77 - 3.70 (m, 4H), 3.08 - 3.01 (m, 4H), 2.47 (s, 3H), 1.31 (s, 9H). | 568.2 |
| 96 | (DMSO-d6) : δ 9.58 (s, 1H), 9.37 (s, 1H), 7.97 - 7.88 (m, 3H), 7.66 - 7.52 (m, 4H), 7.43 (d, J = 7.7 Hz, 1H), 7.19 (t, J = 7.9 Hz, 1H), 7.07 (s, 2H), 6.91 - 6.84 (m, 2H), 3.77 - 3.69 (m, 7H), 3.07 - 3.00 (m, 4H), 1.33 (s, 9H). | 554.2 |
| 97 | (DMSO-d6) : δ 10.31 (s, 1H), 9.41 (s, 1H), 7.81 (d, J = 7.9 Hz, 1H), 7.67 (d, J = 3.9 Hz, 1H), 7.60 (s, 2H), 7.51 (dd, J = 7. 7, 1.4 Hz, 1H), 7.39 (t, J = 7.8 Hz, 1H), 7.16 (s, 2H), 7.03 (d, J = 3.8 Hz, 1H), 6.93 - 6.85 (m, 2H), 5.63 (t, J = 5.9 Hz, 1H), 4.74 (d, J = 5.8 Hz, 2H), 3.77 - 3.69 (m, 4H), 3.08 - 3.00 (m, 4H), 1.38 (s, 9H). | 560.2 |
| 98 | (Chloroform-d) δ 8.02 (dd, J = 7.5, 1.6 Hz, 2H), 7.59 - 7.39 (m, 4H), 7.05 (d, J = 8.3 Hz, 1H), 7.02 - 6.84 (m, 4H), 5.33 - 5.04 (m, 2H), 4.62 (s, 2H), 4. 14 - 3.98 (m, 1H), 3.98 - 3.91 (m, 1H), 3.91 - 3.82 (m, 4H), 3.38 - 3.28 (m, 1H), 3.19 - 3.11 (m, 4H), 3.02 (d, J = 15.6 Hz, 1H), 1.99 - 1.90 (m, 1H), 1.11 - 0.99 (m, 2H), 0.81 - 0.75 (m, 2H). | 564.3 |
| 99 | (DMSO-d6) : δ 10.32 (s, 1H), 9.56 (s, 1H), 8.12 (d, J = 9.2 Hz, 1H), 8.00 (d, J = 3.0 Hz, 1H), 7.97 (dd, J = 8.0, 1.4 Hz, 1H), 7.90 (d, J = 8.5 Hz, 2H), 7.58 (d, J = 8.5 Hz, 2H), 7.51 (dd, J = 7.8, 1.4 Hz, 1H), 7. 44 - 7.36 (m, 2H), 7.33 - 7.17 (m, 2H), 5.69 - 5.62 (m, 1H), 4.77 (d, J = 5.2 Hz, 2H), 3.78 - 3.71 (m, 4H), 3.13 - 3.07 (m, 4H), 1.33 (s, 9H). | 555.4 |
| 100 | (Chloroform-d) δ 8.06 (dd, J = 7.4, 1.9 Hz, 1H), 7.88 (d, J = 8.1 Hz, 1H), 7.60 - 7.39 (m, 7H), 7.17 - 6.97 (m, 1H), 6.92 (d, J = 8.9 Hz, 2H), 5.28 (s, 1H), 4.92 (s, 2H), 4.51 (s, 2H), 3.97 - 3.72 (m, 5H), 3.23 - 3.10 (m, 4H), 1.40 (s, 9H). | 566.0 |
| 101 | (DMSO-d6) : δ 10.27 (s, 1H), 9.40 (s, 1H), 7.87 (s, 1H), 7.62 - 7.57 (m, 2H), 7.53 - 7.45 (m, 2H), 7.38 (t, J = 7.9 Hz, 1H), 7.14 (s, 2H), 6.88 (d, J = 9.1 Hz, 2H), 5.67 (t, J = 5.7 Hz, 1H), 4.76 (d, J = 5.8 Hz, 2H), 3.77 - 3.69 (m, 4H), 3.07 - 3.00 (m, 4H), 2.77 (t, J = 5. 8 Hz, 2H), 2.66 - 2.58 (m, 2H), 1.85 - 1.71 (m, 4H). | 558.2 |
| 102 | (DMSO-d6) : δ 10.33 (s, 1H), 9.58 (s, 1H), 7.95 - 7.89 (m, 1H), 7.70 - 7.37 (m, 8H), 7.22 (d, J = 7.9 Hz, 1H), 6.90 (d, J = 8.7 Hz, 2H), 4.77 (s, 2H), 3.77 - 3.70 (m, 4H), 3.09 - 3. 02 (m, 4H), 2.85 - 2.75 (m, 4H), 1.83 - 1.72 (m, 4H). | 552.2 |
| 103 | (DMSO-d6) : δ 10.43 (s, 1H), 9.43 (s, 1H), 8.39 (s, 1H), 7.70 (d, J = 7.9 Hz, 1H), 7.64 - 7.54 (m, 3H), 7.41 (t, J = 7.9 Hz, 1H), 7.18 (s, 2H), 6.92 - 6.85 (m, 2H), 5.52 (s, 1H), 4.70 (s, 2H), 3.77 - 3.69 (m, 4H), 3.08 - 3.00 (m, 4H), 1.42 (s, 9H). | 561.1 |

(continued)

| | | |
|---|---|---|
| 104 | (DMSO-d6): δ 10.39 (s, 1H), 9.42 (s, 1H), 8.00 - 7.92 (m, 1H), 7.90 (d, J = 8.5 Hz, 2H), 7.69 - 7.56 (m, 4H), 7.49 (dd, J = 7.8, 1.4 Hz, 1H), 7.40 (t, J = 7.8 Hz, 1H), 7.16 s, 2H), 6.90 (d, J = 9.0 Hz, 2H), 5.68 (t, J = 5.6 Hz, 1H), 4.79 (d, J = 5.6 Hz, 2H), 3.50 - 3.40 (m, 4H), 3.05 - 2.95 m, 4H), 1.42 (s, 9H), 1.33 (s, 9H). | 653.3 |
| 105 | (DMSO-d6) : δ 10.39 (s, 1H), 9.57 (s, 1H), 7.98 (d, J = 7.8 Hz, 1H), 7.94 - 7.85 (m, 2H), 7.75 - 7.67 (m, 2H), 7.67 - 7.52 (m, 4H), 7.50 (dd, J = 7.8, 1.4 Hz, 1H), 7.41 (t, J = 7.8 Hz, 1H), 7.20 (d, J = 8.4 Hz, 2H), 5.67 (t, J = 5.7 Hz, 1H), 4.81 (d, J = 5.7 Hz, 2H), 3.57 (s, 2H), 3.39 (t, J = 6.1 Hz, 4H), 3.20 (s, 6H), 2.61 (t, J = 6.1 Hz, 4H), 1.33 (s, 9H). | 614.3 |
| 106 | (DMSO-d6) : δ 10.38 (s, 1H), 9.39 (s, 1H), 8.02 - 7.94 (m, 1H), 7.93 - 7.82 (m, 2H), 7.76 - 7.53 (m, 4H), 7.49 (dd, J = 7.8, 1.4 Hz, 1H), 7.40 (t, J = 7.8 Hz, 1H), 7.13 (s, 2H), 6.88 (d, J = 9.0 Hz, 2H), 5.68 (t, J = 5.7 Hz, 1H), 4.80 (d, J = 5.7 Hz, 2H), 4.73 (t, J = 5.7 Hz, 1H), 3.96 - 3.88 (m, 1H), 3.66 - 3.57 (m, 1H), 3.57 - 3.38 (m, 4H), 2.66 - 2.58 (m, 1H), 2.41 - 2.33 (m, 1H), 1.33 (s, 9H). | 584.3 |
| 107 | (DMSO-d6) : δ 10.38 (s, 1H), 9.36 (s, 1H), 8.02 - 7.94 (m, 1H), 7.93 - 7.86 (m, 2H), 7.68 - 7.51 (m, 4H), 7.49 (dd, J = 7.8, 1.5 Hz, 1H), 7.40 (t, J = 7.8 Hz, 1H), 7.12 (s, 2H), 6.86 (d, J = 9.0 Hz, 2H), 5.68 (t, J = 5.7 Hz, 1H), 4.79 (d, J = 5.7 Hz, 2H), 4.64 (d, J = 4.2 Hz, 1H), 3.64 - 3.54 (m, 1H), 3 . 50 - 3.38 (m, 2H), 2.83 - 2.70 (m, 2H), 1.85 - 1.75 (m, 2H), 1.53 -1.40 (m, 2H), 1.33 (s, 9H). | 568.3 |
| 108 | (DMSO-d6) : δ 10.31 (s, 1H), 9.82 (s, 1H), 7.89 (d, J = 8.3 Hz, 2H), 7.53 (d, J = 7.5 Hz, 1H), 7.40 (d, J = 7.8 Hz, 1H), 7.39 - 7.15 (m, 5H), 6.68 (s, 1H), 3.65 - 3.55 (m, 4H), 3.55 - 3.40 (m, 4H), 2.33 (s, 3H), 1.36 (s, 9H). | 557.3 |
| 109 | (DMSO-d6) : δ 9.80 (s, 1H), 9.74 (s, 1H), 7.89 (d, J = 8.2 Hz, 2H), 7.51 (d, J = 7.6 Hz, 1H), 7.41 (d, J = 7.7 Hz, 1H), 7.37 - 7.28 (m, 3H), 7.27 - 7. 19 (m, 2H), 7.18 (d, J = 3.4 Hz, 1H), 6.30 (d, J = 3.4 Hz, 1H), 3.66 - 3.55 (m, 4H), 3.56 - 3.43 (m, 4H), 2.33 (s, 3H), 1.32 (s, 9H). | 556.3 |

**Table 2-6**

| | | |
|---|---|---|
| 110 | (DMSO-d6): δ 10.11 (s, 1H), 9.37 - 9.17 (m, 1H), 9.08 (s, 1H), 7.91 (d, J = 8.1 Hz, 2H), 7.58 - 7.42 (m, 7H), 7.07 - 6.93 (m, 2H), 6.68 (d, J = 8.4 Hz, 2H), 5.40 (s, 2H), 1.75 (s, 3H), 1.33 (s, 9H). | 527.2 |
| 111 | (DMSO-d6) : δ 10.39 (s, 1H), 9.58 (s, 1H), 7.98 (d, J = 8.0 Hz, 1H), 7.90 (d, J = 8.4 Hz, 2H), 7.73 (d, J = 8.1 Hz, 2H), 7.66 - 7.54 (m, 3H), 7.50 (d, J = 7.6 Hz, 1H), 7.41 (t, J = 7.9 Hz, 1H), 7.28 - 7.16 (m, 3H), 5.67 (t, J = 5.7 Hz, 1H), 5.05 (t, J = 5.7 Hz, 1H), 4.81 (d, J = 5.5 Hz, 2H), 4.43 (d, J = 5.7 Hz, 2H), 1.33 (s, 9H). | 499.2 |
| 112 | (DMSO-d6) : δ 10.39 (s, 1H), 9.62 (s, 1H), 9.46 (s, 1H), 7.98 (d, J = 8.0 Hz, 1H), 7.90 (d, J = 8.5 Hz, 2H), 7.74 (d, J = 8.3 Hz, 2H), 7.58 (d, J = 8.5 Hz, 2H), 7.50 (dd, J = 7.8, 1.4 Hz, 1H), 7.41 (t, J = 7.9 Hz, 1H), 7.32 - 7. 09 (m, 4H), 5.66 (t, J = 5.6 Hz, 1H), 4.80 (d, J = 5.6 Hz, 2H), 2.92 (s, 3H), 1.33 (s, 9H). | 562.2 |
| 113 | (DMSO-d6) : δ10.39 (s, 1H), 9.54 (s, 1H), 9.44 (s, 1H), 7.97 (d, J = 8.2 Hz, 1H), 7.90 (d, J = 8.3 Hz, 2H), 7.85 - 7.67 (m, 1H), 7.58 (d, J = 8.5 Hz, 2H), 7.49 (d, J = 7.9 Hz, 1H), 7.41 (t, J = 7.8 Hz, 1H), 7.32-7.13 (m, 3H), 6.86 (t, J = 9.4 Hz, 1H), 5.66 (t, J = 5.7 Hz, 1H), 4.78 (d, J = 5.6 Hz, 2H), 1.33 (s, 9H). | 503.2 |
| 114 | (DMSO-d6) : δ 10.39 (s, 1H), 9.41 (s, 1H), 8.05 - 7. 95 (m, 2H), 7.90 (d, J = 8.3 Hz, 2H), 7.70 -7.55 (m, 4H), 7.49 (d, J = 7.7 Hz, 1H), 7.41 (d, J = 7.7 Hz, 1H), 7.14 (s, 2H), 6.88 (d, J = 8.7 Hz, 2H), 5.69 (s, 1H), 4.80 (s, 2H), 3.64 (s, 2H), 3.35 - 3.25 (m, 4H), 1.33 (s, 9H). | 567.3 |
| 115 | (DMSO-d6): δ 10.59 (s, 1H), 10.47 (s, 1H), 8.90 (s, 1H), 8.11 - 8.03 (m, 1H), 7.94 - 7.82 (m, 4H), 7.63 - 7.55 (m, 3H), 7.53 - 7.39 (m, 3H), 5.65 (s, 1H), 4.91 (s, 2H), 3 .62 - 3.57 (m, 4H), 3.56 - 3.45 (m, 4H), 1.33 (s, 9H). | 567.2 |
| 116 | (DMSO-d6) : δ 13.08 (s, 1H), 9.80 (s, 1H), 9.36 (s, 1H), 7.89 (d, J = 8.1 Hz, 2H), 7.34 (d, J = 8.1 Hz, 2H), 7.33 - 7.15 (m, 2H), 6.97 (t, J = 8.1 Hz, 1H), 6.70 - 6.63 (m, 1H), 6.55 - 6.45 (m, 1H), 3.66 - 3.55 (m, 4H), 3.54 - 3.45 (m, 2H), 3.36 - 3.26 (m, 2H), 2.03 (s, 3H), 1.30 (s, 9H). | 556.3 |
| 117 | (DMSO-d6) : δ 10.37 (s, 1H), 9.25 (s, 1H), 8.97 (s, 1H), 7.94 (d, J = 8.1 Hz, 1H), 7.92 - 7.84 (m, 2H), 7.58 (d, J = 8.5 Hz, 2H), 7.49 (d, J = 7.5 Hz, 1H), 7.43 - 7.25 (m, 2H), 7.40 (t, J = 7.9 Hz, 1H), 7.10 (s, 2H), 6.68 (d, J = 9.0 Hz, 1H), 5.70 - 5.60 (m, 1H), 4.77 (d, J = 5.8 Hz, 2H), 2.10 (s, 3H), 1.33 (s, 9H). | 499.2 |

(continued)

| | | |
|---|---|---|
| 118 | (DMSO-d6): δ 10.41 (s, 1H), 10.01 (s, 1H), 8. 03 - 7.95 (m, 3H), 7.90 (d, J = 8.2 Hz, 2H), 7.72 (d, J = 8.7 Hz, 2H), 7.58 (d, J = 8.3 Hz, 2H), 7.51 (d, J = 7.8 Hz, 1H), 7.42 (t, J = 7.8 Hz, 1H), 7.35 (s, 2H), 7.21 (s, 2H), 5.66 (s, 1H), 4.83 (d, J = 5.6 Hz, 2H), 1.33 (s, 9H). | 548.2 |
| 119 | (DMSO-d6) : δ 10.38 (s, 1H), 9.82 (s, 1H), 9.54 (s, 1H), 8.02 - 7.85 (m, 3H), 7.75 - 7.62 (m, 2H), 7.58 (d, J = 8.0 Hz, 2H), 7.54 - 7.45 (m, 3H), 7.44 - 7.37 (m, 1H), 7.20 (s, 2H), 5.66 (s, 1H), 4.79 (s, 2H), 2.01 (s, 3H), 1.33 (s, 9H). | 526.2 |
| 120 | (DMSO-d6) : δ 10.38 (s, 1H), 9.49 (s, 1H), 9.19 (s, 1H), 7.96 (d, J = 7.8 Hz, 1H), 7.90 (d, J = 8.4 Hz, 2H), 7.68 - 7.54 (m, 4H), 7.54 - 7. 47 (m, 1H), 7.41 (t, J = 7.8 Hz, 1H), 7.35 (d, J = 8.4 Hz, 2H), 7.19 (s, 2H), 5.66 (t, J = 5.7 Hz, 1H), 4.79 (d, J = 5.7 Hz, 2H), 1.46 (s, 9H), 1.33 (s, 9H). | 584.3 |
| 121 | (DMSO-d6) : δ 10.40 (s, 1H), 9.49 (s, 1H), 9.27 (s, 1H), 7.99 (d, J = 8.0 Hz, 1H), 7.90 (d, J = 8.2 Hz, 2H), 7.58 (d, J = 8.2 Hz, 2H), 7.50 (d, J = 7.7 Hz, 1H), 7.41 (t, J = 7.7 Hz, 1H), 7.27 (s, 2H), 7.20 - 7.16 (m, 1H), 7.13 (s, 1H), 7.04 (t, J = 8.1 Hz, 1H), 6.43 - 6.37 (m, 1H), 5.67 (t, J = 5.8 Hz, 1H), 4.81 (d, J = 5.8 Hz, 2H), 1.33 (s, 9H). | 485.2 |
| 122 | (DMSO-d6) : δ 10.40 (s, 1H), 9.60 (s, 1H), 7.98 (d, J = 7.8 Hz, 1H), 7.90 (d, J = 8.3 Hz, 2H), 7.74 (s, 1H), 7.66 (s, 1H), 7.58 (d, J = 8.3 Hz, 2H), 7.52 (d, J = 8.3 Hz, 1H), 7.41 (t, J = 7.8 Hz, 1H), 7.23 (t, J = 7.8 Hz, 1H), 7.20 (s, 2H), 6.96 (d, J = 7.8 Hz, 1H), 5.66 (t, J = 5.7 Hz, 1H), 5.14 (t, J = 5.7 Hz, 1H), 4.81 (d, J = 5.7 Hz, 2H), 4.47 (d, J = 5.7 Hz, 2H), 1.33 (s, 9H). | 499.2 |
| 123 | (DMSO-d6) : δ 10.37 (s, 1H), 9.20 (s, 1H), 7.96 - 7.92 (m, 1H), 7.90 (m, 3H), 7.58 (d, J = 8.2 Hz, 2H), 7.50 (d, J = 7.8 Hz, 1H), 7.40 (t, J = 7.8 Hz, 1H), 7.25 (s, 2H), 7.09 (s, 2H), 5.66 (t, J = 5.8 Hz, 1H), 4.78 (d, J = 5.6 Hz, 2H), 2.14 (s, 6H), 1.33 (s, 9H). | 513.3 |
| 124 | (DMSO-d6) : δ 10.40 (s, 1H), 9.46 (s, 1H), 8.03 - 7. 95 (m, 1H), 7.90 (d, J = 8.2 Hz, 2H), 7.72 - 7.55 (m, 2H), 7.58 (d, J = 8.2 Hz, 2H), 7.49 (d, J = 7.8 Hz, 1H), 7.41 (t, J = 7.8 Hz, 1H), 7.17 (s, 2H), 6.97 (d, J = 8.6 Hz, 2H), 5.69 (s, 1H), 4.80 (s, 2H), 3.77 - 3.60 (m, 4H), 3.21 - 3.05 (m, 4H), 1.33 (s, 9H). | 602.2 |
| 125 | (DMSO-d6) : δ 10.38 (s, 1H), 9.36 (s, 1H), 7.97 (d, J = 7.9 Hz, 1H), 7.90 (d, J = 8.5 Hz, 2H), 7.58 (d, J = 8.5 Hz, 2H), 7.52 (s, 2H), 7.49 (dd, J = 7.8, 1.4 Hz, 1H), 7.40 (t, J = 7.8 Hz, 1H), 7.12 (s, 2H), 6.85 (d, J = 9.0 Hz, 2H), 5.68 (t, J = 5.7 Hz, 1H), 4.79 (d, J = 5.7 Hz, 2H), 4.76 (d, J = 4.7 Hz, 1H), 3.64 - 3.54 (m, 1H), 3.53 - 3.46 (m, 1H), 3.43 - 3.35 (m, 1H), 2.45 - 2.55 (m, 1H), 1.92 - 1.83 (m, 1H), 1.80 - 1.68 (m, 1H), 1.59 - 1.46 (m, 1H), 1.33 (s, 9H), 1.30 - 1.16 (m, 1H). | 568.3 |
| 126 | (DMSO-d6) : δ 10.38 (s, 1H), 9.38 (s, 1H), 8.02 - 7.94 (m, 1H), 7.90 (d, J = 8.5 Hz, 2H), 7.65 - 7. 51 (m,4H), 7.49 (dd, J = 7.8, 1.4 Hz, 1H), 7.40 (t, J = 7.8 Hz, 1H), 7.13 (s, 2H), 6.86 (d, J = 9.0 Hz, 2H), 5.68 (t, J = 5.8 Hz, 1H), 4.85 - 4.75 (m, 2H), 3.04 - 2.96 (m, 4H), 2.88 - 2.83 (m, 4H), 1.33 (s, 9H). | 553.3 |
| 127 | (DMSO-d6) : δ 10.27 (s, 1H), 9.39 (s, 1H), 7.86 (d, J = 7.8 Hz, 1H), 7.64 - 7.53 (m, 2H), 7.51 (s, 1H), 7.49 (dd, J = 7. 8, 1.4 Hz, 1H), 7.38 (t, J = 7.8 Hz, 1H), 7.13 (s, 2H), 6.88 (d, J = 8.9 Hz, 2H), 5.67 (t, J = 5.7 Hz, 1H), 4.83 - 4.65 (m, 3H), 3.92 (d, J = 10.6 Hz, 1H), 3.63 (t, J = 10.6 Hz, 1H), 3.58 - 3.45 (m, 3H), 3.45 - 3.39 (m, 2H), 2.77 (t, J = 5.8 Hz, 2H), 2.65 - 2.58 (m, 2H), 2.42 - 2.33 (m, 1H), 1.85 - 1.70 (m, 4H). | 588.2 |
| 128 | (DMSO-d6) : δ 10.27 (s, 1H), 9.36 (s, 1H), 7.88 - 7.81 (m, 1H), 7.54 (s, 2H), 7.51 (s, 1H), 7. 48 (dd, J = 7.8, 1.4 Hz, 1H), 7.38 (t, J = 7.8 Hz, 1H), 7.12 (s, 2H), 6.86 (d, J = 8.9 Hz, 2H), 5.67 (t, J = 5.7 Hz, 1H), 4.75 (d, J = 5.7 Hz, 2H), 4.64 (d, J = 4.2 Hz, 1H), 3.59 (dd, J = 8.7, 4.5 Hz, 1H), 3.49 - 3.39 (m, 2H), 2.88 - 2.70 (m, 4H), 2.62 (t, J = 5.7 Hz, 2H), 1.91 - 1.64 (m, 6H), 1.47 (q, J = 9.8 Hz, 2H). | 572.2 |
| 129 | (DMSO-d6) : δ 10.27 (s, 1H), 9.57 (s, 1H), 7.87 (d, J = 7.9 Hz, 1H), 7.70 (d, J = 7.9 Hz, 2H), 7.53 - 7.49 (m, 1H), 7.48 (d, J = 1.4 Hz, 1H), 7.39 (t, J = 7.9 Hz, 1H), 7.28 - 7.12 (m, 4H), 5.66 (t, J = 5.7 Hz, 1H), 4.77 (d, J = 5.7 Hz, 2H), 3.57 (s, 2H), 3.47 - 3.36 (m, 4H), 3.21 (s, 6H), 2.78 (t, J = 5.8 Hz, 2H), 2.65 - 2.56 (m, 6H), 1.85 - 1.70 (m, 4H). | 618.3 |
| 130 | (DMSO-d6) : δ 10.28 (s, 1H), 9.60 (s, 1H), 7.88 (d, J = 8.0 Hz, 1H), 7.70 - 7.60 (m, 3H), 7.53 - 7. 44 (m, 2H), 7.39 (t, J = 7.9 Hz, 1H), 7.26 - 7.15 (m, 4H), 5.67 (s, 1H), 4.78 (s, 2H), 3.70 - 3.49 (m, 2H), 3.08 - 2.90 (m, 1H), 2.84 (s, 3H), 2.82 - 2.71 (m, 2H), 2.67 - 2.57 (m, 3H), 2.05 (s, 3H), 1.90 - 1.66 (m, 4H). | 599.2 |

**Table 2-7**

| | | |
|---|---|---|
| 131 | (DMSO-d6) δ 10.26 (s, 1H), 9.44 (s, 1H), 7.85 (d, J = 8.0 Hz, 1H), 7. 55 - 7.48 (m, 3H), 7.39 (t, J = 7.9 Hz, 1H), 7.30 - 7.09 (m, 3H), 6.88 (d, J = 8.8 Hz, 1H), 5.65 (t, J = 5.8 Hz, 1H), 4.76 (d, J = 5.8 Hz, 2H), 4.13 - 3.99 (m, 4H), 3.71-3.59 (m, 4H), 3.31 (s, 6H), 2.81 - 2.74 (m, 2H), 2.65 - 2.57 (m, 2H), 1.85 - 1.70 (m, 4H). | 621.3 |
| 132 | (DMSO-d6) : δ 10.27 (s, 1H), 9.60 (s, 1H), 7.88 (d, J = 7.9 Hz, 1H), 7.72 (d, J = 8.1 Hz, 2H), 7. 53 - 7.46 (m, 2H), 7.39 (t, J = 7.9 Hz, 1H), 7.30 - 7.09 (m, 4H), 5.66 (t, J = 5.7 Hz, 1H), 4.77 (d, J = 5.6 Hz, 2H), 3.58 - 3.54 (m, 4H), 3.40 (s, 2H), 2.78 (t, 2H), 2.62 (t, 2H), 2.33 (t, 4H), 1.88 - 1.69 (m, 4H). | 572.2 |
| 133 | (DMSO-d6) : δ 10.27 (s, 1H), 9.65 (s, 1H), 8.17 (s, 1H), 7.92 - 7.83 (m, 1H), 7.81 - 7.68 (m, 3H), 7.53 - 7.45 (m, 2H), 7.39 (t, J = 7.9 Hz, 1H), 7.33 - 7.09 (m, 4H), 6.89 (d, J = 1.1 Hz, 1H), 5.65 (s, 1H), 5.12 (s, 2H), 4.77 (s, 2H), 2.81 - 2.74 (m, 2H), 2.62 (t, J = 5.8 Hz, 2H), 1.85 - 1.70 (m, 4H). | 553.2 |
| 134 | (DMSO-d6) : δ 10.27 (s, 1H), 9.46 (s, 1H), 7.86 (d, J = 7.9 Hz, 1H), 7.75 - 7.52 (m, 2H), 7.53 - 7.45 (m, 2H), 7.38 (t, J = 7.9 Hz, 1H), 7.17 (s, 2H), 6.92 - 6.83 (m, 2H), 5.66 (t, J = 5.7 Hz, 1H), 4.75 (d, J = 5.7 Hz, 2H), 4.12 - 3.98 (m, 2H), 3.67 - 3.61 (m, 2H), 3.30 (s, 3H), 2.77 (t, J = 5.9 Hz, 2H), 2.62 (t, J = 5.9 Hz, 2H), 1.81 - 1.74 (m, 4H). | 547.2 |
| 135 | (Chloroform-d) δ 8.70 (s, 1H), 8.19 (dd, J = 8.1, 1.2 Hz, 1H), 7.64 (dd, J = 7.7, 1.3 Hz, 1H), 7. 48 - 7.29 (m, 4H), 7.10 - 6.94 (m, 1H), 6.90 (d, J = 8.9 Hz, 2H), 5.27 (s, 2H), 4.70 (s, 2H), 4.01 (t, J = 6.4 Hz, 2H), 2.85 - 2.75 (m, 2H), 2.67 - 2.60 (m, 2H), 2.52 - 2.43 (m, 2H), 2.27 (s, 6H), 2.3 - 2.2 (m, 1H), 2.03 - 1.91 (m, 2H), 1.92 - 1.78 (m, 4H). | 574.3 |
| 136 | (DMSO-d6) δ 10.26 (s, 1H), 9.47 - 9.16 (m, 1H), 9.10 (s, 1H), 7.85 (d, J = 8.0 Hz, 1H), 7.51 (s, 1H), 7.48 (dd, J = 7.7, 1.4 Hz, 1H), 7.38 (t, J = 7.8 Hz, 1H), 7.21 - 6.92 (m, 2H), 6.69 (d, J = 8.7 Hz, 2H), 5.65 (t, J = 5.7 Hz, 1H), 4.74 (d, J = 5.8 Hz, 2H), 3.65-3.52 (m, 1H), 2.77 (t, J = 5.8 Hz, 2H), 2.62 (t, J = 5.7 Hz, 2H), 1.90 - 1.69 (m, 4H). | 489.2 |
| 137 | (DMSO-d6) : δ 10.35 (s, 1H), 9.40 (s, 1H), 7.95 (d, J = 7.9 Hz, 1H), 7.91 - 7.81 (m, 2H), 7.64 - 7.56 (m, 2H), 7.52 - 7.35 (m, 2H), 7.31 - 7.19 (m, 2H), 7.14 (s, 2H), 6.92 - 6.84 (m, 2H), 5.68 (s, 1H), 4.78 (d, J = 3.4 Hz, 2H), 3.76-3.69 (m, 4H), 3.07 - 3.00 (m, 4H), 2.08 - 1.96 (m, 1H), 1.10 - 0.97 (m, 2H), 0.81 - 0.74 (m, 2H). | 538.2 |
| 138 | (Chloroform-d) δ 8.81 (s, 1H), 8.18 (dd, J = 8.2, 1.3 Hz, 1H), 7.60 (d, J = 7.7 Hz, 1H), 7.50 - 7.10 (m, 5H), 6.89 (d, J = 8.9 Hz, 2H), 5.72 - 5.12 (m, 2H), 4.85 - 4.49 (m, 2H), 4.11 (td, J = 6.4, 5.7, 4.4 Hz, 2H), 3.82 - 3.68 (m, 4H), 2.91 - 2.71 (m, 4H), 2.64 (t, J = 6.0 Hz, 2H), 2.61 - 2.54 (m, 4H), 1.92 - 1.74 (m, 4H). | 602.2 |
| 139 | (DMSO-d6) : δ 10.26 (s, 1H), 9.40 (s, 1H), 7.84 (d, J = 8.0 Hz, 1H), 7. 66 - 7.56 (m, 3H), 7.52 - 7.43 (m, 2H), 7.38 (t, J = 7.9 Hz, 1H), 7.25 - 7.01 (m, 2H), 6.92 - 6.84 (m, 2H), 5.65 (t, J = 5.7 Hz, 1H), 4.75 (d, J = 5.5 Hz, 2H), 3.77 - 3.69 (m, 4H), 3.08 - 3.00 (m, 4H), 2.90 - 2.81 (m, 1H), 2.79 - 2.69 (m, 1H), 2.26 - 2.14 (m, 1H), 1.94 - 1.82 (m, 2H), 1.58 - 1.35 (m, 1H), 1.04 (d, J = 6.5 Hz, 3H). | 572.2 |
| 140 | (DMSO-d6): δ 12.94 (s, 1H), 10.40 (s, 1H), 9.61 (s, 1H), 8.28 (s, 1H), 8.22 - 7.95 (m, 2H), 7.90 (d, J = 8.1 Hz, 2H), 7.63 - 7.54 (m, 3H), 7.51 (d, J = 7.6 Hz, 1H), 7.49 - 7.35 (m, 2H), 7.21 (s, 2H), 5.69 (s, 1H), 4.80 (s, 2H), 1.33 (s, 9H). | 509.2 |
| 141 | (DMSO-d6): δ 10.52 (s, 1H), 10.43 (s, 1H), 10.41 (s, 1H), 9.44 (s, 1H), 8.04 - 7.95 (m, 1H), 7.90 (d, J = 8.4 Hz, 2H), 7.58 (d, J = 8.4 Hz, 2H), 7.50 (d, J = 7.8 Hz, 1H), 7.41 (t, J = 7.8 Hz, 1H), 7.33 (s, 2H), 7.13 (s, 2H), 6.82 (d, J = 8.4 Hz, 1H), 5.68 (t, J = 5.7 Hz, 1H), 4.85 - 4.76 (m, 2H), 1.33 (s, 9H). | 525.2 |
| 142 | (DMSO-d6) : δ 10.42 (s, 1H), 9.84 (s, 1H), 8.36 (s, 1H), 8.17 (s, 1H), 8.00 (d, J = 7.8 Hz, 1H), 7.90 (d, J = 8.4 Hz, 2H), 7.64 - 7.55 (m, 3H), 7.54 (d, J = 7.8 Hz, 1H), 7.47 - 7.40 (m, 2H), 7.34 (s, 1H), 7.23 (s, 1H), 5.67 (t, J = 5.4 Hz, 1H), 4.85 (d, J = 5.4 Hz, 2H), 3.84 (s, 3H), 1.33 (s, 9H). | 527.2 |
| 143 | (DMSO-d6) : δ 10.27 (s, 1H), 9.37 (s, 1H), 7.92 - 7.83 (m, 1H), 7.55 (s, 2H), 7.51 (s, 1H), 7. 48 (dd, J = 7.8, 1.4 Hz, 1H), 7.38 (t, J = 7.8 Hz, 1H), 7.13 (s, 2H), 6.84 (d, J = 8.9 Hz, 2H), 5.67 (t, J= 5.4 Hz, 1H), 4.82 - 4.71 (m, 3H), 3.64 - 3.55 (m, 1H), 3.54 - 3.47 (m, 1H), 3.42 - 3.25 (m, 2H), 2.77 (t, J = 5.9 Hz, 2H), 2.65 - 2.58 (m, 2H), 1.93 - 1.83 (m, 1H), 1.83 - 1.67 (m, 5H), 1.61 - 1.45 (m, 1H), 1.30 - 1.18 (m, 1H). | 572.2 |

(continued)

| | | |
|---|---|---|
| 144 | (DMSO-d6): δ 12.34 (s, 1H), 10.41 (s, 1H), 9.59 (s, 1H), 8.49 (s, 1H), 8.17 - 7.95 (m, 3H), 7.90 (d, J = 8.5 Hz, 2H), 7.58 (d, J = 8.5 Hz, 2H), 7.55 - 7.37 (m, 3H), 7.27 - 7.06 (m, 2H), 5.80 - 5.64 (m, 1H), 4.82 (s, 2H), 1.33 (s, 9H). | 509.3 |
| 145 | (DMSO-d6) : δ 10.17 (s, 1H), 9.40 (s, 1H), 8.00 (d, J = 8.0 Hz, 1H), 7.76 (t, J = 8.1 Hz, 1H), 7.60 (s, 3H), 7.48 (dd, J = 7. 8, 1.4 Hz, 1H), 7.40 (t, J = 7.9 Hz, 1H), 7.27 - 6.95 (m, 3H), 6.92 - 6.84 (m, 2H), 5.47 (t, J = 5.6 Hz, 1H), 4.76 (d, J = 5.7 Hz, 2H), 3.77 - 3.69 (m, 4H), 3.08 - 3.00 (m, 4H), 2.10 - 1.96 (m, 1H), 1.11 - 1.00 (m, 2H), 0.85 - 0.74 (m, 2H). | 556.2 |
| 146 | (DMSO-d6) : δ 10.39 (s, 1H), 9.39 (s, 1H), 8.00 - 7. 93 (s, 1H), 7.90 (d, J = 8.5 Hz, 2H), 7.64 - 7.51 (m, 4H), 7.49 (dd, J = 7.8, 1.5 Hz, 1H), 7.40 (t, J = 7.8 Hz, 1H), 7.13 (s, 2H), 6.87 (d, J = 8.8 Hz, 2H), 5.69 (t, J = 5.7 Hz, 1H), 4.79 (d, J = 5.7 Hz, 2H), 3.10 - 3.02 (m, 4H), 2.47 - 2.38 (m, 4H), 2.21 (s, 3H), 1.33 (s, 9H). | 567.3 |
| 147 | (DMSO-d6) : δ 10.27 (s, 1H), 9.39 (s, 1H), 7.90 - 7.83 (m, 1H), 7.65 - 7.50 (m, 2H), 7.51 (s, 1H), 7.48 (dd, J = 7. 9, 1.4 Hz, 1H), 7.38 (t, J = 7.9 Hz, 1H), 7.14 (s, 2H), 6.88 - 6.83 (m, 2H), 5.67 (t, J = 5.7 Hz, 1H), 4.75 (d, J = 5.7 Hz, 2H), 3.01 - 2.93 (m, 4H), 2.86 - 2.80 (m, 4H), 2.77 (t, J = 5.9 Hz, 2H), 2.65 - 2.56 (m, 2H), 1.85 - 1.71 (m, 4H). | 557.2 |
| 148 | (DMSO-d6) : δ 10.40 (s, 1H), 9.70 (s, 1H), 8.02 - 7.95 (m, 1H), 7.95 - 7.86 (m, 2H), 7.81 (d, J = 8.2 Hz, 2H), 7.64 - 7.54 (m, 2H), 7.50 (dd, J = 7.7, 1.4 Hz, 1H), 7.42 (t, J = 7.8 Hz, 1H), 7.35 - 7.22 (m, 4H), 5.67 (t, J = 5.6 Hz, 1H), 4.81 (d, J = 5.6 Hz, 2H), 3.96 (s, 2H), 1.33 (s, 9H). | 508.2 |
| 149 | (DMSO-d6) δ 10.41 (s, 1H), 9.88 (s, 1H), 8.00 (d, J = 8.0 Hz, 1H), 7. 96 - 7.87 (m, 4H), 7.81 (d, J = 8.3 Hz, 3H), 7.58 (d, J = 8.3 Hz, 2H), 7.51 (d, J = 7.7 Hz, 1H), 7.42 (t, J = 7.8 Hz, 1H), 7.38 - 7.26 (m, 2H), 7.19 (s, 1H), 5.66 (s, 1H), 4.83 (d, J = 5.6 Hz, 2H), 1.33 (s, 9H). | 512.2 |
| 150 | (DMSO-d6) δ 10.39 (s, 1H), 9.6 - 9.4 (s, 1H), 8.04 - 7.92 (m, 1H), 7.90 (d, J = 8.3 Hz, 2H), 7.7 - 7.55(m, 1H), 7.58 (d, 4H), 7.48 (d, J = 7.7 Hz, 1H), 7.40 (t, J = 7.8 Hz, 1H), 7.25 - 7.1 (m, 1H), 6.95 - 6.76 (m, 4H), 5.98 (t, J = 2.1 Hz, 2H), 5.68 (t, J = 5.4 Hz, 1H), 4.82 - 4.67 (m, 2H), 4.23 (d, J = 5.3 Hz, 2H), 4.18 (d, J = 5.4 Hz, 2H), 1.33 (s, 9H). | 578.3 |

**Table 2-8**

| | | |
|---|---|---|
| 151 | (DMSO-d6) δ 10.39 (s, 1H), 9.55 - 9.4 (m, 1H), 7.96 (s, 1H), 7.90 (d, J = 8.3 Hz, 2H), 7.73 - 7. 58 (m, 2H), 7.58 (d, J = 8.3 Hz, 2H), 7.49 (d, J = 7.5 Hz, 1H), 7.40 (t, J = 7.8 Hz, 1H), 7.25 - 7.10 (m, 2H), 6.88 (d, J = 8.6 Hz, 2H), 5.68 (t, J = 5.7 Hz, 1H), 4.79 (d, J = 5.9 Hz, 2H), 4.62 (t, J = 5.1 Hz, 1H), 4.05 (s, 2H), 3.72 (t, J = 5.1 Hz, 2H), 3.53 - 3.43 (m, 4H), 1.31 (s, 9H). | 573.3 |
| 152 | (DMSO-d6) δ 10.40 (s, 1H), 9.55 - 9.4 (m, 1H), 8. 0 - 7.93 (m, 1H), 7.90 (d, J = 8.0 Hz, 2H), 7. 7 - 7.59 (m, 2H), 7.58 (d, J = 8.1 Hz, 2H), 7.49 (d, J = 7.9 Hz, 1H), 7.40 (t, J = 7.7 Hz, 1H), 7.3 - 7.1 (m, 2H), 6.87 (d, J = 8.5 Hz, 2H), 4. 89 - 4.71 (m, 2H), 4. 11 - 3.93 (m, 2H), 3.8 - 3.3 (m, 5H), 2.76 (t, J = 6.1 Hz, 2H), 1.7 - 1.6 (m, 4H), 1.33 (s, 9H). | 582.3 |
| 153 | (DMSO-d6) δ 13.0 - 12.5 (m, 1H), 10.45 (s, 1H), 9.78 (s, 1H), 8.24 (s, 1H), 8.02 (s, 1H), 7.90 (d, J = 8.2 Hz, 2H), 7.56 (dd, J = 20. 9, 10.8 Hz, 5H), 7.40 (q, J = 8.5 Hz, 2H), 7.35-7.1 (m, 2H), 4.86 (s, 2H), 5.9-5.6(m, 1H),1.33 (s, 9H). | 513.2 |
| 154 | (DMSO-d6) δ 10.59 (s, 1H), 10.39 (s, 1H), 9.58 (s, 1H), 7.97 (d, J = 7.8 Hz, 1H), 7.90 (d, J = 8.4 Hz, 2H), 7.67 - 7.50 (m, 2H), 7.52 - 7.36 (m, 2H), 7.35 - 7.15 (m, 4H), 6.80 (d, J = 8.5 Hz, 1H), 5.67 (t, J = 5.6 Hz, 1H), 4.79 (d, J = 5.6 Hz, 2H), 4.54 (s, 2H), 1.33 (s, 9H). | 540.2 |
| 155 | (DMSO-d6) δ 10.40 (s, 1H), 9.58 (s, 1H), 8.29 - 8.06 (m, 2H), 7.97 (s, 1H), 7.90 (d, J = 7.8 Hz, 2H), 7.58 (d, J = 7.6 Hz, 2H), 7.51 (d, J = 6.8 Hz, 2H), 7.42 (t, J = 8.2 Hz, 1H), 7.21 (s, 2H), 5.70 (s, 1H), 5.18 - 4.74 (m, 2H), 4.66 (s, 1H), 4.36 - 3.99 (m, 2H), 2.01 - 1.85 (m, 3H), 1.33 (s, 9H), 1.09 (t, J = 7.1 Hz, 2H). | 567.3 |
| 156 | (DMSO-d6) δ 10.40 (s, 1H), 9.64 (s, 1H), 7.98 (d, J = 7.8 Hz, 1H), 7.90 (d, J = 8.3 Hz, 2H), 7.85 - 7.72 (m, 2H), 7.58 (d, J = 8.3 Hz, 2H), 7.54 - 7.45 (m, 3H), 7.43 (s, 1H), 7.26 (s, 2H), 5.68 (t, J = 5.8 Hz, 1H), 4.81 (d, J = 5.7 Hz, 2H), 4.42 (t, J = 8.0 Hz, 2H), 4.04 (t, J = 8.0 Hz, 2H), 1.33 (s, 9H). | 554.2 |

(continued)

| | | |
|---|---|---|
| 157 | (DMSO-d6) δ 10.33 (s, 1H), 10.26 (s, 1H), 9.81 - 9.19 (m, 2H), 8.00 - 7.86 (m, 3H), 7.74 (d, J = 8.4 Hz, 2H), 7.61 (s, 2H), 7.49 (d, J = 7.7 Hz, 1H), 7.41 (t, J = 7.8 Hz, 1H), 7.22 (s, 2H), 6.89 (d, J = 8.6 Hz, 2H), 4.77 (s, 2H), 3.84 - 3.58 (m, 4H), 3.13 - 2.92 (m, 4H), 2.09 (s, 3H). | 555.2 |
| 158 | (DMSO-d6) δ 10.43 (s, 1H), 9.45 (s, 1H), 8.04 (t, J = 7.5 Hz, 1H), 7.92 (d, J = 10.3 Hz, 1H), 7.88 (d, J = 8.2 Hz, 1H), 7.77 (d, J = 8.1 Hz, 1H), 7.71 - 7.59 (m, 2H), 7.56 (d, J = 7.8 Hz, 1H), 7.43 (t, J = 7.9 Hz, 1H), 7.18 (s, 2H), 6.88 (d, J = 8.6 Hz, 2H), 5.44 (t, J = 6.1 Hz, 1H), 4. 73 (d, J = 6.0 Hz, 2H), 3.73 (t, J = 4.6 Hz, 4H), 3.04 (t, J = 4.6 Hz, 4H). | 584.2 |
| 159 | (DMSO-d6) : δ 10.49 - 10.29 (m, 1H), 9.12 (s, 1H), 8.06 - 7.83 (m, 3H), 7.66 - 7.35 (m, 8H), 6.68 (d, J = 8.6 Hz, 2H), 5.72 - 5.59 (m, 1H), 4.90 - 4.72 (m, 2H), 3.53 - 3.40 (m, 2H), 2.63 - 2.52 (m, 6H), 1.33 (s, 9H), 1.01 - 0.90 (m, 6H). | 584.3 |
| 160 | (DMSO-d6) : δ 10.39 (s, 1H), 9.64 (s, 1H), 8.01 - 7.94 (m, 1H), 7.94 - 7.86 (m, 2H), 7.81 - 7.70 (m, 3H), 7.62 - 7.54 (m, 2H), 7.53 - 7.36 (m, 3H), 7.23 (d, J = 23.0 Hz, 2H), 7.19 - 7.11 (m, 2H), 6.25 (t, J = 2.1 Hz, 1H), 5.66 (t, J = 5.6 Hz, 1H), 5.26 (s, 2H), 4.80 (d, J = 5.6 Hz, 2H), 1.33 (s, 9H). | 549.3 |
| 161 | (DMSO-d6) : δ 10.40 (s, 1H), 9.65 (s, 1H), 8.02 - 7.95 (m, 1H), 7.94 - 7.86 (m, 2H), 7.80 (s, 1H), 7.75 - 7.65 (m, 2H), 7.62 - 7.54 (m, 2H), 7.50 (dd, J = 7.7, 1.4 Hz, 1H), 7.46 - 7.37 (m, 2H), 7.37 - 7.04 (m, 3H), 6.85 - 6.77 (m, 1H), 6.24 (s, 1H), 5.67 (t, J = 5.6 Hz, 1H), 5.30 (s, 2H), 4.81 (d, J = 5.7 Hz, 2H), 1.33 (s, 9H). | 549.3 |
| 162 | (DMSO-d6) : δ 10.39 (s, 1H), 9.68 (s, 1H), 8.66 (s, 1H), 8.01 - 7.94 (m, 2H), 7.94 - 7.86 (m, 2H), 7.79 - 7.68 (m, 2H), 7.62 - 7.54 (m, 2H), 7.54 - 7.38 (m, 2H), 7.37 - 7.03 (m, 3H), 6.91 - 6.84 (m, 1H), 5.64 (t, J = 5.7 Hz, 1H), 5.39 (s, 2H), 4.80 (d, J = 5.7 Hz, 2H), 1.33 (s, 9H). | 550.3 |
| 163 | (DMSO-d6) : δ 10.40 (s, 1H), 9.58 (s, 1H), 7.98 (d, J = 7.9 Hz, 1H), 7.90 (d, J = 8.5 Hz, 2H), 7.74 (s, 1H), 7.67 (s, 1H), 7.58 (d, J = 8.5 Hz, 2H), 7.51 (dd, J = 7. 7, 1.4 Hz, 1H), 7. 41 (t, J = 7.9 Hz, 1H), 7.23 (t, J = 7.7 Hz, 1H), 7.30 - 7.10 (br s, 2H), 6.93 (d, J = 7.7 Hz, 1H), 5.68 (t, J = 5.7 Hz, 1H), 4.83 (d, J = 5.7 Hz, 2H), 3.62 - 3.49 (m, 4H), 3.43 (s, 2H), 2.38 - 2.26 (m, 4H), 1.33 (s, 9H). | 568.3 |
| 164 | (DMSO-d6) : δ 10.39 (s, 1H), 9.67 (s, 1H), 8.62 (s, 1H), 8.02 - 7.94 (m, 2H), 7.94 - 7.86 (m, 2H), 7.77 (d, J = 8.2 Hz, 2H), 7.62 - 7.54 (m, 2H), 7.49 (dd, J = 7.7, 1.4 Hz, 1H), 7.41 (t, J = 7.8 Hz, 1H), 7.29 - 7.18 (m, 4H), 5.66 (t, J = 5.6 Hz, 1H), 5.34 (s, 2H), 4.81 (d, J = 5.6 Hz, 2H), 1.33 (s, 9H). | 550.3 |
| 165 | (DMSO-d6) δ 10.53 - 10.18 (m, 1H), 9.73 - 9.29 (m, 2H), 8.16 - 7.80 (m, 3H), 7.80 - 7.49 (m, 4H), 7.40 (t, J = 8.0 Hz, 1H), 7.22 (s, 2H), 7.1 - 6.9 (m, 1H), 6.0 - 5.5 (m, 1H), 5.07 - 4.41 (m, 2H), 1.33 (s, 9H). | 459.2 |
| 166 | (DMSO-d6) δ 10.39 (s, 1H), 9.46 (s, 1H), 8.03 - 7. 93 (m, 1H), 7.90 (d, J = 8.3 Hz, 2H), 7.73 - 7.61 (m, 2H), 7.58 (d, J = 8.3 Hz, 2H), 7.49 (d, J = 7.6 Hz, 1H), 7.40 (t, J = 7.8 Hz, 1H), 7.18 (s, 2H), 6.87 (d, J = 8.7 Hz, 2H), 5.68 (t, J = 5.7 Hz, 1H), 4.79 (d, J = 5.7 Hz, 2H), 4.00 (t, J = 5.8 Hz, 2H), 2.61 (t, J = 5.9 Hz, 2H), 2.21 (s, 6H), 1.33 (s, 9H). | 556.3 |
| 167 | (DMSO-d6) δ 10.38 (s, 1H), 9.51 (s, 1H), 8.02 - 7.94 (m, 1H), 7.90 (d, J = 8.2 Hz, 2H), 7.79 - 7.62 (m, 2H), 7.58 (d, J = 8.3 Hz, 2H), 7.53 - 7.37 (m, 3H), 7.31 - 7.05 (m, 2H), 6.82 (s, 1H), 5.67 (t, J = 5.6 Hz, 1H), 4.80 (d, J = 5.6 Hz, 2H), 3.82 (t, J = 8.0 Hz, 2H), 3.38 (t, J = 8.0 Hz, 3H), 1.33 (s, 9H). | 553.3 |
| 168 | (DMSO-d6) δ 10.58 (s, 1H), 9.43 (s, 1H), 8.16 (d, J = 8.1 Hz, 2H), 7.95 (d, J = 8.1 Hz, 2H), 7.89 - 7.78 (m, 1H), 7.74 - 7.50 (m, 3H), 7.43 (t, J = 7.9 Hz, 1H), 7.32 - 7.00 (m, 2H), 6.88 (d, J = 8.6 Hz, 2H), 5.59 (t, J = 6.0 Hz, 1H), 4.75 (d, J = 6.2 Hz, 2H), 3.73 (t, J = 4.7 Hz, 4H), 3.04 (t, J = 4.6 Hz, 4H). | 566.2 |
| 169 | (DMSO-d6) δ 10.18 (s, 1H), 9.39 (s, 1H), 8.11 - 7.90 (m, 1H), 7.81 (d, J = 8.7 Hz, 2H), 7.69 - 7.49 (m, 2H), 7.43 (d, J = 7.3 Hz, 1H), 7.37 (t, J = 7.8 Hz, 1H), 7.21 - 6.98 (m, 2H), 6.88 (d, J = 8.8 Hz, 2H), 6.79 (d, J = 8.7 Hz, 2H), 5.72 (s, 1H), 4.80 (s, 2H), 3.73 (t, J = 4.6 Hz, 4H), 3.03 (t, J = 5.1 Hz, 4H), 3.01 (s, 6H). | 541.3 |
| 170 | (DMSO-d6) δ 10.38 (s, 1H), 9.41 (s, 1H), 8.07 - 7.93 (m, 1H), 7.89 (d, J = 7.9 Hz, 2H), 7.71 - 7.53 (m, 2H), 7.49 (d, J = 7.3 Hz, 1H), 7.46 - 7.34 (m, 3H), 7.25 - 6.98 (m, 2H), 6.88 (d, J = 8.6 Hz, 2H), 5.84 - 5.60 (m, 1H), 4.79 (s, 2H), 3.73 (t, J = 4.7 Hz, 4H), 3.04 (t, J = 4.7 Hz, 5H), 1.24 (d, J = 6.9 Hz, 6H). | 540.3 |

(continued)

| | | |
|---|---|---|
| 171 | (DMSO-d6) δ 10.48 - 10.17 (m, 1H), 9.54 - 9.16 (m, 1H), 8.17 - 7.90 (m, 1H), 7.82 (s, 1H), 7.74 (d, J = 8.0 Hz, 1H), 7.69 - 7.52 (m, 2H), 7.48 (d, J = 7.4 Hz, 1H), 7.40 (t, J = 7.2 Hz, 2H), 7.10 (dd, J = 19.7, 11.7 Hz, 2H), 6.88 (d, J = 8.7 Hz, 2H), 5.67 (t, J = 5.6 Hz, 1H), 4.78 (d, J = 5.7 Hz, 2H), 3.73 (t, J = 4.7 Hz, 4H), 3.04 (t, J = 4.6 Hz, 4H), 2.94 (td, J = 7.4, 4.2 Hz, 4H), 2.07 (td, J = 7.5, 3.5 Hz, 2H). | 538.2 |

**Table 2-9**

| | | |
|---|---|---|
| 172 | (DMSO-d6) δ 10.41 (s, 1H), 9.87 (s, 1H), 8.05 - 7.93 (m, 3H), 7.90 (d, J = 8.2 Hz, 2H), 7.58 (d, J = 8.3 Hz, 2H), 7.51 (d, J = 7.7 Hz, 1H), 7.42 (t, J = 7.9 Hz, 1H), 7.38 - 7.16 (m, 5H), 6.88 (s, 1H), 5.69 (t, J = 5.7 Hz, 1H), 4.84 (d, J = 5.5 Hz, 2H), 2.26 (s, 3H), 1.33 (s, 9H). | 549.3 |
| 173 | (DMSO-d6) : δ 10.38 (s, 1H), 9.63 (s, 1H), 7.98 (d, J = 7.8 Hz, 1H), 7. 94 - 7.87 (m, 2H), 7.79 - 7.75 (m, 1H), 7.73 (d, J = 8.3 Hz, 2H), 7.60 - 7.56 (m, 2H), 7.49 (dd, J = 7.8, 1.5 Hz, 1H), 7.43 - 7.37 (m, 2H), 7.23 (d, J = 8.6 Hz, 2H), 7.30 - 7.15 (br s, 2H), 6.43 - 6.38 (m, 1H), 6.22 (td, J = 6.6, 1.4 Hz, 1H), 5.66 (t, J = 5.6 Hz, 1H), 5.03 (s, 2H), 4.80 (d, J = 5.6 Hz, 2H), 1.33 (s, 9H). | 576.3 |
| 174 | (DMSO-d6) δ 10.38 (s, 1H), 9.61 - 9.22 (m, 1H), 8.71 - 8.27 (m, 1H), 7.96 (d, J = 8.0 Hz, 1H), 7. 92 - 7.85 (m, 3H), 7.61 - 7.54 (m, 2H), 7.49 (d, J = 7.7 Hz, 1H), 7.40 (t, J = 7.8 Hz, 1H), 7.20 (s, 2H), 6.81 (d, J = 9.0 Hz, 1H), 5.68 (t, J = 5.7 Hz, 1H), 4.78 (d, J = 5.7 Hz, 2H), 3.70 (t, J = 4.7 Hz, 4H), 3.36 (t, J = 4.7 Hz, 4H), 1.33 (s, 9H). | 555.3 |
| 175 | (DMSO-d6) : δ 10.29 (s, 1H), 9.41 (s, 1H), 7.86 (d, J = 7.8 Hz, 1H), 7.67 - 7.54 (m, 3H), 7.49 (dd, J = 7.7, 1.4 Hz, 1H), 7.39 (t, J = 7.9 Hz, 1H), 7.15 (s, 2H), 6.92 - 6.84 (m, 2H), 5.67 (t, J = 5.7 Hz, 1H), 4.76 (d, J = 5.7 Hz, 2H), 3.77 - 3.69 (m, 4H), 3.07 - 3.00 (m, 4H), 2.96 - 2.87 (m, 2H), 2.79 - 2.71 (m, 2H), 2.46 - 2.35 (m, 2H). | 544.1 |
| 176 | (DMSO-d6) δ 10.37 (s, 1H), 9.41 (s, 1H), 7.95 (s, 1H), 7.88 (d, J = 7.9 Hz, 2H), 7.74 - 7.52 (m, 2H), 7.49 (d, J = 7.6 Hz, 1H), 7.44 - 7.35 (m, 3H), 7.28 - 6.97 (m, 2H), 6.88 (d, J = 8.7 Hz, 2H), 5.68 (t, J = 5.8 Hz, 1H), 4.78 (d, J = 4.8 Hz, 2H), 3.73 (t, J = 4.5 Hz, 4H), 3.03 (t, J = 4.8 Hz, 4H), 2.60 (t, J = 11.4 Hz, 1H), 1.81 (d, J = 10.6 Hz, 4H), 1.63 - 1.14 (m, 6H). | 580.3 |
| 177 | (DMSO-d6) δ 11.60 - 11.11 (m, 1H), 10.38 (s, 1H), 9.42 (s, 1H), 8.07 - 7.77 (m, 4H), 7.67 - 7.34 (m, 5H), 7.22 - 6.90 (m, 3H), 5.69 (t, J = 5.7 Hz, 1H), 4.79 (d, J = 7.3 Hz, 2H), 3.72 (t, J = 5.2 Hz, 4H), 3.55 - 3.37 (m, 4H), 1.33 (s, 9H). | 594.3 |
| 178 | (DMSO-d6) δ 10.39 (s, 1H), 9.51 (s, 1H), 8.30 - 7. 78 (m, 4H), 7.58 (d, J = 8.3 Hz, 2H), 7.51 (d, J = 7.8 Hz, 1H), 7.48 - 7.31 (m, 3H), 7.17 (s, 2H), 5.68 (t, J = 5.7 Hz, 1H), 4.80 (d, J = 5.8 Hz, 2H), 4.24 (t, J = 6.3 Hz, 2H), 3.53 (t, J = 4.5 Hz, 4H), 2.60 (t, J = 6.6 Hz, 2H), 2.52 (s, 3H), 2.47 - 2.32 (m, 4H), 1.33 (s, 9H). | 636.3 |
| 179 | (DMSO-d6) δ 9.69 (s, 1H), 8.02 (s, 1H), 7.80 - 7.73 (m, 1H), 7.63 (s, 1H), 7.51 - 7.41 (m, 2H), 7.37 - 7.17 (m, 2H), 6.95 (s, 1H), 6.89 (d, J = 12.7 Hz, 1H), 5.20 - 5.04 (m, 1H), 4.94 - 4.79 (m, 1H), 4.60 - 4.49 (m, 1H), 4.40 - 4.28 (m, 1H), 4.15 - 4.03 (m, 2H), 3.93 - 3.64 (m, 4H), 3.26 - 3.15 (m, 1H), 3.13 - 3.03 (m, 1H), 2.05 - 1.94 (m, 1H), 1.10 - 1.00 (m, 2H), 0.86 - 0.77 (m, 2H). | 531.1 |
| 180 | (DMSO-d6) δ 9.69 (s, 1H), 8.00 (s, 1H), 7.80 - 7.72 (m, 1H), 7.59 (s, 1H), 7.52 - 7.40 (m, 2H), 7.29 (d, J = 17.5 Hz, 2H), 6.95 (d, J = 1.6 Hz, 1H), 6.89 (dd, J = 12.7, 1.7 Hz, 1H), 5.20 - 4.96 (m, 1H), 4.61 - 4.48 (m, 1H), 4.39 - 4.29 (m, 1H), 3.93 - 3.83 (m, 1H), 3.81 (s, 3H), 3.80 - 3.75 (m, 1H), 3.26 - 3.15 (m, 1H), 3.13 - 3.03 (m, 1H), 2.05 - 1.94 (m, 1H), 1.10 - 1.00 (m, 2H), 0.85 - 0.77 (m, 2H). | 501.0 |
| 181 | (DMSO-d6) δ 9.71 - 9.41 (m, 1H), 7.75 (dd, J = 6.9, 2.2 Hz, 1H), 7.69 - 7.54 (m, 2H), 7.53 - 7.45 (m, 2H), 7.43 (d, J = 1.9 Hz, 1H), 7.39 - 7.23 (m, 3H), 6.90 (d, J = 8.9 Hz, 2H), 4.60 (d, J = 11.9 Hz, 1H), 4.37 (d, J = 12.0 Hz, 1H), 3.99 - 3.89 (m, 1H), 3.87 - 3.78 (m, 1H), 3.78 - 3.71 (m, 4H), 3.23 - 3.15 (m, 2H), 3.09 - 3.02 (m, 4H), 1.74 (s, 6H). | 609.1 |
| 182 | (DMSO-d6) δ 9.41 (s, 1H), 9.10 (s, 1H), 7.74 (dd, J = 7.4, 1.8 Hz, 1H), 7.58 - 7.37 (m, 4H), 7.23 (s, 2H), 6.95 (d, J = 1.6 Hz, 1H), 6.89 (dd, J = 12.6, 1.6 Hz, 1H), 6.77 - 6.60 (m, 2H), 5.06 (d, J = 11.9 Hz, 1H), 4.54 (d, J = 10.5 Hz, 1H), 4.33 (t, J = 10.7 Hz, 1H), 3.93 - 3.70 (m, 2H), 3.20 (s, 1H), 3.08 (dd, J = 16.1, 5.6 Hz, 1H), 2.07 - 1.92 (m, 1H), 1.13 - 0.98 (m, 2H), 0.86 - 0.69 (m, 2H). | 513.1 |

(continued)

| | | |
|---|---|---|
| 183 | (DMSO-d6) δ 9.53 (s, 1H), 7.75 (dd, J = 7.4, 1.8 Hz, 1H), 7.71 - 7.53 (m, 2H), 7.53 - 7.40 (m, 2H), 7.38 - 7.02 (m, 2H), 6.95 (d, J = 1.7 Hz, 1H), 6.92 - 6.82 (m, 3H), 5.03 (s, 1H), 4.56 (dd, J = 12.0, 4.5 Hz, 1H), 4.34 (dd, J = 11.9, 9.5 Hz, 1H), 3.97 (d, J = 6.4 Hz, 1H), 3.92 - 3.72 (m, 2H), 3.29 - 3.14 (m, 1H), 3.13 - 3.00 (m, 1H), 2.40 - 2.27 (m, 3H), 2.14 (s, 6H), 2.08 - 1.91 (m, 1H), 1.90 - 1.74 (m, 2H), 1 . 15 - 0.95 (m, 2H), 0 . 90 - 0.74 (m, 2H). | 598.1 |
| 184 | (DMSO-d6) δ 10.39 (s, 1H), 9.46 (s, 1H), 7.97 (d, J = 7.8 Hz, 1H), 7.90 (d, J = 8.1 Hz, 2H), 7.75 - 7.61 (m, 2H), 7.58 (d, J = 8.3 Hz, 2H), 7.49 (d, J = 7.6 Hz, 1H), 7.40 (t, J = 7.8 Hz, 1H), 7.17 (s, 2H), 6.87 (d, J = 8.8 Hz, 2H), 5.67 (t, J = 5.7 Hz, 1H), 4.92 - 4.60 (m, 3H), 3.94 (t, J = 5.0 Hz, 2H), 3.76 - 3.65(m, 2H), 1.33 (s, 9H). | 529.0 |
| 185 | (DMSO-d6) δ 10.42 (s, 1H), 9.81 (s, 1H), 8.18 (s, 1H), 8.05 - 7.85 (m, 5H), 7.68 (s, 1H), 7.62 - 7.20 (m, 8H), 7.08 (s, 1H), 5.74-5.65 (m, 1H), 4.87 - 4.77 (m, 2H), 1.33 (s, 9H). | 535.4 |
| 186 | (DMSO-d6) δ 10.53 (s, 1H), 10.43 (s, 1H), 8.27 (d, J = 8.7 Hz, 2H), 8.04 (d, J = 8.0 Hz, 1H), 8.00 (d, J = 8.7 Hz, 2H), 7.90 (d, J = 8.1 Hz, 2H), 7.65 - 7.40 (m, 6H), 5.67 (t, J = 5.6 Hz, 1H), 4.86 (d, J = 5.4 Hz, 2H), 1.33 (s, 9H). | 601.2 |
| 187 | (DMSO-d6) δ 13.70 (br s, 1H), 10.42 (s, 1H), 9.75 (s, 1H), 8.25 (s, 1H), 8.05 - 7.95 (m, 1H), 7.97 - 7.85 (m, 2H), 7.64 - 7.55 (m, 4H), 7.55 - 7.49 (m, 1H), 7.43 (t, J = 7.9 Hz, 1H), 7.27 (br s, 2H), 5.74 - 5.66 (m, 1H), 4.88 - 4.77 (m, 2H), 1.33 (s, 9H). | 577.4 |
| 188 | (DMSO-d6) δ 10.38 (s, 1H), 9.49 (s, 1H), 7.99 - 7.85 (m, 3H), 7.64 - 7.49 (m, 3H), 7.41 (t, J = 7.9 Hz, 1H), 7.36 - 7.09 (m, 4H), 5.65 (t, J = 5.9 Hz, 1H), 4.87 - 4.73 (m, 2H), 4.42 4.32 (m, 1H), 3.86 (t, J = 6.5 Hz, 2H), 3.52 - 5.58 (m, 2H), 3.33 (s, 6H), 1.81 - 1.69 (m, 2H), 1.33 (s, 9H). | 603.2 |
| 189 | (DMSO-d6) δ 9.50 (s, 1H), 8.08 (s, 1H), 7.75 (dd, J = 7.4, 1.8 Hz, 1H), 7.61 (m, 2H), 7.50 - 7.43 (m, 2H), 7.27 (br s, 2H), 6.96 - 6.86 (m, 4H), 5.04 (br s, 1H), 4.56 (dd, J = 11.8, 4. 5 Hz, 1H), 4.34 (dd, J = 11.8, 9.6 Hz, 1H), 3.93 - 3.84 (m, 1H), 3.84 - 3.74 (m, 1H), 3.56 - 3.46 (m, 4H), 3.25 - 3.14 (m, 1H), 3.13 - 2.98 (m, 5H), 2.05 - 1.95 (m, 1H), 1.10 - 1.03 (m, 2H), 0.85 - 0.74 (m, 2H). | 609.3 |
| 190 | (DMSO-d6) δ 9.76 (s, 1H), 7.81 (d, J = 8.3 Hz, 2H), 7. 76 (dd, J = 7. 5, 1.8 Hz, 1H), 7. 48 (t, J = 7.5 Hz, 1H), 7.45 (dd, J = 7.5, 1.8 Hz, 1H), 7.35 - 7.20 (br s, 2H), 7.27 (d, J = 8.3 Hz, 2H), 6.95 (s, 1H), 6.89 (dd, J = 12.6, 1.7 Hz, 1H), 4. 99 (dd, J = 9.3, 4.6 Hz, 1H), 4.60 (dd, J = 11.9, 4.6 Hz, 1H), 4.35 (dd, J = 11.9, 9.3 Hz, 1H), 3. 97 (s, 2H), 3. 94 - 3.84 (m, 1H), 3.84 - 3.75 (m, 1H), 3.25 - 3.16 (m, 1H), 3.14 - 3.03 (m, 1H), 2.05 - 1.95 (m, 1H), 1.09 - 1.03 (m, 2H), 0.85 - 0.77 (m, 2H). | 536.3 |

**Table 2-10**

| | | |
|---|---|---|
| 191 | (DMSO-d6) δ 9.46 (s, 1H), 7.75 (dd, J = 7.6, 1.8 Hz, 1H), 7.64 - 7.52 (m, 2H), 7.47 (t, J = 7. 6, 1H), 7. 43 (dd, J = 7. 6, 1.8 Hz, 1H), 7.25 (br s, 2H), 6.97 - 6.85 (m, 4H), 5.05 (br s, 1H), 4.56 (dd, J = 11.9, 4.5 Hz, 1H), 4.34 (dd, J = 11.9, 9.6 Hz, 1H), 3. 92 - 3.75 (m, 2H), 3.26 - 3.15 (m, 1H), 3.12 - 3.02 (m, 1H), 3.03 - 2.95 (m, 4H), 2.87 - 2.78 (m, 4H), 2.04 - 1.96 (m, 1H), 1.08 - 1.02 (m, 2H), 0.85 - 0.76 (m, 2H). | 581.4 |
| 192 | (DMSO-d6) δ 10.38 (s, 1H), 9.40 (s, 1H), 7.98 - 7.79 (m, 3H), 7.62 - 7.57 (m, 2H), 7.52 - 7.44 (m, 1H), 7.44 - 7.32 (m, 3H), 7.17 - 7.12 (m, 2H), 6.92 - 6.84 (m, 2H), 5.68 (t, J = 5.7 Hz, 1H), 4.78 (d, J = 5.8 Hz, 2H), 3.75 - 3.69 (m, 4H), 3.07 - 3.00 (m, 4H), 1.43 (s, 3H), 0.98 - 0.90 (m, 2H), 0.88 - 0.81 (m, 2H). | 552.1 |
| 193 | (DMSO-d6) δ 9.47 (s, 1H), 7.75 (dd, J = 7.3, 1.8 Hz, 1H), 7. 63 - 7.51 (m, 2H), 7.50 - 7.40 (m, 2H), 7.25 (br s, 2H), 6.95 (s, 1H), 6.92 - 6.84 (m, 3H), 5.05 (br s, 1H), 4.56 (dd, J = 11.9, 4.5 Hz, 1H), 4.34 (dd, J = 11.9, 9.6 Hz, 1H), 3.92 - 3.83 (m, 1H), 3.83 - 3.75 (m, 1H), 3.26 - 3.15 (m, 1H), 3.15 - 3.03 (m, 5H), 2.48 - 2.40 (m, 4H), 2.22 (s, 3H), 2.03 - 1.95 (m, 1H), 1.10 - 1.02 (m, 2H), 0.84 - 0.78 (m, 2H). | 595.4 |

(continued)

| | | |
|---|---|---|
| 194 | (DMSO-d6) δ 9.72 (s, 1H), 7.91 (s, 1H), 7.75 (dd, J = 7.4, 1.8 Hz, 1H), 7. 54 - 7.43 (m, 2H), 7.43 - 7.23 (m, 2H), 7.16 (t, J = 9.3 Hz, 1H), 6.95 (d, J = 1.6 Hz, 1H), 6.89 (dd, J = 12.6, 1.7 Hz, 1H), 4.96 (d, J = 7.6 Hz, 1H), 4.59 (dd, J = 12.0, 4.6 Hz, 1H), 4.45 (dt, J = 8.7, 4. 5 Hz, 1H), 4.35 (dd, J = 11.9, 9.3 Hz, 1H), 4.02 - 3.61 (m, 5H), 3.45 (ddd, J = 11.9, 9.3, 2.9 Hz, 2H), 3.21 (ddd, J = 15.0, 9.6, 5.0 Hz, 1H), 3.07 (dt, J = 15.9, 5.2 Hz, 1H), 2.07 - 1.83 (m, 3H), 1.59 (dtd, J = 13.0, 9.0, 4.0 Hz, 2H), 1.11 - 0.98 (m, 2H), 0.87 - 0.71 (m, 2H). | 615.1 |
| 195 | (DMSO-d6) δ 12.57 (s, 1H), 9.69 - 9.38 (m, 1H), 8.05 (s, 1H), 7.87 - 7.71 (m, 1H), 7.70 - 7.52 (m, 1H), 7.52 - 7.40 (m, 1H), 7.30 (s, 1H), 6. 95 (d, J = 1.6 Hz, 1H), 6.89 (dd, J = 12.7, 1.7 Hz, 1H), 5.25 - 4.91 (m, 1H), 4.54 (dd, J = 12.0, 4.5 Hz, 1H), 4.34 (dd, J = 11.9, 9.7 Hz, 1H), 3.99 - 3.73 (m, 2H), 3.21 (ddd, J = 15.1, 9.6, 5. 1 Hz, 1H), 3.08 (dt, J = 15.9, 5.2 Hz, 1H), 2.00 (ddd, J = 13.3, 8.6, 4.9 Hz, 1H), 1.12 - 0.98 (m, 3H), 0 . 90 - 0.72 (m, 3H). | 487.1 |
| 196 | (DMSO-d6) δ 9.66 - 9.35 (m, 1H), 8.17 (s, 1H), 7.75 (dd, J = 7.4, 1.8 Hz, 1H), 7.71 - 7.58 (m, 2H), 7. 57 - 7.40 (m, 2H), 7.39 - 7.01 (m, 2H), 6.97 - 6.93 (m, 1H), 6.92 - 6.85 (m, 3H), 5.13 - 4.94 (m, 1H), 4.62 - 4.51 (m, 1H), 4.41 - 4.28 (m, 1H), 4.09 - 4.03 (m, 2H), 3.93 - 3.83 (m, 1H), 3.83 - 3.73 (m, 1H), 3.60-3.55 (m, 4H), 3.13 - 3.06 (m, 2H), 2.71 - 2.64 (m, 2H), 2.48 - 2.44 (m, 4H), 2.05 - 1.94 (m, 1H), 1.09 - 1.01 (m, 2H), 0.85 - 0.77 (m, 2H). | 626.1 |
| 197 | (DMSO-d6) δ 9.55 (s, 1H), 7.75 (dd, J = 7.4, 1.8 Hz, 1H), 7.72 - 7.57 (m, 2H), 7.50 - 7.42 (m, 2H), 7.38 - 7.05 (m, 2H), 6.95 (s, 1H), 6.93 - 6.84 (m, 3H), 5.15 - 4.95 (m, 1H), 4.61 4.51 (m, 2H), 4.38 - 4.30 (m, 1H), 4.05 - 3.96 (m, 2H), 3.92 - 3.74 (m, 2H), 3.59-3.52 (m, 2H), 3.22 - 3.16 (m, 1H), 3.12 - 3.03 (m, 1H), 2.05 - 1.96 (m, 1H), 1.89 - 1.80 (m, 2H), 1.09 - 1.04 (m, 2H), 0.84 - 0.79 (m, 2H). | 571.1 |
| 198 | (DMSO-d6) δ 9.69 (s, 1H), 7.77 - 7.70 (m, 3H), 7.50 - 7.42 (m, 2H), 7.36 (br s, 2H), 7.19 (d, J = 8.5 Hz, 2H), 6.95 (s, 1H), 6.89 (dd, J = 12.7, 1.6 Hz, 1H), 4. 99 (dd, J = 9.4, 4.6 Hz, 1H), 4. 59 (dd, J = 12.0, 4.6 Hz, 1H), 4.38 - 4.30 (m, 1H), 4.23 (s, 2H), 3.92 - 3.83 (m, 1H), 3.83 - 3.74 (m, 1H), 3.26 - 3.15 (m, 1H), 3.12 - 3.03 (m, 1H), 2.04 - 1.96 (m, 1H), 1.09 - 1.02 (m, 2H), 0.84 - 0.77 (m, 2H). | 579.3 |
| 199 | (DMSO-d6) δ 10.40 (s, 1H), 9.55 (s, 1H), 8.03 - 7.94 (m, 1H), 7.90 (d, J = 8.3 Hz, 2H), 7.82 - 7.64 (m, 2H), 7.58 (d, J = 8.4 Hz, 2H), 7.49 (d, J = 6.9 Hz, 1H), 7.41 (t, J = 7.9 Hz, 1H), 7.34 - 7.09 (m, 2H), 6.99 (d, J = 9.0 Hz, 2H), 5.68 (t, J = 5.5 Hz, 1H), 4.80 (d, J = 5.6 Hz, 2H), 4.75 - 4.63 (m, 2H), 1.33 (s, 9H). | 567.2 |
| 200 | (Methanol-d4) δ 10.39 (s, 1H), 9.46 (s, 1H), 8. 02 - 7.94 (m, 1H), 7.90 (d, J = 8.4 Hz, 2H), 7.73 - 7.54 (m, 4H), 7.49 (dd, J = 7.7, 1.4 Hz, 1H), 7.40 (t, J = 7.8 Hz, 1H), 7.28 - 6.99 (m, 2H), 6.86 (d, J = 8.7 Hz, 2H), 5.75 - 5.62 (m, 1H), 4.86 - 4.73 (m, 2H), 4.60 - 4.46 (m, 1H), 4.05 - 3.93 (m, 2H), 3.61 - 3.48 (m, 2H), 1.90 - 1.77 (m, 2H), 1.33 (s, 9H). | 543.2 |
| 201 | (DMSO-d6) δ 10.42 - 10.35 (m, 1H), 9.46-9.39 (m, 1H), 8.01 - 7.96 (m, 1H), 7.88 (d, J = 8.5 Hz, 2H), 7.64 - 7.53 (m, 4H), 7.51 - 7.47 (m, 1H), 7. 43 - 7.38 (m, 1H), 7.20 - 7.10 (m, 2H), 6.88 (d, J = 8.9 Hz, 2H), 5.73 - 5.68 (m, 1H), 4. 83 - 4.77 (m, 2H), 4. 75 - 4.71 (m, 1H), 3.77 - 3.69 (m, 4H), 3.50 - 3.44 (m, 2H), 3.06 - 3.00 (m, 4H), 1.26 (s, 6H). | 570.4 |
| 202 | (DMSO-d6) δ 10.41 (s, 1H), 9.82 (s, 1H), 8.00 (d, J = 8.0 Hz, 1H), 7. 97 - 7.85 (m, 7H), 7.58 (d, J = 8.5 Hz, 2H), 7.52 (d, J = 7.8 Hz, 1H), 7.43 (t, J = 7.9 Hz, 1H), 7.32 (s, 2H), 5.68 (t, J = 5.6 Hz, 1H), 4.83 (d, J = 5.1 Hz, 2H), 1.33 (s, 9H). | 537.0 |
| 203 | (DMSO-d6) δ 10.40 (s, 1H), 9.61 (s, 1H), 7.98 (d, J = 8.1 Hz, 1H), 7.90 (d, J = 8.2 Hz, 2H), 7.80 - 7.68 (m, 3H), 7.58 (d, J = 8.2 Hz, 2H), 7.49 (d, J = 7.6 Hz, 1H), 7.41 (t, J = 7.8 Hz, 1H), 7.33 - 7.10 (m, 4H), 5.78 - 5.61 (m, 1H), 4.87 - 4.74 (m, 2H), 4.31 - 4.19 (m, 1H), 4.07 - 3.93 (m, 2H), 2.81 - 2.64 (m, 2H), 1.33 (s, 9H). | 568.2 |
| 204 | (DMSO-d6) δ 10.42 (s, 1H), 10.17 (s, 1H), 8.11 (d, J = 8.6 Hz, 2H), 8.02 (d, J = 8.0 Hz, 1H), 7.90 (d, J = 8.2 Hz, 2H), 7.67 - 7.28 (m, 8H), 5.67 (t, J = 5.6 Hz, 1H), 4.85 (d, J = 5.4 Hz, 2H), 3.66 - 3.58 (m, 4H), 2.87 - 2.79 (m, 4H), 1.33 (s, 9H). | 618.4 |
| 205 | (DMSO-d6) δ 11.98 (s, 1H), 10.4 - 9.4 (m, 1H), 7.75 (dd, J = 7.4, 1.8 Hz, 1H), 7.53 - 7.39 (m, 2H), 7.39 - 7.00 (m, 2H), 6.95 (d, J = 1.6 Hz, 1H), 6.89 (dd, J = 12.8, 1.7 Hz, 1H), 6.79 - 5.85 (m, 1H), 5.50 - 4.77 (m, 1H), 4.53 (d, J = 10.1 Hz, 1H), 4.31 (t, J = 10.5 Hz, 1H), 4.00-3.71 (m, 2H), 3.22 (ddd, J = 15.3, 9.7, 5.3 Hz, 1H), 3.07 (dt, J = 16.0, 5.2 Hz, 1H), 2.19 (s, 3H), 1.99 (ddd, J = 8.3, 5.0, 3.3 Hz, 1H), 1.09 - 0.97 (m, 2H), 0.86 - 0.73 (m, 2H). | 501.0 |

(continued)

| | | |
|---|---|---|
| 206 | (DMSO-d6) δ 12.50 (s, 1H), 9.69 (s, 1H), 7.95 - 7.66 (m, 3H), 7.54 - 7.40 (m, 3H), 7.36 (dd, J = 12.4, 1.9 Hz, 1H), 7.30 (s, 2H), 5.22 - 4.98 (m, 1H), 4.63 - 4.50 (m, 1H), 4.40 - 4.31 (m, 1H), 3.98 - 3.78 (m, 2H), 3.26 - 3.15 (m, 2H), 1.74 (s, 6H). | 514.0 |
| 207 | (DMSO-d6) δ 12.96 - 12.07 (m, 1H), 10.74 - 9.39 (m, 1H), 7.90 - 7.70 (m, 1H), 7.70 - 7.54 (m, 1H), 7. 53 - 7.39 (m, 2H), 7.38 - 7.05 (m, 2H), 6.95 (d, J = 1.7 Hz, 1H), 6.89 (dd, J = 12.6, 1.7 Hz, 1H), 6.81 - 6.21 (m, 1H), 5.47 - 4.81 (m, 1H), 4.54 (d, J = 11.2 Hz, 1H), 4.32 (t, J = 10.9 Hz, 1H), 4.03 - 3.63 (m, 2H), 3.28 - 3.13 (m, 1H), 3.13 - 2.98 (m, 1H), 2.09 - 1.84 (m, 1H), 1.14 - 0.95 (m, 2H), 0.93 - 0.64 (m, 2H). | 487.0 |
| 208 | (DMSO-d6) δ 10.38 (s, 1H), 9.40 (s, 1H), 7.99 - 7.86 (m, 3H), 7.67 - 7.54 (m, 4H), 7.53 - 7.43 (m, 1H), 7.40 (t, J = 7.8 Hz, 1H), 7.14 (s, 2H), 6.88 (d, 2H), 5.68 (t, J = 5.8 Hz, 1H), 5.16 (s, 1H), 4.79 (d, J = 5.5 Hz, 2H), 3.77 - 3.69 (m, 4H), 3.07 - 3.00 (m, 4H), 1.46 (s, 6H). | 556.0 |

**Table 2-11**

| | | |
|---|---|---|
| 209 | (DMSO-d6) δ 10.43 (s, 1H), 9.41 (s, 1H), 8.01 - 7.94 (m, 3H), 7.65 - 7.48 (m, 5H), 7.41 (t, J = 7.8 Hz, 1H), 7.15 (s, 2H), 6.88 (d, J = 8.8 Hz, 2H), 5.66 (t, J = 5.9 Hz, 1H), 4.78 (d, J = 5.9 Hz, 2H), 3.73 (t, J = 4.7 Hz, 4H), 3.08 - 3.00 (m, 4H), 1.69 (d, J = 22.2 Hz, 6H). | 558.4 |
| 210 | (DMSO-d6) δ 10.28 - 9.43 (m, 1H), 7.85 - 7.68 (m, 1H), 7.56 (d, J = 2.3 Hz, 1H), 7.51-7.39 (m, 2H), 7.36 - 7.00 (m, 2H), 6.95 (d, J = 1.6 Hz, 1H), 6.89 (dd, J = 12.8, 1.7 Hz, 1H), 6.85 - 6.62 (m, 1H), 5.50 - 4.79 (m, 1H), 4.52 (dd, J = 11.9, 4. 7 Hz, 1H), 4.30 (dd, J = 11.9, 9.8 Hz, 1H), 3.95 - 3.76 (m, 2H), 3.74 (s, 3H), 3.28 - 3.16 (m, 1H), 3.14 - 2.95 (m, 1H), 2.13 - 1.88 (m, 1H), 1.13 - 0.96 (m, 2H), 0.91 - 0.58 (m, 2H). | 501.0 |
| 211 | (DMSO-d6) δ 9.70 (s, 1H), 8.05 (s, 1H), 7.82 - 7.72 (m, 1H), 7.58 (s, 1H), 7.54 - 7.40 (m, 2H), 7.38 - 7.12 (m, 2H), 6.98 - 6.93 (m, 1H), 6.89 (dd, J = 12.7, 1.6 Hz, 1H), 5.16 (dd, J = 9.7, 4.5 Hz, 1H), 4.61 - 4.49 (m, 1H), 4.39 - 4.29 (m, 1H), 4.13 - 4.01 (m, 2H), 3.93 - 3.74 (m, 2H), 3.26 - 3.15 (m, 1H), 3.13 - 3.03 (m, 1H), 2.04 - 1.95 (m, 1H), 1.42 - 1.29 (m, 3H), 1 . 09 - 1.01 (m, 2H), 0 . 85 - 0.77 (m, 2H). | 515.0 |
| 212 | (DMSO-d6) δ 9.70 (s, 1H), 8.01 (s, 1H), 7.76 (dd, J = 7.2, 1.9 Hz, 1H), 7.64 (s, 1H), 7.50 - 7.41 (m, 2H), 7.33 (br s, 2H), 6.95 (s, 1H), 6.89 (dd, J = 12.6, 1.7 Hz, 1H), 5.15 (dd, J = 9.7, 4. 5 Hz, 1H), 4. 54 (dd, J = 11.9, 4. 4 Hz, 1H), 4.39 - 4.30 (m, 1H), 4.24 - 4.16 (m, 2H), 3.92 - 3.84 (m, 1H), 3.84 - 3.75 (m, 1H), 3.72 - 3.60 (m, 2H), 3.23 (s, 3H), 3.24 - 3.15 (m, 1H), 3.13 - 3.04 (m, 1H), 2.04 - 1.96 (m, 1H), 1.08 - 1.03 (m, 2H), 0.84 - 0.77 (m, 2H). | 545.3 |
| 213 | (DMSO-d6) δ 9.88 - 9.02 (m, 1H), 7.93 - 7.63 (m, 1H), 7.53 - 7.34 (m, 2H), 7.32 - 7.05 (m, 2H), 7.03 - 6.75 (m, 3H), 6.69 - 6.42 (m, 1H), 6.12 - 5.91 (m, 1H), 5.28 - 4.98 (m, 1H), 4.69 - 4.36 (m, 1H), 4.33 (t, J = 10.8 Hz, 1H), 4.03 - 3.68 (m, 2H), 3.65-3.51 (m, 3H), 3.28 - 3.16 (m, 1H), 3.15 - 3.02 (m, 1H), 2.13 - 1.87 (m, 1H), 1.12 - 0.94 (m, 2H), 0.89 - 0.67 (m, 2H). | 500.1 |
| 214 | (DMSO-d6) δ 9.69 (s, 1H), 8.06 (s, 1H), 7.80 - 7.72 (m, 1H), 7.67 - 7.40 (m, 3H), 7.40 - 7.16 (m, 2H), 6.98 - 6.85 (m, 2H), 5.26 - 4.95 (m, 1H), 4.71 - 4.43 (m, 1H), 4.39 - 4.29 (m, 1H), 3.95 - 3.73 (m, 4H), 3.27 - 3.14 (m, 1H), 3.14-3.02 (m, 1H), 2.05 - 1.94 (m, 1H), 1.32 - 1.13 (m, 1H), 1.10 - 1.00 (m, 2H), 0.86 - 0.77 (m, 2H), 0.58 - 0.49 (m, 2H), 0.41 - 0.32 (m, 2H). | 541.1 |
| 215 | (DMSO-d6) δ 9.58 (s, 1H), 8.74 (s, 1H), 7.78 - 7.71 (m, 1H), 7.67 - 7.51 (m, 1H), 7.52 - 7.40 (m, 2H), 7.38 - 7.30 (m, 2H), 6.98 - 6.85 (m, 2H), 5.05 (s, 1H), 4.62 - 4.53 (m, 1H), 4.40-4.29 (m, 1H), 3.95 - 3.71 (m, 2H), 3.70 - 3.61 (m, 8H), 3.27 - 3.14 (m, 1H), 3.13 - 3.02 (m, 1H), 2.05 - 1.94 (m, 1H), 1.10 - 0.99 (m, 2H), 0.86 - 0.77 (m, 2H). | 584.1 |
| 216 | (DMSO-d6) δ 12.27 (s, 1H), 8.95 (s, 1H), 7.79 - 7.72 (m, 1H), 7.51 - 7.39 (m, 3H), 7.19 - 7.11 (m, 2H), 7.03 - 6.84 (m, 3H), 5.13 (s, 1H), 4.87 - 4.82 (m, 1H), 4.55 - 4.50 (m, 1H), 3.96 - 3.71 (m, 2H), 3.22 - 3.17 (m, 1H), 3.11 - 3.06 (m, 1H), 1.24 (s, 3H), 1.13 - 1.00 (m, 2H), 0.89 - 0.77 (m, 2H). | 501.4 |

(continued)

| | | |
|---|---|---|
| 217 | (DMSO-d6) δ 11.46 (s, 1H), 9.35 (s, 1H), 7.95 - 7.53 (m, 3H), 7.51 - 7.40 (m, 2H), 7.33 - 7.28 (m, 2H), 6.97 - 6.85 (m, 2H), 6.34 (s, 1H), 5.07 - 5.02 (m, 1H), 4.56 - 4.51 (m, 1H), 4.38 - 4.28 (m, 1H), 3.89 - 3.84 (m, 1H), 3.81 - 3.76 (m, 1H), 3.20 - 3.15 (m, 1H), 3.12 - 3.05 (m, 1H), 2.05 - 1.95 (m, 1H), 1.13 - 1.00 (m, 2H), 0.86 - 0.77 (m, 2H). | 514.4 |
| 218 | (DMSO-d6) δ 9.96 - 9.16 (m, 1H), 8.19 - 7.86 (m, 1H), 7.84 - 7.72 (m, 1H), 7.62 - 6.99 (m, 5H), 6.95 (d, J = 1.6 Hz, 1H), 6.89 (dd, J = 12.7, 1.7 Hz, 1H), 5.31 - 4.91 (m, 1H), 4.69 - 4.47 (m, 1H), 4.45 - 4.22 (m, 2H), 4.05 - 3.72 (m, 4H), 3.62 - 3.40 (m, 2H), 3.26 - 3.13 (m, 1H), 3.12 - 3.00 (m, 1H), 2.05 - 1.75 (m, 5H), 1.18 - 0.91 (m, 2H), 0.86 - 0.72 (m, 2H). | 571.1 |
| 219 | (DMSO-d6) δ 9.83 - 9.27 (m, 1H), 7.88 - 7.71 (m, 1H), 7.64 (d, J = 2.1 Hz, 1H), 7.54 - 7.08 (m, 4H), 6.95 (d, J = 1.6 Hz, 1H), 6.93 - 6.76 (m, 2H), 5.32 - 4.88 (m, 1H), 4.69 - 4.47 (m, 1H), 4.34 (d, J = 11.8 Hz, 1H), 4.29 - 4.02 (m, 2H), 3.98 - 3.61 (m, 5H), 3.26 - 3.16 (m, 1H), 3.15 - 2.98 (m, 1H), 2.04 - 1.93 (m, 1H), 1.35 - 1.19 (m, 3H), 1.12 - 0.98 (m, 2H), 0.88 - 0.69 (m, 2H). | 572.0 |
| 220 | (DMSO-d6) δ 9.50 (s, 1H), 7.75 (dd, J = 7.3, 1.8 Hz, 1H), 7.62 (s, 2H), 7.50 - 7.42 (m, 2H), 7.28 (br s, 2H), 6.98 - 6.82 (m, 4H), 5. 05 (s, 1H), 4.56 (dd, J = 11.8, 4.5 Hz, 1H), 4.34 (dd, J = 11.8, 9.6 Hz, 1H), 3.92 - 3.83 (m, 1H), 3.82 - 3.74 (m, 1H), 3 . 62 - 3. 53 (m, 4H), 3.26 - 3.16 (m, 1H), 3.13 - 2.98 (m, 5H), 2.04 (s, 3H), 2.02 - 1.95 (m, 1H), 1.08 - 1.04 (m, 2H), 0.85 - 0.76 (m, 2H). | 623.3 |
| 221 | (DMSO-d6) δ 10.00 - 9.87 (m, 1H), 7.83 - 7.67 (m, 1H), 7. 52 - 7.41 (m, 3H), 7.41 - 7.36 (m, 1H), 7.34 - 7.10 (m, 2H), 6.95 (d, J = 1.6 Hz, 1H), 6.89 (dd, J = 12.7, 1.7 Hz, 1H), 5. 11 - 4.94 (m, 1H), 4.58 - 4.44 (m, 1H), 4.38 - 4.22 (m, 1H), 3.92 - 3.74 (m, 2H), 3.65 (s, 3H), 3.25 - 3.14 (m, 1H), 3.12 - 3.02 (m, 1H), 2.05 - 1.94 (m, 1H), 1.10 - 0.99 (m, 2H), 0.85 - 0.77 (m, 2H). | 501.0 |
| 222 | (DMSO-d6) δ 9.69 (s, 1H), 8.07 (s, 1H), 7.79 - 7.73 (m, 1H), 7.56 (s, 1H), 7.53 - 7.40 (m, 2H), 7.39 - 7.25 (m, 2H), 6.95 (s, 1H), 6.89 (dd, J = 12.7, 1.6 Hz, 1H), 5.23 - 5.10 (m, 1H), 4.66 - 4.49 (m, 1H), 4.47 - 4.28 (m, 2H), 3.95 - 3.72 (m, 2H), 3.25 - 3.15 (m, 1H), 3.13 - 3.01 (m, 1H), 2.07 - 1.92 (m, 1H), 1.48 - 1.32 (m, 6H), 1.10 - 0.99 (m, 2H), 0.86 - 0.77 (m, 2H). | 529.1 |
| 223 | (DMSO-d6) δ 9.69 (s, 1H), 8.09 (s, 1H), 7.76 (dd, J = 7.2, 2.0 Hz, 1H), 7.56 (s, 1H), 7.52 - 7.42 (m, 2H), 7.32 (s, 2H), 6.95 (s, 1H), 6.93 - 6.85 (m, 1H), 5.16 (dd, J = 9.8, 4.5 Hz, 1H), 4.60 - 4.49 (m, 1H), 4.34 (t, J = 10.8 Hz, 1H), 4.16 - 4.05 (m, 1H), 3.92 - 3.84 (m, 1H), 3.84 - 3.75 (m, 1H), 3 . 26 - 3. 15 (m, 1H), 3.13 - 2.95 (m, 4H), 2.62 - 2.54 (m, 1H), 2.03 - 1.96 (m, 1H), 1.96 - 1.87 (m, 2H), 1.84 - 1.66 (m, 2H), 1.08 - 1.02 (m, 2H), 0.85 - 0.78 (m, 2H). | 570.3 |
| 224 | (DMSO-d6) δ 12.15 (s, 1H), 9.84 - 9.26 (m, 1H), 7.86 - 7.69 (m, 1H), 7.57 (d, J = 2.0 Hz, 1H), 7.52 - 7.41 (m, 2H), 7.37 - 7.17 (m, 2H), 6.95 (d, J = 1.7 Hz, 1H), 6.91 - 6.86 (m, 1H), 6.84 - 6.65 (m, 1H), 5. 25 - 4.93 (m, 1H), 4.54 (dd, J = 12.0, 4.1 Hz, 1H), 4.34 (t, J = 10.7 Hz, 1H), 3.99 - 3.69 (m, 4H), 3.75 - 3.51 (m, 1H), 3.21 (ddd, J = 14.9, 9.6, 5.2 Hz, 1H), 3.10 - 2.98 (m, 1H), 2.06 - 1.90 (m, 1H), 1.05 (dt, J = 8.3, 3.1 Hz, 2H), 0.93 - 0.73 (m, 2H). | 544.0 |
| 225 | (DMSO-d6) δ 9.70 (s, 1H), 8.04 (s, 1H), 7.76 (dd, J = 7.2, 1.9 Hz, 1H), 7.59 (s, 1H), 7.56 - 7.39 (m, 2H), 7.32 (s, 2H), 6.95 (d, J = 1.6 Hz, 1H), 6.89 (dd, J = 12.7, 1.6 Hz, 1H), 5.15 (dd, J = 9.7, 4.6 Hz, 1H), 4.59 (t, J = 4.6 Hz, 1H), 4.57 - 4.45 (m, 1H), 4.34 (t, J = 10.9 Hz, 1H), 4.11 (t, J = 7.1 Hz, 2H), 3.92 - 3.83 (m, 1H), 3.83 - 3.75 (m, 1H), 3.43 - 3.35 (m, 2H), 3.26 - 3.15 (m, 1H), 3.13 - 3.04 (m, 1H), 2.04 - 1.96 (m, 1H), 1.96 - 1.85 (m, 2H), 1.08 - 1.03 (m, 2H), 0.85 - 0.78 (m, 2H). | 545.3 |

**Table 2-12**

| | | |
|---|---|---|
| 226 | (DMSO-d6) δ 9.49 (s, 1H), 7.74 (dd, J = 7.3, 1.8 Hz, 1H), 7. 63 - 7.58 (m, 2H), 7.51 - 7.39 (m, 2H), 7.23 (s, 2H), 6.89 (d, J = 8.8 Hz, 2H), 6.51 (s, 1H), 5.05 (s, 1H), 4.59 - 4.50 (m, 1H), 4.35 (t, J = 10.7 Hz, 1H), 4.24 - 4.16 (m, 2H), 4.09 - 3.98 (m, 1H), 3.96-3.87 (m, 1H), 3.73 (t, J = 4.8 Hz, 4H), 3.05 (t, J = 4.8 Hz, 4H), 2.57 (s, 2H), 2.42 (s, 2H), 1.25 - 1.18 (m, 6H). | 581.2 |
| 227 | (DMSO-d6) δ 9.48 (s, 1H), 7.78 - 7.71 (m, 1H), 7.65 - 7.57 (m, 2H), 7.51 - 7.40 (m, 2H), 7.36 - 7.09 (m, 2H), 6.93 - 6.85 (m, 2H), 5.09 (s, 1H), 4.58 - 4.49 (m, 1H), 4.40 - 4.30 (m, 1H), 4.06 - 3.86 (m, 2H), 3.77 - 3.62 (m, 4H), 3.08 - 3.01 (m, 4H), 3.00 - 2.82 (m, 2H), 2.82 - 2.71 (m, 4H), 1.88 - 1.74 (m, 4H). | 584.4 |

(continued)

| | | |
|---|---|---|
| 228 | (DMSO-d6) δ 10.13 (s, 1H), 7.95 - 7.67 (m, 2H), 7.62 - 7.28 (m, 5H), 7.22 (d, J = 5.2 Hz, 1H), 6.98 - 6.85 (m, 2H), 5.06 (s, 1H), 4.57 (s, 1H), 4.40 - 4.30 (m, 1H), 3.93 - 3.74 (m, 2H), 3.27 - 3.15 (m, 1H), 3.13 - 3.03 (m, 1H), 2.05 - 1.94 (m, 1H), 1.12 - 1.00 (m, 2H), 0.86 - 0.77 (m, 2H). | 503.1 |
| 229 | (DMSO-d6) δ 9.47 (s, 1H), 7.75 (dd, J = 7.2, 1.9 Hz, 1H), 7.52 - 7.40 (m, 2H), 7.31 (s, 1H), 7.19 (s, 2H), 6.95 (s, 1H), 6.89 (dd, J = 12.8, 1.6 Hz, 1H), 6.68 - 6.64 (m, 1H), 6.09 - 6.03 (m, 1H), 5.22 (dd, J = 9.9, 4.4 Hz, 1H), 4. 50 (dd, J = 11.8, 4. 4 Hz, 1H), 4.33 (dd, J = 11.8, 9.9 Hz, 1H), 4.25 - 4.14 (m, 1H), 3.92 - 3.74 (m, 2H), 3.27 - 3.14 (m, 1H), 3.14-3.03 (m, 1H), 2.04 - 1.96 (m, 1H), 1.39 (d, J = 6.7 Hz, 6H), 1.08 - 1.03 (m, 2H), 0.84 - 0.78 (m, 2H). | 528.3 |
| 230 | (DMSO-d6) δ 9.63 (s, 1H), 9.04 (dd, J = 7.1, 1. 7 Hz, 1H), 8. 71 (s, 1H), 8.50 (dd, J = 4.0, 1.7 Hz, 1H), 7.86 - 7.69 (m, 1H), 7.57 - 7.35 (m, 2H), 7.35 - 7.13 (m, 2H), 7.11 - 6.77 (m, 3H), 5.14 - 5.09 (m, 1H), 4.60 - 4.52 (m, 1H), 4.39 - 4.28 (m, 1H), 3.97 - 3.75 (m, 2H), 3.31 - 3.14 (m, 1H), 3.12 - 3.03 (m, 1H), 2.05 - 1.94 (m, 1H), 1.10 - 1.00 (m, 2H), 0.88 - 0.77 (m, 2H). | 538.0 |
| 231 | (DMSO-d6) δ 9.68 - 8.98 (m, 1H), 7.88 - 7.69 (m, 1H), 7.58 - 7.38 (m, 2H), 7.27 (t, J = 2.1 Hz, 1H), 7.24 - 7.12 (m, 2H), 7.05 - 6.93 (m, 1H), 6.89 (dd, J = 12.7, 1.8 Hz, 1H), 6.68 - 6.48 (m, 1H), 6.14 - 6.00 (m, 1H), 5.29 - 4.97 (m, 1H), 4.76 - 4.45 (m, 1H), 4.33 (dd, J = 12.0, 7.3 Hz, 1H), 4.05 - 3.68 (m, 3H), 3.26 - 3.14 (m, 1H), 3.13 - 2.97 (m, 1H), 2.07 - 1.85 (m, 1H), 1.48 - 1.24 (m, 4H), 1.13 - 1.01 (m, 2H), 0.81 (dt, J = 6.7, 4.4 Hz, 2H). | 514.0 |
| 232 | (DMSO-d6) δ 9.90 - 9.00 (m, 1H), 7.89 (d, J = 5.0 Hz, 1H), 7.75 (d, J = 7.2 Hz, 1H), 7.51 - 7.37 (m, 3H), 7.36 - 7.05 (m, 2H), 6.99-6.83 (m, 3H), 6.73 - 6.61 (m, 1H), 5.29 - 4.95 (m, 1H), 4.53 (d, J = 11.0 Hz, 1H), 4.33 (t, J = 10.8 Hz, 1H), 4.01 - 3.70 (m, 4H), 3.28 - 3.13 (m, 1H), 3.13 - 2.96 (m, 1H), 2.71 - 2.66 (m, 3H), 2.04 - 1.91 (m, 1H), 1.09-1.02 (m, 2H), 0.84 - 0.79 (m, 2H). | 557.0 |
| 233 | (DMSO-d6) δ 10.05 (s, 1H), 8.50 (s, 1H), 8.11 (s, 1H), 7.76 (d, J = 7.3 Hz, 1H), 7.64 - 7.39 (m, 4H), 6.95 (d, J = 1.6 Hz, 1H), 6.89 (dd, J = 12.7, 1.6 Hz, 1H), 5.10 - 4.91 (m, 1H), 4.66 - 4.52 (m, 1H), 4.42 - 4.30 (m, 1H), 3.94 - 3.73 (m, 2H), 3.49 (s, 3H), 3.25 - 3.15 (m, 1H), 3.14 - 3.03 (m, 1H), 2.04 - 1.94 (m, 1H), 1.08 - 1.04 (m, 2H), 0.84 - 0.78 (m, 2H). | 565.2 |
| 234 | (DMSO-d6) δ 9.57 - 8.96 (m, 1H), 7.96 - 7.67 (m, 1H), 7.64 - 7.31 (m, 3H), 7.31 - 6.96 (m, 2H), 6.95 (d, J = 1.6 Hz, 1H), 6.89 (dd, J = 12.6, 1.7 Hz, 1H), 6.68 - 6.51 (m, 1H), 6.17 - 5.92 (m, 1H), 5.21 (dd, J = 9.9, 4.5 Hz, 1H), 5.07 - 4.71 (m, 2H), 4.68 - 4.43 (m, 1H), 4.42 - 4.19 (m, 1H), 4.03 - 3.71 (m, 3H), 3.72 - 3.52 (m, 2H), 3.33 - 3.16 (m, 1H), 3.15 - 2.76 (m, 1H), 2.05 - 1.92 (m, 1H), 1.12 - 0.99 (m, 2H), 0.88 - 0.76 (m, 2H). | 530.1 |
| 235 | (DMSO-d6) δ9.72 (s, 1H), 8.07 (s, 1H), 7.79 - 7.74 (m, 1H), 7.64 (s, 1H), 7.56 - 7.40 (m, 2H), 7.33 (br s, 2H), 6.95 (d, J = 1.6 Hz, 1H), 6.89 (dd, J = 12.7, 1.6 Hz, 1H), 5.14 (dd, J = 9.7, 4.5 Hz, 1H), 4.99 - 4.92 (m, 1H), 4.54 (dd, J = 12.2, 4.5 Hz, 1H), 4.04 - 4.30 (m, 1H), 4.03 - 3.75 (m, 6H), 3.26 - 3.16 (m, 1H), 3.12 - 3.03 (m, 1H), 2.43 - 2.27 (m, 2H), 2.04 - 1.95 (m, 1H), 1 . 09 - 1.02 (m, 2H), 0.85 - 0.77 (m, 2H). | 557.2 |
| 236 | (DMSO-d6) δ9.72 (s, 1H), 8.07 (s, 1H), 7.79 - 7.74 (m, 1H), 7.64 (s, 1H), 7.56 - 7.40 (m, 2H), 7.34 (s, 2H), 6.95 (d, J = 1.6 Hz, 1H), 6.89 (dd, J = 12.7, 1.6 Hz, 1H), 5.15 (dd, J = 9.7, 4.5 Hz, 1H), 4.99 - 4.92 (m, 1H), 4.54 (dd, J = 11.9, 4.5 Hz, 1H), 4.40 - 4.30 (m, 1H), 4.03 - 3.75 (m, 6H), 3.26 - 3.16 (m, 1H), 3.13 - 3.04 (m, 1H), 2.43 - 2.25 (m, 2H), 2.04 - 1.95 (m, 1H), 1.09 - 1.02 (m, 2H), 0.85 - 0.77 (m, 2H). | 557.2 |
| 237 | (DMSO-d6) δ9.70 (s, 1H), 8.04 (s, 1H), 7.80 - 7.74 (m, 1H), 7.63 (s, 1H), 7.52 - 7.40 (m, 2H), 7.38 - 7.20 (m, 2H), 6.95 (s, 1H), 6.89 (dd, J = 12.7, 1.7 Hz, 1H), 5. 15 (dd, J = 9.7, 4. 5 Hz, 1H), 4. 54 (dd, J = 11.9, 4. 5 Hz, 1H), 4.39 - 4.30 (m, 1H), 4.21 - 4.11 (m, 2H), 3.93 - 3.83 (m, 1H), 3.83 - 3.74 (m, 1H), 3.60 - 3.45 (m, 4H), 3.26 - 3.16 (m, 1H), 3.13 - 3.03 (m, 1H), 2.75 - 2.62 (m, 2H), 2.46 - 2.35 (m, 4H), 2.04 - 1.96 (m, 1H), 1.08 - 1.00 (m, 2H), 0.85 - 0.76 (m, 2H). | 600.3 |
| 238 | (DMSO-d6) δ 9.69 (s, 1H), 8.09 (s, 1H), 7.81 - 7.72 (m, 1H), 7.55 - 7.40 (m, 3H), 7.37 - 7.32 (m, 2H), 6.98 - 6.85 (m, 2H), 5.19 - 5.10 (m, 1H), 4. 58 - 4.50 (m, 1H), 4.39 - 4.28 (m, 1H), 3.90 - 3.75 (m, 2H), 3.71 - 3.61 (m, 1H), 3.28 - 3.14 (m, 1H), 3.14 - 3.03 (m, 1H), 2.05 - 1.94 (m, 1H), 1.18 - 0.63 (m, 8H). | 527.3 |

(continued)

| | | |
|---|---|---|
| 239 | (DMSO-d6) δ 9.68 (s, 1H), 8.06 (s, 1H), 7.83 - 7.73 (m, 1H), 7.67 - 7.52 (m, 1H), 7.52 - 7.40 (m, 2H), 7.34 - 7.29 (m, 2H), 6.98 - 6.85 (m, 2H), 5.20 - 5.12 (m, 1H), 4.68 - 4.50 (m, 2H), 4.39 - 4.29 (m, 1H), 3.92 - 3.75 (m, 2H), 3.27 - 3.14 (m, 1H), 3.12 - 3.03 (m, 1H), 2.27 - 1.39 (m, 9H), 1.10 - 1.00 (m, 2H), 0.86 - 0.77 (m, 2H). | 555.2 |
| 240 | (DMSO-d6) δ 9.80 (s, 1H), 8.14 - 7.99 (m, 1H), 7.85 (s, 1H), 7.82 - 7.70 (m, 1H), 7.53 - 7.41 (m, 2H), 7.41 - 7.03 (m, 2H), 6.95 (d, J = 1.6 Hz, 1H), 6.89 (dd, J = 12.6, 1.7 Hz, 1H), 5.17 - 4.96 (m, 3H), 4.63 - 4.50 (m, 1H), 4.42 - 4.29 (m, 1H), 3.94 - 3.74 (m, 2H), 3.26 - 3.15 (m, 1H), 3.13 - 3.02 (m, 1H), 2.05 - 1.93 (m, 1H), 1.10 - 0.99 (m, 2H), 0 . 86 - 0.77 (m, 2H). | 569.2 |
| 241 | (DMSO-d6) δ 9.67 (s, 1H), 8.11 (s, 1H), 7.76 (d, J = 7.3 Hz, 1H), 7.56 (s, 1H), 7.51 - 7.42 (m, 2H), 7.40 - 7.27 (m, 2H), 6.95 (s, 1H), 6.89 (d, J = 12.7 Hz, 1H), 5.21 - 5.12 (m, 1H), 4.65 - 4.48 (m, 1H), 4.41 - 4.29 (m, 1H), 3.92 - 3.73 (m, 2H), 3.25 - 3.14 (m, 1H), 3.13 - 3.03 (m, 1H), 2.04 - 1.95 (m, 1H), 1.56 - 1.45 (m, 9H), 1.10 - 1.01 (m, 2H), 0.85 - 0.76 (m, 2H). | 543.3 |
| 242 | (DMSO-d6) δ 9.72 (s, 1H), 7.84 (s, 1H), 7.75 (dd, J = 7.3, 1.9 Hz, 1H), 7.61 - 7.10 (m, 5H), 7.06 - 6.93 (m, 2H), 6.89 (d, J = 12.8 Hz, 1H), 5.10 - 4.84 (m, 1H), 4.60 (dd, J = 11.7, 4.1 Hz, 1H), 4.35 (dd, J = 11.9, 9.0 Hz, 1H), 3.96 - 3.83 (m, 1H), 3.83 - 3.77 (m, 1H), 3.74 (t, J = 4.6 Hz, 4H), 3.27 - 3.13 (m, 1H), 3.15 - 3.01 (m, 1H), 2.95 (t, J = 4.6 Hz, 4H), 2.12 - 1.89 (m, 1H), 1 . 13 - 0.98 (m, 2H), 0.90 - 0.72 (m, 2H). | 600.6 |

**Table 2-13**

| | | |
|---|---|---|
| 243 | (DMSO-d6) δ 9.55 (s, 1H), 7.78 (d, J = 7.2 Hz, 1H), 7.55 (s, 1H), 7.51 - 7.43 (m, 2H), 7.38 (s, 1H), 7.20 (s, 2H), 7.12 (t, J = 8.1 Hz, 1H), 6.95 (s, 1H), 6.89 (d, J = 12.6 Hz, 1H), 6.60 (d, J = 8.2 Hz, 1H), 5.15 - 4.88 (m, 1H), 4.60 (d, J = 11.0 Hz, 1H), 4.35 (t, J = 10.5 Hz, 1H), 3.97 - 3.84 (m, 1H), 3.84 - 3.65 (m, 5H), 3.28 - 3.14 (m, 1H), 3.15 - 2.97 (m, 5H), 2.11 - 1.87 (m, 1H), 1.12 - 0.95 (m, 2H), 0.90 - 0.65 (m, 2H). | 582.4 |
| 244 | (DMSO-d6) δ 9.49 - 9.23 (m, 1H), 7.74 (d, J = 7.3 Hz, 1H), 7.63 - 7.38 (m, 4H), 7.30 - 7.01 (m, 2H), 6.95 (s, 1H), 6.89 (d, J = 12.7 Hz, 1H), 6.49 (d, J = 8.6 Hz, 2H), 5.26 - 4.85 (m, 1H), 4.70 - 4.42 (m, 1H), 4.40 - 4.23 (m, 1H), 3.96 - 3.65 (m, 2H), 3.28-3.11 (m, 5H), 3.13 - 2.96 (m, 1H), 2.08 - 1.83 (m, 5H), 1.06 (dd, J = 8.5, 5.7 Hz, 2H), 0.87 - 0.73 (m, 2H). | 566.2 |
| 245 | (DMSO-d6) δ 9.36 (s, 1H), 7.75 (d, J = 7.3 Hz, 1H), 7.56 - 7.37 (m, 4H), 7.29 - 7.04 (m, 2H), 6.95 (s, 1H), 6.89 (d, J = 12.7 Hz, 1H), 6.62 (d, J = 8.6 Hz, 2H), 5.20 - 5.03 (m, 1H), 3.23 - 3.15 (m, 1H), 4.59 - 4.47 (m, 1H), 4.38 - 4.27 (m, 1H), 3.93 - 3.72 (m, 2H), 3.31 - 3.26 (m, 4H), 3.12 - 3.02 (m, 1H), 2.05 - 1.94 (m, 1H), 1 . 11 - 1.01 (m, 8H), 0.84 - 0.77 (m, 2H). | 568.2 |
| 246 | (DMSO-d6) δ 9.37 (s, 1H), 7.75 (d, J = 7.5 Hz, 1H), 7. 57 - 7.35 (m, 4H), 7.30 - 7.05 (m, 2H), 6.95 (s, 1H), 6.92 - 6.85 (m, 1H), 6.65 (d, J = 8.8 Hz, 2H), 5.19 - 5.04 (m, 1H), 4.69 - 4.59 (m, 1H), 4.58 - 4.48 (m, 1H), 4.39 - 4.27 (m, 1H), 3.91 - 3.72 (m, 2H), 3.60 - 3.47 (m, 2H), 3.36 - 3.33 (m, 2H), 3.25 - 3.14 (m, 1H), 3.13 - 3.02 (m, 1H), 2.89 (s, 3H), 2.05 - 1.93 (m, 1H), 1.10 - 1.00 (m, 2H), 0.86 - 0.76 (m, 2H). | 570.2 |
| 247 | (DMSO-d6) δ 9.24 - 8.55 (m, 1H), 7.77 (dd, J = 7.3, 2.0 Hz, 1H), 7.65 - 7.35 (m, 4H), 7.18 (s, 1H), 6.95 (d, J = 1.6 Hz, 1H), 6.89 (dd, J = 12.6, 1.7 Hz, 1H), 4. 98 (dd, J = 9.7, 4.6 Hz, 1H), 4.56 (d, J = 9.8 Hz, 1H), 4.40 - 4.17 (m, 1H), 4.12 - 3.95 (m, 2H), 3.97 - 3.70 (m, 2H), 3.28 - 3.16 (m, 1H), 3.14 - 3.03 (m, 1H), 3.00 (s, 3H), 2.05 - 1.92 (m, 1H), 1.10 - 0.98 (m, 2H), 0.87 - 0.74 (m, 2H). | 516.0 |
| 248 | (DMSO-d6) δ 9.48 (s, 1H), 7.75 (dd, J = 7.4, 1.8 Hz, 1H), 7.56 - 7.40 (m, 2H), 7.31 - 7.27 (m, 1H), 7.25 - 7.10 (m, 2H), 6.95 (d, J = 1.6 Hz, 1H), 6.89 (dd, J = 12.7, 1.6 Hz, 1H), 6.65 - 6.60 (m, 1H), 6.10 - 6.05 (m, 1H), 5.21 (dd, J = 9.8, 4.4 Hz, 1H), 4. 51 (dd, J = 12.0, 4. 4 Hz, 1H), 4.38-4.28 (m, 1H), 3.94 - 3.75 (m, 2H), 3.68 (d, J = 7.0 Hz, 2H), 3.27-3.15 (m, 1H), 3.13 - 3.02 (m, 1H), 2.03 - 1.96 (m, 1H), 1.21 - 1.08 (m, 1H), 1 . 08 - 1 . 02 (m, 2H), 0.84 - 0.78 (m, 2H), 0.56 - 0.45 (m, 2H), 0.36 - 0.26 (m, 2H). | 540.3 |

(continued)

| | | |
|---|---|---|
| 249 | (DMSO-d6) δ 9.50 (s, 1H), 7.75 (d, J = 7.8 Hz, 1H), 7.51 - 7.40 (m, 2H), 7.29 (s, 1H), 7.19 (br s, 2H), 6.95 (d, J = 1.6 Hz, 1H), 6.89 (dd, J = 12.6, 1.6 Hz, 1H), 6.70 - 6.64 (m, 1H), 6.15 - 6.11 (m, 1H), 5.20 (dd, J = 9.5, 4.5 Hz, 1H), 4.75 - 4 67(m, 1H), 4.56 - 4.46 (m, 1H), 4.37 - 4.28 (m, 1H), 4.05 - 3.70 (m, 6H), 3.26 - 3.15 (m, 1H), 3.13 - 3.03 (m, 1H), 2.44 - 2.35 (m, 1H), 2.14 - 2.04 (m, 1H), 2.04 - 1.95 (m, 1H), 1.08 - 1.03 (m, 2H), 0.85 - 0.78 (m, 2H). | 556.2 |
| 250 | (DMSO-d6) δ9.50 (s, 1H), 7.75 (d, J = 7.8 Hz, 1H), 7.52 - 7.40 (m, 2H), 7.29 (s, 1H), 7.23 - 7.15 (m, 2H), 6.95 (d, J = 1.6 Hz, 1H), 6.89 (dd, J = 12.7, 1.6 Hz, 1H), 6.70 - 6.64 (m, 1H), 6.15 - 6.11 (m, 1H), 5.20 (dd, J = 9.8, 4. 4 Hz, 1H), 4.76 - 4.67 (m, 1H), 4.56 - 4.46 (m, 1H), 4.37 - 4.28 (m, 1H), 4.08 - 3.70 (m, 6H), 3.26 - 3.15 (m, 1H), 3.12 - 3.03 (m, 1H), 2.44 - 2.35 (m, 1H), 2.15 - 2.04 (m, 1H), 2.04 - 1.95 (m, 1H), 1.08 - 1.02 (m, 2H), 0.84 - 0.78 (m, 2H). | 556.2 |
| 251 | (DMSO-d6) δ 9.71 - 9.17 (m, 1H), 7.94 - 7.68 (m, 1H), 7.54 - 7.41 (m, 2H), 7.38 (d, J = 1.8 Hz, 1H), 7.34 - 7.21 (m, 2H), 7.21 - 7.07 (m, 2H), 6.95 (d, J = 1.7 Hz, 1H), 6.89 (dd, J = 12.7, 1.7 Hz, 1H), 6.84 - 6.68 (m, 1H), 5.27 - 4.89 (m, 1H), 4.53 (dd, J = 11.8, 4.5 Hz, 1H), 4.33 (dd, J = 11.8, 9.9 Hz, 1H), 4.01 - 3.65 (m, 5H), 3.28 - 3.12 (m, 1H), 3.15 - 2.92 (m, 1H), 2.11 - 1.82 (m, 1H), 1.12 - 0.98 (m, 2H), 0.87 - 0.73 (m, 2H). | 543.0 |
| 252 | (DMSO-d6) δ 9.71 (s, 1H), 8.13 (s, 1H), 7.76 (dd, J = 7.1, 2.0 Hz, 1H), 7.60 - 7.41 (m, 3H), 7.34 (s, 2H), 6.95 (s, 1H), 6.89 (dd, J = 12.7, 1.7 Hz, 1H), 5.16 (dd, J = 9.7, 4. 5 Hz, 1H), 4. 53 (dd, J = 12.0, 4. 5 Hz, 1H), 4.39 - 4.30 (m, 1H), 4.08 - 3.97 (m, 1H), 3.92 - 3.83 (m, 1H), 3.83 - 3.74 (m, 1H), 3.25 - 3.15 (m, 1H), 3.12 - 2.99 (m, 1H), 2.90 - 2.78 (m, 2H), 2.50 - 2.40 (m, 2H), 2.21 (s, 3H), 2.09 - 1.85 (m, 5H), 1.08 - 0.97 (m, 2H), 0.85 - 0.76 (m, 2H). | 584.3 |
| 253 | (DMSO-d6) δ9.74 (s, 1H), 7.95 (s, 1H), 7.76 (dd, J = 7.2, 2. 0 Hz, 1H), 7.67 (s, 1H), 7.51 - 7.42 (m, 2H), 7.38 - 7.20 (m, 2H), 6.95 (s, 1H), 6.93 - 6.85 (m, 1H), 5.15 (dd, J = 9.7, 4.5 Hz, 1H), 5.08 (s, 2H), 4.54 (dd, J = 11.9, 4.5 Hz, 1H), 4.39 - 4.30 (m 1H), 3.92 - 3.83 (m, 1H), 3.83 - 3.74 (m, 1H), 3.65 - 3.54 (m, 4H), 3.54 - 3.40 (m, 4H), 3.26 - 3.15 (m, 1H), 3.13 - 3.04 (m, 1H), 2.04 - 1.95 (m, 1H), 1.08 - 1.01 (m, 2H), 0.85 - 0.76 (m, 2H). | 614.3 |
| 254 | (DMSO-d6) δ 9.63 - 8.93 (m, 1H), 7.86 - 7.66 (m, 1H), 7.52 - 7.35 (m, 2H), 7.33 - 7.05 (m, 2H), 7.03 - 6.93 (m, 1H), 6.89 (dd, J = 12.5, 1.7 Hz, 1H), 6.62 - 6.50 (m, 1H), 6.12 - 5.97 (m, 1H), 5.26 - 4.88 (m, 1H), 4.65 - 4.45 (m, 1H), 4.39 - 4.25 (m, 1H), 4.02 - 3.75 (m, 5H), 3.77 - 3.62 (m, 2H), 3.39 - 3.31 (m, 2H), 3.29 - 3.15 (m, 2H), 3.13 - 2.98 (m, 1H), 2.12 - 1. 72 (m, 1H), 1.40 (d, J = 12.8 Hz, 2H), 1.19 (t, J = 11.2 Hz, 2H), 1.12 - 0.97 (m, 2H), 0.90 - 0.68 (m, 2H). | 584.0 |
| 255 | (DMSO-d6) δ 11.20 (s, 1H), 9.61 (s, 1H), 7.84 - 7.69 (m, 1H), 7.51 - 7.35 (m, 4H), 7.30 - 7.13 (m, 2H), 6.99 - 6.83 (m, 3H), 6.82 - 6.69 (m, 1H), 5.23 - 5.11 (m, 1H), 4. 59 - 4.47 (m, 1H), 4.39 - 4.25 (m, 1H), 3.94 - 3.72 (m, 2H), 3.26 - 3.15 (m, 1H), 3.13 - 3.01 (m, 1H), 2.05 - 1.94 (m, 1H), 1.09 - 1.02 (m, 2H), 0.86 - 0.77 (m, 2H). | 529.2 |
| 256 | (DMSO-d6) δ 10.52 (s, 1H), 8.35 (s, 1H), 7.76 (d, J = 7.3 Hz, 1H), 7. 55 - 7.22 (m, 4H), 6.95 (s, 1H), 6.89 (d, J = 12.6 Hz, 1H), 5.03 - 4.85 (m, 1H), 4.62 - 4.49 (m, 1H), 4.40 - 4.27 (m, 1H), 4.04 (s, 3H), 3.95 - 3.72 (m, 2H), 3.26 - 3.15 (m, 1H), 3.12 - 3.02 (m, 1H), 2.05 - 1.94 (m, 1H), 1.10 - 0.98 (m, 2H), 0.88 - 0.75 (m, 2H). | 501.8 |
| 257 | (DMSO-d6) δ 9.72 (s, 1H), 8.03 (s, 1H), 7.82 - 7.70 (m, 1H), 7.62 (s, 1H), 7.53 - 7.41 (m, 2H), 7.39 - 7.21 (m, 2H), 6.95 (s, 1H), 6.89 (d, J = 12.7 Hz, 1H), 5.20 - 5.10 (m, 1H), 4.62 - 4.47 (m, 1H), 4.40 - 4.27 (m, 1H), 4.01 - 3.70 (m, 6H), 3.28 - 3.15 (m, 3H), 3.13 - 3.01 (m, 1H), 2.13 - 1.92 (m, 2H), 1.47 - 1.34 (m, 2H), 1.31 - 1.14 (m, 2H), 1 . 11 - 1.00 (m, 2H), 0.87 - 0.78 (m, 2H). | 585.2 |
| 258 | (DMSO-d6) δ 9.84 (s, 1H), 7.72 (dd, J = 7.1, 2.0 Hz, 1H), 7.62 - 7.43 (m, 4H), 7.41 - 7.31 (m, 1H), 7.15 (d, J = 2.3 Hz, 1H), 6.95 (s, 1H), 6.89 (d, J = 12.5 Hz, 1H), 6.56 (dd, J = 7.5, 2.4 Hz, 1H), 4.93 - 4.81 (m, 1H), 4.70 - 4.58 (m, 1H), 4.42 - 4.30 (m, 1H), 3.95 - 3.83 (m, 1H), 3.82 - 3.72 (m, 1H), 3.34 (s, 3H), 3.27 - 3.13 (m, 1H), 3.13 - 3.02 (m, 1H), 2.06 - 1.93 (m, 1H), 1.14 - 0.99 (m, 2H), 0.88 - 0.75 (m, 2H). | 528.4 |

Table 2-14

| | | |
|---|---|---|
| 259 | (DMSO-d6) δ 9.58 - 9.07 (m, 1H), 8.45 - 7.88 (m, 1H), 7.75 (dd, J = 7.2, 2.0 Hz, 1H), 7.66 - 7.16 (m, 5H), 7.05 - 6.79 (m, 2H), 6.50 - 6.23 (m, 1H), 5.26 - 4.75 (m, 1H), 4.60 - 4.51 (m, 1H), 4.39 - 4.28 (m, 1H), 3.93 - 3.75 (m, 2H), 3.45 (s, 3H), 3.27 - 2.99 (m, 2H), 2.07 - 1.92 (m, 1H), 1.14 - 0.96 (m, 2H), 0.92 - 0.70 (m, 2H). | 528.0 |
| 260 | (DMSO-d6) δ 9.83 - 9.38 (m, 1H), 7.96 (s, 1H), 7.84 - 7.73 (m, 1H), 7.66 (s, 1H), 7.51 - 7.40 (m, 2H), 7.38 - 7.19 (m, 2H), 6.98 - 6.85 (m, 2H), 5.19 - 5.11 (m, 1H), 4.96 (s, 2H), 4.59 - 4.51 (m, 1H), 4.39 - 4.29 (m, 1H), 3.90 - 3.77 (m, 2H), 3.55-3.25 (m, 4H), 3.25 - 2.99 (m, 2H), 2.05 - 1.95 (m, 1H), 1 . 95 - 1.70 (m, 4H), 1.13 - 0.96 (m, 2H), 0.92 - 0.72 (m, 2H). | 598.2 |
| 261 | (DMSO-d6) δ 9.73 (s, 1H), 8.05 (s, 1H), 7.89 (s, 1H), 7.75 (d, J = 7.2 Hz, 1H), 7.66 (s, 1H), 7.55 - 7.40 (m, 1H), 7.40 - 7.19 (m, 2H), 7.01 - 6.85 (m, 2H), 5.29 - 4.89 (m, 1H), 4.64 - 4.50 (m, 3H), 4.39 - 4.10 (m, 5H), 3.96 - 3.71 (m, 2H), 3.26 - 3.01 (m, 2H), 2.05 - 1.94 (m, 1H), 1.29 - 1.10 (m, 3H), 1.10 - 1.00 (m, 2H), 0.90 - 0.73 (m, 2H). | 571.2 |
| 262 | (DMSO-d6) δ 9.70 (s, 1H), 8.04 (s, 1H), 7.80 - 7.73 (m, 1H), 7.59 (s, 1H), 7.52 - 7.40 (m, 2H), 7.37 - 7.23 (m, 2H), 6.95 (s, 1H), 6.93 - 6.85 (m, 1H), 5.15 (dd, J = 9.7, 4.5 Hz, 1H), 4.53 (dd, J = 11.9, 4.5 Hz, 1H), 4.39 - 4.29 (m, 1H), 4.01 (t, J = 7.1 Hz, 2H), 3.92 - 3.83 (m, 1H), 3.83 - 3.74 (m, 1H), 3.26 - 3.15 (m, 1H), 3.12 - 3.03 (m, 1H), 2.04 - 1.95 (m, 1H), 1 . 84 - 1.70 (m, 2H), 1 . 08 - 1.01 (m, 2H), 0.89 - 0.78 (m, 5H). | 529.3 |
| 263 | (DMSO-d6) δ 10.52 (s, 1H), 8.22 (s, 1H), 7.75 (d, J = 7.2 Hz, 1H), 7.53 - 7.31 (m, 4H), 6.98 - 6.85 (m, 2H), 4.96 - 4.91 (m, 1H), 4.61 - 4.52 (m, 1H), 4.38 - 4.27 (m, 1H), 4.06 (s, 3H), 3.93 - 3.74 (m, 2H), 3.28 - 2.99 (m, 2H), 2.05 - 1.94 (m, 1H), 1.12 - 1.00 (m, 2H), 0.86 - 0.77 (m, 2H). | 500.6 [M-H] |
| 264 | (DMSO-d6) δ 8.26 (s, 1H), 7.90 - 7.60 (m, 2H), 7.59 - 7.38 (m, 3H), 7.40 - 7.12 (m, 2H), 7.02 - 6.78 (m, 2H), 5.16 - 4.69 (m, 1H), 4.66 - 4.16 (m, 2H), 3.99 - 3.66 (m, 2H), 3.25 - 2.99 (m, 2H), 2.10 - 1.88 (m, 1H), 1.05 (dt, J = 8.4, 3.3 Hz, 2H), 0.87 - 0.75 (m, 2H). | 488.4 |
| 265 | (DMSO-d6) δ 8.31 (s, 1H), 7.81 - 7.74 (m, 1H), 7.52 - 7.10 (m, 5H), 6.97 - 6.85 (m, 2H), 5.69 - 5.49 (m, 1H), 4.50 - 4.42 (m, 1H), 4.27 - 4.22 (m, 1H), 3.87 - 3.80 (m, 2H), 3.76 (s, 3H), 3.25 - 2.94 (m, 2H), 2.05 - 1.96 (m, 1H), 1.10 - 1.00 (m, 2H), 0.89 - 0.78 (m, 2H). | 502.4 |
| 266 | (DMSO-d6) δ 9.75 (s, 1H), 8.01 (s, 1H), 7.80 - 7.72 (m, 1H), 7.67 (s, 1H), 7.60 - 7.12 (m, 6H), 6.98 - 6.85 (m, 2H), 5.20 - 5.11 (m, 1H), 4.69 (s, 2H), 4.58 - 4.49 (m, 1H), 4.39 - 4.28 (m, 1H), 4.02 - 3.64 (m, 2H), 3.26 - 2.93 (m, 2H), 2.05 - 1.94 (m, 1H), 1.10 - 1.00 (m, 2H), 0.86 - 0.77 (m, 2H). | 544.3 |
| 267 | (DMSO-d6) δ 9.94 - 9.24 (m, 1H), 7.76 (d, J = 7.6 Hz, 1H), 7.61 (s, 1H), 7.57 - 7.37 (m, 3H), 7.28 (d, J = 14.2 Hz, 2H), 6.95 (s, 1H), 6.89 (d, J = 12.7 Hz, 1H), 6.78 - 6.43 (m, 1H), 5.28 - 4.88 (m, 1H), 4.71 - 4.45 (m, 1H), 4.43 - 4.22 (m, 1H), 4.03 (t, J = 5.9 Hz, 2H), 3.96 - 3.68 (m, 2H), 3.65 - 3.39 (m, 2H), 3.29 - 3.14 (m, 1H), 3.14 - 2.87 (m, 1H), 2.16 - 1.78 (m, 1H), 1.13 - 0.95 (m, 2H), 0.93 - 0.56 (m, 2H). | 555.2 |
| 268 | (DMSO-d6) δ 9.84 - 9.23 (m, 1H), 7.95 - 7.66 (m, 1H), 7.61 - 7.38 (m, 3H), 7.40 - 7.06 (m, 2H), 6.95 (d, J = 1.6 Hz, 1H), 6.89 (dd, J = 12.7, 1.6 Hz, 1H), 6.74 - 6.57 (m, 1H), 5.26 - 4.91 (m, 1H), 4.54 (dd, J = 11.8, 4.4 Hz, 1H), 4.34 (t, J = 10.9 Hz, 1H), 4.11 (t, J = 6.1 Hz, 2H), 4.01 - 3.73 (m, 2H), 3.71 - 3.54 (m, 2H), 3.28 - 3.13 (m, 1H), 3.13 - 3.02 (m, 1H), 2.99 - 2.89 (m, 3H), 2.09 - 1.89 (m, 1H), 1.13 - 1.00 (m, 2H), 0.87 - 0.74 (m, 2H). | 569.2 |
| 269 | (DMSO-d6) δ 9.94 - 9.34 (m, 1H), 7.92 - 7.67 (m, 1H), 7.59 - 7.40 (m, 3H), 7.33 (d, J = 22.9 Hz, 2H), 7.04 (d, J = 1.8 Hz, 1H), 6.95 (d, J = 1.6 Hz, 1H), 6.89 (dd, J = 12.7, 1.7 Hz, 1H), 5.30 - 4.90 (m, 1H), 4.54 (dd, J = 11.8, 4.5 Hz, 1H), 4.46 - 4.22 (m, 1H), 4.03 - 3.58 (m, 5H), 3.27 - 3.13 (m, 1H), 3.11 - 2.94 (m, 1H), 2.17 - 1.90 (m, 1H), 1.09 - 0.93 (m, 2H), 0.88 - 0.70 (m, 2H). | 525.0 |
| 270 | (DMSO-d6) δ 9.69 (s, 1H), 8.10 (s, 1H), 7.90 - 7.67 (m, 1H), 7.58 (s, 1H), 7.54 - 7.38 (m, 2H), 7.40 - 7.19 (m, 2H), 7.04 - 6.79 (m, 2H), 5.29 - 4.93 (m, 1H), 4.73 - 4.19 (m, 2H), 3.97 - 3.70 (m, 2H), 3.64 - 3.52 (m, 1H), 3.27 - 3.14 (m, 1H), 3.14 - 3.03 (m, 1H), 2.05 - 1.94 (m, 1H), 1.54 - 1.42 (m, 3H), 1.29 - 1.16 (m, 1H), 1.10 - 0.97 (m, 2H), 0.90 - 0.77 (m, 2H), 0.63 - 0.54 (m, 1H), 0.50 - 0.40 (m, 1H), 0.36 - 0.28 (m, 2H). | 555.1 |

(continued)

| | | |
|---|---|---|
| 271 | (DMSO-d6) δ 9.71 (s, 1H), 8.06 (s, 1H), 7.76 (dd, J = 7.3, 1.9 Hz, 1H), 7.57 (s, 1H), 7.53 - 7.40 (m, 2H), 7.39 - 7.24 (m, 2H), 6.95 (d, J = 1.6 Hz, 1H), 6.89 (dd, J = 12.7, 1.7 Hz, 1H), 5.22 - 5.09 (m, 1H), 4.66 - 4.48 (m, 1H), 4.40 - 4.28 (m, 1H), 4.23 - 4.10 (m, 1H), 3.94 - 3.72 (m, 2H), 3.25 - 3.14 (m, 1H), 3.13 - 3.02 (m, 1H), 2.04 - 1.94 (m, 1H), 1.88 - 1.63 (m, 2H), 1.41 (d, J = 6.7 Hz, 3H), 1.08-1.02 (m, 2H), 0.84 - 0.79 (m, 2H), 0.74 (t, J = 7.3 Hz, 3H). | 543.3 |
| 272 | (DMSO-d6) δ 9.65 - 9.06 (m, 1H), 7.89 - 7.68 (m, 1H), 7.55 - 7.39 (m, 2H), 7.38 - 7.02 (m, 3H), 6.95 (d, J = 1.7 Hz, 1H), 6.89 (dd, J = 12.7, 1.6 Hz, 1H), 6.48 (d, J = 1.8 Hz, 1H), 5.40 - 4.90 (m, 1H), 4.68-4.41 (m, 1H), 4.43 - 4.20 (m, 1H), 4.05 - 3.72 (m, 2H), 3.73 - 3.51 (m, 3H), 3.26 - 3.14 (m, 1H), 3.15 - 2.92 (m, 7H), 2.07 - 1.90 (m, 1H), 1 . 08 - 0.97 (m, 2H), 0.90 - 0.69 (m, 2H). | 571.2 |
| 273 | (DMSO-d6) δ 9.71 (s, 1H), 8.05-7.91 (m, 1H), 7.90 - 7.72 (m, 1H), 7.67 - 7.51 (m, 1H), 7.52 - 7.40 (m, 2H), 7.40 - 7.19 (m, 2H), 6.98 - 6.85 (m, 2H), 5.20 - 5.03 (m, 1H), 4.63 - 4.49 (m, 1H), 4.39 - 4.29 (m, 1H), 3.93 - 3.74 (m, 4H), 3.30 - 3.14 (m, 1H), 3.14 - 3.03 (m, 1H), 2.05 - 1.94 (m, 1H), 1.10 - 0.99 (m, 2H), 1.02 - 0.93 (m, 3H), 0.90 - 0.77 (m, 2H), 0.66 - 0.57 (m, 2H), 0.40 - 0.32 (m, 2H). | 555.0 |
| 274 | (DMSO-d6) δ 9.72 (s, 1H), 8.01 (s, 1H), 7.81 - 7.73 (m, 1H), 7.65 (s, 1H), 7.56 - 7.41 (m, 2H), 7.38 - 7.25 (m, 2H), 6.95 (d, J = 1.7 Hz, 1H), 6.89 (dd, J = 12.7, 1.7 Hz, 1H), 6.10 - 5.92 (m, 1H), 5.22 - 5.08 (m, 3H), 4.75 - 4.66 (m, 2H), 4. 61 - 4. 50 (m, 1H), 4.40 - 4.30 (m, 1H), 3.91 - 3.84 (m, 1H), 3.84 - 3.75 (m, 1H), 3.25 - 3.16 (m, 1H), 3.12 - 3.03 (m, 1H), 2.04 - 1.95 (m, 1H), 1.08 - 1.02 (m, 2H), 0.84 - 0.78 (m, 2H). | 527.3 |
| 275 | (DMSO-d6) δ 9.89 - 9.34 (m, 1H), 8.14 - 7.87 (m, 1H), 7.85 - 7.55 (m, 2H), 7.53 - 7.40 (m, 2H), 7.41 - 7.01 (m, 2H), 6.95 (d, J = 1.6 Hz, 1H), 6.89 (dd, J = 12.7, 1.7 Hz, 1H), 6. 54 - 6.10 (m, 1H), 5.25 - 4.88 (m, 1H), 4.66 - 4.47 (m, 3H), 4.42 - 4.25 (m, 1H), 3.96 - 3.83 (m, 1H), 3.83 - 3.73 (m, 1H), 3.25 - 3.14 (m, 1H), 3.13 - 3.01 (m, 1H), 2.06 - 1.89 (m, 1H), 1.13 - 0.95 (m, 2H), 0.88 - 0.70 (m, 2H). | 551.2 |

**Table 2-15**

| | | |
|---|---|---|
| 276 | (DMSO-d6) δ 9.99 - 9.07 (m, 1H), 8.09 (s, 1H), 7.97 - 7.78 (m, 1H), 7.65 - 7.51 (m, 2H), 7.50 - 7.41 (m, 1H), 7.40 - 7.35 (m, 2H), 7.33 (d, J = 7.5 Hz, 1H), 7.28 (d, J = 1.6 Hz, 1H), 7.00 (dd, J = 13.1, 1.7 Hz, 1H), 6.62 (dd, J = 7.5, 2.1 Hz, 1H), 5.32 - 4.89 (m, 1H), 4.49 (dd, J = 12.1, 5.0 Hz, 1H), 4.08 (dd, J = 12.1, 9.2 Hz, 1H), 3.79 - 3.52 (m, 1H), 2.18 - 1.96 (m, 1H), 1.15-1.06 (m, 2H), 1.07 - 1.00 (m, 2H), 1.00 - 0.91 (m, 2H), 0.92 - 0.83 (m, 2H). | 525.2 |
| 277 | (DMSO-d6) δ9.48 (s, 1H), 7.78 - 7.72 (m, 1H), 7.54-7.19 (m, 5H), 6.97 - 6.94 (m, 1H), 6.89 (dd, J = 12.6, 1.7 Hz, 1H), 6.64 - 6.60 (m, 1H), 6.06 - 6.02 (m, 1H), 5.19 (dd, J = 9.7, 4. 5 Hz, 1H), 4. 51 (dd, J = 11.9, 4. 5 Hz, 1H), 4.37 - 4.29 (m, 1H), 3.93 - 3.83 (m, 1H), 3.83 - 3.75 (m, 1H), 3.42 - 3.35 (m, 1H), 3.25 - 3.16 (m, 1H), 3.12 - 3.04 (m, 1H), 2.04 - 1.95 (m, 1H), 1.08 - 1.01 (m, 2H), 0.93 - 0.77 (m, 6H). | 526.3 |
| 278 | (DMSO-d6) δ 9.69 (s, 1H), 7.93 (s, 1H), 7.75 (dd, J = 7.3, 1.9 Hz, 1H), 7. 71 (s, 1H), 7.54 - 7.40 (m, 2H), 7.38 - 7.15 (m, 2H), 6.95 (d, J = 1.6 Hz, 1H), 6.89 (dd, J = 12.7, 1.6 Hz, 1H), 5.19 - 5.08 (m, 1H), 4.69 (s, 1H), 4.59 - 4.49 (m, 1H), 4.39 - 4.28 (m, 1H), 3.96 (s, 2H), 3.92 - 3.82 (m, 1H), 3.84 - 3.74 (m, 1H), 3.26 - 3.14 (m, 1H), 3.13 - 3.02 (m, 1H), 2.05 - 1.94 (m, 1H), 1.11 - 1.00 (m, 8H), 0.86 - 0.78 (m, 2H). | 559.2 |
| 279 | (Methanol-d4) δ 9.45 (s, 1H), 8.92 (d, J = 6.0 Hz, 1H), 8.41 (s, 1H), 8.01 (d, J = 7.6 Hz, 1H), 7.74 - 7.16 (m, 5H), 6.94 - 6.77 (m, 2H), 4.79 - 4.44 (m, 2H), 4.10 - 3.74 (m, 2H), 3.20 - 2.90 (m, 2H), 2.07 - 1.88 (m, 1H), 1.14 - 1.04 (m, 2H), 0.86 - 0.74 (m, 2H). | 499.4 |
| 280 | (DMSO-d6) δ 9.95 (s, 1H), 8.82 (s, 1H), 7.89 - 7.75 (m, 4H), 7.56 - 7.42 (m, 6H), 7.34 - 7.25 (m, 1H), 6.98 - 6.85 (m, 2H), 5.18 - 5.07 (m, 1H), 4.62 - 4.54 (m, 1H), 4.41 - 4.31 (m, 1H), 4.01 - 3.68 (m, 2H), 3.26 - 2.98 (m, 2H), 2.04 - 1.95 (m, 1H), 1.13 - 1.01 (m, 2H), 0.86 - 0.77 (m, 2H). | 563.2 |
| 281 | (DMSO-d6) δ 9.77 (s, 1H), 8.22 (s, 1H), 7.82 - 7.70 (m, 2H), 7.52 - 7.40 (m, 2H), 7.37 - 7.32 (m, 2H), 6.98 - 6.85 (m, 2H), 5.57 - 5.45 (m, 1H), 5.18 - 5.10 (m, 1H), 4.97 - 4.83 (m, 4H), 4.59 - 4.50 (m, 1H), 4.40 - 4.29 (m, 1H), 3.93 - 3.74 (m, 2H), 3.27 - 3.01 (m, 2H), 2.05 - 1.94 (m, 1H), 1.10 - 1.00 (m, 2H), 0.86 - 0.77 (m, 2H). | 543.2 |

(continued)

| | | |
|---|---|---|
| 282 | (DMSO-d6) δ 9.84 (s, 1H), 7.81 - 7.73 (m, 2H), 7.52 - 7.40 (m, 2H), 7.37 - 7.32 (m, 2H), 6.98 - 6.85 (m, 2H), 6.76 (s, 1H), 5.16 - 5.08 (m, 1H), 4.60 - 4.52 (m, 1H), 4.40 - 4.24 (m, 3H), 3.93 - 3.74 (m, 2H), 3.27 - 3. 02 (m, 2H), 2.99 - 2.91 (m, 2H), 2.05 - 1.94 (m, 1H), 1.13 - 1.00 (m, 2H), 0.89 - 0.77 (m, 2H). | 540.6 |

Test Example 1

Test of Activity Inhibition against BTK

Method for Measurement of Kinase Activity

Kinase activity was measured by a mobility shift assay (MSA) method using QuickScout Screening Assist (trademark) MSA (commercially available kit, manufactured by Carna Biosciences, Inc.). FITC-labeled SRCtide peptide supplied with the kit was used as a substrate of a kinase reaction. Using an assay buffer [20 mM HEPES, 0.01% Triton X-100 (trademark), 2 mMdithiothreitol, pH 7.5], a substrate mixed solution was prepared by adjusting to the substrate (4 μM), $MgCl_2$ (20 mM), and ATP (120 μM). An enzyme solution was prepared by diluting kinase (BTK; manufactured by Carna Biosciences, Inc., Catalog No. 08-080) with an assay buffer so that the concentration becomes 0.2 nM. A 10 mM DMSO solution of a test compound was further diluted with DMSO to give diluted solutions (having ten concentrations of 0.00003 mM, 0.0001 mM, 0.0003 mM, 0.001 mM, 0.003 mM, 0.01 mM, 0.03 mM, 0.1 mM, 0.3 mM, and 1 mM) and then each of them was diluted 25 times with the assay buffer to give a drug solution (4% DMSO solution). After mixing 5 μL of the drug solution or a control solution (4% DMSO-assay buffer), 5 μL of a substrate mixed solution, and 10 μL of an enzyme solution in a well of a 384-well plate made of polypropylene, the mixture was reacted at room temperature for 1 hour and the reaction was terminated by adding 60 μL of a termination buffer supplied with the kit. Subsequently, the amounts of the substrate (S) and the phosphorylated substrate (P) in the reaction solution were measured according to the protocol of the assay kit, using LabChip EZ Reader II System (manufactured by Caliper Life Sciences Inc.).

Method for Evaluation of BTK Inhibitory Activity

The heights of the respective peaks of the isolated substrate and phosphorylated substrate were designated S and P. Those containing the assay buffer added therein in place of the enzyme solution were measured as a blank.
Inhibition ratio (%) of a test compound was calculated according to the following equation:

$$\text{Inhibition ratio (\%)} = (1 - (C - A)/(B - A)) \times 100$$

where A, B, and C represent P/(P + S) of a blank well, P/(P + S) of a control solution well, and P/(P + S) of a well containing the compound added therein, respectively.
$IC_{50}$ value was calculated by regression analysis of the inhibition ratio and the test compound concentration (logarithm).

Evaluation Results

$IC_{50}$ value of the compound of the present invention against BTK exhibited strong inhibitory activity of 1 μM or less. BTK inhibitory activity of a representative compound is shown in Table 3.

**Table 3**

| Test compounds (Number of Example) | BTK $IC_{50}$ (nM) |
|---|---|
| 1 | 10 |
| 2 | 12 |
| 4 | 10 |
| 5 | 3.6 |
| 7 | 8.2 |

(continued)

| Test compounds (Number of Example) | BTK $IC_{50}$ (nM) |
|---|---|
| 8 | 13.1 |
| 9 | 12.6 |
| 10 | 12.8 |

The results reveal that the test compound (the compound (I) of the present invention) has strong BTK inhibitory activity.

Test Example 2

Test of Activity Inhibition against Dephosphorylated BTK

Preparation of Dephosphorylated BTK

Dephosphorylated BTK was obtained by adding a λ protein phosphatase (Code No. P07535, manufactured by New England BioLabs Inc.) and $MnCl_2$ to a biotinylated BTK protein BTN-BTK (manufactured by Carna Biosciences, Inc.) enzyme solution so as to adjust the concentrations to 10 U/μg and 2 mM, respectively, reacting the mixture at 4°C overnight, removing the λ protein phosphatase through anti-DYKDDDDK-tag antibody agarose gel chromatography, and performing buffer exchange using 10DG Desalting Column.

Measurement of kinase activity and evaluation of dephosphorylated BTK inhibitory activity were carried out in the same manner as in Test Example 1. Provided that adjustment was made so that the concentration of ATP becomes 200 μM, and the concentration of dephosphorylated BTK becomes 0.6 nM in place of kinase (BTK; Catalog No. 08-080, manufactured by Carna Biosciences, Inc.) in the measurement of dephosphorylated kinase activity.

Evaluation Results

$IC_{50}$ value of the present invention compound against dephosphorylated BTK was 1 μM or less, and thus revealing that the present invention compound exhibits strong inhibitory activity. Dephosphorylated BTK inhibitory activity of representative compounds are shown in [Table 4-1] and [Table 4-2].

**Table 4-1**

| Test compounds (Number of Example) | Dephosphorylated BTK $IC_{50}$ (nM) |
|---|---|
| 1 | 2.1 |
| 2 | 6.4 |
| 4 | 4.2 |
| 5 | 0.7 |
| 7 | 2.5 |
| 8 | 3.5 |
| 9 | 2.1 |
| 10 | 3.2 |
| 17 | 5.1 |
| 19 | 6.1 |
| 24 | 3.4 |
| 25 | 3.5 |
| 30 | 1.3 |
| 31 | 1.5 |
| 32 | 0.8 |

(continued)

| Test compounds (Number of Example) | Dephosphorylated BTK $IC_{50}$ (nM) |
|---|---|
| 34 | 4.0 |
| 37 | 2.7 |
| 38 | 1.2 |
| 39 | <0.3 |
| 40 | 0.7 |
| 42 | 6.5 |
| 44 | 2.2 |
| 45 | 1.7 |
| 46 | 5.2 |
| 47 | 1.6 |
| 48 | 3.4 |
| 49 | 1.1 |
| 50 | 1.0 |
| 51 | 2.0 |
| 52 | 1.6 |
| 53 | 1.8 |
| 54 | 3.6 |
| 55 | 2.3 |
| 56 | 1.2 |
| 57 | 1.4 |
| 58 | 1.0 |
| 59 | 1.1 |
| 60 | 0.9 |
| 61 | 0.8 |
| 62 | 3.4 |
| 63 | 2.4 |
| 64 | 3.7 |
| 65 | 0.6 |
| 66 | 3.2 |
| 70 | 1.0 |
| 73 | 1.3 |
| 74 | 0.7 |
| 76 | 4.3 |
| 77 | 3.4 |
| 78 | 1.2 |
| 79 | 0.6 |
| 80 | 2.1 |
| 81 | 2.2 |

(continued)

| Test compounds (Number of Example) | Dephosphorylated BTK $IC_{50}$ (nM) |
|---|---|
| 83 | 1.8 |
| 85 | 5.8 |
| 86 | 4.0 |
| 87 | 1.4 |
| 88 | 1.8 |
| 89 | 0.6 |
| 90 | 0.7 |
| 91 | 1.1 |
| 92 | 0.9 |
| 93 | 1.3 |
| 94 | 0.4 |
| 96 | 7.7 |
| 97 | 4.0 |
| 98 | 4.2 |
| 99 | 10.6 |
| 100 | 3.8 |
| 101 | 0.8 |
| 102 | 8.7 |
| 103 | 15.2 |
| 104 | 5.0 |
| 105 | 0.9 |
| 106 | 0.7 |
| 107 | 0.8 |
| 110 | 17.9 |
| 111 | 1.0 |
| 112 | 1.1 |
| 113 | 2.6 |
| 114 | 1.0 |
| 115 | 9.4 |
| 117 | 0.9 |
| 118 | 0.8 |
| 119 | 0.8 |
| 120 | 5.5 |
| 121 | 1.2 |
| 122 | 0.8 |
| 123 | 1.4 |
| 124 | 0.5 |
| 125 | 0.8 |

(continued)

| Test compounds (Number of Example) | Dephosphorylated BTK $IC_{50}$ (nM) |
|---|---|
| 126 | 0.5 |
| 127 | 0.5 |
| 128 | 0.6 |
| 129 | 0.6 |
| 130 | 0.5 |
| 131 | 0.4 |
| 132 | 0.5 |
| 133 | 0.4 |
| 134 | 0.9 |
| 135 | 1.3 |
| 136 | 1.3 |
| 137 | 2.1 |
| 138 | 1.7 |
| 139 | 1.6 |
| 140 | 1.7 |
| 141 | 1.2 |
| 142 | 3.1 |
| 143 | 0.9 |
| 144 | 1.9 |
| 145 | 1.8 |
| 146 | 0.4 |
| 147 | 0.3 |
| 148 | 0.8 |
| 149 | 0.5 |
| 150 | 0.8 |
| 151 | 1.3 |
| 152 | 1.2 |
| 153 | 4.9 |
| 154 | 0.9 |
| 155 | 0.8 |
| 156 | 0.5 |
| 159 | 5.9 |
| 160 | 0.8 |
| 161 | 1.1 |
| 162 | 0.5 |
| 163 | 1.0 |
| 164 | 0.5 |
| 165 | 0.7 |

(continued)

| Test compounds (Number of Example) | Dephosphorylated BTK $IC_{50}$ (nM) |
|---|---|
| 166 | 1.0 |
| 167 | 1.6 |
| 168 | 17.6 |
| 169 | 4.7 |
| 170 | 2.5 |
| 171 | 26.0 |
| 172 | 0.6 |
| 173 | 0.6 |
| 174 | 1.5 |
| 175 | 3.6 |
| 179 | 0.9 |
| 180 | 0.7 |
| 181 | 1.6 |
| 182 | 3.6 |
| 183 | 2.3 |

**Table 4-2**

| Test compounds (Number of Example) | Dephosphorylated BTK $IC_{50}$ (nM) |
|---|---|
| 184 | 0.8 |
| 185 | 1.2 |
| 187 | 5.6 |
| 188 | 0.4 |
| 189 | 1.2 |
| 190 | 1.4 |
| 191 | 1.0 |
| 192 | 0.7 |
| 193 | 1.1 |
| 194 | 0.9 |
| 195 | 0.8 |
| 196 | 3.4 |
| 197 | 2.4 |
| 198 | 3.7 |
| 200 | 0.8 |
| 201 | 3.4 |
| 202 | 1.3 |
| 203 | 0.4 |
| 204 | 1.3 |

(continued)

| Test compounds (Number of Example) | Dephosphorylated BTK $IC_{50}$ (nM) |
|---|---|
| 205 | 9.9 |
| 206 | 1.0 |
| 208 | 5.9 |
| 209 | 1.4 |
| 211 | 0.7 |
| 212 | 0.9 |
| 213 | 1.1 |
| 214 | 0.3 |
| 218 | 0.2 |
| 219 | 0.8 |
| 220 | 2.3 |
| 222 | 0.4 |
| 223 | 0.6 |
| 224 | 6.6 |
| 225 | 1.2 |
| 226 | 0.6 |
| 227 | 3.5 |
| 228 | 2.4 |
| 229 | 2.5 |
| 231 | 1.7 |
| 232 | 0.7 |
| 233 | 4.2 |
| 234 | 2.7 |
| 235 | 0.8 |
| 236 | 0.8 |
| 237 | 0.8 |
| 238 | 0.9 |
| 239 | 0.5 |
| 240 | 1.1 |
| 241 | 0.6 |
| 242 | 1.5 |
| 243 | 2.3 |
| 244 | 4.6 |
| 245 | 5.6 |
| 246 | 2.7 |
| 248 | 3.2 |
| 249 | 1.0 |
| 250 | 1.5 |

(continued)

| Test compounds (Number of Example) | Dephosphorylated BTK $IC_{50}$ (nM) |
|---|---|
| 251 | 0.4 |
| 252 | 0.8 |
| 253 | 0.8 |
| 254 | 1.4 |
| 255 | 0.5 |
| 257 | 0.6 |
| 260 | 0.9 |
| 261 | 1.5 |
| 262 | 0.8 |
| 266 | 1.3 |
| 267 | 1.5 |
| 268 | 1.2 |
| 269 | 0.8 |
| 270 | 0.7 |
| 271 | 0.4 |
| 272 | 1.1 |
| 273 | 2.4 |
| 274 | 1.0 |
| 275 | 1.7 |
| 276 | 0.6 |
| 277 | 3.3 |
| 278 | 2.2 |

**Test Example 3-1**

Test of Inhibition against Self-Phosphorylation Activity of Intracellular BTK

Culture of Cells to be used

Ramos cells (2G6.4C10, ATCC Inc., No. CRL-1923) were cultured in a T75 flask, in a 5% $CO_2$ incubator, using an RPMI-1640 medium (GIBCO Inc.) containing 10% FBS (AusGene Inc.) and 5% penicillin-streptomycin (Nacalai Inc.) added therein.

Addition of Test Compound

The cultured Ramos cells were diluted with an RPM-1640 medium (hereinafter referred to as a medium) from which serum was removed so that the cell density becomes $7.5 \times 10^6$ cells/mL, and then kept warm at 37°C for 45 minutes. After the cell suspension was dispensed into 1 ml aliquots in a 2.0 mL tube, and 500 μL of a test compound solution prepared by diluting a 1 mM DMSO solution of a test compound was added so that the concentration becomes 3 μM in the medium, followed by incubation at 37°C for 1 hour under the conditions that the final concentration of the test compound is 1 μM. Thereafter, IgM (Invitrogen, H15100) diluted with the medium was added so that the final concentration becomes 10 μg/mL, followed by incubation at 37°C for 10 minutes.

Extraction of Protein

To pellets obtained by recovering cells by centrifugal operation, 100 μL of Lysis buffer [RIPABuffer (×1) (Cell Signaling Technology, Inc.) containing 1% Phosphatase inhibitor Cacktail 3 (Sigma Co., No. P0044), 1% Phosphatase inhibitor Cacktail (Nacalai Inc., No.07575), and 1 mM phenylmethylsulfonylfluoride (PMSF) added therein] was added, followed by gentle stirring and further standing for 10 minutes. The supernatant was recovered by a centrifugal operation (at 15,000 rpm for 15 minutes) and the amount of protein was quantitatively determined. The supernatant was mixed with SDS-sample buffer and the mixture was reacted at 95°C for 5 minutes thereby modifying the protein to obtain a sample solution. The sample solution (5 μL each) was applied into each well of a 4-20% gradient acrylamide gel (Cosmo Bio Co., Ltd., No. 414879) and then electrophoresis was carried out. Thereafter, the protein in the gel was transferred to a PVDF membrane, using an iBlot gel transfer system (Life Technologies Corporation).

Detection of BTK or Phosphorylated BTK

The transferred PVDF membrane was subjected to a blocking treatment with 2% ECL prime blocking Reagent (GE Healthcare Ltd.) and then reacted at 4°C overnight, using an anti-BTKmouse antibody (No. 611116, BD Transduction Laboratories) or an anti-phosphorylated BTK rabbit antibody (pY223, No. 2207-1, EPITOMICS, Inc.) as a primary antibody. The unreacted primary antibody was washed with TBST buffer (10 mM Tris-HCl (pH 7.5), 150 mM NaCl, 0.1% Tween 20) and then reacted in TBST buffer containing 2% ECL prime blocking Reagent added therein at room temperature for 1 hour, using a HRP-labeled anti-mouse IgG goat antibody (No. 62-6520, Life Technologies Corporation) or an anti-rabbit IgG goat antibody (No. 65-6120, Life Technologies Corporation) as a secondary antibody. The unreacted secondary antibody was washed with TBST buffer and reacted according to the attached protocol, using ECL Prime Western Blotting Detection System (GE Healthcare Ltd.), and then each band was detected by chemiluminescence using a CCD camera (ATTO, Light-CaptureII). The detected band was digitized by densitometry (ATTO CS Analyzer ver3.0) and an inhibition ratio was calculated from the intensity of a band in each group on the assumption that luminescence of a band of phosphorylated BTK of the compound no-addition and IgM stimulated group is 100%, whereas, luminescence of a band of phosphorylated BTK of the compound no-addition and IgM non-stimulated group is 0%. Each band of phosphorylated BTK was corrected by the total BTK.

Combinations of primary antibody and secondary antibody as well as dilute concentrations used in the present test are as follows.

**Table 5**

| | Primary antibody (dilute concentration) | Secondary antibody (dilute concentration) |
|---|---|---|
| 1 | Anti-BTK mouse antibody (1/4,000) | Anti-mouse IgG goat antibody (1/5,000) |
| 2 | Anti-phosphorylated BTK rabbit antibody (1/500) | Anti-rabbit IgG goat antibody (1/5,000) |

In the present test, as shown in Table 6, the present invention compounds strongly inhibited self-phosphorylation activity of intracellular BTK at the concentration of 1 μM.

**Table 6**

| Test compounds (Number of Example) | BTK phosphorylation inhibition ratio (%) |
|---|---|
| 1 | 107 |
| 2 | 85 |

**Test Example 3-2**

Test 2 of Inhibition against Self-Phosphorylation Activity of

Intracellular BTK

Extraction of Protein and Detection of BTK or Phosphorylated BTK was carried out in the same manner as in Test Example 3-1, and the inhibition ratio of each test compounds was calculated.

Addition of Test Compound

The cultured Ramos cells were diluted with an RPM-1640 medium (hereinafter referred to as a medium) from which serum was removed so that the cell density becomes 7.5 × $10^6$ cells/mL, and then kept warm at 37°C for 45 minutes. After the cell suspension was dispensed into 1 ml aliquots in a 2.0 mL tube, and 500 μL of a test compound solution prepared by diluting a 1 mM DMSO solution of a test compound was added so that the concentration becomes 0.9 μM in the medium, followed by incubation at 37°C for 1 hour under the conditions that the final concentration of the test compound is 0.3 μM. Thereafter, IgM (Invitrogen, H15100) diluted with the medium was added so that the final concentration becomes 10 μg/mL, followed by incubation at 37°C for 10 minutes.

The results obtained at a test compound concentration of 0.3 μM are shown in Table 7. The intracellular BTK autophosphorylation inhibiting activity was indicated with the mark "***" when 70% or more, with the mark "**" when 50% or more and less than 70%, and with the mark "*" when 30% or more and less than 50%.

In the present test, as shown in Table 7, the present invention compounds strongly inhibited self-phosphorylation activity of intracellular BTK at the concentration of 0.3 μM.

**Table 7**

| Test compounds (Number of Example) | BTK phosphorylation inhibitory activity |
|---|---|
| 1 | *** |
| 2 | ** |
| 5 | *** |
| 30 | *** |
| 31 | *** |
| 32 | *** |
| 33 | *** |
| 40 | *** |
| 44 | *** |
| 70 | *** |
| 180 | *** |
| 211 | *** |
| 213 | *** |
| 214 | *** |

These results (Test Example 3-1, 3-2) show that the compound of the present invention also has strong inhibitory effect on "self-phosphorylation activity effect of intracellular BTK".

Industrial Applicability

The compound provided by the present invention is useful as a preventive or therapeutic pharmaceutical (pharmaceutical composition) for diseases which are known to be involved in abnormal cell response through BTK, for example, self-immune diseases, inflammatory diseases, bone diseases, and cancers such as lymphoma. The compound is also useful, as a BTK inhibitor, for reagents to be used in tests and researches.

## Claims

1. A triazine derivative represented by the following formula (I) :

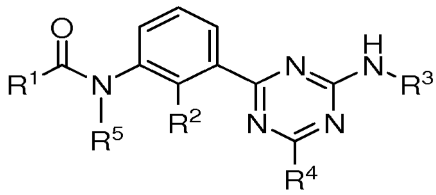

(I)

wherein

$R^1$ represents a substituted or unsubstituted aryl group, a substituted or unsubstituted heterocyclic ring, a substituted or unsubstituted heterocyclic fused ring, or a substituted or unsubstituted alkynyl group,
$R^2$ represents a hydrogen atom, a halogen atom, a substituted or unsubstituted lower alkyl group, or a substituted or unsubstituted alkoxy group,
$R^3$ represents a substituted or unsubstituted aryl group, a substituted or unsubstituted heterocyclic ring, or a substituted or unsubstituted heterocyclic fused ring,
$R^4$ represents a hydrogen atom, a substituted or unsubstituted lower alkyl group, a substituted or unsubstituted alkoxy group, a substituted or unsubstituted amino group, or a halogen atom, and
$R^5$ represents a hydrogen atom, a substituted or unsubstituted lower alkyl group, or $R^1$ and $R^5$ may be combined to form a saturated or unsaturated 5- to 6-membered ring, thereby forming a multiply fused ring,
or a pharmaceutically acceptable salt thereof.

**2.** The triazine derivative according to claim 1,
wherein $R^1$ is a substituted or unsubstituted aryl group,
or a pharmaceutically acceptable salt thereof.

**3.** The triazine derivative according to claim 1,
wherein $R^2$ is a substituted or unsubstituted lower alkyl group,
or a pharmaceutically acceptable salt thereof.

**4.** The triazine derivative according to claim 1,
wherein $R^5$ and $R^1$ are combined to form a saturated or unsaturated 5- to 6-membered ring, thereby forming a multiply fused ring,
or a pharmaceutically acceptable salt thereof.

**INTERNATIONAL SEARCH REPORT**

International application No.
PCT/JP2013/056266

A. CLASSIFICATION OF SUBJECT MATTER
*C07D251/42*(2006.01)i, *A61K31/53*(2006.01)i, *A61K31/5377*(2006.01)i, *A61P19/00*(2006.01)i, *A61P29/00*(2006.01)i, *A61P35/00*(2006.01)i, *A61P35/02*(2006.01)i, *A61P37/06*(2006.01)i, *A61P43/00*(2006.01)i

According to International Patent Classification (IPC) or to both national classification and IPC

B. FIELDS SEARCHED

Minimum documentation searched (classification system followed by classification symbols)
C07D251/42, A61K31/53, A61K31/5377, A61P19/00, A61P29/00, A61P35/00, A61P35/02, A61P37/06, A61P43/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>A | WO 2009/091388 A2 (PROGENICS PHARMACEUTICALS, INC.), 23 July 2009 (23.07.2009), table 39 (En54-57) & EP 2231624 A2 & US 2012/0009151 A1 | 1,2<br>3,4 |
| A | WO 2008/121742 A2 (PHARMACYCLICS, INC.), 09 October 2008 (09.10.2008), entire text & EP 2201840 A1 & US 2008/0076921 A1 & JP 2010-526768 A | 1-4 |
| A | WO 2008/076883 A2 (ABRAXIS BIOSCIENCE, INC.), 26 June 2008 (26.06.2008), entire text & EP 2425840 A1 & US 2008/0176853 A1 & JP 2010-513313 A | 1-4 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>19 March, 2013 (19.03.13) | Date of mailing of the international search report<br>02 April, 2013 (02.04.13) |
| Name and mailing address of the ISA/<br>Japanese Patent Office<br><br>Facsimile No. | Authorized officer<br><br><br>Telephone No. |

Form PCT/ISA/210 (second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **SATTERTHWAITE, A. B. ; WITTE, O N.** *Immunol. Rev.,* 2000, vol. 175, 120-127
- **KUROSAKI T.** *Curr. Opin. Immunol.,* 2000, vol. 12, 276-281
- **DAVIS R. E. et al.** *Nature,* 2010, vol. 463, 88-92
- **ELLMEIER W. et al.** *FEBS J.,* 2011, vol. 278, 1990-2000
- **HALCOMB K. E.** *Mol. Immunol.,* 2008, vol. 46 (2), 233-241
- **JANSSON L. ; HOLMDAHL R.** *Clin. Exp. Immunol.,* 1993, vol. 94, 459-465
- **T. W. GREENE.** Protective Groups in Organic Synthesis. John Wiley&Sons, Inc, 1999
- **N. MIYAURA et al.** *J. Am. Chem. Soc.,* 1985, vol. 107, 972
- **N. MIYAURA ; A. SUZUKI.** *Chem. Rev.,* 1995, vol. 95, 2457
- **T. W. GREENE.** Protective Groups in Organic Synthesis. John Wiley & Sons, Inc, 1999